# EUROPEAN PATENT SPECIFICATION

(11) **EP 1 323 716 B1**
(45) Date of publication and mention of the grant of the patent: **14.01.2009**
(21) Application number: 01972687.6
(22) Date of filing: 04.10.2001
(51) Int. Cl.: C07D 307/79, C07D 405/04, C07D 491/056, C07D 407/12, C07D 491/107, C07D 409/12, C07D 405/12, C07D 307/81, A61K 31/4035, A61K 31/407, A61K 31/343, A61K 31/36, A61K 31/438, A61K 31/381, A61K 31/443, A61K 31/496, A61K 31/5377, A61K 31/4709, A61P 25/28, A61P 25/00

(54) **PROMOTERS FOR THE PROLIFERATION AND DIFFERENTIATION OF STEM CELLS AND/OR NEURON PRECURSOR CELLS**
PROMOTOREN ZUR PROLIFERATION UND DIFFERENZIERUNG VON STAMMZELLEN UND/ODER VORSTUFEN VON NEURONENZELLEN
PROMOTEURS DE PROLIFERATION ET DE DIFFERENCIATION DE CELLULES EMBRYONNAIRES ET/OU DE PRECURSEURS NEURONAUX

(30) Priority: 05.10.2000 JP 2000306801
(43) Date of publication of application: 02.07.2003
(73) Proprietor: Takeda Pharmaceutical Company Limited, Osaka (JP)
(72) Inventor: OKAWA, Shigenori, Takatsuki-shi, Osaka 569-1121 (JP); MIYAMOTO, Masaomi, Takarazuka-shi, Hyogo 665-0841 (JP); OKURA, Masahiro, Ikeda-shi, Osaka 563-0028 (JP)
(74) Representative: Rickard, Timothy Mark Adrian
(86) International application number: PCT/JP2001/008739
(87) International publication number: WO 2002/028850

(56) References cited:
- JP-A- 10 509 592
- JP-A- 11 049 765
- JP-A- 2000 226 388

## Description

### Technical Field

The present invention relates to an agent for promoting the proliferation or differentiation of a stem cell and/or neural progenitor cell comprising a benzofuran derivative.

### Background Art

A neurodegenerative-disease is a disease in which a selective neuronal death takes place progressively, and major known neurodegenerative diseases are Alzheimer's disease, Perkinson's disease, amyotropic lateral sclerosis (ALS) and Huntington's disease.

A current medication therapy mainly employs a substitution therapy that compensates for the depletion of neurotransmitters accompanying neurodegeneration. A dopaminergic agent such as L-dopa which is a precursor of dopamine is employed to treat Parkinson's disease, while an acetylcholine decomposition enzyme inhibitor is employed to treat Alzheimer's disease, the both being used as a substitution therapy agent or a symptomatic therapy agent. However, such a substitution therapy agent or a symptomatic therapy agent does not suppress the progress of neurodegeneration, and its effect becomes attenuated gradually with progression of the disease. Accordingly, the development of an agent that suppresses the progress of neurodegeneration and promotes the regeneration of the remaining nerve ending is desired. However, currently no agent having such effects has been identified. In addition, it is believed that most of neurocytes have been degenerated at the time of the onset of a neurodegenerative disease, and thus a sufficient functional regeneration is not considered to be achieved only by suppression of degeneration or by promotion of nerve ending regeneration.

On the other hand, a concept of the regeneration ability of a central nervous system has recently been changed substantially. That is, it had been understood for a long time that once any neurodegeneration occurs in a central nervous system, it is difficult to recover a function of the nerve because a nerve is never generated and supplemented again. However, a new understanding that the central nervous system of a mature mammal including human possesses a neural stem cell or neural progenitor cell that enables the neogenesis of a nerve was proposed many times recently, and therefore a possibility of the regeneration of a damaged nervous tissue and a function thereof by means of activating an intrinsic neural stem cell was started to be investigated [Nature Medicine, Vol.4, page 1313-1317, 1998, Nature Medicine, Vol.6, page 271-277, 2000]. In addition, an investigation of a neural regeneration medical treatment by means of transplantation of a neural stem cell prepared from an embryonic stem cell, aborted fetal brain or a tissue of a patient himself was also started [Nature, Vol.405, page 951-955, 2000, Eur. J. Neurosci., Vol.10, page 2026-2036, 1998].

A benzofuran derivative that has an activity for promoting the regeneration of a nerve and is useful as a prophylactic and therapeutic agent against a neurodegenerative disease is disclosed in WO 98/55454 and WO 00/34262, which however contain no description with regard to promoting the proliferation or differentiation of a neural stem cell or neural progenitor cell.

### Object of the Invention

Based on a current understanding, a substance that enables the proliferation or differentiation of a neural stem cell or neural progenitor cell is a polymeric in vivo factor, which should be introduced into a brain surgically when being employed in a treatment. The present invention is intended to enable a treatment of a neurodegenerative disease, cerebrovascular disease or cranial trauma by means of developing a compound which migrates in brain satisfactorily, enhances the proliferation (autorepruduction) of a neural stem cell or neural progenitor cell to promote the differentiation into a neurocyte whereby regenerating a neurocyte that had once been damaged upon neurodegeneration Such a compound may not only useful in preparing a neural stem cell and neural progenitor cell from an embryonic stem cell and a nervous tissue but also be capable of promoting post-transplantation engraftment and differentiation

### Summary of the invention

According to the invention there is provided
1. A use of a compound represented by Formula: 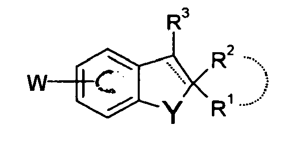 wherein R¹ and R² are same or different and each is a hydrogen atom, an optionally substituted hydrocarbon group or an optionally substituted heterocyclic group or R¹ and R² may be taken together with the adjacent carbon atom to form an optionally substituted 3- to 8-membered homocyclic or heterocyclic ring
   R³ is a hydrogen atom, an optionally substituted hydrocarbon group or an optionally substituted heterocyclic group,
   ^{...} is a single bond or a double bond,
   W is (i) a group represented by Formula: 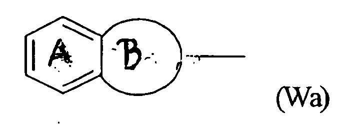 wherein Ring A is an optionally substituted benzene ring, Ring B is an optionally substituted 5-to 7-membered nitrogen-containing heterocyclic ring,
   (ii) a group represented by Formula: 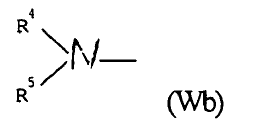 Wherein R⁴ is (1) an aliphatic hydrocarbon group which is substituted by an optionally substituted aromatic group and which may have a further substituent or (2) an optionally substituted aromatic ring-containing acyl group, R⁵ is a hydrogen atom, a C₁₋₆ alkyl or an acyl group, or,
   (iii) a group represented by Formula: 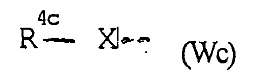 wherein R^{4c} is an optionally substituted aromatic group, an optionally substituted aliphatic hydrocarbon group or an acyl group, X is an oxygen atom or an optionally oxidized sulfur atom,
   Y is an oxygen atom, an optionally oxidized sulfur atom or an optionally substituted imino, Ring C is a benzene ring which may have a further substituent in addition to the group represented by W or a salt thereof in the production of an agent for promoting the engraftment or differentiation in neural stem cell and/or neurocyte transplantation or in the production of an agent for promoting the proliferation or differentiation of a neural stem cell and/or neurocyte for transplantation, wherein the cells are not of human embryonic origin
2. The use according to Paragraph 1 wherein ^{...} is a single bond.
3. The use according to Paragraph 1 wherein Y is an oxygen atom.
4. The use according to Paragraph 1 wherein W is a group represented by Formula (Wa).
5. The use according to Paragraph 1 wherein each of R¹ and R² is a hydrogen atom or a C₁₋₆alkyl group, R³ is a hydrogen atom or a phenyl group which may have 1 to 3 substituents selected from C₁₋₆alkyl and halogen, the Ring C is a benzene ring which may further have 1 to 3 substituents selected from C₁₋₆alkyl and C₁₋₆alkoxy, ^{...} is a single bond, Y is an oxygen atom, the group represented by Formula (Wa) is a group represented by Formula: 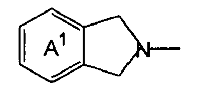 wherein Ring A¹ is a benzene ring which may have 1 to 3 substituents selected from halogen, C₁₋₆ alkoxy and C₁₋₆ alkylenedioxy.
6. The use according to Paragraph 5 wherein the group represented by Formula (Wa) is a substituent on the 5-position of the benzofuran ring.
7. The use according to Paragraph 4 comprising [1] 2-[2,2,4,6,7-pentamethyl-3-(4-methylphenyl)-2,3-dihydro-1-benzofuran-5-yl]isoindoline, [2] 5,6-dimethoxy-2-[2,2,4,6,7-pentamethyl-3-(4-methylphenyl)-2,3-dihydro-1-benzofuran-5-yl]isoindoline, [3] 5,6-dimethoxy-2-[3-(4-isopropylphenyl)-2,2,4,6,7-pentamethyl-2,3-dihydro-1-benzofuran-5-yl]isoindoline, [4] 5,6-dimethoxy-2-[2,2,4,6,7-Pentamethyl-3-(4-methylphenyl)-2,3-dihydro-1-benzofuran-5-yl]-2H-isoindole, [5] 6-[3-(4-isopropylphenyl)-2,2,4,6,7-pentamethyl-2,3-dihydro-1-benzofuran-5-yl]-6,7-dihydro-5H-[1,3]dioxolo[4,5-f]isoindole, [6] 6-[2,2,4,6,7-pentamethyl-3-(4-methylphenyl)-2,3-dihydro-1-benzofuran-5-yl]-6H-[1,3]dioxolo[4,5-f]isoindole, [7] 6-(2,2,4,6,7-pentamethyl-3-phenyl-2,3-dihydro-1-benzofuran-5-yl)-6,7-dihydro-5H-[1,3]dioxolo[4,5-f]isoindole, [8] (R)-5,6-dimethoxy-2-[2,2,4,6,7-pentamethyl-3-(4-methylphenyl)-2,3-dihydro-1-benzofuran-5-yl]isoindoline or [9] (R)-5,6-dimethoxy-2-[2,2,4,6,7-pentamethyl-3-(4-methylphenyl)-2,3-dihydro-1-benzofuran-5-yl]isoindoline hydrochloride.
8. The use according to Paragraph 4 comprising (R)-5,6-dimethoxy-2-[2,2,4,6,7-pentamethyl-3-(4-methylphenyl)-2,3-dihydro-1-benzofuran-5-yl]isoindoline.
9. The use according to Paragraph 1 wherein W is a group represented by Formula (Wb).
10. The use according to Paragraph 9 wherein each of R¹ and R² is a methyl group, R³ is a phenyl group which may have 1 to 3 substituents selected from fluorine, methyl and isopropyl, the Ring C is a benzene ring which may further have 1 to 3 substituents selected from C₁₋₆ alkyl and C₁₋₆ alkoxy, Y is an oxygen atom, R⁴ is a benzyl orphenethyl group which may have 1 to 3 substituents selected from fluorine, methoxy and methylenedioxy and R⁵ is a hydrogen atom or a methyl group.
11. The use according to Paragraph 9 comprising (1) N-(4-fluorobenzyl)-2,2,4,6,7-pentamethyl-3-phenyl-2,3-dihydro-1-benzofuran-5-amine, (2)N-benzyl-3-(4-isopropylphenyl)-2,2,4,6,7-pentamethyl-2,3-dihydro-1-benzofuran-5-amine, (3) 3-(4-isopropylphenyl-N-(4-methoxybenzyl)-N,2,2,3,4,6,7-hexamethyl-2,3-dihydro-1-benzofuran-5-amine, (4) 3(4-isopropylphenyl)-N-[2-(4-methoxyphenyl)ethyl]-2,2,4,6,7-pentamethyl-2,3-dihydro-1-benzofuran-5-amine, (5) N-(4-fluorobenzyl)-3-(4-isopropylphenyl)-2,2,4,6,7-pentamethyl-2,3-dihydro-1-benzofuran-5-amine, (6) N-(1,3-benzodioxyl-5-ylmethyl)-3-(4-isopropylphenyl)-2,2,4,6,7-pentamethyl-2,3-dihydro-1-benzofuran-5-amine, (7) N-(4-fluorobenzyl)-3-(4-fluorophenyl)-2,2,4,6,7-pentamethyl-2,3-dihydro-1-benzofuran-5-amine, (8) N-(4-methoxybenzyl)-2,2,4,6,7-pentamethyl-3-(4-methylphenyl)-2,3-dihydro-1-benzofuran-5-amine, (9) N-(4-fluorobenzyl)-2,2,4,6,7-pentamethyl-3-(4-methylphenyl)-2,3-dihydro-1-benzofuran-5-amine, (10) 3-(4-isopropylphenyl)-N-(4-methoxybenzyl)-2,4,6,7-tetramethyl-1-benzofuran-5-amine, (11) N-(4-fluorobenzyl)-3,4-isopropylphenyl)-2,4,6,7-tetramethyl-1-benzofuran-5-amine, (12) N-(4-fluorobenzyl)-3-(4-fluorophenyl)-2,4,6,7,-tetramethyl-1-benzofuran-5-amine, (13) N-(4-fluorobenzyl)-3-(4-isopropylphenyl)-1',4,6,7-tetramethylspiro[benzofuran-2(3H), 4'-pyperidine]-5-amine or (14) (R)-N-(4-fluorobenzyl)-3-(4-isopropylphenyl)-2,2,4,6,7-pentamethyl-2,3-dihydro-1-benzofuran-5-amine hydrochloride.
12. The use according to Paragraph 1 wherein W is a group represented by Formula(Wc).
13. The use according to Paragraph 12 comprising a compound represented by Formula: 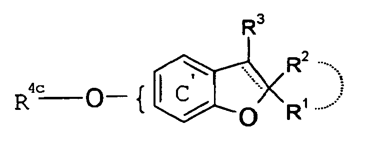 wherein each of R¹ and R² is C₁₋₆-alkyl which may have a phenyl-substituted 6-membered saturated cyclic amino, or R¹ and R² are taken together with the adjacent carbon atom to form a C₁₋₆ alkyl- or C₇-₁₆ aralkyl-substituted piperidine;
   R³ is (i) a hydrogen atom, or,
   (ii) phenyl which may have 1 to 3 substituents selected from (1) C₁₋₆alkyl, (2) d-C₁₋₆ alkylamino and (3) 6-membered saturated cyclic amino which may have C₁₋₆alkyl; R^{4c} is (i) phenyl which may have 1 to 3 substituents selected from nitro and C₁₋₆alkyl-carboxamide,
   (ii) C₁₋₆alkyl or C₂₋₆alkenyl having 1 to 3 phenyl,quinolyl or pyridyl which may have 1 to 3 substituents selected from C₁₋₆alkoxy, C₁₋₆ alkylthio, C₁₋₆ alkoxy-carbon, C₁₋₆ alkylsulfonyl and C₁₋₆alkylsulfinyl and optionally further having phenyl, carboxy or C₁₋₆-alkoxy-carbonyl as additional substituents, or,
   (iii) acyl represented by Formula: -(C=O)-R^{5"} wherein R^{5"} is C₁₋₆alkoxy-substituted phenyl; and,
   the Ring C' is a benzene ring which may further have 1 to 3 C₁₋₆ alkyl, or a salt thereof
14. The use according to Paragraph 12 comprising
   3-(4-isopropylphenyl)-5-(4-methoxybenzyloxy)-2,2,4,6,7-pentamethyl-2,3-dihydrobenzofuran, 3-(4-methylphenyl)-2,4,6,7-tetramethylbenzofuran-5-yl-4-methoxybenzoate, 3-(4-isopropylphenyl)-2,4,6,7-tetramethylbenzofuran-5-yl 4-methoxybenzoate, 3-(4-isopropylphenyl)-5-(4-methoxybenzyroxy)-2,4,6,7-tetramethylbenzofuran, 3-(4-isopropylphenyl)-5-(4-methoxybenzyloxy)-1',4,6,7-tetramethylspiro[benzofuran-2(3H), 4'-piperidine] or a salt thereof
15. A method for culturing a stem cell, neural progenitor cell and/or neurocyte wherein the cells are not of embryonic origin comprising culturing the stem cell, neural progenitor cell and/or neurocyte in the presence of a compound represented by Formula: 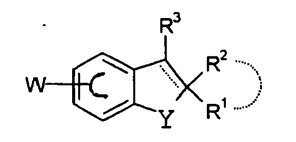 wherein R¹ and R² are same or different and each is a hydrogen atom, an optionally substituted hydrocarbon group or an optionally substituted heterocyclic group or R¹ and R² may be taken together with the adjacent carbon atom to form an optionally substituted 3- to 8-membered homocyclic or heterocyclic ring,
   R³ is a hydrogen atom, an optionally substituted hydrocarbon group or an optionally substituted heterocyclic group,
   ^{...} is a single bond or a double bond,
   W is (i) a group represented by Formula: 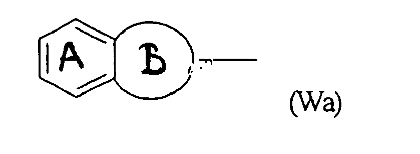 wherein Ring A is an optionally substituted benzene ring Ring B is an optionally substituted 5-to 7-membered nitrogen-containing heterocyclic ring
   (ii) a group represented by Formula: 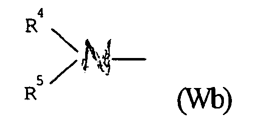 wherein R⁴ is (1) an aliphatic hydrocarbon group which is substituted by an optionally substituted aromatic group and which may have a further substituent or (2) an optionally substituted aromatic ring-containing acyl group, R⁵ is a hydrogen atom, a C₁₋₆alkyl or an acyl group, or,
   (iii) a goup represented by Formula: 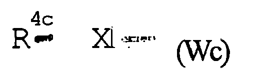 wherein R^{4c} is an optionally substituted aromatic group, an optionally substituted aliphatic hydrocarbon group or an acyl group, X is an oxygen atom or an optionally oxidized sulfur atom,
   Y is an oxygen atom, an optionally oxidized sulfur atom or an optionally substituted imino, Ring C is a benzene ring which may have a further substituent in addition to the group represented by W or a salt thereof:
16. The method according to Paragraph 15 wherein ^{...} is a single bond.
17. The method according to Paragraph 15 wherein Y is an oxygen atom.
18. The method according to Paragraph 15 wherein W is a group represented by Formula (Wa).
19. The method according to Paragraph 15 wherein each of R¹ and R² is a hydrogen atom or a C₁₋₆alkyl goup, R³ is a hydrogen atom or a phenyl group which may have 1 to 3 substituents selected from C₁₋₆ alkyl and halogen, the Ring C is a benzene ring which may further have 1 to 3 substituents selected from C₁₋₆ alkyl and C₁₋₆ alkoxy, = is a single bond, Y is an oxygen atom, the group represented by Formula (Wa) is a group represented by Formula: 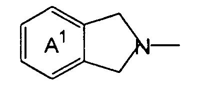 wherein Ring A¹ is a benzene ring which may have 1 to 3 substituents selected from halogen, C₁₋₆alkoxy and C₁₋₆alkylenedioxy.
20. The method according to Paragraph 19 wherein the group represented by Formula (Wa) is a substituent on the 5-position of the benzofuran ring
21. The method according to Paragraph 18 comprising [1] 2-[2,2,4,6,7-pentamethyl-3-(4-methylphenyl)-2,3-dihydro-1-benzofuran-5-yl]isoindoline, [2] 5,6-dimethoxy-2-[2,2,4,6,7-pentamethyl-3-(4-methylphenyl)-2,3-dihydro-1-benzofuran-5-yl]isoindoline, [3] 5,6-dimethoxy-2-[3-(4-isopropylphenyl)-2,2,4,6,7-pentamethyl-2,3-dihydro-1-benzofuran-5-yl]isoindoline, [4] 5,6-dimethoxy-2-[2,2,4,6,7-pentamethyl-3-(4-methylphenyl)-2,3-dihydro-1-benzofuran-5-yl]-2H-isoindole, [5] 6-[3-(4-sopropylphenyl)-2,2,4,6,7-pentamethyl-2,3-dihydro-1-benzofuran-5-yl]-6,7-dihydro-5H-[1,3]dioxolo[4,5-f]isoindole, [6] 6-[2,2,4,6,7-pentamethyl-3-(4-methylphenyl)-2,3-dihydro-1-benzofuran-5-yl]-6H-[1,3]dioxolo[4,5-f]isoindole, [7] 6-(2,2,4,6,7-pentamethyl-3-phenyl-2,3-dihydro)-1-benzofuran-5-yl)-6,7-dihydro-5H-[1,3]dioxolo[4,5-f]isoindole, [8] (R)-5,6-dimethoxy-2-[2,2,4,6,7-pentamethyl-3-(4-methylphenyl)-2,3-dihydro-1-benzofuran-5-yl]isoindoline or [9] (R)-5,6-dimethoxy-2-[2,2,4,6,7-pentamethyl-3-(4-methylphenyl)-2,3-dihydro-1-benzofuran-5-yl]isoindoline hydrochloride.
22. The method according to Paragraph 18 comprising (R)-5,6-dimethoxy-2-[2,2,4,6,7-pentamethyl-3-(4-methylphenyl)-2,3-dihydro-1-benzofuran-5-yl]isoindoline.
23. The method according to Paragraph 15 wherein W is a group represented by Formula (Wb).
24. The method according to Paragraph 23 wherein each of R¹ and R² is a methyl group, R³ is a phenyl group which may have 1 to 3 substituents selected from fluorine, methyl and isopropyl, the Ring C is a benzene ring which may further have 1 to 3 substituents selected from C₁₋₆ alkyl and C₁₋₆ alkoxy, Y is an oxygen atom, R⁴ is a benzyl orphenethyl group which may have 1 to 3 substituents selected from fluorine, methoxy and methylenedioxy and R⁵ is a hydrogen atom or a methyl group.
25. The method according to Paragraph 23 comprising (1) N-(4-fluorobenzyl)-2,2,4,6,7-pentamethyl-3-phenyl-2,3-dihydro-1-benzofuran-5-amine, (2) N-benzyl-3-(4-isopropylphenyl)-2,2,4,6,7-pentamethyl-2,3-dihydro-1-benzofuran-5-amine (3) 3-(4-isopropylphenyl-N-(4-methoxybenzyl)-N,2,2,4,6,7-hexamethyl-2,3-dihydro-1-benzofuran-5-amine, (4) 3-(4-isopropylphenyl)-N-[2(4-methoxyphenyl)ethyl]-2,2,4,6,7-pentamethyl-2,3 dihydro-1-benzofuran-5-amine, (5) N-(4-fluorobenzyl)-3-(4-isopropylphenyl)-2,2,4,6,7-pentamethyl-2,3-dihydro-1-benzofuran-5-amine, (6) N-(1-benzodioxol-5-ylmethyl)-3-(4-isopropylphenyl)-2,2,4,6,7-pentamethyl-2,3-dihydro-1-benzofuran-5-amine, (7) N-(4-fluorobenzyl)-3-(4-fluorophenyl)-2,2,4,6,7-pentamethyl-2,3-dihydro-1-benzofuran-5-amine, (8) N-(4-methoxybenzyl)-2,2,4,6,7-pentamethyl-3-(4-methylphenyl)-2,3-dihydro-1-benzofuran-5-amine, (9) N-(4-fluorobenzyl)-2,2,4,6,7-pentamethyl-3-(4-methylphenyl)-2,3 dihydro-1-benzofuran-5-amine, (10) 3-(4-isopropylphenyl)-N-(4-methoxybenzyl)-2,4,6,7-tetramethyl-1-benzofuran-5-amine, (11) N-(4-fluorobenzyl)-3-(4-isopropylphenyl)-2,4,6,7-tetramethyl-1-benzofuran-5-amine, (12) N-(4-fluorobenzyl)-3-(4-fluorophenyl)-2,4,6,7-tetramethyl-1-benzofuran-5-amine, (13) N-(4-fluorobenzyl)-3-(4-isopropylphenyl)-1',4,6,7-tetramethylspiro[benzofuran-2-(3H), 4'-pyperidine]-5-amine or (14) (R)-N-(4-fluorobenzyl)-3-(4-isopropylphenyl)-2,2,4,6,7-pentamethyl-2,3-dihydro-1-benzofuran-5-amine hydrochloride.
26. The method according to Paragraph 15 wherein W is a group represented by Formula (Wc).
27. The method according to Paragraph 26 comprising a compound represented by Formula: 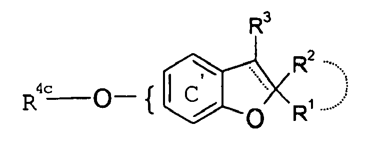 wherein each of R¹ and R² is C₁₋₆-alkyl which may have a phenyl-substituted 6-membered saturated cyclic amino, or R¹ and R² are taken together with the adjacent carbon atom to form a C₁₋₆ alkyl- or C₇₋₁₆ aralkyl-substituted piperidine;
   R³ is (i) a hydrogen atom, or,
   (ii) phenyl which may have 1 to 3 substituents selected from (1) C₁₋₆alkyl, (2) di-C₁₋₆ alkylamino and (3) 6-membered saturated cyclic amino which may have C₁₋₆alkyl;
   R^{4c} is (i) phenyl which may have 1 to 3 substituents selected from nitro and C₁₋₆alkyl-carboxamide,
   (ii)C₁₋₆-alkyl or C₂₋₆-alkenyl having 1 to 3 phenyl, quinolyl or pyridyl which may have 1 to 3 substituents selected from C₁₋₆ alkoxy, C₁₋₆ alkylthio, C₁₋₆ alkoxy-carbonyl, C₁₋₆alkylsulfonyl and C₁₋₆alkylsulfinyl and optionally further having phenyl, carboxy or C₁₋₆alkoxy-carbonyl as additional substituents, or,
   (iii) acyl represented by Formula: -(C=O)-R^{5"} wherein R^{5"} is C₁₋₆ alkoxy-substituted phenyl; and,
   the Ring C' is a benzene ring which may further have 1 to 3 C₁₋₆ alkyl, or a salt thereof.
28. The method according to Paragraph 26 comprising:
   3-(4-isoprophylphenyl)-5-(4-methoxybenzyloxy)-2,2,4,6,7-pentamethyl-2,3-dihydrobenzofuran, 3-(4-methylphenyl)-2,4,6,7-tetramethylbenzofuran-5-yl-4-methoxybenzoate, 3-(4-isopropylphenyl)-2,4,6,7-tetramethylbenzofuran-5-yl-4-methoxybenzoabe, 3-(4-isopropylphenyl)-5-(4-methoxybenzyloxy)-2,4,6,7-tetramethylbenzofuran, 3-(4-isopropylphenyl)-5-methoxybenzyloxy)-1',4,6,7-tetramethylspiro[benzofuran-2-(3H)-4'-piperidine] or a salt thereof
29. The method according to Paragraph 15 wherein the stem cell is an embryonic stem cell or a neural stem cell.
30. The method according to Paragraph 15 whereby a cell for tansplantation therapy is prepared.

### Brief Description of Drawings

Figure 1 shows a graph indicating a differentiation promoting effect of a test compound on a neural stem cell in an Experimental Example described below.

### Detailed Description of the Invention

In the formula shown above, --- is a single bond or a double bond. Preferably, --- is a single bond.

In the formula shown above, R¹ and R² are same or different and each is a hydrogen atom, an optionally substituted hydrocarbon group or an optionally substituted heterocyclic group or R¹ and R² may be taken together with the adjacent carbon atom to form an optionally substituted 3- to 8-membered homocyclic or heterocyclic ring.

In the formula shown above, when --- is a double bond, then R² is not present. Thus, in the formula shown above:
(i) when --- is a single bond, the moiety: 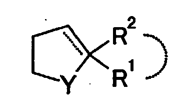 is the moiety: 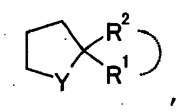
(ii) when --- is a double bond, the moiety: 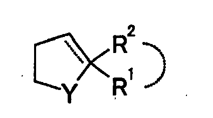 is the moiety: 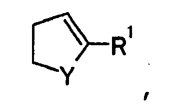 but in this specification (i) and (ii) are sometimes unified to be represented just by Formula:

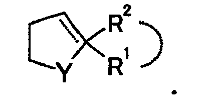

A "hydrocarbon group" in the "optionally substituted hydrocarbon group" represented by R¹ or R² may for example be a linear or branched or cyclic hydrocarbon group (e.g., alkyl, alkenyl, alkynyl, cycloalkyl, aryl). Among those listed above, a linear or branched or cyclic hydrocarbon group having 1 to 16 carbon atoms is preferred.

Preferred "alkyl" may for example be C₁₋₆ alkyl (e.g., methyl, ethyl, propyl, isopropyl, butyl, isobutyl, sec-butyl, tert-butyl, pentyl, hexyl).

Preferred "alkenyl" may for example be C₂₋₆ alkenyl (e.g., vinyl, allyl, isopropenyl, butenyl, isobutenyl, sec-butenyl).

Preferred "alkynyl" may for example be C₂₋₆ alkynyl (e.g., ethynyl, propargyl, butynyl, 1-hexynyl).

Preferred "cycloalkyl" may for example be C₃₋₆ cycloalkyl (e.g., cyclopropyl, cyclobutyl, cyclopentyl, cyclohexyl).

Preferred "aryl" mayor example be C₆₋₁₄ aryl (e.g., phenyl, 1-naphthyl, 2-naphthyl, biphenylyl, 2-anthryl).

A "substituent" in the "optionally substituted hydrocarbon group" represented by R¹ or R² may for example be (1) a halogen atom (e.g., fluorine, chlorine, bromine, iodine), (2) C₁₋₃ alkylenedioxy (e.g., methylenedioxy, ethylenedioxy), (3) nitro, (4) cyano, (5) optionally halogenated C₁₋₆ alkyl, (6) optionally halogenated C₂₋₆ alkynyl, (7) optionally halogenated C₂₋₆ alkynyl (8) optionally halogenated C₃₋₆ cycloalkyl, (9) C₆₋₁₄ aryl (e.g., phenyl, 1-naphthyl, 2-naphthyl, biphenylyl, 2-anthryl), (10) optionally halogenated C₁₋₆ alkoxy, (11) optionally halogenated C₁₋₆ alkylhtio or mercapto, (12) hydroxy, (13) amino, (14) mono-C₁₋₆ alkylamino (e.g., methylamino, ethylamino and the like), (15) mono-C₆₋₁₄ arylamino (e.g., phenylamino, 1-naphthylamino, 2-naphthylamino), (16) di-C₁₋₆ alkylamino (e.g., dimethylamino, diethylamino), (17) di-C₆₋₁₄ arylamino (e.g., diphenylamino), (18) acyl, (19) acylamino, (20) acyloxy, (21) optionally substituted 5- to 7-membered saturated cyclic amino, (22) a 5- to 10-membered aromatic heterocyclic group (e.g., 2- or 3-thienyl, 2-, 3-or 4-pyridyl, 2-, 3-, 4-, 5- or 8-quinolyl, 1-, 3-, 4- or 5-isoquinolyl, 1-, 2- or 3-indolyl, 2-benzothiazolyl, 2-benzo [b] thienyl, benzo[b]furanyl), (23) sulfo, (24) C₆₋₁₄ aryloxy (e.g., phenyloxy, naphthyloxy).

The "hydrocarbon group" may have 1 to 5, preferably 1 to 3 of the above listed substituents in any substitutable positions, and when the number of the substituents is 2 or more, then each substituent may be same to or different from each other.

The "optionally halogenated C₁₋₆ alkyl," mentioned above may for example be C₁₋₆ alkyl (e.g., methyl, ethyl, propyl, isopropyl, butyl, isobutyl, sec-butyl, tert-butyl, pentyl, hexyl) which may have 1 to 5, preferably 1 to 3 halogen atoms (e.g., fluorine, chlorine, bromine, iodine). Those exemplified typically are methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-tricluorohexyl and the like.

The "optionally halogenated C₂₋₆ alkenyl" mentioned above may for example be C₂₋₆ alkenyl (e.g., vinyl, allyl, isopropenyl, butenyl, isobutenyl, sec-butenyl) which may have 1 to 5, preferably 1 to 3 halogen atoms (e.g., fluorine, chlorine, bromine, iodine). Those exemplified typically are vinyl, allyl, isopropenyl, butenyl, isobutenyl, sec-butenyl, 3,3,3-trifluoro-1-propenyl, 4,4,4-trifluoro-1-butenyl and the like.

The "optionally halogenated C₂₋₆ alkynyl" mentioned above may for example be C₂₋₆ alkynyl (e.g., ethynyl, propargyl, butynyl, 1-hexynyl) which may have 1 to 5, preferably 1 to 3 halogen atoms (e.g., fluorine, chlorine, bromine, iodine). Those exemplified typically are ethynyl, propargyl, butynyl, 1-hexynyl, 3,3,3-trifluoro-1-propynyl, 4,4,4-trifluoro-1-butynyl and the like.

The "optionally halogenated C₃₋₆ cycloalkyl" mentioned above may for example be C₃₋₆ cycloalkyl (e.g., cyclopropyl, cyclobutyl, cyclopentyl, cyclohexyl) which may have 1 to 5, preferably 1 to 3 halogen atoms (e.g., fluorine, chlorine, bromine, iodine). Those exemplified typically are cyclopropyl, cyclobutyl, cyclopentyl, cyclohexyl, 4,4-dichlorocyclohexyl, 2,2,3,3-tetrafluorocyclopentyl, 4-chlorocyclohexyl and the like.

The "optionally halogenated C₁₋₆ alkoxy" mentioned above may for example be C₁₋₆ alkoxy (e.g., methoxy, ethoxy, propoxy, isopropoxy, butoxy, isobutoxy, sec-butoxy, pentyloxy, hexyloxy) which may have 1 to 5, preferably 1 to 3 halogen atoms (e.g., fluorine, chlorine, bromine, iodine). Those exemplified typically are methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy and the like.

The "optionally halogenated C₁₋₆ alkylthio" mentioned above may for example be C₁₋₆ alkylthio (e.g., methylthio, ethylthio, propylthio, isopropylthio, butylthio, sec-butylthio, tert-butylthio) which may have 1 to 5, preferably 1 to 3 halogen atoms (e.g., fluorine, chlorine, bromine, iodine). Those exemplified typically are methylthio, difluoromethylthio, trifluoromethylthio, ethylthio, propylthio, isopropylthio, butylthio, 4,4,4-trifluorobutylthio, pentylthio, hexylthio and the like.

The "acyl" mentioned above may for example be formyl, carboxy, carbamoyl, C₁₋₆ alkyl-carbonyl (e.g., acetyl, propionyl), C₃₋₆ cycloalkyl-carbonyl (e.g., cyclopropylcarbonyl, cyclopentylcarbonyl, cyclohexylcarbonyl), C₁₋₆ alkoxy-carbonyl (e.g., methoxycarbonyl, ethoxycarbonyl, propoxycarbonyl, tert-butoxycarbonyl), C₆₋₁₄ aryl-carbonyl (e.g., benzoyl, 1-naphthoyl, 2-naphthoyl), C₇₋₁₆ aralkyl-carbonyl (e.g., phenylacetyl, phenylpropionyl), C₆₋₁₄ aryloxy-carbonyl (e.g., phenoxycarbonyl), C₇₋₁₆ aralkyloxy-carbonyl (e.g., benzyloxycarbonyl, phenethyloxycarbonyl), 5- or 6-membered heterocyclic carbonyl (e.g., nicotinoyl, isonicotinoyl, 2-thenoyl, 3-thenoyl, 2-furoyl, 3-furoyl, morpholinocarbonyl, thiomorpholinocarbonyl, piperidinocarbonyl, 1-pyrrolidinylcarbonyl), mono-C₁₋₆ alkyl-carbamoyl (e.g., methylcarbamoyl, ethylcarbamoyl), di-C₁₋₆ alkyl-carbamoyl (e.g., dimethylcarbamoyl, diethylcarbamoyl, ethylmethylcarbamoyl), C₆₋₁₄ aryl-carbamoyl (e.g., phenylcarbamoyl, 1-naphthylcarbamoyl, 2-naphthylcarbamoyl), thiocarbamoyl, 5- or 6-membered heterocyclic carbamoyl (e.g., 2-pyridylcarbamoyl, 3-pyridylcarbamoyl, 4-pyridylcarbamoyl, 2-thienylcarbampyl, 3-thienylcarbamoyl), C₁₋₆ alkylsulfonyl (e.g., methylsulfonyl, ethylsulfonyl), C₆₋₁₄ arylsulfonyl (e.g., phenylsulfonyl, 1-naphthylsulfoniyl, 2-naphthylsulfonyl), C₁₋₆ alkylsulfinyl (e.g., methylsulfinyl, ethylsulfinyl), C₆₋₁₄ arylsulfinyl (e.g., phenylsulfinyl, 1-naphthylsulfinyl, 2-naphthylsulfinyl).

The "acylamino" mentioned above may for example be formylamino, C₁₋₆ alkyl-carbonylamino (e.g., acetylamino), C₆₋₁₄ aryl-carbonylamino (e.g., phenylcarbonylamino, naphthylcarbonylamino), C₁₋₆ alkoxy-carbonylamino (e.g., methoxycarbonylamino, ethoxycarbonylamino, propoxycarbonylamino, butoxycarbonylamino), C₁₋₆ alkylsulfonylamino (e.g., methylsulfonylamino, ethylsulfonylamino), C₆₋₁₄ arylsulfonylamino (e.g., phenylsulfonylamino, 2-naphthylsulfonylamino, 1-naphthylsulfonylamino).

The "acyloxy" mentioned above may for example be formyloxy, C₁₋₆ alkyl-carbonyloxy (e.g., acetoxy, propionyloxy), C₆₋₄ aryl-carbonyloxy (e.g., benzoyloxy, naphthylcarbonyloxy), C₁₋₆ alkoxy-carbonyloxy (e.g., methoxycarbonyloxy, ethoxycarbonyloxy, propoxycarbonyloxy, butoxycarbonyloxy), mono-C₁₋₆ alkyl-carbamoyloxy (e.g., methylcarbamoyloxy, ethylcarbamoyloxy), di-C₁₋₆ alkyl-carbamoyloxy (e.g., dimethylcarbamoyloxy, diethylcarbamoyloxy), C₆₋₁₄ arylcarbamoyloxy (e.g., phenylcarbamoyloxy, naphthylcarbamoyloxy), nicotinoyloxy and the like.

A "5- to 7-membered saturated cyclic amino" in the "optionally substituted 5- to 7-membered saturated cyclic amino" mentioned above may for example be morpholino, thiomorpholino, piperazin-1-yl, piperidino, pyrrolidin-1-yl and the like. A "substituent" in such an "optionally substituted 5- to 7-membered saturated cyclic amino" may for example be C₁₋₆ alkyl (e.g., methyl, ethyl, propyl, isopropyl, butyl, isobutyl, sec-butyl, tert-butyl, pentyl, hexyl), C₆₋₁₄ aryl (e.g., phenyl, 1-naphthyl, 2-naphthyl, biphenylyl, 2-anthryl), 5- to 10-mebered aromatic heterocyclic group (e.g., 2- or 3-thienyl, 2-, 3- or 4-pyridyl, 2-, 3-, 4-, 5- or 8-quinolyl, 1-, 3-, 4- or 5-isoquinolyl, 1-, 2- or 3-indolyl, 2-benzothiazolyl, 2-benzo[b]thienyl, benzo[b]furanyl), 1 to 3 of which may be employed.

A "substituent" in the "optionally substituted heterocyclic group" represented by R¹ or R² may for example be a 5- to 14-membered heterocyclic group (aromatic heterocyclic group, saturated or unsaturated non-aromatic heterocyclic group) containing 1 to 4 heteroatoms selected from nitrogen, sulfur and oxygen atoms in addition to carbon atoms.

Such "aromatic heterocyclic group" may for example be a 5- to 14-membered, preferably 5- to 10-membered aromatic heterocyclic group containing one or more (for example 1 to 4) heteroatoms selected from nitrogen, sulfur and oxygen atoms in addition to carbon atoms. Those exemplified typically are a monovalent group formed by removing any hydrogen atom from an aromatic heterocyclic ring such as thiophene, benzothiophene, benzofuran, benzimidazole, benzoxazole, benzothiazole, benzisothiazole, naphtho[2,3-b]thiophene, furane, isoindolidine, xantholene, phenoxathiine, pyrrole, imidazole, pyrazole, pyridine, pyrazine, pyrimidine, pyridazine, indole, isoindole, 1H-indazole, purine, 4H-quinolidine, isoquinoline, quinoline, phthalazine, naphthylidine, quinoxaline, quinazoline, cinnoline, carbazole, β-carboline, phenanthridine, acridine, phenazine, thiazole, isothiazole, phenothiazine, oxazole, isoxazole, furazane or phenoxazine, or a ring formed by condensation of any of the ring listed above (preferably monocyclic ring) with one ore more (preferably 1 or 2) aromatic rings (e.g., benzene ring, etc.) and the like.

A preferred "aromatic heterocyclic group" may for example be a 5- or 6-membered aromatic heterocyclic group which may be fused with a single benzene ring. Those exemplified typically are 2-, 3- or 4-pyridyl, 2-, 3-, 4-, 5- or 8-quinolyl, 1-, 3-, 4- or 5-isoquinolyl, 1-, 2- or 3-indolyl, 2-benzothiazolyl, 2-benzo[b]thienyl, benzo[b]furanyl, 2- or 3-thienyl and the like. Those employed more preferably are 2- or 3-thienyl, 2-, 3- or 4-pyridyl, 2- or 3-quinolyl, 1-isoquinolyl, 1- or 2-indolyl, 2-benzothiazolyl and the like.

A "non-aromatic heterocyclic group" may for example be a 3- to 8-membered (preferably 5- to 6-membered) saturated or unsaturated (preferably saturated) non-aromatic heterocyclic group (aliphatic heterocyclic group) such as oxylanyl, azethidinyl, oxetanyl, thietanyl, pyrrolidinyl, tetrahydrofuryl, thiolanyl, piperidyl, tetrahydropyranyl, morpholinyl, thiomorpholinyl, piperazinyl and the like.

A "substituent" in the "optionally substituted heterocyclic group" represented by R¹ or R² is similar to the "substituent" in the "optionally substituted hydrocarbon group" represented by R¹ or R² described above, and the same number of such substituents is employed.

A "3- to 8-membered homocyclic ring" in the "optionally substituted 3- to 8-membered homocyclic ring" formed from R¹ and R² may for example be a C₃₋₈ cycloalkane such as cyclopropane, cyclobutane, cyclopentan, cyclohexane and the like.

A "3- to 8-membered heterocyclic ring" in the "optionally substituted 3- to 8-membered heterocyclic ring" formed from R¹ and R² may for example be a 3- to 8-membered heterocyclic ring containing 1 to 4 heteroatoms selected from nitrogen, sulfur and oxygen atoms in addition to carbon atoms, such as aziridine, azetidine, morpholine, thiomorpholine, piperazine, piperidine, pyrrolidine, hexamethyleneimine, heptamethyleneimine, hexahydropyrimidine and the like.

A "substituent" in the "optionally substituted 3- to 8-membered homocyclic or heterocyclic ring" formed from R¹ and R² is similar to the "substituent" in the "optionally substituted hydrocarbon group" represented by R¹ or R² described above, and the same number of such substituents is employed.

The "optionally substituted hydrocarbon group" and "optionally substituted heterocyclic group" represented by R³ are similar to the "optionally substituted hydrocarbon group" and "optionally substituted heterocyclic group" represented by R¹ or R² described above.

In the formula shown above, W is:
(i) a group represented by Formula: 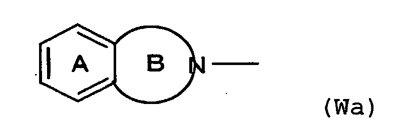 wherein Ring A is an optionally substituted benzene ring, Ring B is an optionally substituted 5- to 7-membered nitrogen-containing heterocyclic ring,
(ii) a group represented by Formula: 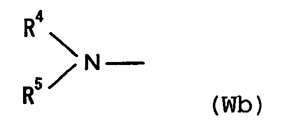 wherein R⁴ is (1) an aliphatic hydrocarbon group which is substituted by an optionally substituted aromatic group and which may have a further substituent or (2) an optionally substituted aromatic ring-containing acyl group, R⁵ is a hydrogen atom, a C₁₋₆ alkyl or an acyl group, or,
(iii) a group represented by Formula:

   R^{4c} - X- (Wc)
wherein R^{4c} is an optionally substituted aromatic group, an optionally substituted aliphatic hydrocarbon group or an acyl group, X is an oxygen atom or an optionally oxidized sulfur atom.

When W is Wa, R³ in the formula shown above is preferably a hydrogen atom, an optionally substituted hydrocarbon group or an optionally substituted heterocyclic group (hereinafter sometimes referred to as R^{3a}).

In the formula shown above, the Ring A is an optionally substituted benzene ring.

A "substituent" in the "optionally substituted benzene ring" represented by the Ring A is similar to the "substituent" in the "optionally substituted hydrocarbon group" represented by R¹ or R² described above, and the "optionally substituted benzene ring" may have 1 to 4 (preferably 1 or 2) such substitutents at any substitutable positions, and when the number of such substituents is 2 or more, the substituents may be the same as or different from each other.

In the formula shown above, the Ring B is an optionally substituted 5- to 7-membered nitrogen-containing heterocyclic ring.

The "5- to 7-membered nitrogen-containing heterocyclic ring" represented by the Ring B may for example be a 5- to 7-membered nitrogen-containing heterocyclic ring such as pyrrole (e.g., 1H-pyrrole), dihydropyrrole (e.g., 2,5-dihydro-1H-pyrrole), dihydropyridine (e.g., 1,2-dihydropyridine), tetrahyrdopyridine (e.g., 1,2,3,4-tetrahydropyridine), azepine (e.g., 1H-azepine), dihydroazepine (e.g., 2,3-dihydro-1H-azepine, 2,5-dihydro-1H-azepine, 2,7-dihydro-1H-azepine), tetrahydroazepine (e.g., 2,3,6,7-tetrahydro-1H-azepine, 2,3,9,7-tetrahydro-1H-azepine) and the like.

A "substituent" in the "optionally substituted 5- to 7-membered nitrogen-containing heterocyclic ring" represented by the Ring B is similar to the "substituent" in the "optionally substituted hydrocarbon group" represented by R¹ or R² described above, and the same number of such substituents is employed. The substituent on the Ring B may also be an oxo group and the like.

A group represented by Formula: 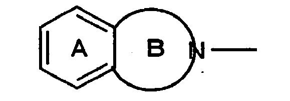 wherein each symbol is as defined above, is more typically a group represented by Formulae: 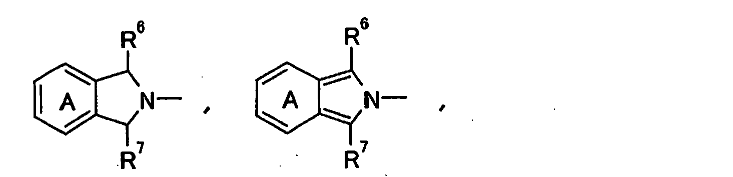 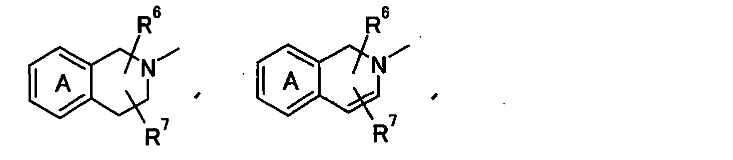 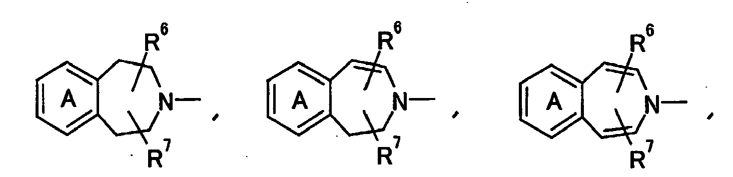 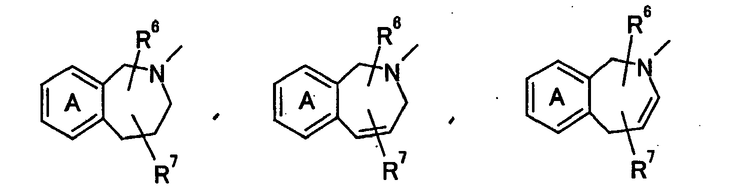 wherein R⁶ and R⁷ are same or different and each is a hydrogen atom, a halogen or an optionally substituted hydrocarbon group, and Ring A is as defined above, preferably a group represented by Formulae: 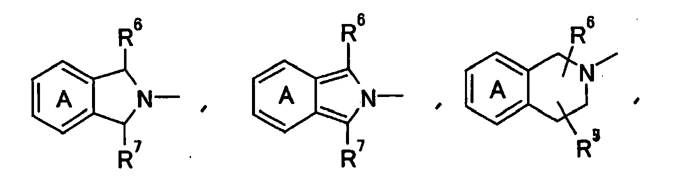 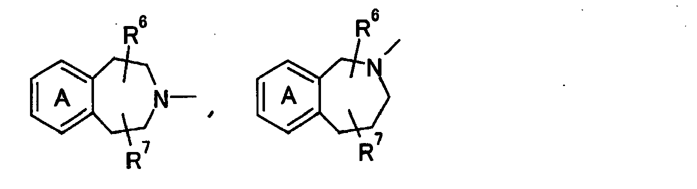 wherein each symbol is as defined above, more preferably a group represented by Formulae: 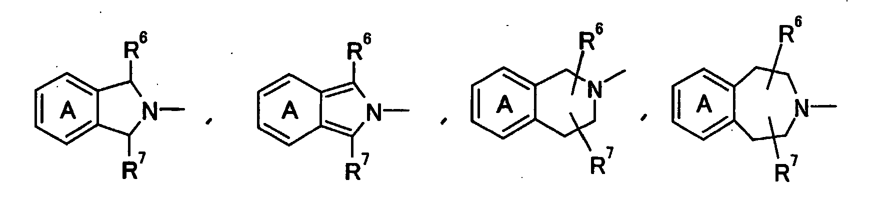 wherein each symbol is as defined above, especially a group represented by Formulae: 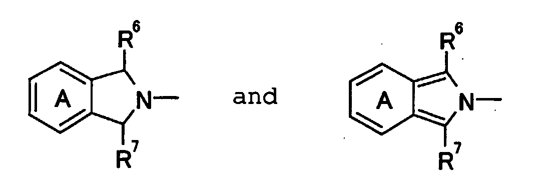 wherein each symbol is as defined above.

The "halogen" or "optionally substituted hydrocarbon group" represented by R⁶ and R⁷ is similar to the "halogen" or "optionally substituted hydrocarbon group" as "substituent" on the Ring B described above.

In the formula shown above, the Ring C is a benzene ring which may further have a substituent in addition to the group represented by W.

The Ring C may have 1 to 3 (preferably 1) groups represented by W at any substitutable positions, and when the number of the substituents is 2 or more, then they may be the same as or different from each other.

A "substituent" which the Ring C may further have is similar to the "substituent" in the "optionally substituted hydrocarbon group" represented by R¹ or R² described above. A "C₁₋₆ alkyl" group as a "substituent" on the Ring C may be substituted for example by a "4- to 8-membered lactone which may be substituted for example by hydroxy (for example, 3-hydroxy-8-valerolactone) or the like. The Ring C may have 1 to 3 (preferably 3) such substituents at any substitutable position, and when the number of the substituents is 2 or more, then they may be the same as or different from each other.

The Ring C is preferably a benzene ring substituted by three C₁₋₆ alkyl groups such as methyl.

When W is Wa, then the Ring C in the formula shown above is preferably a benzene ring which may further have a substituent selected from a halogen, optionally halogenated lower alkyl, optionally halogenated lower alkoxy and optionally halogenated lower alkylthio in addition to a group represented by Formula: 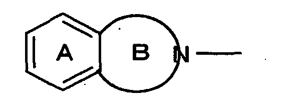 wherein each symbol is as defined above, (hereinafter sometimes referred to as Ring C¹) .

The ring C¹ may have 1 to 3 (preferably 1) substituents represented by Formula: 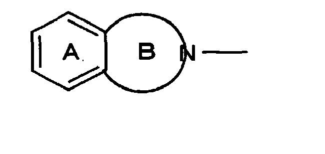 at any substitutable positions, and when the number of the substituents is 2 or more, then they may be the same as or different from each other.

The "halogen" as a "substituent" which the ring C¹ may further have may for example be fluorine, chlorine, bromine or iodine. The "optionally halogenated lower alkyl" may for example be C₁₋₆ alkyl (e.g., methyl, ethyl, propyl, isopropyl, butyl, isobutyl, sec-butyl, tert-butyl, pentyl, hexyl) which may have 1 to 5, preferably 1 to 3 halogen atoms (e.g., fluorine, chlorine, bromine, iodine), and those exemplified typically are methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl and the like.

The "optionally halogenated lower alkoxy" may for example be C₁₋₆ alkoxy (e.g., methoxy, ethoxy, propoxy, isopropoxy, butoxy, isobutoxy, sec-butoxy, pentyloxy, hexyloxy) which may have 1 to 5, preferably 1 to 3 halogen atoms (e.g., fluorine, chlorine, bromine, iodine). Those exemplified typically are methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy and the like. An "optionally halogenated lower alkylthio" group mentioned above may for example be C₁₋₆ alkylthio (e.g., methylthio, ethylthio, propylthio, isopropylthio, butylthio, sec-butylthio, tert-butylthio) which may have 1 to 5, preferably 1 to 3 halogen atoms (e.g., fluorine, chlorine, bromine, iodine). Those exemplified typically are methylthio, difluoromethylthio, trifluoromethylthio, ethylthio, propylthio, isopropylthio, butylthio, 4,4,4-trifluorobutylthio, pentylthio, hexylthio and the like.

The Ring C¹ may have 1 to 3 (preferably 3) such substituents at any substitutable positions, and when the number of the substituents is 2 or more, then they may be the same as or different from each other.

When W is Wb, then R³ in the formula shown above is preferably an optionally substituted C₆₋₁₄ aryl group (hereinafter sometimes referred to as R^{3b}).

A "C₆₋₁₄ aryl" group in the "optionally substituted C₆₋₁₄ aryl" represented by R^{3b} may for example be C₆₋₁₄ aryl such as phenyl, 1-naphthyl, 2-naphthyl, biphenylyl, anthryl and the like.

A "substituent" in such "optionally substituted C₆₋₁₄ aryl" is similar to the "substituent" in the "optionally substituted hydrocarbon group" represented by R¹ or R² described above, and the same number of such substituents is employed.

In the formula shown above, R⁴ is (1) an aliphatic hydrocarbon group which is substituted by an optionally substituted aromatic group and which may further have a substituent, or (2) an acyl group which may contain an optionally substituted aromatic group.

An "aromatic group" in the "optionally substituted aromatic group" as a substituent on the "aliphatic hydrocarbon group which has an optionally substituted aromatic group and which may further have a substituent" represented by R⁴ may for example be an aromatic hydrocarbon group and an aromatic heterocyclic group.

Such "aromatic hydrocarbon group" may for example be a monocyclic or fused polycyclic (dicyclic or tricyclic) aromatic hydrocarbon group having 6 to 14 carbon atoms. Those exemplified typically are C₆₋₄ aryl groups such as phenyl, 1-naphthyl, 2-naphthyl, biphenylyl, anthryl and the like, preferably C₆₋₁₀ aryl such as phenyl, 1-naphthyl, 2-naphthyl and the like.

Such "aromatic heterocyclic group" may for example be a 5- to 14-membered, preferably 5- to 10-membered aromatic heterocyclic group containing one or more (for example 1 to 4) heteroatoms selected from nitrogen, sulfur and oxygen atoms in addition to carbon atoms. Those exemplified typically are a monovalent group formed by removing any hydrogen atom from an aromatic heterocyclic ring such as thiophene, benzothiophene, benzofuran, benzimidazole, benzoxazole, benzothiazole, benzisothiazole, naphtho[2,3-b]thiophene, furane, isoindolidine, xantholene, phenoxathiine, pyrrole, imidazole, pyrazole, pyridine, pyrazine, pyrimidine, pyridazine, indole, isoindole, 1H-indazole, purine, 4H-quinolidine, isoquinoline, quinoline, phthalazine, naphthylidine, quinoxaline, quinazoline, cinnoline, carbazole, β-carboline, phenanthridine, acridine, phenazine, thiazole, isothiazole, phenothiazine, oxazole, isoxazole, furazane or phenoxazine, or a ring formed by condensation of any of the ring listed above (preferably monocyclic ring) with one ore more (preferably 1 or 2) aromatic rings (e.g., benzene ring, etc.) and the like.

A preferred "aromatic heterocyclic group" may for example be a 5- or 6-membered aromatic heterocyclic group which may be fused with a single benzene ring. Those exemplified typically are 2-, 3- or 4-pyridyl, 2-, 3-, 4-, 5- or 8-quinolyl, 1-, 3-, 4- or 5-isoquinolyl, 1-, 2- or 3-indolyl, 2-benzothiazolyl, 2-benzo[b]thienyl, benzo[b]furanyl, 2- or 3-thienyl and the like. Those employed more preferably are 2- or 3-thienyl, 2-, 3- or 4-pyridyl, 2- or 3-quinolyl, 1-isoquinolyl, 1- or 2-indolyl, 2-benzothiazolyl and the like.

A "substituent" in the "optionally substituted aromatic group" is similar to the "substituent" in the "optionally substituted hydrocarbon group" represented by R¹ or R² described above, and the same number of such substituents is employed.

An "aliphatic hydrocarbon group" in the "aliphatic hydrocarbon group which has an optionally substituted aromatic group and which may further have a substituent" represented by R⁴ may for example be alkyl, alkenyl, alkynyl, cycloalkyl and the like. Those preferred especially are C₁₋₁₀ alkyl, C₂₋₁₀ alkenyl, C₂₋₁₀ alkynyl, C₃₋₁₀ cycloalky and the like.

Preferred "alkyl" may for example be C₁₋₆ alkyl (e.g., methyl, ethyl, propyl, isopropyl, butyl, isobutyl, sec-butyl, tert-butyl, pentyl, hexyl).

Preferred "alkenyl" may for example be C₂₋₆ alkenyl (e.g., vinyl, allyl, isopropenyl, butenyl, isobutenyl, sec-butenyl).

Preferred "alkynyl" may for example be C₂₋₆ alkynyl (e.g., ethynyl, propargyl, butynyl, 1-hexynyl).

Preferred "cycloalkyl" may for example be C₃₋₆ cycloalkyl (e.g., cyclopropyl, cyclobutyl, cyclopentyl, cyclohexyl).

Among those listed above, a C₁₋₆ alkyl group is preferred.

The "aliphatic hydrocarbon group" mentioned above may have 1 to 3 "optionally substituted aromatic groups" at any substitutable positions, and when the number of such substituents is 2 or more, then they may be the same as or different from each other.

A "substituent" which the "aliphatic hydrocarbon group" may further have is similar to the "substituent" in the "optionally substituted hydrocarbon group" represented by R¹ or R² described above, and the same number of such substituents is employed.

An "acyl group" in the "acyl group which may contain an optionally substituted aromatic group" represented by R⁴ is similar to the "acyl group" as "substituent" in the "optionally substituted hydrocarbon group" represented by R¹ or R² described above.

An "optionally substituted aromatic group" in the "acyl group which may contain an optionally substituted aromatic group" represented by R⁴ is similar to the "optionally substituted aromatic group" in the "aliphatic hydrocarbon group which has an optionally substituted aromatic group and which may further have a substituent" represented by R⁴ described above.

Those exemplified typically as the "acyl group which may contain an optionally substituted aromatic group" represented by R⁴ are preferably C₆₋₁₄ aryl-carbonyl (e.g., benzoyl, 1-naphthoyl, 2-naphthoyl), C₇₋₁₆ aralkyl-carbonyl (e.g., phenylacetyl, phenylpropionyl), C₆₋₁₄ aryloxy-carbonyl (e.g., phenoxycarbonyl), C₇₋₁₆ aralkyloxy-carbonyl (e.g., benzyloxycarbonyl, phenethyloxycarbonyl), 5- or 6-membered heterocyclic carbonyl (e.g., nicotinoyl, isonicotinoyl, 2-thenoyl, 3-thenoyl, 2-furoyl, 3-furoyl, morpholinocarbonyl, thiomorpholinocarbonyl, piperidinocarbonyl, 1-pyrrolidinylcarbonyl), C₆₋₁₄ aryl-carbamoyl (e.g., phenylcarbamoyl, 1-naphthylcarbamoyl, 2-naphthylcarbamoyl), 5- or 6-membered heterocyclic carbamoyl (e.g., 2-pyridylcarbamoyl, 3-pyridylcarbamoyl, 4-pyridylcarbamoyl, 2-thienylcarbamoyl, 3-thienylcarbamoyl), C₆₋₁₄ arylsulfonyl (e.g., phenylsulfonyl, 1-naphthylsulfonyl, 2-naphthylsulfonyl), C₆₋₁₄ arylsulfinyl (e.g., phenylsulfinyl, 1-naphthylsulfinyl, 2-naphthylsulfinyl).

In the formula shown above, R⁵ is a hydrogen atom, a C₁₋₆ alkyl group or an acyl group.

The C₁₋₆ alkyl group represented by R⁵ may for example be methyl, ethyl, propyl, isopropyl, butyl, isobutyl, sec-butyl, tert-butyl, pentyl, hexyl and the like.

The "acyl group" represented by R⁵ is similar to the "acyl group" as "substituent" in the "optionally substituted hydrocarbon group" represented by R¹ or R² described above.

When W is Wb, the Ring C in the formula shown above is a benzene ring which may further have a substituent in addition to a group represented by Formula: -NR⁴(R⁵) (hereinafter sometimes referred to as ring C²).

The Ring C² may have 1 to 3 groups represented by Formula: -NR⁴(R⁵) at any substitutable positions, and when the number of the substituents is 2 or more, then each substituent may be same to or different from each other.

A "substituent" which the Ring C² may further have in addition to a group represented by Formula: -NR⁴ (R⁵) may for example be a halogen atom (e.g., fluorine, chlorine, bromine, iodine), C₁₋₃ alkylenedioxy (e.g., methylenedioxy, ethylenedioxy), nitro, cyano, optionally halogenated C₁₋₆ alkyl, optionally halogenated C₂₋₆ alkenyl, optionally halogenated C₂₋₆ alkynyl, optionally halogenated C₃₋₆ cycloalkyl, C₆₋₁₄ aryl (e.g., phenyl, 1-naphthyl, 2-naphthyl, biphenylyl, 2-anthryl), optionally halogenated C₁₋₆ alkoxy, hydroxy, amino, mono-C₁₋₆ alkylamino (e.g., methylamino, ethylamino), mono-C₆₋₁₄ arylamino (e.g., phenylamino, 1-naphthylamino, 2-naphthylamino), di-C₁₋₆ alkylamino (e.g., dimethylamino, diethylamino), di -C₆₋₁₄ arylamino (e.g., diphenylamino), acyl, acylamino, optionally substituted 5-to 7-membered saturated cyclic amino, 5- to 10-membered aromatic heterocyclic group (e.g., 2- or 3-thienyl, 2-, 3-or 4-pyridyl, 2-, 3-, 4-, 5- or 8-quinolyl, 1-, 3-, 4- or 5-isoquinolyl, 1-, 2- or 3-indolyl, 2-benzothiazolyl, 2-benzo[b]thienyl, benzo[b]furanyl), sulfo and the like.

Such "optionally halogenated C₁₋₆ alkyl", "optionally halogenated C₂₋₆ alkenyl", "optionally halogenated C₂₋₆. alkynyl", "optionally halogenated C₃₋₆ cycloalkyl", "optionally halogenated C₁₋₆ alkoxy", "acyl", "acylamino" and "optionally substituted 5- to 7-membered saturated cyclic amino" may for example be similar to those described as "substituents" in the "optionally substituted hydrocarbon group" represented by R¹ or R² described above.

R^{4c} is an optionally substituted aromatic group, an optionally substituted aliphatic hydrocarbon group or an acyl group.

An "aromatic group" in the "optionally substituted aromatic group" represented by R^{4c} may for example be an aromatic hydrocarbon group, aromatic heterocyclic group and the like.

Such "aromatic hydrocarbon group" may for example be a monocyclic or fused polycyclic (dicyclic or tricyclic) aromatic hydrocarbon group having 6 to 14 carbon atoms. Those exemplified typically are C₆₋₁₄ aryl groups such as phenyl, 1-naphthyl, 2-naphthyl, biphenylyl, anthryl and the like.

Such "aromatic heterocyclic group" may for example be a 5- to 14-membered, preferably 5- to 10-membered aromatic heterocyclic group containing one or more (for example 1 to 4) heteroatoms selected from nitrogen, sulfur and oxygen atoms in addition to carbon atoms. Those exemplified typically are a monovalent group formed by removing any hydrogen atom from an aromatic heterocyclic ring such as thiophene, benzothiophene, benzofuran, benzimidazole, benzoxazole, benzothiazole, benzisothiazole, naphtho[2,3-b]thiophene, furane, isoindolidine, xantholene, phenoxathiine, pyrrole, imidazole, pyrazole, pyridine, pyrazine, pyrimidine, pyridazine, indole, isoindole, 1H-indazole, purine, 4H-quinolidine, isoquinoline, quinoline, phthalazine, naphthylidine, quinoxaline, quinazoline, cinnoline, carbazole, β-carboline, phenanthridine, acridine, phenazine, thiazole, isothiazole, phenothiazine, oxazole, isoxazole, furazane or phenoxazine, or a ring formed by condensation of any of the ring listed above (preferably monocyclic ring) with one ore more (preferably 1 or 2) aromatic rings (e.g., benzene ring, etc.) and the like.

A preferred "aromatic heterocyclic group" may for example be a 5- or 6-membered aromatic heterocyclic group which may be fused with a single benzene ring. Those exemplified typically are 2-, 3- or 4-pyridyl, 2-, 3-, 4-, 5- or 8-quinolyl, 1-, 3-, 4- or 5-isoquinolyl, 1-, 2- or 3-indolyl, 2-benzothiazolyl, 2-benzo[b]thienyl, benzo[b]furanyl, 2- or 3-thienyl and the like. Those employed more preferably are 2- or 3-thienyl, 2-, 3- or 4-pyridyl, 2- or 3-quinolyl, 1-isoquinolyl, 1- or 2-indolyl, 2-benzothiazolyl and the like.

A "substituent" in the "optionally substituted aromatic group" may for example be a halogen atom (e.g., fluorine, chlorine, bromine, iodine), C₁₋₃ alkylenedioxy (e.g., methylenedioxy, ethylenedioxy), nitro, cyano, optionally halogenated C₁₋₆ alkyl, optionally halogenated C₂₋₆ alkenyl, optionally halogenated C₂₋₆ alkynyl, optionally halogenated C₃₋₆ cycloalkyl, optionally halogenated C₁₋₆ alkoxy, optionally halogenated C₁₋₆ alkylthio, hydroxy, amino, mono-C₁₋₆ alkylamino (e.g., methylamino, ethylamino, propylamino, isopropylamino, butylamino), di-C₁₋₆ alkylamino (e.g., dimethylamino, diethylamino, dipropylamino, dibutylamino, ethylmethylamino), optionally substituted 5-to 7-membered saturated cyclic amino, acyl, acylamino, acyloxy, sulfo, C₆₋₁₄ aryl (e.g., phenyl, 1-naphthyl, 2-naphthyl), C₆₋₁₄ aryloxy (e.g., phenyloxy, naphthyloxy) and the like.

Such "optionally halogenated C₁₋₆ alkyl", "optionally halogenated C₂₋₆ alkenyl", "optionally halogenated C₂₋₆ alkynyl", "optionally halogenated C₃₋₆ cycloalkyl", "optionally halogenated C₁₋₆ alkoxy", "optionally halogenated C₁₋₆ alkylthio", "optionally substituted 5- to 7-membered saturated cyclic amino", "acyl", "acylamino" and "acyloxy" may for example be similar to those described as "substituents" in an "optionally substituted hydrocarbon group" represented by R¹ or R² described above.

The "aromatic group" mentioned above may have 1 to 3 substituents listed above at any substitutable positions, and when the number of the substituents is 2 or more, then they may be the same as or different from each other.

The "optionally substituted aromatic group" mentioned above is preferably phenyl, 2-, 3- or 4-pyridyl, 2- or 3-quinolyl, 1-isoquinolyl which may be substituted by 1 to 3 substituents selected from a halogen atom, C₁₋₃ alkylenedioxy, nitro, cyano, optionally halogenated C₁₋₆ alkyl, optionally halogenated C₂₋₆ alkenyl, optionally halogenated C₂₋₆ alkynyl, optionally halogenated C₃₋₆ cycloalkyl, optionally halogenated C₁₋₆ alkoxy, optionally halogenated C₁₋₆ alkylthio, hydroxy, amino, mono-C₁₋₆ alkylamino, di-C₁₋₆ alkylamino, optionally substituted 5- to 7-membered saturated cyclic amino, acyl, acylamino, acyloxy, sulfo, C₆₋₁₄ aryl and C₆₋₁₄ aryloxy.

An "aliphatic hydrocarbon group" in the "optionally substituted aliphatic hydrocarbon group" represented by R^{4C} may for example be alkyl, alkenyl, alkynyl, cycloalkyl and the like. Those preferred especially are C₁₋₁₀ alkyl, C₂₋₁₀ alkenyl, C₂₋₁₀ alkynyl, C₃₋₁₀ cycloalky and the like.

Preferred "alkyl" may for example be C₁₋₆ alkyl (e.g., methyl, ethyl, propyl, isopropyl, butyl, isobutyl, sec-butyl, tert-butyl, pentyl, hexyl).

Preferred "alkenyl" may for example be C₂₋₆ alkenyl (e.g., vinyl, allyl, isopropenyl, butenyl, isobutenyl, sec-butenyl).

Preferred "alkynyl" may for example be C₂₋₆ alkynyl (e.g., ethynyl, propargyl, butynyl, 1-hexynyl).

Preferred "cycloalkyl" may for example be C₃₋₆ cycloalkyl (e.g., cyclopropyl, cyclobutyl, cyclopentyl, cyclohexyl).

Among those listed above, a C₁₋₆ alkyl group is preferred.

A "substituent" which the "aliphatic hydrocarbon group" may have is similar to the "substituent" in the "optionally substituted hydrocarbon group" represented by R¹ or R² described above, and the same number of such substituents is employed.

Such "substituent" may for example be acyl (e.g., carboxy, C₁₋₆ alkyl-carbonyl, C₁₋₆ alkoxy-carbonyl, C₆₋₁₄ aryl-carbonyl) and the like.

The "acyl group" represented by R^{4C} is similar to the "acyl group" as "substituent" in the "optionally substituted hydrocarbon group" represented by R¹ or R² described above.

The "optionally oxidized sulfur atom" represented by X or Y may for example be S, SO and SO₂.

A "substituent" in the "optionally substituted imino" represented by Y may for example be optionally substituted hydrocarbon group and acyl.

Such "optionally substituted hydrocarbon group" may for example be similar to the "optionally substituted hydrocarbon group" represented by R¹ or R² described above.

Such "acyl" may for example be the "acyl group" as "substituent" in the "optionally substituted hydrocarbon group" represented by R¹ or R² described above.

The "optionally substituted imino" represented by Y is preferably imino, C₁₋₆ alkylimino (e.g., methylimino, ethylimino), C₆₋₁₄ arylimino (e.g., phenylimino, 1-naphthylimino, 2-naphthylimino), C₇₋₁₆ aralkylimino (e.g., benzylimino) and the like.

Each of X and Y is preferably an oxygen atom.

As described above, a compound (I) of the present invention includes a compound (Ia) represented by Formula: 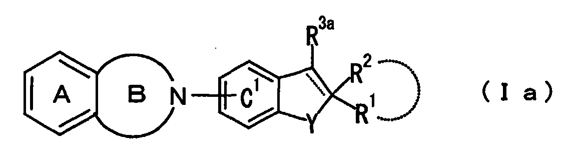 wherein each symbol is as defined above, a compound (Ib) represented by Formula: 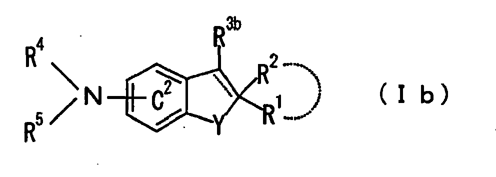 wherein each symbol is as defined above and a compound (Ic) represented by Formula: 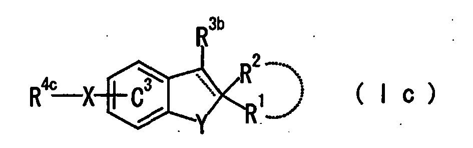 wherein each symbol is as defined above.

In the Compound (Ia) shown above, R¹ and R² are same or different and preferably each is a hydrogen atom or an optionally substituted C₁₋₆ alkyl group (especially a C₁₋₃ alkyl group such as methyl), or R¹ and R² are taken together with the adjacent carbon atom to form an optionally substituted 3- to 8-membered homocyclic or heterocyclic ring, and more preferably each of R¹ and R² is a C₁₋₆ alkyl group. When --- is a double bond, then R² is not present, and R¹ is preferably an optionally substituted C₁₋₆ alkyl group, especially a C₁₋₃ alkyl group such as methyl.

A preferred R³ may for example be an optionally substituted C₆₋₁₄ aryl group.

A preferred Ring A may for example be a benzene ring which may have 1 to 3 substituents selected from halogen, C₁₋₆ alkyl, C₁₋₆ alkoxy and C₁₋₆ alkylenedioxy.

A preferred Ring B may for example be a 5- to 7-membered nitrogen-containing heterocyclic ring which may be substituted by 1 to 2 C₁₋₆ alkyl groups.

A preferred Ring C¹ may for example be a benzene ring which may further be substituted by 1 to 3 substituents selected from C₁₋₆ alkyl and C₁₋₆ alkoxy groups.

A group represented by Formula: 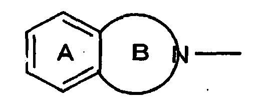 wherein each symbol is as defined above is preferably a group represented by Formulae: 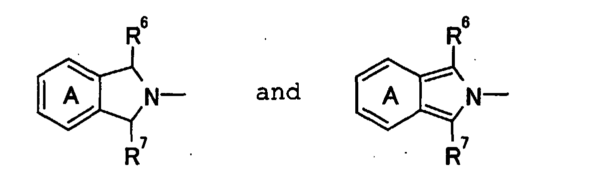 wherein each symbol is as defined above. Specifically, in the above formulae, each of R⁶ and R⁷ is preferably a hydrogen atom, and the Ring A is preferably a benzene ring which may have 1 to 3 substituents selected from halogen, C₁₋₆ alkyl, C₁₋₆ alkoxy and C₁₋₆ alkylenedioxy.

The position at which the Ring C¹ is substituted by a group represented by Formula: 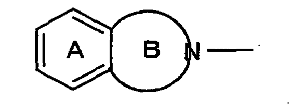 wherein each symbol is as defined in Claim 1, is preferably the 5-position on the benzofuran ring or dihydrobenzofuran ring.

In an especially preferred Compound (Ia), each of R¹ and R² is a hydrogen atom or a C₁₋₆ alkyl group (especially a C₁₋₃ alkyl group such as methyl), R^{3a} is a hydrogen atom or a phenyl group which may have 1 to 3 substituents selected from C₁₋₆ alkyl (especially a C₁₋₃ alkyl group such as methyl, ethyl, propyl, isopropyl) and halogen atoms (especially fluorine), the Ring A is a benzene ring which may have 1 to 3 substituents selected from halogen, C₁₋₆ alkyl (especially a C₁₋₃ alkyl such as methyl), C₁₋₆ alkoxy (especially, a C₁₋₃ alkoxy such as methoxy) and C₁₋₆ alkylenedioxy (especially, a C₁₋₃ alkylenedioxy such as methylenedioxy), the Ring B is a 5- to 7-membered nitrogen-containing heterocyclic ring which may be substituted by 1 or 2 C₁₋₆ alkyl groups, the Ring C¹ is a benzene ring which may further have 1 to 3 substituents selected from C₁₋₆ alkyl (especially a C₁₋₃ alkyl such as methyl) and C₁₋₆ alkoxy (especially, a C₁₋₃ alkoxy such as methoxy) groups, and Y is an oxygen atom, and in a particularly preferred compound the group represented by Formula: 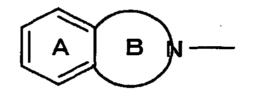 wherein each symbol is as defined above is a group represented by Formula: 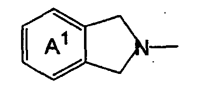 wherein Ring A¹ is a benzene ring which may have 1 to 3 substituents selected from halogen, C₁₋₆ alkoxy and C₁₋₆ alkylenedioxy.

When --- is a double bond, then R² is not present, and a preferred R¹ may for example be a C₁₋₆ alkyl group, especially a C₁₋₃ alkyl group such as methyl. While other symbols are preferably as defined above, a particularly preferred compound is a compound wherein R^{3a} is a phenyl group which may have 1 to 3 C₁₋₆ alkyl (especially C₁₋₃ alkyl such as methyl, ethyl, propyl, isopropyl) groups, the Ring A is a benzene ring which may be substituted by 1 to 3 C₁₋₆ alkoxy (especially methoxy) groups, the Ring B is a 5- to 7-membered nitrogen-containing heterocyclic ring, the Ring C¹ is a benzene ring which may further be substituted by 1 to 3 C₁₋₆ alkyl (especially C₁₋₃ alkyl such as methyl) groups (especially a benzene ring substituted by 3 C₁₋₆ alkyl groups such as methyl groups), and Y is an oxygen atom. One especially preferred is a compound wherein the group represented by Formula: 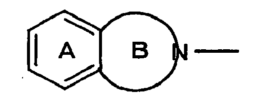 wherein each symbol is as defined above is a group represented by Formula:

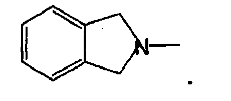

Examples of a Compound (Ia) are preferably the compounds produced in the Example 1a to Example 22a described below, among those preferred are:
[1] 2-[2,2,4,6,7-pentamethyl-3-(4-methylphenyl)-2,3-dihydro-1-benzofuran-5-yl]isoindoline (Example 4a) or a salt thereof,
[2] 5,6-dimethoxy-2-[2,2,4,6,7-pentamethyl-3-(4-methylphenyl)-2,3-dihydro-1-benzofuran-5-yl]isoindoline (Example 6a) or a salt thereof,
[3] 5,6-dimethoxy-2-[3-(4-isopropylphenyl)-2,2,4,6,7-pentamethyl-2,3-dihydro-1-benzofuran-5-yl]isoindoline (Example 11a) or a salt thereof,
[4] 6-[3-(4-isopropylphenyl)-2,2,4,6,7-pentamethyl-2,3-dihydro-1-benzofuran-5-yl]-6,7-dihydro-5H-[1,3]dioxolo[4,5-f]isoindole (Example 12a) or a salt thereof,
[5] 6-[2,2,4,6,7-pentamethyl-3-(4-methylphenyl)-2,3-dihydro-1-benzofuran-5-yl]-6H-[1,3]dioxolo[4,5-f]isoindole (Example 14a) or a salt thereof,
[6] 6-(2,2,4,6,7-pentamethyl-3-phenyl-2,3-dihydro-1-benzofuran-5-yl)-6,7-dihydro-5H-[1,3]dioxolo[4,5-f]isoindole (Example 16a) or a salt thereof,
[7] (R)-5,6-dimethoxy-2-[2,2,4,6,7-pentamethyl-3-(4-methylphenyl)-2,3-dihydro-1-benzofuran-5-yl]isoindoline (Example 17a),
[8] (R)-5,6-dimethoxy-2-[2,2,4,6,7-pentamethyl-3-(4-methylphenyl)-2,3-dihydro-1-benzofuran-5-yl]isoindoline hydrochloride (Example 19a),
[9] 5,6-dimethoxy-2-[2,2,4,6,7-pentamethyl-3-(4-methylphenyl)-2,3-dihydro-1-benzofuran-5-yl]-2H-isoindole (Example 23a) or a salt thereof, among which those preferred especially are:
   [1] 5,6-dimethoxy-2-[2,2,4,6,7-pentamethyl-3-(4-methylphenyl)-2,3-dihydro-1-benzofuran-5-yl]isoindoline (Example 6a),
   [2] 6-[3-(4-isopropylphenyl)-2,2,4,6,7-pentamethyl-2,3-dihydro-1-benzofuran-5-yl]-6,7-dihydro-5H-[1,3]dioxolo[4,5-f]isoindole (Example 12a),
   [3] (R)-5,6-dimethoxy-2-[2,2,4,6,7-pentamethyl-3-(4-methylphenyl)-2,3-dihydro-1-enzofuran-5-yl]isoindoline (Example 17a),
   [4] (R)-5,6-dimethoxy-2-[2,2,4,6,7-pentamethyl-3-(4-methylphenyl)-2,3-dihydro-1-benzofuran-5-yl]isoindoline hydrochloride (Example 19a),
   [5] 5,6-dimethoxy-2-[2,2,4,6,7-pentamethyl-3-(4-methylphenyl)-2,3-dihydro-1-benzofuran-5-yl]-2H-isoindole (Example 23a).

In the Compound (Ib) described above, preferably R¹ and R² are same or different and each is a hydrogen atom or an optionally substituted C₁₋₆ alkyl group (especially C₁₋₃ alkyl group such as methyl) or R¹ and R² are taken together with the adjacent carbon atom to form an optionally substituted 3- to 8-membered homocyclic ring (a C₃₋₈ cycloalkane such as cyclopropane, cyclobutane, cyclopentane, cyclohexane), and more preferably R¹ and R² are same or different and each is a hydrogen atom or a C₁₋₆ alkyl group (especially C₁₋₃ alkyl group such as methyl) or R¹ and R² are taken together with the adjacent carbon atoms to form a 3-to 8-membered homocyclic ring. Among those, each of R¹ and R² is preferably a C₁₋₆ alkyl group, especially methyl.

A preferred R^{3b} may for example be a phenyl group which may have 1 to 3 substituents selected from halogen (especially, fluorine) and C₁₋₆ alkyl (especially C₁₋₃ alkyl such as methyl, ethyl, propyl, isopropyl), and more preferred one is a phenyl group which may be substituted by fluorine, methyl or isopropyl.

A preferred R⁴ may for example be (1) a C₁₋₆ alkyl group substituted by an aromatic group (especially, a C₆₋₁₄ aryl group such as phenyl or a 5- or 6-membered aromatic heterocyclic group containing 1 to 3 heteroatoms selected from nitrogen, oxygen, sulfur and the like in addition to carbon atoms such as thienyl and pyridyl) which may have 1 to 3 substituents selected from halogen, C₁₋₆ alkoxy and C₁₋₃ alkylenedioxy, or (2) an acyl group containing an aromatic group (especially, a C₆₋₁₄ aryl group such as phenyl) which may have 1 to 3 substituents selected from halogen, C₁₋₆ alkoxy and C₁₋₃ alkylenedioxy, and more preferably (1) a C₁₋₆ alkyl group (especially C₁₋₃ alkyl such as methyl) substituted by a C₆₋₁₄ aryl group (especially, phenyl), thienyl or pyridyl which may have 1 to 3 substituents selected from halogen (especially, fluorine, chlorine), C₁₋₆ alkoxy (especially C₁₋₃ alkoxy such as methoxy) and C₁₋₃ alkylenedioxy (especially, methylenedioxy) or (2) a C₆₋₁₄ aryl-carbonyl group (especially, phenylcarbonyl group), C₇₋₁₆ aralkyl-carbonyl group (especially, benzylcarbonyl group), C₆₋₁₄ aryl-sulfonyl group (especially, phenylsulfonyl group), nicotinoyl group or thenoyl group which may have 1 to 3 substituents selected from halogen (especially, fluorine, chlorine), C₁₋₆ alkoxy (especially C₁₋₃ alkoxy such as methoxy) and C₁₋₃ alkylenedioxy (especially, methylenedioxy). One preferred especially is a benzyl group or a phenethyl group which may have 1 to 3 substituents selected from fluorine, methoxy and methylenedioxy.

A preferred R⁵ may for example be a hydrogen atom, a C₁₋₆ alkyl group (especially C₁₋₃ alkyl such as methyl) or a C₁₋₆ alkyl-carbonyl group (especially C₁₋₃ alkyl-carbonyl group such as acetyl), more preferably it is a hydrogen atom or a methyl group.

A preferred Ring C² may for example be a benzene ring which may be further substituted by 1 to 3 C₁₋₆ alkyl (especially C₁₋₃ alkyl such as methyl) groups, more preferably it is a benzene ring substituted further by 3 methyl groups.

In an especially preferred Compound (Ib), R¹ and R² are same or different and each is a hydrogen atom or a C₁₋₆ alkyl group (especially C₁₋₃ alkyl group such as methyl) or R¹ and R² are taken together with the adjacent carbon atom to form a 3- to 8-membered homocyclic ring;
R^{3b} is a phenyl group which may have 1 to 3 substituents selected from halogen (especially, fluorine) and C₁₋₆ alkyl (especially C₁₋₃ alkyl such as methyl, ethyl, propyl, isopropyl);
R⁴ is (1) a C₁₋₆ alkyl group (especially C₁₋₃ alkyl such as methyl) substituted by a C₆₋₁₄ aryl group (especially, phenyl), thienyl or pyridyl which may have 1 to 3 substituents selected from halogen (especially, fluorine, chlorine), C₁₋₆ alkoxy (especially C₁₋₃ alkoxy such as methoxy) and C₁₋₃ alkylenedioxy (especially, methylenedioxy) or (2) a C₆₋₁₄ aryl-carbonyl group (especially, phenylcarbonyl group), C₇₋₁₆ aralkyl-carbonyl group (especially, benzylcarbonyl group), C₆₋₁₄ aryl-sulfonyl group (especially, phenylsulfonyl group), nicotinoyl group or thenoyl group which may have 1 to 3 substituents selected from halogen (especially, fluorine, chlorine), C₁₋₆ alkoxy (especially C₁₋₃ alkoxy such as methoxy) and C₁₋₃ alkylenedioxy (especially, methylenedioxy);
R⁵ is a hydrogen atom, a C₁₋₆ alkyl group (especially C₁₋₃ alkyl such as methyl) or a C₁₋₆ alkyl-carbonyl group (especially C₁₋₃ alkyl-carbonyl group such as acetyl);
Y is an oxygen atom; and,
the ring C² is a benzene ring further substituted by 1 to 3 C₁₋₆ alkyl (especially C₁₋₃ alkyl such as methyl) groups, and in a further preferred Compound,
each of R¹ and R² is a methyl group;
R^{3b} is a phenyl group optionally substituted by fluorine, methyl or isopropyl;
R⁴ is a benzyl group or a phenethyl group optionally substituted by fluorine, methoxy or methylenedioxy;
R⁵ is a hydrogen atom or a methyl group;
--- is a single bond;
Y is an oxygen atom; and,
the Ring C² is a benzene ring further substituted by 3 methyl groups.

When --- is a double bond, then R² is not present, and R¹ is preferably a C₁₋₆ alkyl group or the like, especially a C₁₋₃ alkyl group such as methyl. While other symbols are preferably as defined above, a particularly preferred compound is a compound wherein R^{3b} is a phenyl group which may have 1 to 3 substituents selected from halogen (especially, fluorine) and C₁₋₆ alkyl (especially C₁₋₃ alkyl such as methyl, ethyl, propyl, isopropyl); R⁴ is (1) a C₁₋₆ alkyl group (especially C₁₋₃ alkyl such as methyl) substituted by a C₆₋₁₄ aryl group (especially, phenyl) which may have 1 to 3 substituents selected from halogen (especially, fluorine) and C₁₋₆ alkoxy (especially C₁₋₃ alkoxy such as methoxy) or (2) a C₆₋₁₄ aryl-carbonyl group (especially, phenylcarbonyl group) or a C₇₋₁₆ aralkyl-carbonyl group (especially, benzylcarbonyl group) which may have 1 to 3 substituents selected from halogen (especially, fluorine) and C₁₋₆ alkoxy (especially C₁₋₃ alkoxy such as methoxy); R⁵ is a hydrogen atom; Y is an oxygen atom; and the Ring C² is a benzene ring substituted further by 1 to 3 C₁₋₆ alkyl (especially C₁₋₃ alkyl such as methyl) groups (especially a benzene ring substituted by 3 C₁₋₃ alkyl groups such as methyl).

Examples of a Compound (Ib) are preferably the compounds produced in the Example 1b to Example 67b described below, among which those preferred are:
(1) N-(4-fluorobenzyl)-2,2,4,6,7-pentamethyl-3-phenyl-2,3-dihydro-1-benzofuran-5-amine (Example 4b) or a salt thereof,
(2) N-benzyl-3-(4-isopropylphenyl)-2,2,4,6,7-pentamethyl-2,3-dihydro-1-benzofuran-5-amine (Example 6b) or a salt thereof,
(3) 3-(4-isopropylphenyl)-N-(4-methoxybenzyl)-N,2,2,4,6,7-hexamethyl-2,3-dihydro-1-benzofuran-5-amine (Example 9b) or a salt thereof,
(4) 3-(4-isbpropylphenyl)-N-[2-(4-methoxyphenyl)ethyl]-2,2,4,6,7-pentamethyl-2,3-dihydro-1-benzofuran-5-amine (Example 11b) or a salt thereof,
(5) N-(4-fluorobenzyl)-3-(4-isopropylphenyl)-2,2,4,6,7-pentamethyl-2,3-dihydro-1-benzofuran-5-amine (Example 19b) or a salt thereof,
(6) N-(1,3-benzodioxol-5-ylmethyl)-3-(4-isopropylphenyl)-2,2,4,6,7-pentamethyl-2,3-dihydro-1-benzofuran-5-amine (Example 23b) or a salt thereof,
(7) N-(4-fluorobenzyl)-3-(4-fluorophenyl)-2,2,4,6,7-pentamethyl-2,3-dihydro-1-benzofuran-5-amine (Example 31b) or a salt thereof,
(8) N-(4-methoxybenzyl)-2,2,4,6,7-pentamethyl-3-(4-methylphenyl)-2,3-dihydro-1-benzofuran-5-amine (Example 33b) or a salt thereof,
(9) N-(4-fluorobenzyl)-2,2,4,6,7-pentamethyl-3-(4-methylphenyl)-2,3-dihydro-1-benzofuran-5-amine (Example 35b) or a salt thereof,
(10) 3-(4-isopropylphenyl)-N-(4-methoxybenzyl)-2,4,6,7-tetramethyl-1-benzofuran-5-amine (Example 45b) or a salt thereof,
(11) N-(4-fluorobenzyl)-3-(4-isopropylphenyl)-2,4,6,7-tetramethyl-1-benzofuran-5-amine (Example 47b) or a salt thereof,
(12) N-(4-fluorobenzyl)-3-(4-fluorophenyl)-2,4,6,7-tetramethyl-1-benzofuran-5-amine (Example 51b) or a salt thereof,
(13) N-(4-fluorobenzyl)-3-(4-isopropylphenyl)-1',4,6,7-tetramethylspiro[benzofuran-2(3H),4'-pyperidine]-5-amine (Example 55b) or a salt thereof,
(14) (R)-N-(4-fluorobenzyl)-3-(4-isopropylphenyl)-2,2,4,6,7-pentamethyl-2,3-dihydro-1-benzofuran-5-amine, hydrochloride (Example 61b) or other salts thereof, and among those preferred especially are:
   [1] N-(4-fluorobenzyl)-3-(4-isopropylphenyl)-2,2,4,6,7-pentamethyl-2,3-dihydro-1-benzofuran-5-amine (Example 19b),
   [2] N-(4-fluorobenzyl)-3-(4-isopropylphenyl)-1',4,6,7-tetramethylspiro[benzofuran-2(3H),4'-pyperidine]-5-amine (Example 55b),
   [3] (R)-N-(4-fluorobenzyl)-3-(4-isopropylphenyl)-2,2,4,6,7-pentamethyl-2,3-dihydro-1-benzofuran-5-amine hydrochloride (Example 61b) and the like.
A group represented by Formula:-X-R^{4c} preferably substituts the 5-position on the backbone structure as shown below.

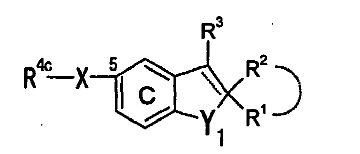

In a preferred Compound (Ic), each of R¹ and R² is C₁₋₆ alkyl which may have 1 to 3 substituents selected from (1) C₆₋₁₄ aryl, (2) C₁₋₆ alkoxy, (3) C₁₋₆ alkylhtio, (4) hydroxy, (5) amino, (6) mono-C₁₋₆ alkylamino, (7) mono-C₆₋₁₄ arylamino, (8) di-C₁₋₆ alkylamino, (9) di-C₆₋₁₄ arylamino, (10) carboxy, (11) C₁₋₆ alkylsulfonyl, (12) C₆₋₁₄ arylsulfonyl, (13) C₁₋₆ alkylsulfinyl, (14) C₆₋₁₄ arylsulfinyl and (15) 5- to 7-membered saturated cyclic amino which may have 1 to 3 substituents selected from C₁₋₆ alkyl, C₆₋₁₄ aryl and 5- to 10-membered aromatic group, or,
R¹ and R² are taken together with the adjacent carbon atom to form a 3- to 8-membered homocyclic or heterocyclic ring which may have 1 to 3 substituents selected from C₁₋₆ alkyl, C₆₋₁₄ aryl, C₇₋₁₆ aralkyl and 5-to 10-membered aromatic heterocyclic group;
R³ is phenyl, 1-naphthyl, 2-naphthyl, 2-thienyl, 3-thienyl, 2-pyridyl, 3-pyridyl, 4-pyridyl, 2-quinolyl, 3-quinolyl, 1-isoquinolyl, 1-indolyl, 2-indolyl or 2-benzothiazolyl which may have 1 to 3 substituents selected from (1) halogen atom, (2) C₁₋₆ alkyl, (3) C₁₋₆ alkoxy, (4) amino, (5) mono-C₁₋₆ alkylamino, (6) di-C₁₋₆ alkylamino and (7) 5- to 7-membered saturated cyclic amino which may have 1 to 3 substituents selected from C₁₋₆ alkyl, C₆₋₁₄ aryl and 5- to 10-membered aromatic group;
R^{4c} is (i) C₁₋₆ alkyl which has a phenyl, 1-naphthyl, 2-naphthyl, 2-thienyl, 3-thienyl, 2-pyridyl, 3-pyridyl, 4-pyridyl, 2-quinolyl, 3-quinolyl, 1-isoquinolyl, 1-indolyl, 2-indolyl or 2-benzothiazolyl which may have 1 to 3 substituents selected from (1) halogen atom, (2) C₁₋₆ alkyl, (3) C₁₋₆ alkoxy, (4) hydroxy, (5) amino, (6) mono-C₁₋₆ alkylamino, (7) di-C₁₋₆ alkylamino, (8) carboxy and (9) 5-to 7-membered saturated cyclic amino which may have 1 to 3 substituents selected from C₁₋₆ alkyl, C₆₋₁₄ aryl and 5- to 10-membered aromatic group and which may further have carboxy or C₁₋₆ alkoxy-carbonyl; or,
(ii) C₁₋₆ alkyl-carbonyl, C₃₋₆ cycloalkyl-carbonyl, C₆₋₁₄ aryl-carbonyl or C₇₋₁₆ aralkyl-carbonyl which may have 1 to 3 substituents selected from (1) halogen atom, (2) C₁₋₆ alkyl, (3) C₁₋₆ alkoxy, (4) hydroxy, (5) amino, (6) mono-C₁₋₆ alkylamino, (7) di-C₁₋₆ alkylamino and (8) carboxy;
X is an oxygen atom;
Y is an oxygen atom;
the Ring C³ is a benzene ring which may have 1 to 3 substituents selected from a halogen atom, optionally halogenated C₁₋₆ alkyl, optionally halogenated C₁₋₆ alkoxy, amino, mono-C₁₋₆ alkylamino and di-C₁₋₆ alkylamino.

In a more preferred compound, each of R¹ and R² is a C₁₋₆ alkyl group which may have 1 to 3 substituents selected from C₆₋₁₄ aryl, C₁₋₆ alkoxy, C₁₋₆ alkylthio, hydroxy, amino, mono-C₁₋₆ alkylamino, mono-C₆₋₁₄ aryl amino, di-C₁₋₆ alkylamino, di-C₆₋₁₄ arylamino, carboxy, C₁₋₆ alkylsulfonyl, C₆₋₁₄ arylsulfonyl, C₁₋₆ alkylsulfinyl and C₆₋₁₄ arylsulfinyl, or,
R¹ and R² are taken together with the adjacent carbon atom to form a piperidine which may have 1 to 3 substituents selected from C₁₋₆ alkyl, C₆₋₁₄ aryl and C₇₋₁₆ aralkyl;
R³ is phenyl which may have 1 to 3 substituents selected from C₁₋₆ alkyl, C₁₋₆ alkoxy, amino, mono-C₁₋₆ alkylamino and di-C₁₋₆ alkylamino;
R⁴ is (i) C₁₋₆ alkyl having a phenyl or pyridyl which may have 1 to 3 substituents selected from a halogen atom, C₁₋₆ alkyl, C₁₋₆ alkoxy, hydroxy, amino, mono-C₁₋₆ alkylamino, di-C₁₋₆ alkylamino and carboxy, or,
(ii) acyl represented by Formula: -(C=O)-R^{5'} wherein R^{5'} is phenyl or phenyl-C₁₋₆ alkyl which may have 1 to 3 substituents selected from a halogen atom, C₁₋₆ alkyl, C₁₋₆ alkoxy, hydroxy, amino, mono-C₁₋₆ alkylamino, di-C₁₋₆ alkylamino and carboxy;
X is an oxygen atom;
Y is an oxygen atom;
the Ring C³ is a benzene ring which may have 1 to 3 substituents selected from a halogen atom, optionally halogenated C₁₋₆ alkyl, optionally halogenated C₁₋₆ alkoxy, amino, mono-C₁₋₆ alkylamino and di-C₁₋₆ alkylamino.

One also preferred is a compound represented by Formula: 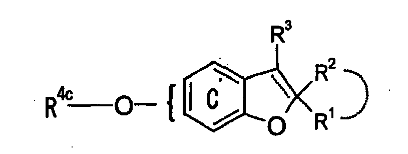 wherein each of R¹ and R² is C₁₋₆ alkyl which may have 6-membered saturated cyclic amino substituted by phenyl, or R¹ and R² are taken together with the adjacent carbon atom to form a pyperidine substituted by C₁₋₆ alkyl or C₇₋₁₆ aralkyl;
R³ is (i) a hydrogen atom, or,
(ii) phenyl which may have 1 to 3 substituents selected from (1) C₁₋₆ alkyl, (2) di-C₁₋₆ alkylamino and (3) 6-membered saturated cyclic amino which may have C₁₋₆ alkyl; R^{4c} is (i) phenyl which may have 1 to 3 substituents selected from nitro and C₁₋₆ alkyl-carboxamide,
(ii) C₁₋₆ alkyl or C₂₋₆ alkenyl having 1 to 3 phenyl, quinolyl or pyridyl which may have 1 to 3 substituents selected from C₁₋₆ alkoxy, C₁₋₆ alkylthio, C₁₋₆ alkoxy-carbonyl, C₁₋₆ alkylsulfonyl and C₁₋₆ alkylsulfinyl and optionally further having phenyl, carboxy or C₁₋₆ alkoxy-carbonyl as additional substituents, or,
(iii) acyl represented by Formula: -(C=O)-R^{5"} wherein R^{5"} is C₁₋₆ alkoxy-substituted phenyl; and,
the Ring C' is a benzene ring which may further have 1 to 3 C₁₋₆ alkyl (especially, a benzene ring substituted by 3 C₁₋₆ alkyl groups such as methyl).

Examples of a Compound (Ic) are preferably the compounds produced in the Example 1c to 33c described below, among those preferred are:
3-(4-isopropylphenyl)-5-(4-methoxybenzyloxy)-2,2,4,6,7-pentamethyl-2,3-dihydrobenzofuran, 3-(4-methylphenyl)-2,4,6,7-tetramethylbenzofuran-5-yl 4-methoxybenzoate,
3-(4-isopropylphenyl)-2,4,6,7-tetramethylbenzofuran-5-yl 4-methoxybenzoate,
3-(4-isopropylphenyl)-5-(4-methoxybenzyloxy)-2,4,6,7-tetramethylbenzofuran,
3-(4-isopropylphenyl)-5-(4-methoxybenzyloxy)-1',4,6,7-tetramethylspiro[benzofuran-2(3H),4'-piperidine],
3-(4-isopropylphenyl)-5-(3-pyridylmethyl)-2,2,4,6,7-pentamethyl-2,3-dihydrobenzofuran and their salts.

Among those listed above, the preferred compounds are:
3-(4-isopropylphenyl)-5-(4-methoxybenzyloxy)-2,2,4,6,7-pentamethyl-2,3-dihydrobenzofuran, 3-(4-methylphenyl)-2,4,6,7-tetramethylbenzofuran-5-yl 4-methoxybenzoate,
3-(4-isopropylphenyl)-2,4,6,7-tetramehylbenzofuran-5-yl 4-methoxybenzoate,
3-(4-isopropylphenyl)-5-(4-methoxybenzyloxy)-2,4,6,7-tetramethylbenzofuran,
3-(4-isopropylphenyl)-5-(4-methoxybenzyloxy)-1',4,6,7-tetramethylspiro[benzofuran-2(3H),4'-piperidine] and their salts.

A salt of a compound described above having an acidic group such as -COOH may for example be a metal salt, ammonium salt and a salt with an organic base, while one having a basic group such as -NH₂ may for example be a salt with an inorganic acid, organic acid, basic or acidic amino acid and the like as well as an intramolecular salts. A preferred metal salt may for example be an alkaline metal salt such as sodium and potassium salts; an alkaline earth metal salt such as calcium salt, magnesium salt and barium salt; as well as aluminum salt. A preferred salt with an organic base may for example be a salt with trimethylamine, triethylamine, pyridine, picoline, ethanolamine, diethanolamine, triethanolamine, dicyclohexylamine or N,N'-dibenzylethylenediamine. A preferred salt with an inorganic acid may for example be a salt with hydrochloric acid, hydrobromic acid, nitric acid, sulfuric acid, phosphoric acid and the like. A preferred salt with an organic acid may for example be a salt with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like. A preferred salt with a basic amino acid may for example be a salt with arginine, lysine or ornithine. A preferred salt with acidic amino acid may for example be a salt with aspartic acid or glutamic acid.

Among those listed above, pharmaceutically acceptable salts are preferred. For example, a compound having an acidic functional group therein is presented as an inorganic salt such as an alkaline metal salt (e.g., sodium salt, potassium salt), alkaline earth metal salt (e.g., calcium salt, magnesium salt, barium salt) as well as ammonium salt, while one having a basic functional group therein is presented as an inorganic salt such as hydrochloride, sulfate, phosphate and hydrobromide or an organic salt such as acetate, maleate, fumarate, succinate, methanesulfonate, p-toluenesulfonate, citrate, tartarate and the like.

A Compound (I) (including Compound (Ia), (Ib) and (Ic)) can be produced by a method known per se, such as those described for example in WO98/55454, WO00/36262, WO95/29907, JP-A-5-194466, USP4,881,967, USP4,212,865 and Tetrahedron Letters, Vol.37, No.51, page 9183-9186 (1996) or analogous methods.

A prodrug of a Compound (I) may be a compound which is converted into a Compound (I) by a reaction with an enzyme or gastric acid or the like under an in vivo physiological condition, that is a compound undergoing an enzymatic oxidation, reduction or hydrolysis to form the Compound (I) and a compound being hydrolyzed by gastric acid or the like to form the Compound (I).

A prodrug for a Compound (I) may for example be a compound obtained by subjecting an amino group of the Compound (I) to acylation, alkylation or phosphorylation (e.g., a compound obtained by subjecting an amino group of the Compound (I) to eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation and tert-butylation); a compound obtained by subjecting a hydroxyl group of the Compound (I) to acylation, alkylation, phosphorylation or boration (e.g., a compound obtained by subjecting a hydroxyl group of the Compound (I) to acetylation, palmitoylation, propanoylation, pivaloylation, succinylation, fumarylation, alanylation, or dimethylaminomethylcarbonylation); a compound obtained by subjecting a carboxyl group of a compound, which is obtained by esterifying or amidating the carboxyl group of the Compound (I), to ethylesterification, phenylesterification, carboxymethylesterification, dimethylaminoesterification, pivaloyloxymethylesterification, ethoxycarbonyloxyethylesterification, phthalidylesterification, (5-methyl-2-oxo-1,3-dioxolen-4-yl)methylesterification, cyclohexyloxycarbonylethylesterification and methylamidation) and the like. Any of these compounds can be produced from a Compound (I) by a method known per se.

A prodrug for a Compound (I) may also be one which is converted into the Compound (I) under a physiological condition, such as those described in "IYAKUHIN no KAIHATSU (Development of Pharmaceuticals)", Vol.7, Design of Molecules, p.163-198, Published by HIROKAWA SHOTEN (1990).

A Compound (I) or a salt or prodrug thereof (hereinafter sometimes just referred to as Compound (I)) has excellent pharmaceutical effects such as neural stem cell autoreplication-promoting effect, neural progenitor cell differentiation-promoting effect, neurotrophic factor-like effect, neurotrophic factor activity-enhancing effect, neurodegeneration inhibiting effect, neuroregeneration promoting effect, antioxidative effect or β-amyloid-induced neuronal death inhibiting effect, and has a low toxicity and reduced side effects, thus exhibiting a pharmaceutical usefulness.

A Compound (I) can be given to a mammal (e.g., mouse, rat, hamster, rabbit, cat, dog, cattle, sheep, monkey, human and the like) as an agent for promoting the proliferation of a stem cell (e.g., embryonic stem cell, neural stem cell), an agent for promoting the differentiation of a neural progenitor cell, or a neurotrophic factor-like substance, a neurotrophic factor activity-enhancing agent and a neurodegeneration inhibitor, whereby inhibiting neuronal death and promoting the regeneration of a neural tissue or function via neural neogenesis and neuroaxonal development. It is useful also in preparing a neural stem cell or neurocyte (including neural progenitor cell) to be transplanted from a brain tissue of a fetus or patient and embryonic stem cell, and it also promotes the engraftment or differentiation of the neural stem cell or neurocyte after transplantation as well as the functional expression thereof.

Accordingly, an agent for promoting the proliferation and/or differentiation of a stem cell and/or neural progenitor cell comprising a Compound (I) is effective, for example, against neurodegenerative disease (e.g., Alzheimer's disease, Perkinson's disease, amyotropic lateral sclerosis (ALS), Huntington's disease, spinocerebeller degeneration and the like), psychoneural disease (e.g., schizophrenia), cranial trauma, spinal damage, cerebrovascular disorder, cerebrovascular dementia and the like, and can be used as a prophylactic and therapeutic agent against these central nervous system diseases.

A Compound (I) has a low toxicity, and can be safely given as it is or as a pharmaceutical composition prepared by mixing with a pharmaceutically acceptable carrier according to a method known per se, for example a tablet (including a sugar-coated tablet, film-coated tablet, buccal disintegration tablet and the like), powder, granule, capsule (including soft capsule), liquid, injection, suppository, sustained release formulation, plaster and the like, orally or parenterally (e.g., topically, rectally, intravenously).

The amount of a Compound (I) in a pharmaceutical composition of the present invention is about 0.01 to about 100% by weight based on the entire composition.

The dose may vary depending on the subject to be treated, the administration route and the disease to be treated. For example, a compound of the present invention as an active ingredient may be given orally to an adult with Alzheimer's disease at about 0.1 to about 20 mg/kg body weight, preferably about 0.2 to about 10 mg/kg body weight, more preferably about 0.5 to about 10 mg/kg body weight, which can be given at a divided dose once to several times a day.

In addition, a compound of the present invention may be used in combination with other active ingredients [e.g., chorine esterase inhibitor (e.g., Aricept (donepezil) and the like), β-secretase inhibitor, β-amyloid production and sedimentation inhibitor, cerebral function activator (e.g., Idebenone, Vinpocetine), Perkinson's disease agent (e.g., L-dopa, Deprenyl, Bromocriptine, Talipexole, Pramipexole, Amantadine), amyotropic lateral sclerosis agent (e.g., riluzole), neurotrophic factor and the like]. Such other active ingredients and a compound of the present invention or a salt thereof may be mixed by a method known per se to be formulated into a single pharmaceutical composition (e.g., tablet, powder, granule, capsule (including a soft capsule), liquid, injection, suppository, sustained release formulation, and the like), or they may be formulated individually and given simultaneously or sequentially to the identical subject. In addition, a pharmaceutical composition of the present invention may be used in combination with an immunossupressing agent or the like on transplantation or after transplantation of a neural stem cell or neural progenitor cell prepared from an embryonic stem cell and neural tissue.

A pharmacologically acceptable carrier employed in the production of a pharmaceutical composition of the present invention may be any of various organic and inorganic carriers customarily employed as a pharmaceutical material, such as an excipient, lubricant, binder and disintegrant for a solid dosage form; a solvent, solubilizer, suspending agent, isotonizing agent, buffering agent and soothing agent for a liquid dosage form. A conventional additive such as a preservative, antioxidant, colorant, sweetener, adsorbent, wetting agent and the like may also be employed if necessary.

An excipient may for example be lactose, sugar, D-mannitol, starch, corn starch, crystalline cellulose, light anhydrous silicic acid and the like.

A lubricant may for example be magnesium stearate, calcium stearate, talc, colloidal silica and the like.

A binder may for example be crystalline cellulose, sugar, D-mannitol, dextrin, hydroxypropyl cellulose, hydroxypropylmethyl cellulose, polyvinylpyrrolidone, starch, sucrose, gelatin, methyl cellulose, sodium carboxymethyl cellulose and the like.

A disintegrant may for example be starch, carboxymethyl cellulose, calcium carboxymethyl cellulose, sodium croscarmellose, sodium carboxymethyl starch, L-hydroxypropyl cellulose and the like.

A solvent may for example be a water for injection, alcohol, propylene glycol, macrogol, sesame oil, corn oil, olive oil and the like.

A solubilizing agent may for example be a polyethylene glycol, propylene glycol, D-mannitol, benzyl benzoate, ethanol, tris-aminomethane, cholesterol, triethanolamine, sodium carbonate, sodium citrate and the like.

A suspending agent may for example be a surfactant such as stearyltriethanolamine, sodium laurylsulfate, laurylaminopropionic acid, lecithin, benzalkonium chloride, benzethonium chloride, glycerin monostearate and the like; a hydrophilic polymer such as polyvinylalcohol, polyvinylpyrrolidone, sodium carboxymethyl cellulose, methyl cellulose, hydroxymethyl cellulose, hydroxyethyl cellulose, hydroxypropyl cellulose and the like.

An isotonizing agent may for example be glucose, D-sorbitol, sodium chloride, glycerin, D-mannitol and the like.

A buffering agent may for example be a buffer solution of a phosphate, acetate, carbonate, citrate and the like.

A soothing agent may for example be benzyl alcohol and the like.

A preservative may for example be p-hydroxybenzoates, chlorobutanol, benzyl alcohol, phenethyl alcohol, dehydroacetic acid, sorbic acid and the like.

An antioxidant may for example be a sulfite, ascorbic acid, α-tocopherol and the like.

The present invention is further illustrated in detail by the following Reference Examples, Examples, Formulation Examples, and Experimental Examples, but these examples are merely examples, which are not intended to limit the present invention and may be varied without departing from the scope of the present invention.

"Room temperature" in the following Reference Examples and Examples usually indicates about 10°C to about 35°C. Unless otherwise stated, % indicates the percent by weight.

Other symbols used in the present specification indicate the following meanings.
s: singlet
d: doublet
dd: doublet of doublets
dt: doublet of triplets
t: triplet
q: quartet
septet: septet
m: multiplet
br: broad
J: coupling constant
Hz: hertz
CDCl₃: deuterated chloroform
DMSO-d₆: deuterated dimethyl sulfoxide
¹H-NMR: proton nuclear magnetic resonance

### [Compounds (Ia)]

### Reference Example 1a

### Ethyl 3-(4-isopropylphenyl)-2-methyl-2-propenoate

To a suspension of sodium hydride (a 60% dispersion in liquid paraffin, 5.92 g, 148 mmol) in N,N-dimethylformamide (150 ml) was added at 0°C triethyl 2-phosphonopropionate (35.0 g, 148 mmol) and the resulting mixture was stirred at the same temperature for 10 minutes. To the reaction solution was added 4-isopropylbenzaldehyde (20.0 g, 135 mmol) and the resulting mixture was stirred at room temperature for 30 minutes. Water was added into the reaction solution and the product was extracted twice with ethyl acetate. The combined extracts were washed with water, dried on magnesium sulfate, and then concentrated under reduced pressure to obtain 30.1 g (96% yield) of the oily title compound.
¹H-NMR (CDCl₃) δ: 1.26 (6H, d, J = 7.0 Hz), 1.35 (3H, t, J = 7.0 Hz), 2.13 (3H, s), 2.92 (1H, septet, J = 7.0 Hz), 4.27 (2H, q, J = 7.0 Hz), 7.21-7.38 (4H, m), 7.67 (1H, s).

### Reference Example 2a

### Ethyl 2-methyl-3-(4-methylphenyl)-2-propenoate

To a suspension of sodium hydride (a 60% dispersion in liquid paraffin, 15.0 g, 375 mmol) in N,N-dimethylformamide (160 ml) was added at 0°C a solution of triethyl 2-phosphonopropionate (87.7 g, 368 mmol) in N,N-dimethylformamide (10 ml) and the resulting mixture was stirred at the same temperature for 1 hour. To the reaction solution was added 4-methylbenzaldehyde (43.3 g, 361 mmol) and the resulting mixture was stirred at room temperature for 1 hour. Water was added into the reaction solution and the product was extracted twice with ethyl acetate. The combined extracts were washed with water, dried on magnesium sulfate, and then concentrated under reduced pressure to obtain 66.7 g (91% yield) of the oily title compound.
¹H-NMR (CDCl₃) δ: 1.34 (3H, t, J = 7.0 Hz), 2.12 (3H, d, J = 1.4 Hz), 2.37 (3H, s), 4.26 (2H, q, J = 7.0 Hz), 7.19 (2H, d, J = 8.4 Hz), 7.31 (2H, d, J = 8.4 Hz), 7.66 (1H, s).

### Reference Example 3a

### Ethyl 3-(4-fluorophenyl)-2-methyl-2-propenoate

By using 4-fluorobenzaldehyde, the title compound was synthesized according to Reference Example 1a. Yield: 97%. An oily substance.
¹H-NMR (CDCl₃) δ: 1.35 (3H, t, J = 7.0 Hz), 2.10 (3H, d, J = 1.2 Hz), 4.28 (2H, q, J = 7.0 Hz), 7.08 (2H, t, J = 8.8 Hz), 7.32-7.43 (2H, m), 7.65 (1H, s).

### Reference Example 4a

### Ethyl (E)-3-(4-isopropylphenyl)-2-propenoate

To a suspension of sodium hydride (a 60% dispersion in liquid paraffin, 10.4 g, 260 mmol) in N,N-dimethylformamide (200 ml) was added at 0°C triethyl 2-phosphonoacetate (58.2 g, 236 mmol) and the resulting mixture was stirred at the same temperature for 10 minutes. To the reaction solution was added 4-isopropylbenzaldehyde (35.0 g, 260 mmol) and the resulting mixture was stirred at room temperature for 30 minutes. Water was added into the reaction solution and the product was extracted twice with ethyl acetate. The combined extracts were washed with water, dried on magnesium sulfate, and then concentrated under reduced pressure to obtain 47.5 g (92% yield) of the oily title compound.
¹H-NMR (CDCl₃) δ: 1.25 (6H, d, J = 7.0 Hz), 1.33 (3H, t, J = 7.0 Hz), 2.92 (1H, septet, J = 7.0 Hz), 4.26 (2H, q, J = 7.0 Hz), 6.40 (1H, d, J = 15.8 Hz), 7.24 (2H, d, J = 8.2 Hz), 7.46 (2H, d, J = 8.2 Hz), 7.67 (1H, d, J = 15.8 Hz).

### Reference Example 5a

### Ethyl (E)-3-(4-fluorophenyl)-2-propenoate

By using 4-fluorobenzaldehyde, the title compound was synthesized according to Reference Example 4a. Yield: 88%. An oily substance.
¹H-NMR (CDCl₃) δ: 1.34 (3H, t, J = 7.0 Hz), 4.26 (2H, q, J = 7.0 Hz), 6.31 (1H, d, J = 15.8 Hz), 7.00-7.11 (2H, m), 7.43-7.58 (2H, m), 7.67 (1H, d, J = 15.8 Hz).

### Reference Example 6a

### 3-(4-Isopropylphenyl)-2-methyl-2-propen-1-ol

To a suspension of ethyl 3-(4-isopropylphenyl)-2-methyl-2-propenoate (9.00 g, 38.7 mmol) and cerium chloride (1.00 g, 4.06 mmol) in tetrahydrofuran (50 ml) was added lithium aluminum hydride (1.47 g, 38.7 mmol) in four portions at -40°C over a period of 30 minutes and the resulting mixture was stirred at the same temperature for 30 minutes. Water was added into the reaction solution and the product was extracted twice with ethyl acetate. The combined extracts were washed with water, dried on magnesium sulfate, and then concentrated under reduced pressure. The residue was subjected to column chromatography on silica gel (hexane-ethyl acetate 8 : 1) to obtain 6.30 g (86% yield) of the oily title compound.
¹H-NMR (CDCl₃) δ: 1.25 (6H, d, J = 7.0 Hz), 1. 91 (3H, d, J = 1.4 Hz), 2.90 (1H, septet, J = 7.0 Hz), 4.17 (2H, d, J = 0.8 Hz), 6.49 (1H, dd, J = 2.6, 1.4 Hz), 7.15-7.25 (4H, m), 1H unidentified.

### Reference Example 7a

### 2-Methyl-3-(4-methylphenyl)-2-propen-1-ol

To a suspension of ethyl 2-methyl-3-(4-methylphenyl)-2-propenoate (26.31 g, 128.8 mmol) and cerium chloride (10.32 g, 41.89 mmol) in tetrahydrofuran (120 ml) was added lithium aluminum hydride (4.89 g, 129 mmol) in four portions at -40°C over a period of 30 minutes and the resulting mixture was stirred at the same temperature for 30 minutes. Water was added into the reaction solution and the product was extracted twice with ethyl acetate. The combined extracts were washed with water, dried on magnesium sulfate, and then concentrated under reduced pressure to obtain 8.87 g (42% yield) of the oily title compound.
¹H-NMR (CDCl₃) δ: 1.87 (3H, s), 2.32 (3H, s), 4.13 (2H,s), 6.46 (1H, s), 7.08-7.22 (4H, m), 1H unidentified.

### Reference Example 8a

### 3-(4-Fluorophenyl)-2-methyl-2-propen-1-ol

By using ethyl 3-(4-fluorophenyl)-2-methyl-2-propenoate, the title compound was synthesized according to Reference Example 6a. Yield: 95%. An oily substance.
¹H-NMR (CDCl₃) δ: 1.98 (3H, d, J = 1.6 Hz), 4.11 (2H, s), 6.58 (1H, s), 7.01 (2H, t, J = 8.8 Hz), 7.18-7.28 (2H, m), 1H unidentified.

### Reference Example 9a

### (E)-3-(4-Isopropylphenyl)-2-propen-1-ol

By using ethyl (E)-3-(4-isopropylphenyl)-2-propenoate, the title compound was synthesized according to Reference Example 6a. Yield: 65%. An oily substance.
¹H-NMR (CDCl₃) δ: 1.24 (6H, d, J = 7.0 Hz), 2.79-3.00 (2H, m), 4.30 (2H, d, J = 5.6 Hz), 6.35 (1H, dt, J = 15.8, 5.6 Hz), 6.59 (1H, d, J = 15.8 Hz), 7.10-7.39 (4H, m).

### Reference Example 10a

### (E)-3-(4-Fluorophenyl)-2-propen-1-ol

By using ethyl (E)-3-(4-fluorophenyl)-2-propenoate, the title compound was synthesized according to Reference Example 6a. Yield: 84%. An oily substance.
¹H-NMR (CDCl₃) δ: 4.31 (2H, d, J = 5.6 Hz), 6.28 (1H, dt, J = 15.8, 5.6 Hz), 6.59 (1H, d, J = 15.8 Hz), 6.90-7.40 (4H, m), 1H unidentified.

### Reference Example 11a

### 1-(3-Bromo-2-methyl-1-propenyl)-4-isopropylbenzene

To a solution of 3-(4-isopropylphenyl)-2-methyl-2-propen-1-ol (6.30 g, 33.1 mmol) in isopropyl ether (50 ml) was added phosphorus tribromide (5.98 g, 22.1 mmol) under ice cooling and the resulting mixture was stirred at room temperature for 30 minutes. Water was added into the reaction solution and the product was extracted with isopropyl ether. The organic layer was washed with water and an aqueous saturated solution of sodium hydrogen carbonate, dried on magnesium sulfate, filtered, and then concentrated under reduced pressure to obtain 7.63 g (91% yield) of the oily title compound.
¹H-NMR (CDCl₃) δ: 1.25 (6H, d, J = 7.0 Hz), 2.03 (3H, d, J = 1.4 Hz), 2.90 (1H, septet, J = 7.0 Hz), 4.15 (2H, d, J = 0.8 Hz), 6,62 (1H, s), 7.14-7.26 (4H, m).

### Reference Example 12a

### 1-(3-Bromo-2-methyl-1-propenyl)benzene

By using 2-methyl-3-phenyl-2-propen-1-ol, the title compound was synthesized according to Reference Example 11a. Yield: 89%. An oily substance.
¹H-NMR (CDCl₃) δ: 2.01 (3H, d, J = 1.4 Hz), 4.13 (2H, d, J = 0.8 Hz), 6.64 (1H, s), 7.19-7.44 (5H, m).

### Reference Example 13a

### 1-(3-Bromo-2-methyl-1-propenyl)-4-methylbenzene

To a solution of 2-methyl-3-(4-methylphenyl)-2-propen-1-ol (11.40 g, 70.27 mmol) in isopropyl ether (100 ml) was added phosphorus tribromide (12.83 g, 47.38 mmol) under ice cooling and the resulting mixture was stirred at room temperature for 30 minutes. Water was added into the reaction solution and the product was extracted with isopropyl ether. The organic layer was washed with water and an aqueous saturated solution of sodium hydrogen carbonate, dried on magnesium sulfate, filtered, and then concentrated under reduced pressure to obtain 12.71 g (80% yield) of the oily title compound.
¹H-NMR (CDCl₃) δ: 2.01 (3H, s), 2.34 (3H, s), 4.13(2H, s), 6,60 (1H, s), 7.09-7.22 (4H, m).

### Reference Example 14a

### 1-(3-Bromo-2-methyl-1-propenyl)-4-fluorobenzene

By using 3-(4-fluorophenyl)-2-methyl-2-propen-1-ol, the title compound was synthesized according to Reference Example 11a. Yield: 79%. An oily substance.
¹H-NMR (CDCl₃) δ: 1.87 (3H, s), 4.17 (2H, s), 6. 48 (1H, s), 7.01 (2H, t, J = 8.8 Hz), 7.18-7.27 (2H, m).

### Reference Example 15a

### 1-[(E)-3-Bromo-1-propenyl]-4-isopropylbenzene

By using (E)-3-(4-isopropylphenyl)-2-propen-1-ol, the title compound was synthesized according to Reference Example 11a. Yield: 72%. An oily substance.
¹H-NMR (CDCl₃) δ: 1.24 (6H, d, J = 7.0 Hz), 2.89 (1H, septet, J = 7.0 Hz), 4.16 (2H, dd, J = 7.8, 0.8 Hz), 6.35 (1H, dt, J = 15.4, 7.8 Hz), 6.63 (1H, d, J = 15.4 Hz), 7.14-7.35 (4H, m).

### Reference Example 16a

### 1-[(E)-3-Bromo-1-propenyl]-4-fluorobenzene

By using (E)-3-(4-fluorophenyl)-2-propen-1-ol, the title compounds was synthesized according to Reference Example 11a. Yield: 61%. An oily substance.
¹H-NMR (CDCl₃) δ: 4.15 (2H, d, J = 7.6 Hz), 6.30 (1H, dt, J = 15.4, 7.6 Hz), 6.61 (1H, d, J = 15.4 Hz), 6.83-7.08 (2H, m), 7.31-7.45 (2H, m).

### Reference Example 17a

### N-[4-[[3-(4-Isopropylphenyl)-2-methyl-2-propenyl]oxy]-2,3,6-trimethylphenyl]formamide

To a solution of N-(4-hydroxy-2,3,6-trimethylphenyl)formamide (3.00 g, 16.7 mmol) in N,N-dimethylformamide (30 ml) was added sodium hydride (a 60% dispersion in liquid paraffin, 0.74 g, 18.4 mmol) at 0°C under a nitrogen atmosphere and the resulting mixture was stirred at the same temperature for 10 minutes. To the reaction solution was added 1-(3-bromo-2-methyl-1-propenyl)-4-isopropylbenzene (4.66 g, 18.4 mmol) and the resulting mixture was stirred at room temperature for 30 minutes. Water was added into the reaction solution and the product was extracted twice with ethyl acetate. The combined extracts were washed with water, dried on magnesium sulfate, and then concentrated under reduced pressure. The resulting residue was crystallized from ethyl acetate-hexane to obtain 3.70 g (63% yield) of the title compound. Melting point: 153-155°C.
¹H-NMR (CDCl₃) δ: 1.26 (6H, d, J = 7.0 Hz), 2.00 (3H, s), 2.07-2.34 (9H, m), 2.91 (1H, septet, J = 7.0 Hz), 4.54 (2H, d, J = 5.4 Hz), 6.59-6.84 (3H, m), 7.17-7.36 (4H, m), 7.98 (0.5H, d, J = 12.0 Hz), 8.41 (0.5H, s).

### Reference Example 18a

### N-[2,3,6-Trimethyl-4-[(2-methyl-3-phenyl-2-propenyl)oxy]phenyl]formamide

By using N-(4-hydroxy-2,3,6-trimethylphenyl)formamide and 1-(3-bromo-2-methyl-1-propenyl)benzene, the title compound was synthesized according to Reference Example 17a. Yield: 41%. Melting point: 152-154°C. (Ethyl acetate-hexane)
¹H-NMR (CDCl₃) δ: 1.98 (3H, d, J = 1.6 Hz), 2.10-2.32 (9H, m), 4.54 (2H, d, J = 5.2 Hz), 6.65 (1H, s), 6.67 (1H, s), 6.69-6.90 (1H, m), 7.11-7.41 (5H, m), 7.98 (0.5H, d, J = 12.0 Hz), 8.41 (0.5H, d, J = 1.4 Hz).

### Reference Example 19a

### N-[2,3,6-Trimethyl-4-[[2-methyl-3-(4-methylphenyl)-2-propenyl]oxy]phenyl]formamide

To a solution of N-(4-hydroxy-2,3,6-trimethylphenyl)formamide (9.31 g, 52.0 mmol) in N,N-dimethylformamide (120 ml) was added sodium hydride (a 60% dispersion in liquid paraffin, 2.11 g, 52.8 mmol) at 0°C under a nitrogen atmosphere and the resulting mixture was stirred at the same temperature for 10 minutes. To the reaction solution was added a solution of 1-(3-bromo-2-methyl-1-propenyl)-4-methylbenzene (12.48 g, 55.44 mmol) in N,N-dimethylformamide (20 ml) and the resulting mixture was stirred at room temperature for 30 minutes. Water was added into the reaction solution and the product was extracted twice with ethyl acetate. The combined extracts were washed with water, dried on magnesium sulfate, and then concentrated under reduced pressure. The resulting residue was crystallized from ethyl acetate-isopropyl ether to obtain 7.34 g (44% yield) of the title compound. Melting point: 167-169°C.
¹H-NMR (CDCl₃) δ: 1.98 (3H, s), 2.07-2.38 (9H, m), 2.35 (3H, s), 4.53 (2H, d, J = 6.6 Hz), 6.61 (1H, s), 6.66 (1H, d, J = 2.4 Hz), 6.82-7.09 (1H, m), 7.11-7.31 (4H, m), 7.98 (0.5H, d, J = 12.2 Hz), 8.38 (0.5H, s).

### Reference Example 20a

### N-[4-[[3-(4-Fluorophenyl)-2-methyl-2-propenyl]oxy]-2,3,6-trimethylphenyl]formamide

By using N-(4-hydroxy-2,3,6-trimethylphenyl)formamide and 1-(3-bromo-2-methyl-1-propenyl)-4-fluorobenzene, the title compound was synthesized according to Reference Example 17a. Yield: 52%. Melting point: 164-165°C. (Ethyl acetate-hexane)
¹H-NMR (CDCl₃) δ: 1.96 (3H, s), 2.12-2.32 (9H, m), 4.53 (2H, d, J = 5.2 Hz), 6.60 (1H, s), 6.66 (1H, s), 6.71-6.95 (1H, m), 7.04 (2H, t, J = 8.8 Hz), 7.22-7.33 (2H, m), 8.04 (0.5H, d, J = 12.0 Hz), 8.40 (0.5H, d, J = 1.4 Hz).

### Reference Example 21a

### N-[4-[[(E)-3-(4-Isopropylphenyl)-2-propenyl]oxy]-2,3,6-trimethylphenyl]formamide

By using N-(4-hydroxy-2,3,6-trimethylphenyl)formamide and 1-[(E)-3-bromo-1-propenyl]-4-isopropylbenzene, the title compound was synthesized according to Reference Example 17a. Yield: 59%. Melting point: 165-167°C. (Ethyl acetate-hexane)
¹H-NMR (CDCl₃) δ: 1.25 (6H, d, J = 6.8 Hz), 2.13-2.27 (9H, m), 2.90 (1H, septet, J = 6.8 Hz), 4.66 (2H, t, J = 5.8 Hz), 6.37 (1H, dt, J = 15.8, 5.8 Hz), 6.65-6.88 (3H, m), 7.16-7.26 (2H, m), 7.35 (2H, d, J = 8.0 Hz), 7.98 (0.5H, d, J = 12.0 Hz), 8.40 (0.5H, d, J = 1.4 Hz).

### Reference Example 22a

### N-[2,3,6-Trimethyl-4-[[(E)-3-phenyl-2-propenyl]oxy]-phenyl]formamide

By using N-(4-hydroxy-2,3,6-trimethylphenyl)formamide and cinnamyl chloride, the title compound was synthesized according to Reference Example 17a. Yield: 44%. Melting point: 197-199°C. (Ethyl acetate-hexane)
¹H-NMR (CDCl₃) δ: 2.05-2.18 (9H, m), 4.62-4.72 (2H, m), 6.35-6.50 (1H, m), 6.62-7.00 (3H, m), 7.24-7.52 (5H, m), 8.00 (0.5H, d, J = 12.0 Hz), 8.39 (0.5H, d, J = 1.6 Hz).

### Reference Example 23a

### N-[4-[[(E)-3-(4-Fluorophenyl)-2-propenyl]oxy]-2,3,6-trimethylphenyl]formamide

By using N-(4-hydroxy-2,3,6-trimethylphenyl)formamide and 1-[(E)-3-bromo-1-propenyl]-4-fluorobenzene, the title compound was synthesized according to Reference Example 17a. Yield: 52%. Melting point: 196-198°C. (Ethyl acetate-hexane)
¹H-NMR (CDCl₃) δ: 2.10-2.32 (9H, m), 4.67 (2H, t, J = 5.0 Hz), 6.37(1H, dt, J = 15.6, 5.0 Hz), 6.59-6.89 (3H, m), 6.92-7.09 (2H, m), 7.32-7.43 (2H, m), 7.99 (0.5H, d, J = 12.0 Hz), 8.42 (0.5H, d, J = 1.4 Hz).

### Reference Example 24a

### N-[4-Hydroxy-3-[1-(4-isopropylphenyl)-2-propenyl]-2,5,6-trimethylphenyl]formamide

A solution of N-[4-[[(E)-3-(4-isopropylphenyl)-2-propenyl]oxy]-2,3,6-trimethylphenyl]formamide (5.80 g, 17.2 mmol) in N,N-dimethylaniline (50 ml) was stirred at 215°C for 6 hours under an argon atmosphere. The reaction mixture was cooled down, then diluted with ethyl acetate, washed with 2 N hydrochloric acid and water, dried on magnesium sulfate, and then concentrated under reduced pressure. The residue was crystallized from ethyl acetate to obtain 3.50 g (60% yield) of the title compound. Melting point: 170-171°C.
¹H-NMR (CDCl₃) δ: 1.18-1.40 (6H, m), 2.11-2.27 (9H, m), 2.77-3.00 (1H, m), 5.00-5.22 (2H, m), 5.30-5.42 (1H, m), 6.30-6.85 (2H, m), 7.10-7.37 (5H, m), 7.97 (0.5H, d, J = 12.2 Hz), 8.43 (0.5H, d, J = 1.4 Hz).

### Reference Example 25a

### N-[4-Hydroxy-3-(1-phenyl-2-propenyl)-2,5,6-trimethylphenyl]formamide

By using N-[2,3,6-trimethyl-4-[[(E)-3-phenyl-2-propenyl]oxy]phenyl]formamide, the title compound was synthesized according to Reference Example 24a. Yield: 78%. Melting point: 144-145°C. (Ethyl acetate)
¹H-NMR (CDCl₃) δ: 2.08-2.27 (9H, m), 5.02-5.41 (3H, m), 6.32-6.52 (1H, m), 6.61-7.03 (2H, m), 7.18-7.42 (5H, m), 7.95 (0.5H, d, J = 12.0 Hz), 8.42 (0.5H, d, J = 1.8 Hz).

### Reference Example 26a

### N-[4-Hydroxy-3-[1-(4-fluorophenyl)-2-propenyl]-2,5,6-trimethylphenyl]formamide

By using N-[4-[[(E)-3-(4-fluorophenyl)-2-propenyl]oxy]-2,3,6-trimethylphenyl]formamide, the title compound was synthesized according to Reference Example 24a. Yield: 66%. Melting point: 168-170°C. (Ethyl acetate)
¹H-NMR (CDCl₃) δ: 2.10-2.29 (9H, m), 5.02-5.22 (1.5H, m), 5.33-5.50 (1.5H, m), 6.35-6.55 (1H, m), 6.72-7.08 (4H, m), 7.18-7.30 (2H, m), 7.96 (0.5H, d, J = 12.2 Hz), 8.42 (0.5H, d, J = 1.4 Hz).

### Reference Example 27a

### 3-(4-Isopropylphenyl)-2,2,4,6,7-pentamethyl-2,3-dihydro-1-benzofuran-5-amine

A solution of N-[4-[[3-(4-isopropylphenyl)-2-methyl-2-propenyl]oxy]-2,3,6-trimethylphenyl]formamide (3.70 g, 10.5 mmol) in N,N-dimethylaniline (20 ml) was stirred at 215°C for 6 hours under an argon atmosphere. The reaction mixture was cooled down, then diluted with ethyl acetate, washed with 2 N hydrochloric acid and water, dried on magnesium sulfate, and then concentrated under reduced pressure to obtain N-[4-hydroxy-3-[1-(4-isopropylphenyl)-2-methyl-2-propenyl]-2,5,6-trimethylphenyl]formamide as a crude product. A mixture of this compound (2.98 g, 8.47 mmol), concentrated hydrochloric acid (20 ml) and methanol (60 ml) was refluxed with heating for 2 hours under a nitrogen atmosphere. The solvent was concentrated under reduced pressure and the resulting residue was neutralized with an 8 N aqueous solution of sodium hydroxide. The product was extracted twice with ethyl acetate. The combined extracts were washed with water, dried on magnesium sulfate, and then concentrated under reduced pressure. The resulting residue was crystallized from isopropyl ether-hexane to obtain 2.23 g (66% yield) of the title compound. Melting point: 130-132°C.
¹H-NMR (CDCl₃) δ: 1.00 (3H, s), 1.21 (6H, d, J = 6.6 Hz), 1.47 (3H, s), 1.78 (3H, s), 2.12 (3H, s), 2.19 (3H, s), 2.40-2.60 (3H, m), 4.08 (1H, s), 6.72-7.00 (2H, m), 7.07 (2H, d, J = 8.0 Hz).

### Reference Example 28a

### 2,2,-4,6,7-Pentamethyl-3-phenyl-2,3-dihydro-1-benzofuran-5-amine

By using N-[2,3,6-trimethyl-4-[(2-methyl-3-phenyl-2-propenyl)oxy]phenyl]formamide, the title compound was synthesized according to Reference Example 27a. Yield: 67%. Melting point: 129-131°C.
¹H-NMR (CDCl₃) δ: 1.00 (3H, s), 1.48 (3H, s), 1.77(3H, s), 2.13 (3H, s), 2.19 (3H, s), 3.20 (2H, br s), 4.12 (1H, s), 6.70-7.30 (5H, m).

### Reference Example 29a

### 2,2,4,6,7-Pentamethyl-3-(4-methylphenyl)-2,3-dihydro-1-benzofuran-5-amine

A solution of N-[2,3,6-trimethyl-4-[[2-methyl-3-(4-methylphenyl)-2-propenyl]oxy]phenyl]formamide (5.43 g, 16.8 mmol) in N,N-dimethylaniline (60 ml) was stirred at 210°C for 6 hours under an argon atmosphere. The reaction mixture was cooled down, then diluted with ethyl acetate, washed with 2 N hydrochloric acid and water, dried on magnesium sulfate, and then concentrated under reduced pressure. A mixture of the resulting residue and a hydrochloric acid-methanol reagent (40 ml) was refluxed with heating for 2 hours under a nitrogen atmosphere. The solvent was concentrated under reduced pressure and the resulting residue was neutralized with an 8 N aqueous solution of sodium hydroxide. The product was extracted twice with ethyl acetate. The combined extracts were washed with water, dried on magnesium sulfate, and then concentrated under reduced pressure. The resulting residue was crystallized from hexane to obtain 2.81 g (57% yield) of the title compound. Melting point: 114-115°C. (Petroleum ether)
¹H-NMR (CDCl₃) δ: 0.99 (3H, s), 1.47 (3H, s), 1.77 (3H, s), 2.12 (3H, s), 2.19 (3H, s), 2.30. (3H, s), 3.23 (2H, br s), 4.08 (1H, s), 6.60-7.23 (4H, m).

### Reference Example 30a

### 3-(4-Fluorophenyl)-2,2,4,6,7-pentamethyl-2,3-dihydro-1-benzofuran-5-amine

By using N-[4-[[3-(4-fluorophenyl)-2-methyl-2-propenyl]oxy]-2,3,6-trimethylphenyl]formamide, the title compound was synthesized according to Reference Example 27a. Yield: 78%. Melting point: 125-127°C. (Petroleum ether)
¹H-NMR (CDCl₃) δ: 0.99 (3H, s), 1.47 (3H, s), 1.77(3H, s), 2.12 (3H, s), 2.19 (3H, s), 3.10 (2H, br s), 4.09 (1H, s), 6.62-7.20 (4H, m).

### Reference Example 31a

### 3-(4-Isopropylphenyl)-2,4,6,7-tetramethyl-1-benzofuran-5-amine hydrochloride

To a suspension of N-[4-hydroxy-3-[1-(4-isopropylphenyl)-2-propenyl]-2,5,6-trimethylphenyl]formamide (3.50 g, 10.4 mmol) and calcium carbonate (1.35 g, 13.5 mmol) in a mixed solvent of tetrahydrofuran (15 ml) and methanol (15 ml) was gradually added benzyltrimethylammonium iododichloride (3.90 g, 11.4 mmol). The reaction mixture was stirred at room temperature for 30 minutes. After filtration of the insoluble material, the solvent was concentrated under reduced pressure. Ethyl acetate and water were added to the residue. The organic layer was separated and the aqueous layer was extracted twice with ethyl acetate. The combined organic layers were successively washed with a 10% aqueous solution of sodium hydrosulfite, water, an aqueous saturated solution of sodium hydrogen carbonate, and an aqueous saturated solution of sodium chloride, dried on magnesium sulfate, and then concentrated under reduced pressure to obtain 4.08 g of N-[2-iodomethyl-3-(4-isopropylphenyl)-4,6,7-trimethyl-2,3- dihydro-1-benzofuran-5-yl]formamide. A solution of this compound (4.08 g, 8.81 mmol) and 1,8-diazabicyclo[5,4,0]-7-undecene (6.58 m, 44.0 mmol) in toluene (30 ml) was stirred at 100°C for 3 hours under an argon atmosphere. Water was added into the reaction solution and the product was extracted twice with ethyl acetate. The combined extracts were washed with 2 N hydrochloric acid and water, dried on magnesium sulfate, and then concentrated under reduced pressure. The resulting residue was subjected to column chromatography on silica gel (hexane-ethyl acetate 20 : 1) to obtain 2.40 g of -[3-(4-isopropylphenyl)-2,4,6,7-tetramethyl-1-benzofuran-5-yl]formamide. A mixture of this compound (2.40 g, 7.18 mmol), concentrated hydrochloric acid (20 ml) and methanol (60 ml) was refluxed with heating for 2 hours under a nitrogen atmosphere. The solvent was concentrated under reduced pressure and the resulting residue was neutralized with an 8 N aqueous solution of sodium hydroxide. The product was extracted twice with ethyl acetate. The combined extracts were washed with water, dried on magnesium sulfate, and then concentrated under reduced pressure to obtain 1.80 g of an oily free base. This free base (0.50 g, 1.63 mmol) was dissolved into a solution of hydrochloric acid in methanol and the solvent was concentrated under reduced pressure. The resulting residue was crystallized from methanol to obtain 0.41 g (41% yield) of the title compound. Melting point: 194-197°C.
¹H-NMR (CDCl₃) δ: 1.29 (6H, d, J = 7.0 Hz), 2.30 (6H, s), 2.41 (3H, s), 2.60 (3H, s), 2.94 (1H, septet, J = 7.0 Hz), 7.13-7.26 (4H, m), 10.1 (2H, br s), 1H unidentified.

### Reference Example 32a

### 2,4,6,7-Tetramethyl-3-phenyl-1-benzofuran-5-amine hydrochloride

By using N-[4-hydroxy-3-(1-phenyl-2-propenyl)-2,5,6-trimethylphenyl]formamide, the title compound was synthesized according to Reference Example 31a. Yield: 26%. Melting point: 189-192°C. (Ethanol-hexane)
¹H-NMR. (CDCl₃) δ: 2. 30 (6H, s) , 2.42 (3H, s) , 2. 60 (3H, s), 7.21-7.37 (5H, m), 10.2 (2H, br s), 1H unidentified.

### Reference Example 33a

### 3-(4-Fluorophenyl)-2,4,6,7-tetramethyl-1-benzofuran-5-amine hydrochloride

By using N-[4-hydroxy-3-[1-(4-fluorophenyl)-2-propenyl]-2,5,6-trimethylphenyl]formamide, the title compound was synthesized according to Reference Example 31a. Yield: 87%. Melting point: 208-210°C. (Ethanol)
¹H-NMR (CDCl₃) δ: 2.29 (6H, s), 2.42 (3H, s), 2.60 (3H, s), 7.03-7.28 (4H, m), 10.2 (2H, br s), 1H unidentified.

### Reference Example 34a

### 5,6-Dichloro-2-(2,2,4,6,7-pentamethyl-3-phenyl-2,3-dihydro-1-benzofuran-5-yl)-1H-isoindole-1,3(2H)-dione

To a solution of 2,2,4,6,7-pentamethyl-3-phenyl-2,3-dihydro-1-benzofuran-5-amine (1.00 g, 3.56 mmol) in tetrahydrofuran (30 ml) was added 4,5-dichlorophthalic anhydride (850.6 mg, 3.92 mmol) under an argon atmosphere and the mixture was refluxed with heating for 13 hours. The reaction mixture was cooled down to room temperature and then 1-ethyl-3-(3-dimethylaminopropyl)carbodiimide (WSC) hydrochloride (760.0 mg, 3.96 mmol) and 1-hydroxy-1H-benzotriazole (HOBT) monohydrate (602.6 mg, 3.93 mmol) were added to the mixture. The resulting mixture was refluxed with heating for 3 hours and then cooled down to room temperature. Water and an 8 N aqueous solution of sodium hydroxide were added into the reaction mixture and the product was extracted twice with ethyl acetate. The combined extracts were washed with an aqueous saturated solution of sodium hydrogen carbonate, dried on magnesium sulfate, filtered, and then concentrated under reduced pressure. The residue was crystallized from ethyl acetate to obtain 1.16 g (68% yield) of the title compound. Melting point: 178-181°C.
¹H-NMR (CDCl₃) δ: 1.02 (3H, s), 1.56 (3H, s), 1.61 (3H, s), 2.01 (3H, s), 2.20 (3H, s), 4.21 (1H, s), 6.8-7.4 (5H, m), 7.99 (1H, s), 8.03 (1H, s).

### Reference Example 35a

### 2-[2,2,4,6,7-Pentamethyl-3-(4-methylphenyl)-2,3-dihydro-1-benzofuran-5-yl]-1H-isoindole-1,3(2H)-dione

To a solution of phthalic anhydride (566.4 mg, 3.82 mmol) in tetrahydrofuran (5 ml) was added a solution of 2,2,4,6,7-pentamethyl-3-(4-methylphenyl)-2,3-dihydro-1-benzofuran-5-amine (987.3 mg, 3.38 mmol) in tetrahydrofuran(10 ml) and the resulting mixture was refluxed with heating for 11 hours. The reaction mixture was cooled down to room temperature and then concentrated under reduced pressure. Sodium acetate (314.6 mg, 3.84 mmol) and acetic anhydride (20 ml) were added into the residue and the resulting mixture was stirred at 90°C for 2 hours. The reaction mixture was cooled down to room temperature and then an 8 N aqueous solution of sodium hydroxide was added into the mixture until it became basic. The product was extracted twice with ethyl acetate. The combined extracts were washed with an aqueous saturated solution of sodium hydrogen carbonate, dried on magnesium sulfate, filtered, and then concentrated under reduced pressure. The residue was crystallized from ethyl acetate to obtain 1.16 g (81% yield) of the title compound. Melting point: 222-224°C.
¹H-NMR (CDCl₃) δ: 1.03 (3H, s), 1.55 (3H, s), 1.64 (3H, s), 2.05 (3H, s), 2.20 (3H, s), 2.30 (3H, s), 4.19 (1H, s), 6.6-7.1 (4H, m), 7.76-7.82 (2H, m), 7.88-7.97 (2H, m).

### Reference Example 36a

### 5,6-Dichloro-2-[2,2,4,6,7-pentamethyl-3-(4-methylphenyl)-2,3-dihydro-1-benzofuran-5-yl]-1H-isoindole-1,3(2H)-dione

By using 2,2,4,6,7-pentamethyl-3-(4-methylphenyl)-2,3-dihydro-1-benzofuran-5-amine, the title compound was synthesized according to Reference Example 34a. Yield: 62%. Melting point: 157-159°C. (Ethyl acetate)
¹H-NMR ( CDCl₃ ) δ: 1.02 (3H, s), 1.54 (3H, s), 1.61 (3H, s), 2.01 (3H, s). 2.19 (3H, s), 2.30 (3H, s), 4.18 (1H, s), 6.8-7.1 (4H, m), 7.99 (1H, s), 8.03 (1H, s) .

### Reference Example 37a

### 2-[3-(4-Fluorophenyl)-2,2,4,6,7-pentamethyl-2,3-dihydro-1-benzofuran-5-yl]-1H-isoindole-1,3(2H)-dione

By using 3-(4-fluorophenyl)-2,2,4,6,7-pentamethyl-2,3-dihydro-1-benzofuran-5-amine, the title compound was synthesized according to Reference Example 35a. Yield: 72%. Melting point: 209-211°C. (Ethyl acetate)
¹H-NMR (CDCl₃) δ: 1.02 (3H, s), 1.55 (3H, s), 1.61 (3H, s), 2.05 (3H, s), 2.20 (3H, s), 4.21 (1H, s), 6.9-7.1 (4H, m), 7.76-7.83 (2H, m), 7.90-7.97 (2H, m).

### Reference Example 38a

### 5,6-Dichloro-2-[3-(4-fluorophenyl)-2,2,4,6,7-pentamethyl-2,3-dihydro-1-benzofuran-5-yl]-1H-isoindole-1,3(2H)-dione

By using 3-(4-fluorophenyl)-2,2,4,6,7-pentamethyl-2,3-dihydro-1-benzofuran-5-amine, the title compound was synthesized according to Reference Example 34a. Yield: 62%. Melting point: 232-233°C. (Ethyl acetate)
¹H-NMR (CDCl₃) δ: 1.02 (3H, s), 1.54 (3H, s), 1.61 (3H, s), 2.01 (3H, s), 2.19 (3H, s), 4.19 (1H, s), 6.8-7.1 (4H, m), 8.00 (1H, s), 8.03 (1H, s).

### Reference Example 39a

### 2-[3-(4-Isopropylphenyl)-2,2,4,6,7-pentamethyl-2,3-dihydro-1-benzofuran-5-yl]-1H-isoindole-1,3(2H)-dione

By using 3-(4-isopropylphenyl)-2,2,4,6,7-pentamethyl-2,3-dihydro-1-benzofuran-5-amine, the title compound was synthesized according to Reference Example 35a. Yield: 97%. Melting point: 180-181°C. (Ethyl acetate-hexane)
¹H-NMR (CDCl₃) δ: 1.02 (3H, s), 1.21 (6H, d, J = 7.0 Hz), 1.55 (3H, s), 1.64 (3H, s), 2.05 (3H, s), 2.20 (3H, s), 2.85 (1H, septet, J = 7.0 Hz), 4.20 (1H, s), 6.7-7.2 (4H, m), 7.75-7.82 (2H, m), 7.87-7.97 (2H, m).

### Reference Example 40a

### 5,6-Dichloro-2-[3-(4-isopropylphenyl)-2,2,4,6,7-pentamethyl-2,3-dihydro-1-benzofuran-5-yl]-1H-isoindole-1,3(2H)-dione

By using 3-(4-isopropylphenyl)-2,2,4,6,7-pentamethyl-2,3-dihydro-1-benzofuran-5-amine, the title compound was synthesized according to Reference Example 34a. Yield: 31%. Melting point: 237-239°C. (Ethyl acetate)
¹H-NMR (CDCl₃) δ: 1.01 (3H, s), 1.21. (6H, d, J = 6.6 Hz), 1.54 (3H, s), 1.62 (3H, s), 2.01 (3H, s), 2.19 (3H, s), 2.85 (1H, septet, J = 6.6 Hz), 4.18 (1H, s), 6.8-7.2 (4H, m), 7.99 (1H, s), 8.03 (1H, s).

### Reference Example 41a

### 2-[3-(4-Isopropylphenyl)-2,4,6,7-tetramethyl-1-benzofuran-5-yl]-1H-isoindole-1,3(2H)-dione

By using 3-(4-isopropylphenyl)-2,4,6,7-tetramethyl-1-benzofuran-5-amine, the title compound was synthesized according to Reference Example 35a. Yield: 71%. Melting point: 232-234°C. (Ethyl acetate-hexane)
¹H-NMR (CDCl₃) δ: 1.27 (6H, d, J = 7.0 Hz), 1.85 (3H, s), 2.14 (3H, s), 2.33 (3H, s), 2.48 (3H, s), 2.94 (1H, septet, J = 7.0 Hz), 7.24 (4H, m), 7.72-7.83 (2H, m), 7.90-8.03 (2H, m).

### Reference Example 42a

### 2-[2,2,4,6,7-Pentamethyl-2,3-dihydro-1-benzofuran-5-yl]-1H-isoindole-1,3(2H)-dione

By using 2,2,4,6,7-pentamethyl-2,3-dihydro-1-benzofuran-5-amine, the title compound was synthesized according to Reference Example 34a. Yield: 77%. Melting point: 166-168°C. (Methanol)
¹H-NMR (CDCl₃) δ: 1.49 (6H, s), 1.95 (3H, s), 1.99 (3H, s), 2.12 (3H, s), 2.97 (2H, s), 7.66-7.83 (2H, m), 7.91-8.01 (2H, m).

### Reference Example 43a

### 4-Methoxy-2,3,6-trimethylaniline

N-(4-Hydroxy-2,3,6-trimethylphenyl)formamide (30.0 g, 167 mmol) was dissolved into a mixed solvent of a 4 N aqueous solution of potassium hydroxide (100 ml) and methanol (300 ml) and dimethyl sulfate (42.0 g, 334 mmol) was added to the resulting solution at room temperature. The resulting mixture was refluxed with heating for 14 hours. After the reaction solution was cooled down, the crystals precipitated were collected by filtration to obtain N-(4-methoxy-2,3,6-trimethylphenyl)formamide as a crude product. To a suspension of this compound in methanol (200 ml) was added concentrated hydrochloric acid (50 ml) and the resulting mixture was refluxed with heating for 3 hours. The reaction mixture was cooled down and neutralized with an 8 N aqueous solution of sodium hydroxide. The product was extracted twice with ethyl acetate. The combined extracts were washed with a 10% aqueous solution of sodium hydrosulfite, dried on magnesium sulfate, and then concentrated under reduced pressure. The residue was crystallized from isopropyl ether to obtain 21.0 g (yield 76%) of the title compound. Melting point: 70-72°C.
¹H-NMR (CDCl₃) δ: 2.11 (3H, s), 2.16 (3H, s), 2.18 (3H, s), 3.16 (1H, br s), 3.74 (3H, s), 6.54 (1H, s).

### Reference Example 44a

### Tert-butyl 4-methoxy-2,3,6-trimethylphenylcarbamate

To a solution of 4-methoxy-2,-3,6-trimethylaniline (21.0 g, 127 mmol) and triethylamine (21.0 ml, 152 mmol) in tetrahydrofuran (150 ml) was added di-tert-butyl dicarbonate (32 ml, 140 mmol) at room temperature and the resulting mixture was refluxed with heating for 14 hours. The solvent was concentrated under reduced pressure. Water was added into the residue and the product was extracted twice with ethyl acetate. The combined organic layers were washed with 1 N hydrochloric acid and an aqueous saturated solution of sodium hydrogen carbonate, dried on magnesium sulfate, filtered, and then concentrated under reduced pressure. The residue was crystallized from ethyl acetate-hexane to obtain 25.2 g (75% yield) of the title compound. Melting point: 104-106°C.
¹H-NMR (CDCl₃) δ: 1.50 (9H, s), 2.12 (3H, s), 2.17 (3H, s), 2.24 (3H, s), 3.78 (3H, s), 5.81 (1H, br s), 6.58 (1H, s).

### Reference Example 45a

### Tert-butyl 3-bromo-4-methoxy-2,5,6-trimethylphenylcarbamate

To a solution of tert-butyl 4-methoxy-2,3,6-trimethylphenylcarbamate (12.7 g, 47.9 mmol) and sodium acetate (4.72 g, 57.5 mg) in acetic acid (50 ml) was added bromine (8.42 g, 52.7 mmol) at room temperature and the resulting mixture was stirred at the same temperature for 1 hour. Water (80 ml) was added into the reaction mixture and then the crystals precipitated were collected by filtration and dissolved into ethyl acetate. This solution was washed with an aqueous saturated solution of sodium hydrogen carbonate and water, dried on magnesium sulfate, filtered, and then concentrated under reduced pressure. The residue was crystallized from methanol to obtain 15.0 g (91% yield) of the title compound. Melting point: 159-161°C.
¹H-NMR (CDCl₃) δ: 1.50 (9H, s), 2.15 (3H, s), 2.24 (3H, s), 2.35 (3H, s), 3.74 (3H, s), 5.92 (1H, br s).

### Reference Example 46a

### 2,2,4,6,7-Pentamethyl-3-(4-methylphenyl)-2,3-dihydro-1-benzofuran-5-amine

To a solution of tert-butyl 3-bromo-4-methoxy-2,5,6-trimethylphenylcarbamate (27.8 g, 80.8 mmol) in tetrahydrofuran (150 ml) was added n-butyllithium (1.6 M, 110 ml, 176 mmol) at -78°C and the reaction mixture was stirred at the same temperature for 20 minutes. To the reaction solution was added 2-methyl-1-(4-methylphenyl)propan-1-one (13.1 g, 80.7 mmol) and the resulting mixture was stirred at room temperature for 1 hour. Water (150 ml) was added into the reaction mixture and the product was extracted three times with ethyl acetate. The combined organic layers were washed with water, dried on magnesium sulfate, filtered, and then concentrated under reduced pressure to obtain 26.0 g of tert-butyl 3-[1-hydroxy-2-methyl-1-(4-methylphenyl)propyl]-4-methoxy-2,5,6-trimethylphenylcarbamate as a crude product. A mixture of this compound and 47% hydrobromic acid (100 ml) was refluxed with heating for 4 hours under an argon atmosphere. The reaction mixture was cooled down to room temperature and neutralized with an 8 N aqueous solution of sodium hydroxide. The product was extracted twice with ethyl acetate. The combined extracts were washed with an aqueous saturated solution of sodium hydrogen carbonate, dried on magnesium sulfate, filtered, and then concentrated under reduced pressure. The residue was crystallized from isopropyl ether-hexane to obtain 14.8 g (62% yield) of the title compound. Melting point: 114-115°C.
¹H-NMR (CDCl₃) δ: 0.99 (3H, s), 1.47 (3H, s), 1.78 (3H, s), 2.12 (3H, s), 2.17 (3H, s), 2.30 (3H, s), 2.80 (2H, br s), 4.08 (1H, s), 6.60-7.10 (4H, m).

### Reference Example 47a

### (+)-2,2,4,6,7-Pentamethyl-3-(4-methylphenyl)-2,3-dihydro-1-benzofuran-5-amine

2,2,4,6,7-Pentamethyl-3-(4-methylphenyl)-2,3-dihydro-1-benzofuran-5-amine was subjected to high performance liquid chromatography (instrument: Waters semi-preparative separation system, column: CHIRALCEL OD (20 (i, d) x 250 mm) manufactured by Daicel Chemical Industries, LTD.), mobile phase: hexane : isopropyl alcohol = 95 : 5, flow rate: 5 ml/min, column temperature: 30°C, sample injection amount: 40 mg) to preparatively separate a fraction with a shorter retention time as the title compound. Melting point: 87-89°C. [α]D = +4.7° (c = 0.495, methanol)

### Reference Example 48a

### (-)-2,2,4,6,7-Pentamethyl-3-(4-methylphenyl)-2,3-dihydro-1-benzofuran-5-amine

2,2,4,6,7-Pentamethyl-3-(4-methylphenyl)-2,3-dihydro-1-benzofuran-5-amine was subjected to high performance liquid chromatography (instrument: Waters semi-preparative separation system, column: CHIRALCEL OD (20 (i, d) x 250 mm) manufactured by Daicel Chemical Industries, LTD.), mobile phase: hexane : isopropyl alcohol = 95 : 5, flow rate: 5 ml/min, column temperature: 30°C, sample injection amount: 40 mg) to preparatively separate a fraction with a longer retention time as the title compound. Melting point: 88-90°C. [α]D = -4.3° (c = 0.499, methanol)

### Example 1a

### 2-(2,2,4,6,7-Pentamethyl-3-phenyl-2,3-dihydro-1-benzofuran-5-yl)isoindoline

A mixture of 2,2,4,6,7-pentamethyl-3-phenyl-2,3-dihydro-1-benzofuran-5-amine (1.00 g, 3.55 mmol), 1,2-bis(bromomethyl)benzene (1.03 g, 3.91 mmol), potassium carbonate (540 mg, 3.91 mmol) and N, N-dimethylformamide (20 ml) was stirred at room temperature for 1 hour. Water was added into the reaction mixture and the product was extracted twice with ethyl acetate. The combined organic layers were washed with water, dried on magnesium sulfate, filtered, and then concentrated under reduced pressure. The residue was subjected to column chromatography on silica gel (hexane-ethyl acetate 10 : 1) to obtain 208 mg (15% yield) of the title compound. Melting point: 164-166°C. (Ethyl acetate-hexane)
¹H-NMR (CDCl₃) δ: 1.02 (3H, s), 1.52 (3H, s), 1.76 (3H, s), 2.18 (3H, s), 4.13 (1H, s), 4.52 (4H, s), 6.70-7.41 (9H, m).

### Example 2a

### 5,6-Dichloro-2-(2,2,4,6,7-pentamethyl-3-phenyl-2,3-dihydro-1-benzofuran-5-yl)isoindoline

To a solution of aluminum chloride (1.01 g, 7.59 mmol) in tetrahydrofuran (30 ml) was added lithium aluminum hydride (276.5 mg, 7.29 mmol) and the resulting mixture was stirred for 10 minutes. To this mixture was added a solution of 5,6-dichloro-2-[2,2,4,6,7-pentamethyl-3-phenyl-2,3-dihydro-1-benzofuran-5-yl]-1H-isoindole-1,3(2H)-dione (907.4 mg, 1.89 mmol) in tetrahydrofuran (10 ml) and the resulting mixture was refluxed with heating for 2 hours. The reaction mixture was cooled down to room temperature and water was added into the mixture. The product was extracted twice with ethyl acetate. The combined extracts were washed with an aqueous saturated solution of sodium hydrogen carbonate, dried on magnesium sulfate, filtered, and then concentrated under reduced pressure. The residue was crystallized from ethyl acetate-hexane to obtain 153 mg (18% yield) of the title compound. Melting point: 194-196°C.
¹H-NMR (CDCl₃) δ: 1.02 (3H, s) , 1.52 (3H, s), 1.74 (3H, s), 2.16 (6H, s), 4.12 (1H, s), 4.45 (4H, s), 6.8-7.4 (7H, m).

### Example 3a

### 5,6-Dimethoxy-2-(2,2,4,6,7-pentamethyl-3-phenyl-2,3-dihydro-1-benzofuran-5-yl)isoindoline

To a solution of 2,2,4,6,7-pentamethyl-3-phenyl-2,3-dihydro-1-benzofuran-5-amine (1.00 g, 3.56 mmol) in tetrahydrofuran (30 ml) were added 1,2-bis(chloromethyl)-4,5-dimethoxybenzene (889.1 mg, 3.78 mmol), sodium carbonate (1.15 g, 10.85 mmol), and tetrabutylammonium iodide (701.4 mg, 1.90 mmol) and the mixture was refluxed with heating for 21 hours. The reaction mixture was cooled down to room temperature and then poured into ice water. The product was extracted twice with ethyl acetate. The combined extracts were washed with an aqueous saturated solution of sodium hydrogen carbonate, dried on magnesium sulfate, filtered, and then concentrated under reduced pressure. The residue was subjected to column chromatography on silica gel to obtain 403 mg (26% yield) of the title compound. Melting point: 154-157°C. (Ethyl acetate)
¹H-NMR (CDCl₃) δ: 1.02 (3H, s), 1.53 (3H, s), 1.76 (3H, s), 2.18 (6H, s), 3.87 (6H, s), 4.13 (1H, s), 4.46 (4H, s), 6.7-7.4 (7H, m).

### Example 4a

### 2-[2,2,4,6,7-Pentamethyl-3-(4-methylphenyl)-2,3-dihydro-1-benzofuran-5-yl]isoindoline

By using 2-[2,2;4,6,7-pentamethyl-3-(4-methylphenyl)-2,3-dihydro-1-benzofuran-5-yl]-1H-isoindole-1,3(2H)-dione, the title compound was synthesized according to Example 2a. Yield: 46%. Melting point: 141-143°C. (Hexane)
¹H-NMR (CDCl₃) δ: 1.02 (3H, s), 1.51 (3H, s), 1.77 (3H, s), 2.17-2.18 (6H, s), 2.31 (3H, s), 4.10 (1H, s), 4.52 (4H, s), 6.8-7.1 (7H, m), 7.24 (4H, s).

### Example 5a

### 5,6-Dichloro-2-[2,2,4,6,7-pentamethyl-3-(4-methylphenyl)-2,3-dihydro-1-benzofuran-5-yl]isoindoline

By using 5,6-dichloro-2-[2,2,4,6,7-pentamethyl-3-(4-methylphenyl)-2,3-dihydro-1-benzofuran-5-yl]-1H-isoindole-1,3(2H)-dione, the title compound was synthesized according to Example 2a. Yield: 25%. Melting point: 201-203°C.
¹H-NMR (CDCl₃ ) δ: 1.01 (3H, s), 1.50 (3H, s), 1.74 (3H, s), 2.16 (6H, s), 2.31 (3H, s), 4.08 (1H, s), 4.45 (4H, s), 6.6-7.1 (4H, m), 7.31 (2H, s).

### Example 6a

### 5,6-Dimethoxy-2-[2,2,4,6,7-pentamethyl-3-(4-methylphenyl)-2,3-dihydro-1-benzofuran-5-yl]isoindoline

To a solution of 2,2,4,6,7-pentamethyl-3-(4-methylphenyl)-2,3-dihydro-1-benzofuran-5-amine (806.1 mg, 2.76 mmol) in tetrahydrofuran (30 ml) were added 1,2-bis(chloromethyl)-4,5-dimethoxybenzene- (686.6 mg, 2.92 mmol), sodium carbonate (878.5 g, 8.29 mmol), and tetrabutylammonium iodide (543.6 mg, 1.47 mmol) and the mixture was refluxed with heating for 11 hours. The reaction mixture was cooled down to room temperature and then poured into ice water. The product was extracted twice with ethyl acetate. The combined extracts were washed with an aqueous saturated solution of sodium hydrogen carbonate, dried on magnesium sulfate, filtered, and then concentrated under reduced pressure. The residue was crystallized from ethyl acetate-hexane to obtain 199.6 mg (16% yield) of the title compound. Melting point: 156-159°C.
¹H-NMR (CDCl₃) δ: 1.02 (3H, s), 1.51 (3H, s), 1.76 (3H, s), 2.17 (6H, s), 2.31 (3H, s), 3.88 (6H, s), 4.10 (1H, s), 4.45 (4H, s), 6.7-7.2 (6H, m).

### Example 7a

### 2-[3-(4-Fluorophenyl)-2,2,4,6,7-pentamethyl-2,3-dihydro-1-benzofuran-5-yl]isoindoline

By using 2-[3-(4-fluorophenyl)-2,2,4,6,7-pentamethyl-2,3-dihydro-1-benzofuran-5-yl]-1H-isoindole-1,3(2H)-dione, the title compound was synthesized according to Example 2a. Yield: 55%. Melting point: 204-205°C. (Ethyl acetate)
¹H-NMR (CDCl₃) δ: 1.02 (3H, s), 1.51 (3H, s), 1.76 (3H, s), 2.17 (3H, s), 2.18 (3H, s), 4.11 (1H, s), 4.52 (4H, s), 6.7-7.1 (4H, m), 7.25 (4H, s).

### Example 8a

### 5,6-Dichloro-2-[3-(4-fluorophenyl)-2,2,4,6,7-pentamethyl-2,3-dihydro-1-benzofuran-5-yl]isoindoline

By using 5,6-dichloro-2-[3-(4-fluorophenyl)-2,2,4,6,7-pentamethyl-2,3-dihydro-1-benzofuran-5-yl]-1H-isoindole-1,3(2H)-dione, the title compound was synthesized according to Example 2a. Yield: 25%. Melting point: 233-238°C. (Ethyl acetate)
¹H-NMR (CDCl₃) δ: 1.01 (3H, s), 1.50 (3H, s), 1.60 (3H, s), 1.74 (3H, s), 2.15 (3H, s), 4.09 (1H, s), 4.45 (4H, s), 6.8-7.1 (4H, m), 7.32 (2H, s).

### Example 9a

### 2-[3-(4-Isopropylphenyl)-2,2,4,6,7-pentamethyl-2,3-dihydro-1-benzofuran-5-yl]isoindoline

By using 2-[3-(4-isopropylphenyl)-2,2,4,6,7-pentamethyl-2,3-dihydro-1-benzofuran-5-yl]-1H-isoindole-1,3(2H)-dione, the title compound was synthesized according to Example 2a. Yield: 57%. Melting point: 113-114°C. (Hexane)
¹H-NMR (CDCl₃) δ: 1.00 (3H, s), 1.22 (6H, d, J = 7.0 Hz), 1.51 (3H, s), 1.77 (3H, s), 2.17 (3H, s), 2.18 (3H, s), 2.86 (1H, septet, J = 7.0 Hz), 4.11 (1H, s), 4.53 (4H, s), 6.7-7.2 (4H, m), 7.24 (4H, s).

### Example 10a

### 5,6-Dichloro-2-[3-(4-isopropylphenyl)-2,2,4;6,7-pentamethyl-2,3-dihydro-1-benzofuran-5-yl]isoindoline

By using 5,6-dichloro-2-[3-(4-isopropylphenyl)-2,2,4,6,7-pentamethyl-2,3-dihydro-1-benzofuran-5-yl]-1H-isoindole-1,3(2H)-dione, the title compound was synthesized according to Example 2a. Yield: 16%. Melting point: 148-150°C. (Hexane)
¹H-NMR (CDCl₃) δ: 1.01-1.06 (3H, s), 1.22 (6H, d, J = 7.0 Hz), 1.50-1.54 (3H, m), 1.74-1.78 (3H, m), 2.16-2.20 (6H, m), 2.86 (1H, septet, J = 7.0 Hz), 4.09-4.13 (1H, m), 4.46 (4H, s), 6.7-8.0 (6H, m).

### Example 11a

### 5,6-Dimethoxy-2-[3-(4-isopropylphenyl)-2,2,4,6,7-pentamethyl-2,3-dihydro-1-benzofuran-5-yl]isoindoline

By using 3-(4-isopropylphenyl)-2,2,4,6,7-pentamethyl-2,3-dihydro-1-benzofuran-5-amine, the title compound was synthesized according to Example 3a. Yield: 68%. Melting point: 153-155°C. (Isopropyl ether-hexane)
¹H-NMR (CDCl₃) δ: 1.01-1.05 (3H, s), 1.22 (6H, d, J = 7.0 Hz), 1.48-1.55 (3H, m), 1.77-1.83 (3H, m), 2.17-2.19 (6H, m), 2.86 (1H, septet, J = 7.0 Hz), 3.87-3.91 (7H, m), 4.10-4.14 (1H, m), 4.48 (3H, s), 6.77 (2H, s), 6.8-7.0 (2H, m), 7.07-7.11 (2H, m).

### Example 12a

### 6-[3-(4-Isopropylphenyl)-2,2,4,6,7-pentamethyl-2,3-dihydro-1-benzofuran-5-yl]-6,7-dihydro-5H-[1,3]dioxolo[4,5-f]isoindole

To a solution of 3-(4-isopropylphenyl)-2,2,4,6,7-pentamethyl-2,3-dihydro-1-benzofuran-5-amine (835.5 mg, 2.58 mmol) in tetrahydrofuran (20 ml) were added 5,6-bis(chloromethyl)-1,3-benzodioxazole (574.5 mg, 2.62 mmol), sodium carbonate (832.8 mg, 7.88 mmol) and tetrabutylammonium iodide (481.6 mg, 1.30 mmol) and the mixture was refluxed with heating for 23 hours. The reaction mixture was cooled down to room temperature and then poured into ice water. The product was extracted twice with ethyl acetate. The combined extracts were washed with an aqueous saturated solution of sodium hydrogen carbonate, dried on magnesium sulfate, filtered, and then concentrated under reduced pressure. The residue was crystallized from isopropyl ether to obtain 395.0 mg (33% yield) of the title compound. Melting point: 175-177°C.
¹H-NMR (CDCl₃) δ: 1.00 (3H, s), 1.22 (6H, d, J = 7.0 Hz), 1.50 (3H, s), 1.76 (3H, s), 2.17 (6H, s), 2.86 (1H, septet, J = 7.0 Hz), 4.10 (1H, s), 4.42 (4H, s), 5.94 (2H, s), 6.89 (2H, s), 6.80-7.11 (4H, m).

### Example 13a

### 2-[3-(4-Isopropylphenyl)-2,4,6,7-tetramethyl-1-benzofuran-5-yl]isoindoline

By using 2-[3-(4-isopropylphenyl)-2,4,6,7-tetramethyl-1-benzofuran-5-yl]-1H-isoindole-1,3(2H)-dione, the title compound was synthesized according to Example 2a. Yield: 70%. Melting point: 126-129°C. (Ethanol)
¹H-NMR (CDCl₃) δ: 1.28 (6H, d, J = 7.0 Hz), 1.97 (3H, s), 2.27 (3H, s), 2.31 (3H, s), 2.44 (3H, s), 2.95 (1H, septet, J = 7.0 Hz), 4.57 (4H, s), 7.25 (8H, s).

### Example 14a

### 6-[2,2,4,6,7-Pentamethyl-3-(4-methylphenyl)-2,3-dihydro-1-benzofuran-5-yl]-6H-[1,3]dioxolo[4,5-f]-isoindole

To a solution of 2,2,4,6,7-pentamethyl-3-(4-methylphenyl)-2,3-dihydro-1-benzofuran-5-amine (799.8 mg, 2.73 mmol) in tetrahydrofuran (30 ml) were added 5,6-bis(chloromethyl)-1,3-benzodioxazole (603.8 mg, 2.76 mmol), sodium carbonate (877.8 mg, 8.28 mmol) and tetrabutylammonium iodide (506.8 mg, 1.37 mmol) and the mixture was refluxed with heating for 23 hours. The reaction mixture was cooled down to room temperature and then poured into ice water. The product was extracted twice with isopropyl ether. The combined extracts were washed with an aqueous saturated solution of sodium chloride, dried on magnesium sulfate, filtered, and then concentrated under reduced pressure. The residue was subjected to column chromatography on silica gel (hexane-ethyl acetate 10 : 1) to obtain 136.8 mg (11% yield) of the title compound. Melting point: 236-242°C. (Ethyl acetate)
¹H-NMR (CDCl₃) δ: 1.06 (3H, s), 1.47 (3H, s), 1.54 (3H, s), 1.82 (3H, s), 2.19 (3H, s), 2.31 (3H, s), 4.12 (1H, s), 5.85 (2H, s), 6.7-7.1 (8H, m).

### Example 15a

### 2-[2,2,4,6,7-Pentamethyl-2,3-dihydro-1-benzofuran-5-yl]isoindoline

By using 2-[2,2,4,6,7-pentamethyl-2,3-dihydro-1-benzofuran-5-yl]-1H-isoindole-1,3(2H)-dione, the title compound was synthesized according to Example 2a. Yield: 84%. Melting point: 161-163°C. (Ethanol)
¹H-NMR (CDCl₃) δ: 1.48 (6H, s), 2.08 (3H, s), 2.11 (3H, s), 2.14 (3H, s), 2.93 (2H, s), 4.56 (4H, s), 7.27 (4H, s).

### Example 16a

### 6-(2,2,4,6,7-Pentamethyl-3-phenyl-2,3-dihydro-1-benzofuran-5-yl)-6,7-dihydro-5H-[1,3]dioxolo[4,5-f]-isoindole

To a solution of 2,2,4,6,7-pentamethyl-3-phenyl-2,3-dihydro-1-benzofuran-5-amine (1.00 g, 3.56 mmol) in tetrahydrofuran (30 ml) were added 5,6-bis(chloromethyl)-1,3-benzodioxazole (604 mg, 2.76 mmol), sodium carbonate (1.17 mg, 11.0 mmol) and tetrabutylammonium iodide (700 mg, 1.90 mmol) and the mixture was refluxed with heating for 15 hours. The reaction mixture was cooled down to room temperature and then poured into ice water. The product was extracted twice with ethyl acetate. The combined extracts were washed with an aqueous saturated solution of sodium chloride, dried on magnesium sulfate, filtered, and then concentrated under reduced pressure. The residue was subjected to column chromatography on silica gel (hexane-ethyl acetate 8 : 1) to obtain 853 mg (56% yield) of the title compound. Melting point: 245-248°C. (Ethyl acetate)
¹H-NMR (CDCl₃) δ: 1.01 (3H, s), 1. 52 (3H, s), 1.76 (3H, s), 2.17 (6H, s), 4.12 (1H, s), 4.43 (4H, s), 5.94 (2H, s), 6.68 (2H, s), 6.8-7.3 (5H, m).

### Example 17a

### (+)-5,6-Dimethoxy-2-[2,2,4,6,7-pentamethyl-3-(4-methylphenyl)-2,3-dihydro-1-benzofuran-5-yl]isoindoline

To a solution of (+)-2,2,4,6,7-pentamethyl-3-(4-methylphenyl)-2,3-dihydro-1-benzofuran-5-amine (6.00 g, 20.3 mmol) in tetrahydrofuran (50 ml) was added under an argon atmosphere 4,5-dimethoxyphthalic anhydride (4.43 g, 21.3 mmol) and the mixture was refluxed with heating for 3 hours. The reaction mixture was cooled down to room temperature and then 1-ethyl-3-(3-dimethylaminopropyl)carbodiimide (WSC) hydrochloride (4.67 g, 24.4 mmol) and 1-hydroxy-1H-benzotriazole (HOBT) monohydrate (3.74 g, 24.4 mmol) were added to the mixture. The resulting mixture was refluxed with heating for 14 hours and then cooled down to room temperature. Water and an 8 N aqueous solution of sodium hydroxide were added into the reaction mixture and the product was extracted twice with ethyl acetate. The combined extracts were washed with an aqueous saturated solution of sodium hydrogen carbonate, dried on magnesium sulfate, filtered, and then concentrated under reduced pressure to obtain 8.40 g of (+)-5,6-dimethoxy-2-[2,2,4,6,7-pentamethyl-3-(4-methylphenyl)-2,3-dihydro-1-benzofuran-5-yl]-1H-isoindole-1,3(2H)-dione as a crude product. To a solution of aluminum chloride (13.6 g, 102 mmol) in tetrahydrofuran (60 ml) was added lithium aluminum hydride (3.87 g, 102 mmol) and the resulting mixture was stirred for 10 minutes. To this mixture was added a solution of the above-described crude product in tetrahydrofuran (30 ml) and the resulting mixture was refluxed with heating for 3 hours. The reaction mixture was cooled down to room temperature and water was added into the mixture. The product was extracted twice with ethyl acetate. The combined extracts were washed with a 1 N aqueous solution of sodium hydroxide, dried on magnesium sulfate, filtered, and then concentrated under reduced pressure. The residue was subjected to column chromatography (hexane-ethyl acetate δ : 1) on silica gel to obtain 6.23 g (68% yield) of the title compound. Melting point: 157-159°C. [α]D = +62.3° (c = 0.488, methanol)
¹H-NMR (CDCl₃) δ: 1.02 (3H, s), 1.51 (3H, s), 1.76 (3H, s), 2.17 (3H, s), 2.18 (3H, s), 2.31 (3H, s), 3.87 (6H, s), 4.10 (1H, s), 4.45 (4H, s), 6.70-7.15 (6H, m).

### Example 18a

### (-)-5,6-Dimethoxy-2-[2,2,4,6,7-pentamethyl-3-(4-methylphenyl)-2,3-dihydro-1-benzofuran-5-yl]isoindoline

By using (-)-2,2,4,6,7-pentamethyl-3-(4-methylphenyl)-2,3-dihydro-1-benzofuran-5-amine, the title compound was synthesized according to Example 17a. Yield: 34%. Melting point: 157-159°C. (Ethanol) [α]D = -61.5° (c = 0.501, methanol)
¹H-NMR (CDCl₃) δ: 1.02 (3H, s), 1.51 (3H, s), 1.76 (3H, s), 2.17 (6H, s), 2.31 (3H, s), 3.88 (6H, s), 4.10 (1H, s), 4.45 (4H, s), 6.74-7.10 (6H, m).

### Example 19a

### (+)-5,6-Dimethoxy-2-[2,2,4,6,7-pentamethyl-3-(4-methylphenyl)-2,3-dihydro-1-benzofuran-5-yl]isoindoline hydrochloride

(+)-5,6-Dimethoxy-2-[2,2,4,6,7-pentamethyl-3-(4-methylphenyl)-2,3-dihydro-1-benzofuran-5-yl]isoindoline (296 mg, 0.65 mmol) was dissolved in ethyl acetate (5.0 ml) and then a 4 N solution of hydrogen chloride in ethyl acetate (0.38 ml) was added into this mixture. The solvent was removed under reduced pressure and the residue was crystallized from a mixed solution of ethyl acetate and diethyl ether (1 : 5). The crystals were collected by filtration and washed with a cold mixed solution of ethyl acetate and diethyl ether (1 : 5) to obtain 291 mg (87% yield) of the titled compound as a crystalline product. Melting point: 170-171°C. [α]D = +44.9° (c = 0.495, chloroform)
¹H-NMR (CDCl₃) δ: 1.05 (3H, s), 1.49 (3H, s), 2.03 (3H, br), 2.18 (3H, s), 2.32 (3H, s), 2.45 (3H, br), 3.86 (6H, s), 4.06 (1H, s), 4.60 (2H, br), 5.70 (2H, br), 6.71 (2H, s), 6.80 (2H, br), 7.07 (2H, brd, J = 6.0 Hz).

### Example 20a

### (-)-5,6-Dimethoxy-2-[2,2,4,6,7-pentamethyl-3-(4-methylphenyl)-2,3-dihydro-1-benzofuran-5-yl]isoindoline hydrochloride

By using (-)-5,6-dimethoxy-2-[2,2,4,6,7-pentamethyl-3-(4-methylphenyl)-2,3-dihydro-1-benzofuran-5-yl]isoindoline, the title compound was synthesized according to Example 19a. Yield: 61%. Melting point: 173-175°C. [α]D = -44.4° (c = 0.501, chloroform)
¹H-NMR (CDCl₃) δ: 1.05 (3H, s), 1.49 (3H, s), 2.05 (3H, br), 2.18 (3H, s), 2.31 (3H, s), 2.48 (3H, br), 3.86 (6H, s), 4.06 (1H, s), 4.55 (2H, br), 5.75 (2H, br), 6.71 (2H, s), 6.85 (2H, br), 7.07 (2H, brd, J = 6.0 Hz).

### Example 21a

### (+)-5,6-Dimethoxy-2-[2,2,4,6,7-pentamethyl-3-(4-methylphenyl)-2,3-dihydro-1-benzofuran-5-yl]isoindoline hydrobromide

(+)-5,6-Dimethoxy-2-[2,2,4,6,7-pentamethyl-3-(4-methylphenyl)-2,3-dihydro-1-benzofuran-5-yl]isoindoline (150 mg, 0.327 mmol) was dissolved into a 25% solution of hydrogen bromide in acetic acid and the mixture was concentrated under reduced pressure. The residue was crystallized from methanol to obtain 92 mg (52% yield) of the title compound. Melting point: 174-177°C. [α]D = +40.2° (c = 0.495, methanol)
¹H-NMR (CDCl₃) δ: 1.02 (3H, s), 1.51 (3H, s), 1.76 (3H, s), 2.17 (3H, s), 2.18 (3H, s), 2.31 (3H, s), 3.87 (6H, s), 4.10 (1H, s), 4.45 (4H, s), 6.70-7.15 (6H, m).

### Example 22a

### (-)-5,6-Dimethoxy-2-[2,2,4,6,7-pentamethyl-3-(4-methylphenyl)-2,3-dihydro-1-benzofuran-5-yl]isoindoline hydrobromide

By using (-)-5,6-dimethoxy-2-[2,2,4,6,7-pentamethyl-3-(4-methylphenyl)-2,3-dihydro-1-benzofuran-5-yl]isoindoline, the title compound was synthesized according to Example 21a. Yield: 46%. Melting point: 171-174°C. [α]D = -40.1° (c = 0.498, methanol)
¹H-NMR (CDCl₃) δ: 1.02 (3H, s), 1.51 (3H, s), 1.76 (3H, s), 2.17 (6H, s), 2.31 (3H, s), 3.88 (6H, s), 4.10 (1H, s), 4.45 (4H, s), 6.74-7.10 (6H, m).

### Example 23a

### 5,6-dimethoxy-2-[2,2,4,6,7-pentamethyl-3-(4-methylphenyl)-2,3-dihydro-1-benzofuran-5-yl]-2H-isoindole

To a solution of 5,6-dimethoxy-2-[2,2,4,6,7-pentamethyl-3-(4-methylphenyl)-2,3-dihydro-1-benzofuran-5-yl]-2H-isoindoline(1.83g, 4mmol)in toluene (50 ml) was added 10 % palladium-carbon (water content 50%, 1.83g)and the resulting mixture was stirred at 100°C for 20 minutes under a nitrogen atmosphere.

The catalyst was removed through filtration, and the filtrate was concentrated under reduced pressure. The residue was crystallized from hexane/ethyl acetate (6:1) to obtain the title compound 1.37g (yield: 75%). [α]D= +92.9.°(c= 0.498 chloroform). m.p.:146-147°C.
¹H-NMR (CDCl₃) δ: 1.06 (3H, s), 1.48 (3H, s), 1.55 (3H, s), 1.82 (3H, s), 2.20 (3H, s), 2.32 (3H, s), 3.90 (3H, s), 3.91 (3H, s), 4.13 (1H, s), 6.82 (2H, s), 6.84 (2H, brs), 6.90 (2H, br), 7.07 (2H, brd, J=7.8Hz).

The chemical structures of the compounds obtained in the above-described Examples are shown below.

**Table 1**

| | | | | | | | | |
|---|---|---|---|---|---|---|---|---|
| 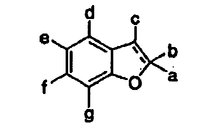 | | | | | | | | |

| example number | a | b | c | d | e | f | g | -̅ -̅ -̅ |
|---|---|---|---|---|---|---|---|---|
| 1a | Me | Me | 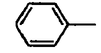 | Me | 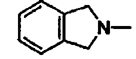 | Me | Me | --- |
| 2a | Me | Me | 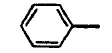 | Me | 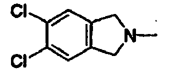 | Me | Me | --- |
| 3a | Me | Me | 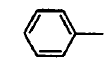 | Me | 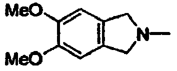 | Me | Me | --- |
| 4a | Me | Me | 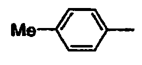 | Me | 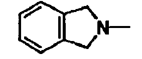 | Me | Me | --- |
| 5a | Me | Me | 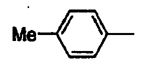 | Me | 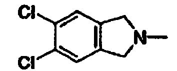 | Me | Me | --- |
| 6a | Me | Me | 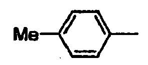 | Me | 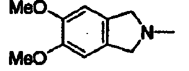 | Me | Me | --- |
| 7a | Me | Me | 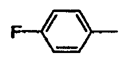 | Me | 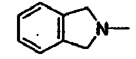 | Me | Me | --- |
| 8a | Me | Me | 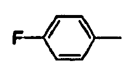 | Me | 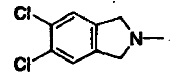 | Me | Me | --- |
| 9a | Me | Me | 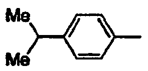 | Me | 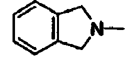 | Me | Me | --- |
| 10a | Me | Me | 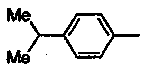 | Me | 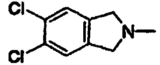 | Me | Me | --- |
| 11a | Me | Me | 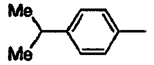 | Me | 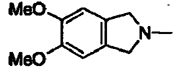 | Me | Me | --- |
| 12a | Me | Me | 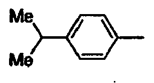 | Me | 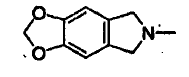 | Me | Me | --- |

**Table 2**

| | | | | | | | | | |
|---|---|---|---|---|---|---|---|---|---|
| 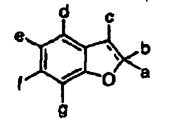 | | | | | | | | | |

| example number | a | b | c | d | e | f | g | -̅ -̅ -̅ | adduct |
|---|---|---|---|---|---|---|---|---|---|
| 13a | Me | - | 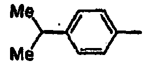 | Me | 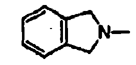 | Me | Me | --- | |
| 14a | Me | Me | 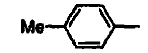 | Me | 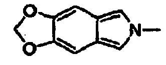 | Me | Me | --- | |
| 15a | Me | Me | H | Me | 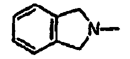 | Me | Me | --- | |
| 16a | Me | Me | 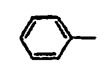 | Me | 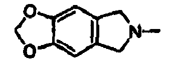 | Me | Me | --- | |
| 17a | Me | Me | 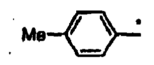 | Me | 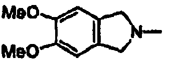 | Me | Me | --- | |
| 18a | Me | Me | 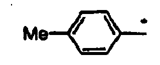 | Me | 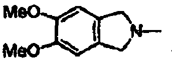 | Me | Me | --- | |
| 19a | Me | Me | 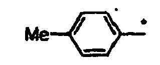 | Me | 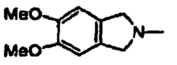 | Me | Me | --- | HCl |
| 20a | Me | Me | 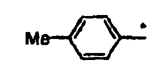 | Me | 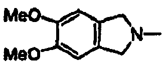 | Me | Me | --- | HCl |
| 21a | Me | Me | 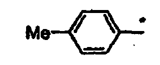 | Me | 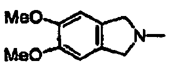 | Me | Me | --- | HBr |
| 22a | Me | Me | 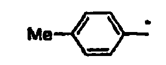 | Me | 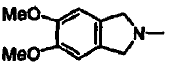 | Me | Me | --- | HBr |
| 23a | Me | Me | 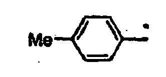 | Me | 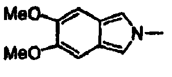 | Me | Me | --- | |

### Formulation Example 1a

| | | |
|---|---|---|
| (1) | The compound obtained in Example 14a | 50 mg |
| (2) | Lactose | 34 mg |
| (3) | Corn starch | 10.6 mg |
| (4) | Corn starch (paste) | 5 mg |
| (5) | Magnesium Stearate | 0.4 mg |
| (6) | Calcium carboxymethyl cellulose | 20 mg |
| | Total | 120 mg |

According to a conventional method, tablets were prepared by mixing the above-described substances (1) to (6), and then subjecting the resulting mixture to a tablet compression process by using a tablet compression machine.

### [Compounds (Ib)]

### Reference Example 1b

### Ethyl 3-(4-isopropylphenyl)-2-methyl-2-propenoate

To a suspension of sodium hydride (a 60% dispersion in liquid paraffin, 5.92 g, 148 mmol) in N,N-dimethylformamide (150 ml) was added triethyl 2-phosphonopropionate (35.0 g, 148 mmol) at 0°C and the resulting mixture was stirred at the same temperature for 10 minutes. To the reaction solution was added 4-isopropylbenzaldehyde (20.0 g, 135 mmol) and the resulting mixture was stirred at room temperature for 30 minutes. Water was added into the reaction solution and the product was extracted twice with ethyl acetate. The combined extracts were washed with water, dried on magnesium sulfate, and then concentrated under reduced pressure to obtain 30.1 g (96% yield) of the oily title compound.
¹H-NMR (CDCl₃) δ: 1.26 (6H, d, J = 7.0Hz) , 1.35 (3H, t, J = 7.0 Hz), 2.13 (3H, s), 2.92 (1H, septet, J = 7.0 Hz), 4.27 (2H, q, J = 7.0 Hz), 7.21-7.38 (4H, m), 7.67 (1H, s).

### Reference Example 2b

### Ethyl 2-methyl-3-(4-methylphenyl)-2-propenoate

By using 4-methylbenzaldehyde, the title compound was synthesized according to Reference Example 1b. Yield: 94%. An oily substance.
¹H-NMR (CDCl₃) δ: 1.34 (3H, t, J = 7 . 0 Hz), 2.12 (3H, d, J = 1.4 Hz), 2.37 (3H, s), 4.26 (2H, q, J = 7.0 Hz), 7.19 (2H, d, J = 8.4 Hz), 7.31 (2H, d, J = 8.4 Hz), 7.66 (1H, s).

### Reference Example 3b

### Ethyl 3-(4-fluorophenyl)-2-methyl-2-propenoate

By using 4-fluorobenzaldehyde, the title compound was synthesized according to Reference Example 1b. Yield: 97%. An oily substance.
¹H-NMR (CDCl₃) δ: 1.35 (3H, t, J = 7.0 Hz), 2.10 (3H, d, J = 1.2 Hz), 4.28 (2H, q, J = 7.0 Hz), 7.08 (2H, t, J = 8.8 Hz), 7.32-7.43 (2H, m), 7.65 (1H, s).

### Reference Example 4b

### Ethyl (E)-3-(4-isopropylphenyl)-2-propenoate

To a suspension of sodium hydride (a 60% dispersion in liquid paraffin, 10.4 g, 260 mmol) in N,N-dimethylformamide, (200 ml) was added triethyl phosphonoacetate (58.2 g, 236 mmol) at 0°C and the resulting mixture was stirred at the same temperature for 10 minutes. To the reaction mixture was added 4-isopropylbenzaldehyde (35.0 g, 260 mmol) and the resulting mixture was stirred at room temperature for 30 minutes. Water was added into the reaction mixture and the product was extracted twice with ethyl acetate. The combined extracts were washed with water, dried on magnesium sulfate, and then concentrated under reduced pressure to obtain 47.5 g (92% yield) of the oily title compound.
¹H-NMR (CDCl₃) δ: 1.25 (6H, d, J = 7.0 Hz), 1.33 (3H, t, J = 7.0 Hz), 2.92 (1H, septet, J = 7.0 Hz), 4.26 (2H, q, J = 7.0 Hz), 6.40 (1H, d, J = 15.8 Hz), 7.24 (2H, d, J = 8.2 Hz), 7.46 (2H, d, J = 8.2 Hz), 7.67 (1H, d, J = 15.8 Hz).

### Reference Example 5b

### Ethyl (E)-3-(4-fluorophenyl)-2-propenoate

By using 4-fluorobenzaldehyde, the title compound was synthesized according to Reference Example 4b. Yield: 88%. An oily substance.
¹H-NMR (CDCl₃) δ: 1.34 (3H, t, J = 7.0 Hz), 4.26 (2H, q, J = 7.0 Hz), 6.31 (1H, d, J = 15.8 Hz), 7.00-7.11 (2H, m), 7.43-7.58 (2H, m), 7.67 (1H, d, J = 15.8 Hz).

### Reference Example 6b

### 3-(4-Isopropylphenyl)-2-methyl-2-propen-1-ol

To a suspension of ethyl 3-(4-isopropylphenyl)-2-methyl-2-propenoate (9.00 g, 38.7 mmol) and cerium chloride (1.00 g, 4.06 mmol) in tetrahydrofuran (50 ml) was added lithium aluminum hydride (1.47 g, 38.7 mmol) in four portions at -40°C for 30 minutes and the resulting mixture was stirred at the same temperature for 30 minutes. Water was added into the reaction mixture and the product was extracted twice with ethyl acetate. The combined extracts were washed with water, dried on magnesium sulfate, and then concentrated under reduced pressure. The residue was subjected to column chromatography on silica gel (hexane-ethyl acetate 8 : 1) to obtain 6.30 g (86% yield) of the oily title compound.
¹H-NMR (CDCl₃) δ: 1.25 (6H, d, J = 7.0 Hz), 1.91 (3H, d, J = 1.4 Hz), 2.90 (1H, septet, J = 7.0 Hz), 4.17 (2H, d, J = 0.8 Hz), 6.49 (1H, dd, J = 2.6, 1.4 Hz), 7.15-7.25 (4H, m), 1H unidentified.

### Reference Example 7b

### 2-Methyl-3-(4-methylphenyl)-2-propen-1-ol

By using ethyl 2-methyl-3-(4-methylphenyl)-2-propenoate, the title compound was synthesized according to Reference Example 6b. Yield: 98%. An oily substance.
¹H-NMR (CDCl₃) δ: 1.87 (3H, s), 2.32 (3H, s), 4.13 (2H,s), 6.46 (1H, s), 7.08-7.22 (4H, m), 1H unidentified.

### Reference Example 8b

### 3-(4-Fluorophenyl)-2-methyl-2-propen-1-ol

By using ethyl 3-(4-fluorophenyl)-2-methyl-2-propenoate, the title compound was synthesized according to Reference Example 6b. Yield: 95%. An oily substance.
¹H-NMR (CDCl₃) δ: 1. 98 (3H, d, J = 1.6 Hz), 4.11 (2H, s), 6.58 (1H, s), 7.01 (2H, t, J = 8.8 Hz), 7.18-7.28 (2H, m), 1H unidentified.

### Reference Example 9b

### (E)-3-(4-Isopropylphenyl)-2-propen-1-ol

To a suspension of ethyl (E)-3-(4-isopropylphenyl)-2-propenoate (20.0 g, 91.6 mmol) in tetrahydrofuran (200 ml) was added lithium aluminum hydride (2.61 g, 68.7 mmol) in four portions at -40°C for 30 minutes and the resulting mixture was stirred at the same temperature for 30 minutes. Water was added into the reaction solution and the product was extracted twice with ethyl acetate. The combined extracts were washed with water, dried on magnesium sulfate, and then concentrated under reduced pressure. The residue was subjected to column chromatography on silica gel (hexane-ethyl acetate 8 : 1) to obtain 10.5 g (65% yield) of the oily title compound.
¹H-NMR (CDCl₃) δ: 1.24 (6H, d, J = 7.0 Hz), 2.79-3.00 (2H, m), 4.30 (2H, d, J = 5. 6 Hz), 6.35 (1H, dt, J = 15.8, 5.6 Hz), 6.59 (1H, d, J = 15.8 Hz), 7.10-7.39 (4H, m).

### Reference Example 10b

### (E)-3-(4-Fluorophenyl)-2-propen-1-ol

By using ethyl (E)-3-(4-fluorophenyl)-2-propenoate, the title compound was synthesized according to Reference Example 6b. Yield: 84%. An oily substance.
¹H NMR (CDCl₃) δ: 4.31 (2H, d, J = 5.6 Hz), 6.28 (1H, dt, J = 15.8, 5.6 Hz), 6.59 (1H, d, J = 15.8 Hz), 6.90-7.40 (4H, m), 1H unidentified.

### Reference Example 11b

### 1-(3-Bromo-2-methyl-1-propenyl)-4-isopropylbenzene

To a solution of 3-(4-isopropylphenyl)-2-methyl-2-propen-1-ol (6.30 g, 33.1 mmol) in isopropyl ether (50 ml) was added phosphorus tribromide (5.98 g, 22.1 mmol) under ice cooling and the resulting mixture was stirred at room temperature for 30 minutes. Water was added into the reaction mixture and the product was extracted with isopropyl ether. The organic layer was washed with water and an aqueous saturated solution of sodium hydrogen carbonate, dried on magnesium sulfate, filtered, and then concentrated under reduced pressure to obtain 7.63 g (91% yield) of the oily title compound.
¹H-NMR (CDCl₃) δ: 1.25 (6H, d, J = 7.0 Hz), 2.03 (3H, d, J = 1.4 Hz ) , 2.90 (1H, septet, J = 7.0 Hz) , 4.15 (2H, d, J =0.8 Hz), 6,62 (1H, s), 7.14-7.26 (4H, m).

### Reference Example 12b-

### 1-(3-Bromo-2-methyl-1-propenyl)-benzene

By using 2-methyl-3-phenyl-2-propen-l-ol, the title compound was synthesized according to Reference Example 11b. Yield: 89%. An oily substance.
¹H-NMR (CDCl₃) δ: 2.01 (3H, d, J = 1.4 Hz), 4.13 (2H, d, J = 0.8 Hz), 6.64 (1H, s), 7.19-7.44 (5H, m).

### Reference Example 13b

### 1-(3-Bromo-2-methyl-1-propenyl)-4-methylbenzene

By using 2-methyl-3-(4-methylphenyl)-2-propen-1-ol, the title compound was synthesized according to Reference Example 11b. Yield: 77%. An oily substance.
¹H-NMR (CDCl₃) δ: 2.01 (3H, s), 2.34 (3H, s), 4.13(2H, s), 6,60 (1H, s), 7.09-7.22 (4H, m).

### Reference Example 14b

### 1-(3-Bromo-2-methyl-1-propenyl)-4-fluorobenzene

By using 3-(4-fluorophenyl)-2-methyl-2-propen-1-ol, the title compound was synthesized according to Reference Example 11b. Yield: 79%. An oily substance.
¹H-NMR (CDCl₃) δ: 1.87 (3H, s), 4.17 (2H, s), 6.48 (1H, s), 7.01 (2H, t, J = 8.8 Hz), 7.18-7.27 (2H, m).

### Reference Example 15b

### 1-[(E)-3-Bromo-1-propenyl]-4-isopropylbenzene

To a solution of (E)-3-(4-isopropylphenyl)-2-propen-1-ol (10.5 g, 59.6 mmol) in isopropyl ether (100 ml) was added phosphorus tribromide (10.7 g, 39.7 mmol) under ice cooling and the resulting mixture was stirred at room temperature for 30 minutes. Water was added into the reaction solution and the product was extracted with isopropyl ether. The organic layer was washed with water and an aqueous saturated solution of sodium hydrogen carbonate, dried on magnesium sulfate, filtered, and then concentrated under reduced pressure to obtain 10.2 g (72% yield) of the oily title compound.
¹H-NMR (CDCl₃) δ: 1.24 (6H, d, J = 7.0 Hz), 2.89 (1H, septet, J = 7.0 Hz), 4.16 (2H, dd, J = 7.8, 0.8 Hz), 6.35 (1H, dt, J = 15.4, 7.8 Hz), 6.63 (1H, d, J = 15.4 Hz), 7.14-7.35 (4H, m).

### Reference Example 16b

### 1-[(E)-3-Bromo-1-propenyl]-4-fluorobenzene

By using (E)-3-(4-fluorophenyl)-2-propen-1-ol, the title compound was synthesized according to Reference Example 11b. Yield: 61%. An oily substance.
¹H-NMR (CDCl₃) δ: 4.15 (2H, d, J = 7.6 Hz), 6.30 (1H, dt, J = 15.4, 7.6 Hz), 6.61 (1H, d, J = 15.4 Hz), 6.83-7.08 (2H, m), 7.31-7.45 (2H, m).

### Reference Example 17b

### N-[4-[[3-(4-Isopropylphenyl)-2-methyl-2-propenyl]oxy]-2,3,6-trimethylphenyl]formamide

To a solution of N-(4-hydroxy-2,3,6-trimethylphenyl)formamide (3.00 g, 16.7 mmol) in N,N-dimethylformamide (30 ml) was added sodium hydride (a 60% dispersion in liquid paraffin, 0.74 g, 18.4 mmol) at 0°C under a nitrogen atmosphere and the resulting mixture was stirred at the same temperature for 10 minutes. To the reaction solution was added 1-(3-bromo-2-methyl-1-propenyl)-4-isopropylbenzene (4.66 g, 18.4 mmol) and the resulting mixture was stirred at room temperature for 30 minutes. Water was added into the reaction solution and the product was extracted twice with ethyl acetate. The combined extracts were washed with water, dried on magnesium sulfate, and then concentrated under reduced pressure. The resulting residue was crystallized from ethyl acetate-hexane to obtain 3.70 g (63% yield) of the title compound. Melting point: 153-155°C.
¹H-NMR (CDCl₃) δ: 1.26 (6H, d, J = 7.0 Hz), 2.00 (3H, s), 2.07-2.34 (9H, m), 2.91 (1H, septet, J = 7.0 Hz), 4.54 (2H, q, J = 7.0 Hz), 6.59-6.84 (3H, m), 7.17-7.36 (4H, m), 7.98 (0.5H, d, J = 12.0 Hz), 8.41 (0.5H, s).

### Reference Example 18b

### N-[2,3,6-Trimethyl-4-[(2-methyl-3-phenyl-2-propenyl)oxy]phenyl]formamide

By using N-(4-hydroxy-2,3,6-trimethylphenyl)formamide and 1-(3-bromo-2-methyl-1-propenyl)benzene, the title compound was synthesized according to Reference Example 17b. Yield: 41%. Melting point: 152-154°C. (Ethyl acetate-hexane)
¹H-NMR (CDCl₃) δ: 1.98 (3H, d, J = 1.60 Hz) ; 2.10-2.32 (9H, m), 4.54 (2H, d, J = 5.2 Hz), 6.65 (1H, s), 6.67 (1H, s), 6.69-6.90 (1H, m), 7.11-7.41 (5H, m), 7.98 (0.5H, d, J = 12.0 Hz), 8.41 (0.5H, d, J = 1.4 Hz).

### Reference Example 19b

### N-[2,3,6-Trimethyl-4-[[2-methyl-3-(4-methylphenyl)-2-propenyl]oxy]phenyl]formamide

By using N-(4-hydroxy-2,3,6-trimethylphenyl)formamide and 1-(3-bromo-2-methyl-1-propenyl)-4-methylbenzene, the title compound was synthesized according to Reference Example 17b. Yield: 57%. Melting point: 167-169°C. (Ethyl acetate-hexane)
¹H-NMR (CDCl₃) δ: 1.98 (3H, s), 2.07-2.38 (9H, m), 2.35 (3H, s), 4.53 (2H, d, J = 6.6 Hz), 6.61 (1H, s), 6.66 (1H, d, J = 2.4 Hz), 6.82-7.09 (1H, m), 7.11-7.31 (4H, m) 7.98 (0.5H, d, J = 12.2 Hz), 8.38 (0.5H, s).

### Reference Example 20b

### N-[4-[[3-(4-Fluorophenyl)-2-methyl-2-propenyl]oxy]-2,3,6-trimethylphenyl]formamide

By using N-(4-hydroxy-2,3,6-trimethylphenyl)formamide and 1-(3-bromo-2-methyl-1-propenyl)-4-fluorobenzene, the title compound was synthesized according to Reference Example 17b. Yield: 52%. Melting point: 164-165°C. (Ethyl acetate-hexane)
¹H-NMR (CDCl₃) δ: 1.96 (3H, s), 2.12-2.32 (9H, m), 4.53 (2H, d, J = 5.2 Hz), 6.60 (1H, s), 6.66 (1H, s), 6.71-6.95 (1H, m), 7.04 (2H, t, J = 8.8 Hz), 7.22-7.33 (2H, m), 8.04 (0.5H, d, J = 12.0 Hz), 8.40 (0.5H, d, J = 1.4 Hz).

### Reference Example 21b

### N-[4-[[(E)-3-(4-Isopropylphenyl)-2-propenyl]oxy]-2,3,6- trimethylphenyl]formamide

To a solution of N-(4-hydroxy-2,3,6-trimethylphenyl)formamide (5.20 g, 29.0 mmol) in N,N-dimethylformamide (30 ml) was added sodium hydride (a 60% dispersion in liquid paraffin, 1.39 g, 3.4.8 mmol) at 0°C under a nitrogen atmosphere and the resulting mixture was stirred at the same temperature for 10 minutes. To the reaction solution was added 1-[(E)-3-bromo-1-propenyl]-4-isopropylbenzene (9.00 g, 37.7 mmol) and the resulting mixture was stirred at room temperature for 30 minutes. Water was added into the reaction solution and the product was extracted twice with ethyl acetate. The combined extracts were washed with water, dried on magnesium sulfate, and then concentrated under reduced pressure. The resulting residue was crystallized from ethyl acetate-hexane to obtain 5.80 g (59% yield) of the title compound. Melting point: 165-167°C.
¹-H-NMR (CDCl₃) δ: 1.25 (6H, d, J = 6.8 Hz), 2.13-2.27 (9H, m), 2.90 (1H, septet, J = 6.8 Hz), 4.66 (2H, t, J = 5.8 Hz), 6.37 (1H, dt, J = 15.8, 5.8 Hz), 6.65-6.88 (3H, m), 7.16-7.26 (2H, m), 7.35 (2H, d, J = 8.0 Hz), 7.98 (0.5H, d, J = 12.0 Hz), 8.40 (0.5H, d, J = 1.4 Hz).

### Reference Example 22b

### N-[2,3,6-Trimethyl-4-[[(E)-3-phenyl-2-propenyl)oxy]-phenyl]formamide

By using N-(4-hydroxy-2,3,6-trimethylphenyl)formamide and cinnamyl chloride, the title compound was synthesized according to Reference Example 17b. Yield: 44%. Melting point: 197-199°C. (Ethyl acetate-hexane)
¹H-NMR (CDCl₃) δ: 2.05-2.18 (9H, m), 4.62-4.72 (2H, m), 6.35-6.50 (1H, m), 6.62-7.00 (3H, m), 7.24-7.52 (5H, m), 8.00 (0.5H, d,J = 12.0 Hz), 8.39 (0.5H, d, J = 1.6 Hz).

### Reference Example 23b

### N-[4-[[(E)-3-(4-Fluorophenyl)-2-propenyl]oxy]-2,3,6-trimethylphenyl]formamide

By using N-(4-hydroxy-2,3,6-trimethylphenyl)formamide and 1-[(E)-3-bromo-1-propenyl]-4-fluorobenzene, the title compound was synthesized according to Reference Example 17b. Yield: 52%. Melting point: 196-198°C. (Ethyl acetate-hexane)
¹H-NMR (CDCl₃) δ: 2.10-2.32 (9H, m), 4.67 (2H, t, J = 5.0 Hz), 6.37(1H, dt, J = 15.6, 5.0 Hz), 6.59-6.89 (3H, m), 6.92-7.09 (2H, m), 7.32-7.43 (2H, m), 7.99 (0.5H, d, J = 12.0 Hz), 8.42 (0.5H, d, J = 1.4 Hz).

### Reference Example 24b

### N-[4-Hydroxy-3-[1-(4-isopropylphenyl)-2-propenyl]-2,5,6- trimethylphenyl]formamide

A solution of N-[4-[[(E)-3-(4-isopropylphenyl)-2-propenyl]oxy]-2,3,6-trimethylphenyl]formamide (5.80 g, 17.2 mmol) in N,N-dimethylaniline (50 ml) was stirred at 215°C for 6 hours under argon atmosphere. The reaction mixture was cooled down, diluted with ethyl acetate, washed with 2 N hydrochloric acid and water, dried on magnesium sulfate, and then concentrated under reduced pressure. The residue was crystallized from ethyl acetates to obtain 3.50 g (60% yield) of the title compound. Melting point: 170-171°C.
¹H-NMR (CDCl₃) δ: 1.18-1.40 (6H, m), 2.11-2.27 (9H, m), 2.77-3:00 (1H, m), 5.00-5.22 (2H, m), 5.30-5.42 (1H, m), 6.30-6.85 (2H, m), 7.10-7.37 (5H, m), 7.97 (0.5H, d, J = 12.2 Hz), 8.43 (0.5H, d, J = 1.4 Hz).

### Reference Example 25b

### N-[4-Hydroxy-3-[1-phenyl-2-propenyl]-2,5,6-trimethylphenyl]formamide

By using N-[2,3,6-trimethyl-4-[[(E)-3-phenyl-2-propenyl]oxy]phenyl]formamide, the title compound was synthesized according to Reference Example 24b. Yield: 78%. Melting point: 144-145°C. (Ethyl acetate)
¹H-NMR (CDCl₃) δ: 2.08-2.27 (9H, m), 5.02-5.41 (3H, m), 6.32-6.52 (1H, m), 6.61-7.03 (2H, m), 7.18-7.42 (5H, m), 7.95 (0.5H, d, J = 12.0 Hz), 8.42 (0.5H, d, J = 1.8 Hz).

### Reference Example 26b

### N-[4-Hydroxy-3-[1-(4-fluorophenyl)-2-propenyl]-2,5,6-trimethylphenyl]formamide

By using N-[4-[[(E)-3-(4-fluorophenyl)-2-propenyl]oxy]-2,3,6-trimethylphenyl]formamide, the title compound was synthesized according to Reference Example 24b. Yield: 66%. Melting point: 168-170°C. (Ethyl acetate)
¹H-NMR (CDCl₃) δ: 2.10-2.29 (9H, m), 5.02-5.22 (1.5H, m), 5.33-5.50 (1.5H, m), 6.35-6.55 (1H, m), 6.72-7.08 (4H, m), 7.18-7.30 (2H, m), 7.96 (0.5H, d, J = 12.2 Hz), 8.42 (0.5H, d, J = 1.4 Hz).

### Reference Example 27b

### 3-(4-Isopropylphenyl)-2,2,4,6,7-pentamethyl-2,3-dihydro-1-benzofuran-5-amine

A solution of N-[4-[[3-(4-isopropylphenyl)-2-methyl-2-propenyl]oxy]-2,3,6-trimethylphenyl]formamide (3.70 g, 10.5 mmol) in N,N-dimethylaniline (20 ml) was stirred at 215°C for 6 hours under an argon atmosphere. The reaction mixture was cooled down, then diluted with ethyl acetate, washed with 2 N hydrochloric acid and water, dried on magnesium sulfate, and then concentrated under reduced pressure to obtain N-[4-hydroxy-3-[1-(4-isopropylphenyl)-2-methyl-2-propenyl]-2,5,6-trimethylphenyl]formamide as a crude product. A mixture of this compound (2.98 g, 8.47 mmol), concentrated hydrochloric acid (20 ml) and methanol (60 ml) was refluxed with heating for 2 hours under a nitrogen atmosphere. The solvent was concentrated under reduced pressure and the resulting residue was neutralized with an 8 N aqueous solution of sodium hydroxide. The product was extracted twice with ethyl acetate. The combined extracts were washed with water, dried on magnesium sulfate, and then concentrated under reduced pressure. The resulting residue was crystallized from isopropyl ether-hexane to obtain 2.23 g (66% yield) of the title compound. Melting point: 130-132°C.
¹H-NMR (CDCl₃) δ: 1.00 (3H, s), 1.21 (6H, d, J = 6.6 Hz), 1.47 (3H, s), 1.78 (3H, s), 2.12 (3H, s), 2.19 (3H, s), 2.40-2.60 (3H, m), 4.08 (1H, s), 6.72-7.00 (2H, m), 7.07 (2H, d, J = 8.0 Hz).

### Reference Example 28b

### 2,2,4,6,7-Pentamethyl-3-phenyl-2,3-dihydro-1-benzofuran-5-amine

By using N-[2,3,6-trimethyl-4-[(2-methyl-3-phenyl-2-propenyl)oxy]phenyl]formamide, the title compound was synthesized according to Reference Example 27b. Yield: 67%. Melting point: 129-131°C. (Petroleum ether)
¹H-NMR (CDCl₃) δ: 1.00 (3H, s), 1.48 (3H, s), 1.77(3H, s), 2.13 (3H, s), 2.19 (3H, s), 3.20 (2H, br s), 4.12 (1H, s), 6.70-7.30 (5H, m).

### Reference Example 29b

### 2,2,4,6,7-Pentamethyl-3-(4-methylphenyl)-2,3-dihydro-1-benzofuran-5-amine

By using N-[2,3,6-trimethyl-4-[[2-methyl-3-(4-methylphenyl)-2-propenyl]oxy]phenyl]formamide, the title compound was synthesized according to Reference Example 27b. Yield: 62%. Melting point: 114-115°C. (Petroleum ether)
¹H-NMR (CDCl₃) δ: 0.99 (3H, s), 1.47 (3H, s), 1.77 (3H, s), 2.12 (3H, s), 2.19 (3H, s), 2.30 (3H, s), 3.23 (2H, br s), 4.08 (1H, s), 6.60-7.23 (4H, m).

### Reference Example 30b

### 3-(4-Fluorophenyl)-2,2,4,6,7-pentamethyl-2,3-dihydro-1-benzofuran-5-amine

By using N-[4-[[3-(4-fluorophenyl)-2-methyl-2-propenyl]oxy]-2,3,6-trimethylphenyl]formamide, the title compound was synthesized according to Reference Example 27b. Yield: 78%. Melting point: 125-127°C. (Petroleum ether)
¹H-NMR (CDCl₃) δ: 0.99 (3H, s), 1.47 (3H, s), 1.77(3H, s), 2.12 (3H, s), 2.19 (3H, s), 3.10 (2H, br s), 4.09 (1H, s), 6.62-7.20 (4H, m).

### Reference Example 31b

### 3-(4-Isopropylphenyl)-2,4,6,7-tetramethyl-1-benzofuran-5-amine hydrochloride

To a suspension of N-[4-hydroxy-3-[1-(4-isopropylphenyl)-2-propenyl]-2,5,6-trimethylphenyl]formamide (3.50 g, 10.4 mmol) and calcium carbonate (1.35 g, 13.5 mmol) in a mixed solvent of tetrahydrofuran (15 ml) and methanol (15 ml) was gradually added benzyltrimethylammonium iododichloride (3.90 g, 11.4 mmol). The reaction solution was stirred at room temperature for 30 minutes. After filtration of the insoluble substances, the solvent was concentrated under reduced pressure. Ethyl acetate and water were added to the residue. The organic layer was separated and the aqueous layer was extracted twice with ethyl acetate. The combined organic layers were successively washed with a 10% aqueous solution of sodium hydrosulfite, water, an aqueous, saturated solution of sodium hydrogen carbonate and an aqueous saturated solution of sodium chloride, dried on magnesium sulfate, and then concentrated under reduced pressure to obtain 4.08 g of N-[2-iodomethyl-3-(4-isopropylphenyl)-4,6,7-trimethyl-2,3- dihydro-1-benzofuran-5-yl]formamide. A solution of this compound (4.08 g, 8.81 mmol) and 1,8-diazabicyclo[5,4,0]-7-undecene (6.58 m, 44.0 mmol) in toluene (30 ml) was stirred at 100°C for 3 hours under an argon atmosphere. Water was added into the reaction solution and the product was extracted twice with ethyl acetate. The combined extracts were washed with 2 N hydrochloric acid and water, dried on magnesium sulfate, and then concentrated under reduced pressure. The residue was subjected to column chromatography on silica gel (hexane-ethyl acetate 20 : 1) to obtain 2.40 g of N-[3-(4-isopropylphenyl)-2,4,6,7-tetramethyl-1-benzofuran-5-yl]formamide. A mixture of this compound (2.40 g, 7.18 mmol), concentrated hydrochloric acid (20 ml) and methanol (60 ml) was refluxed with heating for 2 hours under a nitrogen atmosphere. The solvent was concentrated under reduced pressure and the resulting residue was neutralized with an 8 N aqueous solution of sodium hydroxide. The product was extracted twice with ethyl acetate. The combined extracts were washed with water, dried on magnesium sulfate, and then concentrated under reduced pressure to obtain 1.80 g of an oily free base. The free base (0.50 g, 1.63 mmol) was dissolved into a solution of hydrochloric acid in methanol and the solvent was concentrated under reduced pressure. The resulting residue was crystallized from methanol to obtain 0.41 g (yield 41%) of the title compound.
¹H-NMR (CDCl₃) δ: 1.29 (6H, d, J = 7.0 Hz), 2.30 (6H, s), 2.41 (3H, s), 2.60 (3H, s), 2.94 (1H, septet, J = 7.0 Hz), 7.13-7.26 (4H, m), 10.1 (2H, br s), 1H unidentified.

### Reference Example 32b

### 2,4,6,7-Tetramethyl-3-phenyl-1-benzofuran-5-amine hydrochloride

By using N-[4-hydroxy-3-(1-phenyl-2-propenyl)-2,5,6-trimethylphenyl]formamide, the title compound was synthesized according to Reference Example 31b. Yield: 26%. Melting point: 189-192°C. (Ethanol-hexane)
¹H-NMR (CDCl₃) δ: 2.30 (6H, s), 2.42 (3H, s), 2.60(3H, s), 7.21-7.37 (5H, m), 10.2 (2H, br s), 1H unidentified.

### Reference Example 33b

### 3-(4-Fluorophenyl)-2,4,6,7-tetramethyl-1-benzofuran-5-amine hydrochloride

By using N-[4-hydroxy-3-[1-(4-fluorophenyl)-2-propenyl]-2,5,6-trimethylphenyl]formamide, the title compound was synthesized according to Reference Example 31b. Yield: 87%. Melting point: 208-210°C. (Ethanol)
¹H-NMR (CDCl₃) δ: 2.29 (6H, s), 2.42 (3H, s), 2.60 (3H, s), 7.03-7.28 (4H, m), 10.2 (2H, br s), 1H unidentified.

### Reference Example 34b

### (1-Benzyl-4-piperidyl) (4-isopropylphenyl) (3;4,6-trimethyl-2-methoxyphenyl)methanol

To a solution of 2-methoxy-3,4,6-trimethylbromobenzene (15.48 g, 67.56 mmol) in tetrahydrofuran (200 ml), which was kept at -78°C, was added dropwise a solution of n-butyllithium in hexane (1.59 mol/l, 42 ml, 66.78 mmol) under an argon atmosphere and the mixture was stirred for 30 minutes. To this mixture was added dropwise a solution of 1-benzyl-4-(4-isopropylbenzoyl)piperidine (19.81 g, 61.63 mmol) in tetrahydrofuran (50 ml) and the resulting mixture was stirred at room temperature for 30 minutes. Water was added to the reaction mixture and the product was extracted with ethyl acetate. The extracts were washed with an aqueous saturated solution of sodium chloride, dried on magnesium sulfate, filtered, and then concentrated under reduced pressure. The residue was crystallized from ethyl acetate-hexane to obtain 23.01 g (79% yield) of the title compound. Melting point: 154-156°C
¹H-NMR (CDCl₃) δ: 1.18 (6H, d, J = 7.0 Hz), 1.40-1.47 (2H, m), 1.85-1.96 (4H, m), 2.07 (3H, s), 2.17 (3H, s), 2.26 (1H, m), 2.39 (3H, s), 2.57-2.94 (6H, m), 3.48 (2H, s), 6.18 (1H, br), 6.72 (1H, s), 7.08-7.12 (2H, d, J = 8.0 Hz), 7.12-7.34 (7H, m).

### Reference Example 35b

### 1'-Benzyl-3-(4-isopropylphenyl)-4,6,7-trimethylspiro[benzofuran-2(3H),4'-piperidine]

To a solution of (1-benzyl-4-piperidyl)(4-isopropylphenyl)(3,4,6-trimethyl-2-methoxyphenyl)methanol (5.61 g, 11.89 mmol) in acetic acid (40 ml) was added a 47% hydrobromic acid (50 ml) and the resulting mixture was refluxed with heating for 13 hours. The reaction mixture was cooled down to room temperature and then an 8 N aqueous solution of sodium hydroxide was added into the mixture until it became basic. The product was extracted with ethyl acetate. The extracts were washed with an aqueous saturated solution of sodium chloride, dried on magnesium sulfate, filtered, and then concentrated under reduced pressure. The residue was crystallized from hexane to obtain 4.44 g (76% yield) of the title compound. Melting point: 125-128°C.
¹H-NMR (CDCl₃) δ: 1.20 (6H, d, J = 6.8 Hz), 1.36-1.40 (2H, m), 1.72-1.95 (5H, m), 2.17 (3H, s), 2.23 (3H, s), 2.29-2.91 (5H, m), 3.52 (2H, s), 4.04 (1H, s), 6.48 (1H, m), 6.6-7.2 (4H, m), 7.22-7.32 (5H, m).

### Reference Example 36b

### 3-(4-Isopropylphenyl)-4,6,7-trimethylspiro[benzofuran-2(3H), 4'-piperidine] hydrochloride

To a solution of 1'-benzyl-3-(4-isopropylphenyl)-4,6,7-trimethylspiro[benzofuran-2(3H),4'-piperidine] (10.26 g, 23.34 mmol) in tetrahydrofuran (100 ml) was added α-chloroethyl chloroformate (3.76 g, 26.60 mmol) and the resulting mixture was refluxed with heating for 1 hour. The reaction mixture was cooled down to room temperature and concentrated under reduced pressure. Methanol (80 ml) was added into the resulting residue and the mixture was refluxed for 1 hour. The reaction mixture was cooled down to room temperature and concentrated under reduced pressure. The residue was crystallized from ethanol to obtain 7.32 g (81% yield) of the title compound. Melting point: > 260°C (decomposed).
¹H-NMR (DMSO-d₆ ) δ: 1.17 (6H, d, J = 7.0 Hz), 1.29-1.67 (2H, m), 1.77 (3H, s), 1.95-2.05(2H, m), 2.11 (3H, s), 2.18 (3H, s), 2.78-3.28 (5H, m), 4.31 (1H, s), 6.50 (1H, s), 6.6-7.2 NH, m), 2H unidentified.

### Reference Example 37b

### 3-(4-Isopropylphenyl)-1',4,6,7-tetramethylspiro[benzofuran-2(3H),4'-piperidine]

To a suspension of 3-(4-isopropylphenyl)-4,6,7-trimethylspiro[benzofuran-2(3H),4'-piperidine]hydrochloride (389.6 mg, 1.01 mmol) in acetonitrile (5 ml) was added 37% formalin (2.0 ml) and the mixture was cooled to 0°C. Sodium cyanoborohydride (101.8 mg, 1.62 mmol) was added into this mixture and the mixture was stirred at room temperature for 1 hour. The reaction mixture was concentrated under reduced pressure and an aqueous saturated solution of sodium hydrogen carbonate was added to the residue. The product was extracted twice with ethyl acetate. The combined extracts were washed with water, dried on magnesium sulfate, filtered, and then concentrated under reduced pressure. The residue was subjected to column chromatography (on Chromatorex NHDM1020 (trade name, manufactured by Fuji Silysia Chemical Ltd.); hexane-ethyl acetate 10 : 1) to obtain 145.0 mg (40% yield of the title compound. Melting point: 63-64°C. (Petroleum ether).
¹H-NMR (CDCl₃) δ: 1.21 (6H, d, J = 7.0 Hz), 1.34-1.41 (2H, m), 1.84 (3H, s), 1.87-1.97 (2H, m), 2.04 (3H, s), 2.17 (3H, s), 2.30 (3H, s), 2.32-2.69 (4H, m), 2.85 (1H, septet, J = 7.0 Hz), 4.05 (1H, s), 6.48 (1H, s), 6.6-7.2 (4H, m).

### Reference Example 38b

### 3-(4-Isopropylphenyl)-1',4,6,7-tetramethylspiro[benzofuran-2(3H),4'-piperidine]-5-amine

A solution of nitrosyl tetrafluoroborate (470.7 mg, 4.03 mmol) in acetonitrile (40 ml) was cooled to 0°C. To the solution was added a solution of 3-(4-isopropylphenyl)-1',4,6,7-tetramethylspiro[benzofuran-2(3H),4'-piperidine] (479.1 mg, 1.32 mmol) in acetonitrile (10 ml) and the mixture was stirred for 20 minutes. The reaction mixture was poured into ice water and basified with an 8 N aqueous solution of sodium hydroxide. The product was extracted twice with ethyl acetate. The combined extracts were washed with an aqueous saturated solution of sodium hydrogen carbonate, dried on magnesium sulfate, filtered, and then concentrated under reduced pressure. The residue was dissolved into ethanol (20 ml) and palladium-carbon (59.9 mg) was added into the solution, then the mixture was stirred at 60°C for 18 hours under a hydrogen atmosphere. The reaction mixture was cooled down to room temperature, filtered to remove insoluble materials, and then concentrated under reduced pressure. The residue was subjected to column chromatography (on Chromatorex NHDM1020 (trade name, manufactured by Fuji Silysia Chemical Ltd.); hexane-ethyl acetate 3 : 1) to obtain 402.0 mg (83% yield) of the title compound. Melting point: 123-124°C. (Hexane).
¹H-NMR (CDCl₃) δ: 1.10-1.38 (8H, m), 1.69-2.04 (5H, m), 2.12 (3H, s), 2.22 (3H, m), 2.25-2.51 (7H, m), 2.84 (1H, septet, J = 6.6 Hz), 3.23 (2H, br), 4.05 (1H, s), 6.6-7.1 (4H, m).

### Reference Example 39b

### 4-Methoxy-2,3,6-trimethylaniline

N-(4-Hydroxy-2,3,6-trimethylphenyl)formamide (30.0 g, 167 mmol) was dissolved into a mixed solvent comprising of a 4 N aqueous solution of potassium hydroxide (100 ml) and methanol (300 ml) and then dimethyl sulfate (42.0 g, 334 mmol) was added into the resulting solution. The mixture was refluxed with heating for 14 hours. The reaction mixture was cooled down and the crystals precipitated were collected by filtration to obtain N-(4-methoxy-2,3,6-trimethylphenyl)formamide as a crude product. To a suspension of this compound in methanol (200 ml) was added concentrated hydrochloric acid (50 ml) and the mixture was refluxed with heating for 3 hours. The reaction mixture was cooled down to room temperature and then neutralized with an 8 N aqueous solution of sodium hydroxide. The product was extracted twice with ethyl acetate. The combined extracts were washed with a 10% aqueous solution of sodium hydrosulfite and water, dried on magnesium sulfate, and then concentrated under reduced pressure. The residue was crystallized from isopropyl ether to obtain 21.0 g (76% yield) of the title compound. Melting point: 70-72°C.
¹H-NMR (CDCl₃) δ: 2.11 (3H, s), 2.16 (3H, s), 2.18 (3H, s), 3.16 (1H, br s), 3.74 (3H, s), 6.54 (1H, s).

### Reference Example 40b

### Tert-butyl 4-methoxy-2,3,6-trimethylphenylcarbamate

To a solution of 4-methoxy-2,3,6-trimethylaniline (21.0 g, 127 mmol) and triethylamine (21.0 ml, 152 mmol) in tetrahydrofuran (150 ml) was added di-tert-butyl dicarbonate (32 ml, 140 mmol) at room temperature and the resulting mixture was refluxed with heating for 14 hours. The solvent was concentrated under reduced pressure. Water was added into the residue and the product was extracted twice with ethyl acetate. The combined organic layers were washed with 1N hydrochloric acid and an aqueous saturated solution of sodium hydrogen carbonate, dried on magnesium sulfate, filtered, and then concentrated under reduced pressure. The residue was crystallized from ethyl acetate-hexane to obtain 25.2 g (75% yield) of the title compound. Melting point: 104-106°C.
¹H-NMR (CDCl₃) δ: 1.50 (9H, s), 2.12 (3H, s), 2.17 (3H, s), 2.24 (3H, s), 3.78 (3H, s), 5.81 (1H, br s), 6.58 (1H, s).

### Reference Example 41b

### Tert-butyl 3-bromo-4-methoxy-2,5,6-trimethylphenylcarbamate

To a solution of tert-butyl 4-methoxy-2,3,6-trimethylphenylcarbamate (12.7 g, 47.9 mmol) and sodium acetate (4.72 g, 57.5 mg) in acetic acid (50 ml) was added bromine (8.42 g, 52.7 mmol) at room temperature and the resulting mixture was stirred at the same temperature for 1 hour. Water (80 ml) was added into the reaction mixture. The crystals precipitated were collected by filtration and dissolved into ethyl acetate. The solution was washed with an aqueous saturated solution of sodium hydrogen carbonate, dried on magnesium sulfate, filtered, and then concentrated under reduced pressure. The residue was crystallized from methanol to obtain 15.0 g (91% yield) of the title compound. Melting point: 159-161°C.
¹H-NMR (CDCl₃) δ: 1.50 (9H, s), 2.15 (3H, s), 2.24 (3H, s), 2.35 (3H, s), 3.74 (3H, s), 5.92 (1H, br s).

### Reference Example 42b

### 2,2,4,6,7-Pentamethyl-3-(4-methylphenyl)-2,3-dihydro-1- benzofuran-5-amine

To a solution of tert-butyl 3-bromo-4-methoxy-2,5,6-trimethylphenylcarbamate (27.8 g, 80.8 mmol) in tetrahydrofuran (150 ml) was added n-butyllithium (1.6 M, 110 ml, 176 mmol) at -78°C and the reaction mixture was stirred at the same temperature for 20 minutes. To the reaction solution was added 2-methyl-1-(4-methylphenyl)propan-1-one (13.1 g, 80.7 mmol) and stirred at room temperature for 1 hour. Water (150 ml) was added to the reaction mixture and the product was extracted three times with ethyl acetate. The combined organic layers were washed with water, dried on magnesium sulfate, filtered, and then concentrated under reduced pressure to obtain 26.0 g of tert-butyl 3-[1-hydroxy-2-methyl-1-(4-methylphenyl)propyl]-4-methoxy-2,5,6-trimethylphenylcarbamate as a crude product. A mixture of this compound and 47% hydrobromic acid (100 ml) was refluxed with heating for 4 hours under argon atmosphere. The reaction mixture was cooled down to room temperature and neutralized with an 8 N aqueous solution of sodium hydroxide. The product was extracted twice with ethyl acetate. The combined extracts were washed with an aqueous saturated solution of sodium hydrogen carbonate, dried on magnesium sulfate, and then concentrated under reduced pressure. The residue was crystallized from isopropyl ether-hexane to obtain 14.8 g (62% yield) of the title compound. Melting point: 114-115°C.
¹H-NMR (CDCl₃) δ: 0.99 (3H, s), 1.47 (3H, s), 1.78 (3H, s), 2.12 (3H, s), 2.17 (3H, s), 2.30 (3H, s), 2.80 (2H, br s), 4.08 (1H, s), 6.60-7.10 (4H, m).

### Reference Example 43b

### (+)-3-(4-Isopropylphenyl)-2,2,4,6,7-pentamethyl-2,3-dihydro-1-benzofuran-5-amine

3-(4-Isopropylphenyl)-2,2,4,6,7-pentamethyl-2,3-dihydro-1-benzofuran-5-amine was subjected to high performance liquid chromatography (instrument: Waters semi-preparative separation system, column: CHIRALCEL OD (20 (i, d) x 250 mm) manufactured by Daicel Chemical Industries, LTD.), mobile phase: hexane : isopropyl alcohol = 98 : 2, flow rate: 6 ml/min, column temperature: 30°C, sample injection amount: 40 mg) to preparatively separate a fraction with a shorter retention time as the title compound. Melting point: 72-75°C. [α]D = +2.8° (c = 0.500, methanol)

### Reference Example 44b

### (-)-3-(4-Isopropylphenyl)-2,2,4,6,7-pentamethyl-2,3-dihydro-1-benzofuran-5-amine

3-(4-Isopropylphenyl)-2,2,4,6,7-pentamethyl-2,3-dihydro-1-benzofuran-5-amine was subjected to high performance liquid chromatography (instrument: Waters semi-preparative separation system, column: CHIRALCEL OD (20 (i, d) x 250 mm) manufactured by Daicel Chemical Industries, LTD.), mobile phase: hexane : isopropyl alcohol = 98 : 2, flow rate: 6 ml/min, column temperature: 30°C, sample injection amount: 40 mg) to preparatively separate a fraction with a longer retention time as the title compound. Melting point: 74-76°C. [α]D = -3.3° (c = 0.506, methanol)

### Example 1b

### 4-Methoxy-N-(2,2,4,6,7-pentamethyl-3-phenyl-2,3-dihydro- 1-benzofuran-5-yl)benzamide

To a solution of 2,2,4,6,7-pentamethyl-3-phenyl-2,3-dihydro-1-benzofuran-5-amine (1.60 g, 5.69 mmol) and 4-methoxybenzoyl chloride (1.16 g, 6.82 mmol) in chloroform (20 ml) was added triethylamine (0.87 ml, 6.26 mmol) at room temperature and the mixture was stirred at room temperature for 30 minutes. The solvent was removed under reduced pressure. Water (30 ml) was added into the residue and the product was extracted twice with ethyl acetate. The combined organic layers were washed with 1N hydrochloric acid and an aqueous saturated solution of sodium hydrogen carbonate, dried on magnesium sulfate, filtered, and then concentrated under reduced pressure. The residue was crystallized from methanol to obtain 1.70 g (72% yield) of the title compound. Melting point: 190-192°C.
¹H-NMR (CDCl₃) δ: 1.03 (3H, s), 1.53 (3H, s), 1.80 (3H, s), 2.19 (6H, s), 3.86 (3H, s), 4.16 (1H, s), 6.80-7.36 (8H, m), 7.86 (2H, d, J = 8.8 Hz).

### Example 2b

### N-(4-Methoxybenzyl)-2,2,4,6,7-pentamethyl-3-phenyl-2,3-dihydro-1-benzofuran-5-amine

To a suspension of aluminum chloride (2.25 g, 16.9 mmol) in tetrahydrofuran (20 ml) was gradually added lithium aluminum hydride (640 mg, 16.9 mmol) under ice cooling and the resulting mixture was stirred at the same temperature for 10 minutes. To this mixture was added 4-methoxy-N- (2,2,4,6,7-pentamethyl-3-phenyl-2, 3-dihydro-1-benzofuran-5-yl)benzamide (1.40 g, 3.37 mmol) and the mixture was refluxed with heating for 3 hours. The reaction mixture was poured into ice water and neutralized with an 8 N aqueous solution of sodium hydroxide. The product was extracted twice with ethyl acetate. The combined organic layers were washed with water, dried on magnesium sulfate, filtered, and then concentrated under reduced pressure. The residue was crystallized from methanol to obtain 0.80 g (59% yield) of the title compound. Melting point: 113-115°C.
¹H-NMR (CDCl₃) δ: 1.01 (3H, s), 1.50 (3H, s), 1.78 (3H, s), 1.98 (1H, br s), 2.18 (3H, s), 2.27 (3H, s), 3.79 (3H, s), 3.85 (2H, s), 4.11 (1H, s), 6.80-7.31 (9H, m).

### Example 3b

### 4-Fluoro-N-(2,2,4,6,7-pentamethyl-3-phenyl-2,3-dihydro-1-benzofuran-5-yl)benzamide

By using 2,2,4,6,7-pentamethyl-3-phenyl-2,3-dihydro-1-benzofuran-5-amine and 4-fluorobenzoyl chloride, the title compound was synthesized according to Example 1b. Yield: 92%. Melting point: 156-158°C. (Ethyl acetate)
¹H-NMR (CDCl₃) δ: 1.03 (3H, s), 1.53 (3H, s), 1.80 (3H, s), 2.19 (3H, s), 2.20 (3H, s), 4.17 (1H, s), 6.62-7.35 (8H, m), 7.85-7.94 (2H, m).

### Example 4b

### N-(4-Fluorobenzyl)-2,2,4,6,7-pentamethyl-3-phenyl-2,3-dihydro-1-benzofuran-5-amine

By using 4-fluoro-N-(2,2,4,6,7-pentamethyl-3-phenyl-2,3-dihydro-1-benzofuran-5-yl)benzamide; the title compound was synthesized according to Example 2b. Yield: 60%. Melting point: 93-95°C. (Methanol)
¹H-NMR (CDCl₃) δ: 1.01 (3H, s), 1.52 (3H, s), 1.76 (3H, s), 2.18 (3H, s), 2.26 (3H, s), 2.61(H, br s), 3.88 (2H, s), 4.11 (1H, s), 6.62-7.40 (9H, m).

### Example 5b

### N-[3-(4-Isopropylphenyl)-2,2,4,6,7-pentamethyl-2,3-dihydro-1-benzofuran-5-yl]benzamide

By using 3-(4-isopropylphenyl)-2,2,4,6,7-pentamethyl-2,3-dihydro-l-benzofuran-5-amine and benzoyl chloride, the title compound was synthesized according to Example 1b. Yield: 90%. Melting point: 218-220°C. (Ethyl acetate-hexane).
¹H-NMR (CDCl₃) δ: 1.03 (3H, s), 1.21 (6H, d, J = 7.0 Hz), 1.52 (3H, s), 1.82 (3H, s), 2.19 (6H, s), 2.85 (1H, septet, J = 7.0 Hz), 4.14 (1H, s), 6.70-7.13 (4H, m), 7.30 (1H, br s), 7.42-7.61 (3H, m), 7.85-7.92 (2H, m).

### Example 6b

### N-Benzyl-3-(4-isopropylphenyl)-2,2,4,6,7-pentamethyl-2,3-dihydro-1-benzofuran-5-amine hydrochloride

To a suspension of aluminum chloride (1.18 g, 8.89 mmol) in tetrahydrofuran (20 ml) was gradually added lithium aluminum hydride (337 mg, 8.89 mmol) under ice cooling and the resulting mixture was stirred at the same temperature for 10 minutes. To this mixture was added N-[3-(4-isopropylphenyl)-2,2,4,6,7-pentamethyl-2,3-dihydro-1-benzofuran-5-yl]benzamide (0.76 g, 1.78 mmol) and the mixture was refluxed with heating for 3 hours. The reaction mixture was poured into ice water and neutralized with an 8 N aqueous solution of sodium hydroxide. The product was extracted twice with ethyl acetate. The combined organic layers were washed with water, dried on magnesium sulfate, filtered, and then concentrated under reduced pressure to obtain 0.52 g of an oily free base. The free base (0.52g, 1.26 mmol) was dissolved into a solution of hydrochloric acid in methanol and then solvent was concentrated under reduced pressure. The resulting residue was crystallized from methanol to obtain 0.47 g (59% yield) of the title compound. Melting point: 186-188°C.
¹H-NMR (DMSO-d₆) δ: 0.94 (3H, s), 1.20 (6H, d, J = 6.6 Hz), 1.41 (3H, s), 1.62 (3H, s), 2.10 (3H, s), 2.26 (3H, s), 2.86 (1H, septet, J = 6.6 Hz), 4.14 (1H, s), 4.23-4.58 (2H, s), 6.40-7.42 (9H, m), 10.4 (2H, br s).

### Example 7b

### N-[3-(4-Isopropylphenyl-)-2,2,4,6,7-pentamethyl-2,3-dihydro-1-benzofuran-5-yl]-4-methoxybenzamide

By using 3-(4-isopropylphenyl)-2,2,4,6,7-pentamethyl-2,3-dihydro-1-benzofuran-5-amine and 4-methoxybenzoyl chloride, the title compound was synthesized according to Example 1b. Yield: 42%. Melting point: 202-205°C. (Ethyl acetate-hexane)
¹H-NMR (CDCl₃) δ: 1.02 (3H, s), 1.21 (6H, d, J = 6.8 Hz), 1.49 (3H, s), 1.80 (3H, s), 2.18 (6H, s), 2.85 (1H, septet, J = 6.8 Hz), 3.86 (3H, s), 4.13 (1H, s), 6.62-7.19 (6H, m), 7.23 (1H, s), 7.85 (2H, d, J = 9.2 Hz).

### Example 8b

### 3-(4-Isopropylphenyl)-N-(4-methoxybenzyl)-2,2,4,6,7-pentamethyl-2,3-dihydro-1-benzofuran-5-amine

By using N-[3-(4-isopropylphenyl)-2,2,4,6,7-pentamethyl-2,3-dihydro-1-benzofuran-5-yl]-4-methoxybenzamide, the title compound was synthesized according to Example 2b. Yield: 80%. Melting point: 95-96°C. (Hexane)
¹H-NMR (CDCl₃) δ: 1.00 (3H, s), 1.22 (6H, d, J = 6.8 Hz), 1.49 (3H, s), 1.6-1.7 (1H, br), 1.79 (3H, s), 2.81 (3H, s), 2.27 (3H, s), 2.86 (1H, septet, J = 6.8 Hz), 3.80 (3H, s), 3.86 (2H, s), 4.09 (1H, s), 6.81-6.88 (4H, m), 7.06-7.11 (2H, m), 7.24-7.28 (2H, m).

### Example 9b

### 3-(4-Isopropylphenyl)-N-(4-methoxybenzyl)-N,2,2,4,6,7-hexamethyl-2,3-dihydro-1-benzofuran-5-amine

Sodium hydride (a 60% dispersion in liquid paraffin, 598.8 mg, 14.97 mmol) was washed twice with hexane and then suspended to N,N-dimethylformamide (10 ml). To this suspension was gradually added a solution of 3-(4-isopropylphenyl)-N-(4-methoxybenzyl)-2,2,4,6,7-pentamethyl-2,3-dihydro-1-benzofuran-5-amine (998.9 mg, 2.25 mmol) in N,N-dimethylformamide (30 ml) and the reaction mixture was stirred at 60°C for 30 minutes. To this mixture was added methyl iodide (2.19 g, 15.45 mmol) and the mixture was stirred at the same temperature for 30 minutes. The reaction solution was cooled down to room temperature and water was added to the solution. The product was extracted with ethyl acetate. The extracts were dried on magnesium sulfate, filtered, and concentrated under reduced pressure. The residue was subjected to column chromatography on silica gel (hexane-ethyl acetate 10 : 1) to obtain the title compound (69 % yield) as an oily mixture of rotamers.
¹H-NMR (CDCl₃) δ: 0.97-1.00 (3H, s), 1.20-1.25 (6H, m), 1.50 (3H, m), 1.83-1.88 (3H, m), 2.14-2.16 (3H, m), 2.27-2.28 (3H, m), 2.59-2.67 (3H, m), 2.80-2.94 (1H, m), 3.79-3.80 (3H, m), 4.03-4.06 (2H, m), 4.08-4.10 (1H, m), 6.78-6.87 (4H, m), 7.06-7.30 (4H, m).

### Example 10b

### N-[3-(4-Isopropylphenyl)-2,2,4,6,7-pentamethyl-2,3-dihydro-1-benzofuran-5-yl]-4-methoxyphenylacetamide

By using 3-(4-isopropylphenyl)-2,2,4,6,7-pentamethyl-2,3-dihydro-1-benzofuran-5-amine and 4-methoxyphenylacetyl chloride, the title compound was synthesized according to Example 1b. Yield: 74%. Melting point: 171-173°C. (Methanol)
¹H-NMR (CDCl₃) δ: 0.98 (3H, s), 1.20 (6H, d, J = 6.6 Hz), 1.46 (3H, s), 1.64 (3H, s), 2.03 (3H, s), 2.12 (3H, s), 2.84 (1H, septet, J = 6.6 Hz), 3.68 (2H, s), 3.80 (3H, s), 4.06 (1H, s), 6.45 (1H, br), 6.6-6.9 (2H, m), 6.89 (2H, d, J = 8.6 Hz), 7.05 (2H, d, J = 8.0 Hz), 7.26 (d, 2H, J = 8.6 Hz) .

### Example 11b

### 3-(4-Isopropylphenyl)-N-[2-(4-methoxyphenyl)ethyl]-2,2,4,6,7-pentamethyl-2,3-dihydro-1-benzofuran-5-amine

By using N-[3-(4-isopropylphenyl)-2,2,4,6,7-pentamethyl-2, 3-dihydro-1-benzofuran-5-yl]-4-methoxyphenylacetamide, the title compound was synthesized according to Example 2b. Yield: 66%. Melting point: 63-65°C. (Hexane)
¹H-NMR (CDCl₃) δ: 0.98 (3H, s), 1.21 (6H, d, J = 6.8 Hz), 1.46 (3H, s), 1.68 (3H, s), 1.8-1.9 (1H, br), 2.12 (3H, s), 2.14 (3H, s), 2.76-3.04 (5H, m), 3.78 (3H, s), 4.05 (1H, s), 6.6-7.0 (4H, m), 7.04-7.08 (4H, m), 7.12-7.19 (2H, m).

### Example 12b

### 3-(4-Isopropylphenyl)-N-[2-(4-methoxyphenyl)ethyl]-N,2,2,4,6,7-hexamethyl-2,3-dihydro-1-benzofuran-5-amine

By using 3-(4-isopropylphenyl)-N-[2-(4-methoxyphenyl)ethyl]-2,2,4,6,7-pentamethyl-2,3-dihydro-1-benzofuran-5-amine, the title compound was synthesized according to Example 9b. Yield: 85%. An oily substance.
¹H-NMR (CDCl₃) δ: 0.99 (3H, s), 1.20-1.24 (6H, m), 1.48-1.50 (3H, m), 1.77 (3H, s), 2.14-2.17 (6H, m), 2.58-2.89 (6H, m), 3.1-3.2 (2H, m), 3.76-3.77 (3H, m), 4.06-4.09 (1H, m), 6.74-6.90 (4H, m), 7.00-7.09 (4H, m).

### Example 13b

### N-[3-(4-Isopropylphenyl)-2,2,4,6,7-pentamethyl-2,3-dihydro-1-benzofuran-5-yl]-N-[2-(4-methoxyphenyl)ethyl]acetamide

Sodium hydride (a 60% dispersion in liquid paraffin, 232.1 mg, 5.80 mmol) was washed twice with hexane and then suspended to N,N-dimethylformamide (25 ml). To this suspension was added under an argon atmosphere 3-(4-isopropylphenyl)-N-[2-(4-methoxyphenyl)ethyl]-2,2,4,6,7-pentamethyl-2,3-dihydro-1-benzofuran-5-amine (537.9 mg, 1.18 mmol) and the reaction mixture was stirred at 60°C for 20 minutes. Then, acetyl chloride (0.5 ml, 7.03 mmol) was added into the reaction mixture and stirred at the same temperature for 1 hour. After the reaction mixture was cooled down to room temperature, an aqueous saturated solution of sodium hydrogen carbonate was added to the reaction mixture and the product was extracted twice with ethyl acetate. The combined extracts were washed with water, dried on magnesium sulfate, filtered, and concentrated under reduced pressure. The residue was subjected to column chromatography on silica gel (hexane-ethyl acetate 3 : 1) to obtain the rotamer 1 (Rf = 0.38; hexane-ethyl acetate 3 : 1) of the title compound (46 % yield). Melting point: 134-136°C. (Ethyl acetate-hexane)
¹H-NMR (CDCl₃) δ: 1.03 (3H, s), 1.22 (6H, d, J = 7.0 Hz), 1.54 (3H, s), 1.66 (3H, s), 1.72 (3H, s), 2.12 (3H, s), 2.18 (3H, s), 2.77-2.89 (3H, m), 3.59-3.70 (2H, m), 3.77 (3H, s), 4.11 (1H, s), 6.77-7.13 (8H, m).

### Example 14b

### N-[3-(4-Isopropylphenyl)-2,2,4,6,7-pentamethyl-2,3-dihydro-1-benzofuran-5-yl]-N-[2-(4-methoxyphenyl)ethyl]acetamide

The residue treated in the same manner as described in the Example 13b was subjected to column chromatography on silica gel (hexane-ethyl acetate 3 : 1) to obtain the rotamer 2 (Rf = 0.25; hexane-ethyl acetate 3 : 1) of the title compound (36 % yield). Amorphous.
¹H-NMR (CDCl₃) δ: 1.03 (3H, s), 1.23 (6H, d, J = 6.8 Hz), 1.53 (3H, s), 1.73 (3H, s), 1.75 (3H, s), 2.12 (3H, s), 2.18 (3H, s), 2.67-2.75 (2H, m), 2.80-2.94 (1H, septet, J = 6.8 Hz), 3.57-3.74 (2H, s), 3.77 (3H, s), 4.14 (1H, S), 6.77-7.13 (8H, m).

### Example 15b

### N-[3-(4-Isopropylphenyl)-2,2,4,6,7-pentamethyl-2,3-dihydro-1-benzofuran-5-yl]-3-(4-methoxyphenyl)propionamide

By using 3-(4-isopropylphenyl)-2,2,4,6,7-pentamethyl-2,3-dihydro-1-benzofuran-5-amine and 3-(4-methoxyphenyl)propionyl chloride, the title compound was synthesized according to Example 1b. Yield: 72%. Melting point: 188-191°C. (Ethyl acetate-hexane)
¹H-NMR (CDCl₃) δ: 0.99-1.01 (3H, m), 1.19-1.26 (6H, m), 1.48 (3H, s), 1.64-1.68 (3H, m), 1.99 (3H, s), 2.05-2.13 (5H, m), 2.65-3.04 (3H, m), 3.72-3.77 (3H, m), 4.08 (1H, s), 6.47-7.19 (9H, m).

### Example 16b

### 3-(4-Isopropylphenyl)-N-[3-(4-methoxyphenyl)propyl]-2,2,4,6,7-pentamethyl-2,3-dihydro-1-benzofuran-5-amine

By using N-[3-(4-isopropylphenyl)-2,2,4,6,7-pentamethyl-2,3-dihydro-1-benzofuran-5-yl]-3-(4-methoxyphenyl)propionamide, the title compound was synthesized according to Example 2b. Yield: 99%. Melting point: 62-65°C. (Pentane)
¹H-NMR (CDCl₃) δ: 0.99 (3H, s), 1.21 (6H, d, J = 6.6 Hz), 1.48 (3H, s), 1.78-1.88 (6H, s), 2.15 (3H, s), 2.20 (3H, s), 2.65 (2H, t, J = 7.6 Hz), 2.76 (3H, m), 3.78 (3H, s), 4.08 (1H, s), 6.6-6.8 (4H, m), 7.05-7.12 (4H, m).

### Example 17b

### N-[3-(4-Isopropylphenyl)-2,2,4,6,7-pentamethyl-2,3-dihydro-1-benzofuran-5-yl]-4-methoxybenzenesulfonamide

To a solution of 3-(4-isopropylphenyl)-2,2,4,6,7-pentamethyl-2,3-dihydro-1-benzofuran-5-amine (0.35 g, 1.08 mmol) and 4-methoxybenzenesulfonyl chloride (0.25 g, 1.19 mmol) in chloroform (5 ml) was added triethylamine (0.16 ml, 1.19 mmol) and the mixture was stirred at room temperature for 14 hours. The solvent was concentrated under reduced pressure. Water (20 ml) was added into the residue and the product was extracted twice with ethyl acetate. The combined organic layers were washed with 1N hydrochloric acid and an aqueous saturated solution of sodium hydrogen carbonate, dried on magnesium sulfate, filtered, and then concentrated under reduced pressure. The residue was crystallized from ethyl acetate-hexane to obtain 0.18 g (34% yield) of the title compound. Melting point: 206-208°C.
¹H-NMR (CDCl₃) δ: 0.99 (3H, s), 1.23 (6H, d, J = 6.8 Hz), 1.40 (3H, s), 1.47 (3H, s), 2.10 (3H, s), 2.13 (3H, s), 2.87 (1H, septet, J = 6.8 Hz), 3.80 (3H, s), 3.90 (1H, s), 5.79 (1H, s), 6.70-7.15 (4H, m), 7.09 (2H, d, J = 8.4 Hz), 7.57 (2H, d, J = 8.8 Hz).

### Example 18b

### 4-Fluoro-N-[3-(4-isopropylphenyl)-2,2,4,6,7-pentamethyl- 2,3-dihydro-1-benzofuran-5-yl]benzamide

By using 3-(4-isopropylphenyl)-2,2,4,6,7-pentamethyl-2,3-dihydro-1-benzofuran-5-amine and 4-fluorobenzoyl chloride, the title compound was synthesized according to Example 1b. Yield: 65%. Amorphous.
¹H-NMR (CDCl₃) δ: 1.02 (3H, s), 1.21 (6H, d, J = 6.8 Hz), 1.41 (3H, s), 1.80 (3H, s), 2.17 (3H, s), 2.19 (3H, s), 2.85 (1H, septet, J = 6.8 Hz), 4.13 (1H, s), 6.60-7.31 (7H, m), 7.89 (2H, dd, J = 8.8, 5.2 Hz).

### Example 19b

### N-(4-Fluorobenzyl)-3-(4-isopropylphenyl)-2,2,4,6,7-pentamethyl-2,3-dihydro-1-benzofuran-5-amine hydrochloride

To a suspension of aluminum chloride (1.20 g, 9.00 mmol) in tetrahydrofuran (25 ml) was gradually added lithium aluminum hydride (340 mg, 9.00 mmol) under ice cooling and the resulting mixture was stirred at the same temperature for 10 minutes. To this mixture was added 4-fluoro-N-[3-(4-isopropylphenyl)-2,2,4,6,7-pentamethyl-2,3-dihydro-1-benzofuran-5-yl]benzamide (0.83 g, 1.86 mmol) and the mixture was refluxed with heating for 3 hours. The reaction mixture was poured into ice water and neutralized with an 8 N aqueous solution of sodium hydroxide. The product was extracted twice with ethyl acetate. The combined organic layers were washed with water, dried on magnesium sulfate, filtered, and then concentrated under reduced pressure to obtain 0.51 g of an oily free base. The free base (0.51 g, 1.18 mmol) was dissolved into a solution of hydrochloric acid in methanol and then solvent was concentrated under reduced pressure. The resulting residue was crystallized from methanol to obtain 0.49 g (56% yield) of the title compound. Melting point: 201-204°C.
¹H-NMR (DMSO-d₆) δ : 0.92 (3H, s), 1.19 (6H, d, J = 7.0 Hz), 1.40 (3H, s), 1.54 (3H, s), 2.10 (3H, s), 2.31 (3H, s), 2.85 (1H, septet, J = 6.8 Hz), 4.13 (1H, s), 4.29 (1H, d, J = 12.8 Hz), 4.43 (1H, d, J = 12.8 Hz), 6.20-7.40 (8H, m), 10.4 (2H, br s).

### Example 20b

### 4-Chloro-N-[3-(4-isopropylphenyl)-2,2,4,6,7-pentamethyl-2,3-dihydro-1-benzofuran-5-yl]benzamide

By using 3-(4-isopropylphenyl)-2,2,4,6,7-pentamethyl-2,3-dihydro-1-benzofuran-5-amine and 4-chlorobenzoyl chloride, the title compound was synthesized according to Example 1b. Yield: 71%. Melting point: 201-203°C. (Ethyl acetate-hexane)
¹H-NMR (CDCl₃) δ: 1.02 (3H, s), 1.21 (6H, d, J = 7.0 Hz), 1.51 (3H, s), 1.80 (3H, s), 2.17 (3H, s), 2.19 (3H, s), 2.85 (1H, septet, J = 6.8 Hz), 4.13 (1H, s), 6.62-7.31 (4H, m), 7.24 (1H, br s), 7.44 (2H, d, J = 8.8 Hz), 7.82 (2H, d, J = 8.8 Hz).

### Example 21b

### N-(4-Chlorobenzyl)-3-(4-isopropylphenyl)-2,2,4,6,7-pentamethyl-2,3-dihydro-1-benzofuran-5-amine

By using 4-chloro-N-[3-(4-isopropylphenyl)-2,2,4,6,7-pentamethyl-2,3-dihydro-1-benzofuran-5-yl]benzamide, the title compound was synthesized according to Example 2b. Yield: 37%. Melting point: 93-94°C. (Methanol)
¹H-NMR (CDCl₃) δ: 1.00 (3H, s), 1.22 (6H, d, J = 7.0 Hz), 1.49 (3H, s), 1.58 (1H, br s), 1.74 (3H, s), 2.18 (3H, s), 2.25 (3H, s), 2.86 (1H, septet, J = 6.8 Hz), 3.89 (2H, s), 4.07 (1H, s), 6.63-7.12 (4H, m), 7.25 (4H, s).

### Example 22b

### N-[3-(4-Isopropylphenyl)-2,2,4,6,7-pentamethyl-2,3-dihydro-1-benzofuran-5-yl]-1,3-benzodioxol-5-carboxamide

By using 3-(4-isopropylphenyl)-2,2,4,6,7-pentamethyl-2,3-dihydro-1-benzofuran-5-amine and 1,3-benzodioxol-5-carbonyl chloride, the title compound was synthesized according to Example 1b. Yield: 67%. Melting point: 165-167°C. (Ethyl ether-hexane)
¹H-NMR (CDCl₃) δ: 1.02 (3H, s), 1.21 (6H, d, J = 7.0 Hz), 1.51 (3H, s), 1.80 (3H, s), 2.17 (3H, s), 2.18 (3H, s), 2.85 (1H, septet, J = 7.0 Hz), 4.13 (1H, s), 6.03 (2H, s), 6.63-7.13 (5H, m), 7.17 (1H, br s), 7.35-7.45 (2H, m).

### Example 23b

### N-(1,3-Benzodioxol-5-ylmethyl)-3-(4-isopropylphenyl)-2,2,4,6,7-pentamethyl-2,3-dihydro-1-benzofuran-5-amine

To a suspension of aluminum chloride (847 mg, 6.35 mmol) in tetrahydrofuran (10 ml) was gradually added lithium aluminum hydride (240 mg, 6.35 mmol) under ice cooling and the resulting mixture was stirred at the same temperature for 10 minutes. To this mixture was added N-[3-(4-isopropylphenyl)-2,2,4,6,7-pentamethyl-2,3-dihydro-1-benzofuran-5-yl]-1,3-benzodioxol-5-carboxamide (0.60 g, 1.27 mmol) and the mixture was refluxed with heating for 3 hours. The reaction mixture was poured into ice water and neutralized with an 8 N aqueous solution of sodium hydroxide. The product was extracted twice with ethyl acetate. The combined organic layers were washed with water, dried on magnesium sulfate, filtered, and then concentrated under reduced pressure. The residue was crystallized from methanol to obtain 0.23 g (40% yield) of the title compound. Melting point: 100-102°C.
¹H-NMR (CDCl₃) δ: 1.00 (3H, s), 1.22 (6H, d, J = 7.0 Hz), 1.49 (3H, s), 1.80 (3H, s), 1.86 (1H, br s), 2.17 (3H, s), 2.26 (3H, s), 2.86 (1H, septet, J = 7.0 Hz), 3.82 (2H, s), 4.08 (1H, s), 5.93 (2H, s), 6.62-7.00 (5H, m), 7.08 (2H, d, J = 8.0 Hz).

### Example 24b

### N-[3-(4-Isopropylphenyl)-2,2,4,6,7-pentamethyl-2,3-dihydro-1-benzofuran-5-yl]-2-thiophenecarboxamide

By using 3-(4-isopropylphenyl)-2,2,4,6,7-pentamethyl-2,3-dihydro-1-benzofuran-5-amine and 2-thiophenecarbonyl chloride, the title compound was synthesized according to Example 1b. Yield: 66%. Melting point: 222-224°C. (Ethyl acetate-hexane)
¹H-NMR (CDCl₃) δ: 1.02 (3H, s), 1.21 (6H, d, J = 7.0 Hz), 1.51 (3H, s), 1.82 (3H, s), 2.19 (3H, s), 2.85 (1H, septet, J = 7.0 Hz), 4.13 (1H, s), 6.70-7.20 (6H, m), 7.50 (1H, dd, J = 4.8, 1.2 Hz), 7.63 (1H, dd, J = 3.6, 1.2 Hz).

### Example 25b

### 3-(4-Isopropylphenyl)-2,2,4,6,7-pentamethyl-N-(2-thienylmethyl)-2,3-dihydro-1-benzofuran-5-amine

By using N-[3-(4-isopropylphenyl)-2,2,4,6,7-pentamethyl-2,3-dihydro-1-benzofuran-5-yl]-2-thiophenecarboxamide, the title compound was synthesized according to Example 2b. Yield: 61%. Melting point: 101-103°C. (Methanol)
¹H-NMR (CDCl₃) δ: 1.00 (3H, s), 1.22 (6H, d, J = 7.0 Hz), 1.49 (3H, s), 1.80 (3H, s), 3.00-2.40 (7H, s), 2.86 (1H, septet, J = 7.0 Hz), 4.08 (1H, s), 4.11 (2H, s), 6.71-7.30 (7H, m).

### Example 26b

### N-[3-4-(Isopropylphenyl)-2,2,4,6,7-pentamethyl-2,3-dihydro-1-benzofuran-5-yl]nicotinamide

To a solution of 3-(4-isopropylphenyl)-2,2,4,6,7-pentamethyl-2,3-dihydro-1-benzofuran-5-amine (0.85 g, 2.63 mmol) and nicotinoyl chloride hydrochloride (516 mg, 2.90 mmol) in chloroform (15 ml) was added triethylamine (0.80 ml, 5.80 mmol) and the mixture was stirred at room temperature for 30 minutes. The solvent was concentrated under reduced pressure. Water (30 ml) was added into the residue and the product was extracted twice with ethyl acetate. The combined organic layers were washed with an aqueous saturated solution of sodium hydrogen carbonate, dried on magnesium sulfate, filtered, and then concentrated under reduced pressure. The residue was subjected to column chromatography (hexane-ethyl acetate 5 : 1) on silica gel to obtain 0.72 g (61% yield) of the title compound. Melting point: 214-216°C. (Ethyl ether-hexane)
¹H-NMR (CDCl₃) δ: 1.03 (3H, s), 1.21 (6H, d, J = 7.0 Hz), 1.52 (3H, s), 1.82 (3H, s), 2.19 (6H, s), 2.86 (1H, septet, J = 7.0 Hz), 4.14 (1H, s), 6.70-7.13 (4H, m), 7.31 (1H, br s), 7.44 (1H, dd, J = 7.8, 4.8 Hz), 8.23 (1H, dt, J = 8.0, 2.2 Hz), 8.74-8.79 (1H, m), 9.12 (1H, br s).

### Example 27b

### N-[3-(4-Isopropylphenyl)-2,2,4,6,7-pentamethyl-2,3-dihydro-1-benzofuran-5-yl]isonicotinamide hydrochloride

To a solution of 3-(4-isopropylphenyl)-2,2,4,6,7-pentamethyl-2,3-dihydro-1-benzofuran-5-amine (0.85 g, 2.63 mmol) and isonicotinoyl chloride hydrochloride (516 mg, 2.90 mmol) in chloroform (15 ml) was added triethylamine (0.80 ml, 5.80 mmol) and the mixture was stirred at room temperature for 30 minutes. The solvent was concentrated under reduced pressure. Water (30 ml) was added into the residue and the product was extracted twice with ethyl acetate. The combined organic layers were washed with an aqueous saturated solution of sodium hydrogen carbonate, dried on magnesium sulfate, filtered, and then concentrated under reduced pressure. The residue was subjected to column chromatography (hexane-ethyl acetate 5 : 1) on silica gel to obtain 0.90 g of an oily free base. The free base (0.90 g, 2.10 mmol) was dissolved into a solution of hydrochloric acid in methanol and the solvent was concentrated under reduced pressure to obtain 0.47 g (64% yield) of the amorphous title compound.
¹H-NMR (CDCl₃) δ: 0.99 (3H, s), 1.19 (6H, d, J = 6.8 Hz), 1.49 (3H, s), 1.80 (3H, s), 2.14 (6H, s), 2.83 (1H, septet, J = 6.8 Hz), 4.13 (1H, s), 6.70-7.19 (5H, m), 8.20-9.20 (4H, m), 9.79 (1H, br s).

### Example 28b

### N-[3-(4-Fluorophenyl)-2,2,4,6,7-pentamethyl-2,3-dihydro-1-benzofuran-5-yl]-4-methoxybenzamide

By using 3-(4-fluorophenyl)-2,2,4,6,7-pentamethyl-2,3-dihydro-1-benzofuran-5-amine and 4-methoxybenzoyl chloride, the title compound was synthesized according to Example 1b. Yield: 79%. Melting point: 191-194°C. (Ethyl acetate-hexane)
¹H-NMR (CDCl₃) δ: 1.02 (3H, s), 1.51 (3H, s), 1.79 (3H, s), 2.17 (3H, s), 2.20 (3H, s), 3.86 (3H, s), 4.14 (1H, s), 6.60-7.21 (7H, m), 7.85 (2H, d, J = 8.8 Hz).

### Example 29b

### 3-(4-Fluorophenyl)-N-(4-methoxybenzyl)-2,2,4,6,7-pentamethyl-2,3-dihydro-1-benzofuran-5-amine

By using N-[3-(4-fluorophenyl)-2,2,4,6,7-pentamethyl-2,3-dihydro-1-benzofuran-5-yl]-4-methoxybenzamide, the title compound was synthesized according to Example 2b. Yield: 52%. Melting point: 114-115°C. (Methanol)
¹H-NMR (CDCl₃) δ: 1.00 (3H, s), 1.49 (3H, s), 1.76 (3H, s), 2.18 (3H, s), 2.27 (3H, s), 2.80 (1H, br s), 3.79 (3H, s), 3.85 (2H, s), 4.08 (1H, s), 6.71-7.03 (6H, m), 7.20-7.27 (2H, m).

### Example 30b

### 4-Fluoro-N-[3-(4-fluorophenyl)-2,2,4,6,7-pentamethyl-2,3-dihydro-1-benzofuran-5-yl]benzamide

By using 3-(4-fluorophenyl)-2,2,4,6,7-pentamethyl-2,3-dihydro-l-benzofuran-5-amine and 4-fluorobenzoyl chloride, the title compound was synthesized according to Example 1b. Yield: 75%. Melting point: 140-142°C. (Ethyl acetate-hexane)
¹H-NMR (CDCl₃) δ: 1.02 (3H, s), 1.52 (3H, s), 1.80 (3H, s), 2.19 (6H, s), 4.14 (1H, s), 6.75-7.25 (7H, m), 7.85-7.94 (2H, m).

### Example 31b

### N-(4-Fluorobenzyl)-3-(4-fluorophenyl)-2,2,4,6,7-pentamethyl-2,3-dihydro-1-benzofuran-5-amine

By using 4-fluoro-N-[3-(4-fluorophenyl)-2,2,4,6,7-pentamethyl-2,3-dihydro-1-benzofuran-5-yl]benzamide, the title compound was synthesized according to Example 2b. Yield: 66%. Melting point: 118-120°C. (Ethanol)
¹H-NMR (CDCl₃) δ: 1.00 (3H, s), 1.49 (3H, s), 1.77 (3H, s), 2.18 (3H, s), 2.26 (3H, s), 2.92 (1H, br s), 3.88 (2H, s), 4.08 (1H, s), 6.50-7.21 (6H, m), 7.24-7.41 (2H, m).

### Example 32b

### 4-Methoxy-N-[2,2,4,6,7-pentamethyl-3-(4-methylphenyl)-2,3-dihydro-1-benzofuran-5-yl]benzamide

By using 2,2,4,6,7-pentamethyl-3-(4-methylphenyl)-2,3-dihydro-1-benzofuran-5-amine and 4-methoxybenzoyl chloride, the title compound was synthesized according to Example 1b. Yield: 86%. Melting point: 161-163°C. (Ethyl acetate-hexane)
¹H-NMR (CDCl₃) δ: 1.03 (3H, s), 1.51 (3H, s), 1.79 (3H, s), 2.18 (6H, s), 2.30 (3H, s), 3.86 (3H, s), 4.12 (1H, s), 6.58-7.11 (6H, m), 7.20 (1H, br s), 7.85 (2H, d, J = 8.8 Hz).

### Example 33b

### N-(4-Methoxybenzyl)-2,2,4,6,7-pentamethyl-3-(4-methylphenyl)-2,3-dihydro-1-benzofuran-5-amine

By using 4-methoxy-N-[2,2,4,6,7-pentamethyl-3-(4-methylphenyl)-2,3-dihydro-1-benzofuran-5-yl]benzamide, the title compound was synthesized according to Example 2b. Yield: 58%. Melting point: 97-98°C. (Ethanol)
¹H-NMR (CDCl₃) δ: 1.00 (3H, s), 1.49 (3H, s), 1.78 (3H, s), 2.18 (3H, s), 2.26 (3H, s), 2.31(3H, s), 2.60(1H, br s), 3.79 (3H, s), 3.85 (2H, s), 4.08 (1H, s), 6.58-7.38 (8H, m).

### Example 34b

### 4-Fluoro-N-[2,2,4,6,7-pentamethyl-3-(4-methylphenyl)-2,3-dihydro-1-benzofuran-5-yl]benzamide

By using 2,2,4,6,7-pentamethyl-3-(4-methylphenyl)-2,3-dihydro-1-benzofuran-5-amine and 4-fluorobenzoyl chloride, the title compound was synthesized according to Example 1b. Yield: 43%. Melting point: 148-120°C. (Ethyl acetate-hexane)
¹H-NMR (CDCl₃) δ: 1.03 (3H, s), 1.52 (3H, s), 1.80 (3H, s), 2.19 (6H, s), 2.30 (3H, s), 4.13 (1H, s), 6.60-7.20 (7H, m), 7.85-7.94 (2H, m).

### Example 35b

### N-(4-Fluorobenzyl)-2,2,4,6,7-pentamethyl-3-(4-methylphenyl)-2,3-dihydro-1-benzofuran-5-amine

By using 4-fluoro-N-[2,2,4,6,7-pentamethyl-3-(4-methylphenyl)-2,3-dihydro-1-benzofuran-5-yl]benzamide, the title compound was synthesized according to Example 2b. Yield: 39%. Melting point: 92-94°C. (Methanol)
¹H-NMR (CDCl₃) δ: 1.01 (3H, s), 1.49 (3H, s), 1.77 (3H, s), 2.18 (3H, s), 2.25 (3H, s), 2.31 (3H, s), 2.82 (1H, br s), 3.87 (2H, s), 4.07 (1H, s), 6.60-7.32 (8H, m).

### Example 36b

### Methyl 4-[[3-(4-isopropylphenyl)-2,2,4,6,7-pentamethyl-2,3-dihydro-1-benzofuran-5-ylamino]carbonyl]benzoate

By using 3-(4-isopropylphenyl)-2,2,4,6,7-pentamethyl-2,3-dihydro-1-benzofuran-5-amine and 4-methoxycarbonylbenzoyl chloride, the title compound was synthesized according to Example 1b. Yield: 92%. Melting point: 220-223°C. (Methanol)
¹H-NMR (CDCl₃) δ: 1.03 (3H, s), 1.21 (6H, d, J = 7.0 Hz), 1.52 (3H, s), 1.82 (3H, s), 2.19 (6H, s), 2.85 (1H, septet, J = 7.0 Hz), 3.95 (3H, S), 4.14 (1H, s), 6.88 (2H, br s), 7.07-7.11 (2H, m), 7.30 (1H, s), 7.92-7.96 (2H, m), 8.11-8.16 (2H, m).

### Example 37b

### 4-[[3-(4-Isopropylphenyl)-2,2,4,6,7-pentamethyl-2,3-dihydro-1-benzofuran-5-ylamino]carbonyl]benzoic acid

To a solution of methyl 4-[[3-(4-isopropylphenyl)-2,2,4,6,7-pentamethyl-2,3-dihydro-1-benzofuran-5-ylamino]carbonyl]benzoate (341.7 mg, 0.70 mmol) in a mixed solvent of tetrahydrofuran (10 ml) and methanol (2.5 ml) was added a 1 N aqueous solution of sodium hydroxide (0.75 ml, 0.75 mmol) and the resulting mixture was stirred at room temperature for 3 hours. The reaction mixture was concentrated under reduced pressure and 1N hydrochloric acid was added into the residue. The product was extracted twice with ethyl acetate. The combined extracts were dried on magnesium sulfate, filtered, and concentrated under reduced pressure. The residue was crystallized from ethyl acetate-hexane to obtain the title compound (60% yield). Melting point: 258-261°C.
¹H-NMR (DMSO-d₆) δ: 0.97 (3H, s), 1.17 (6H, d, J = 7.0 Hz), 1.47 (3H, s), 1.71 (3H, s), 2.07 (3H, s), 2.13 (3H, s), 2.84 (1H, septet, J = 7.0 Hz), 4.24 (1H, s), 6.90 (2H, br s), 7.15 (2H, d, J = 7.6 Hz), 8.04 (4H, s), 9.47 (1H, s), 1H unidentified.

### Example 38b

### 5-(4-Methoxybenzylamino)-2,4,6,7-tetramethyl-3-phenyl-1-benzofuran hydrochloride

To a solution of 2,4,6,7-tetramethyl-3-phenyl-1-benzofuran-5-amine (0.50 g, 1.88 mmol) and 4-methoxybenzaldehyde (282 mg, 2.07 mmol) in methanol (15 ml) was added sodium cyanoborohydride (130 mg, 2.07 mmol) at room temperature and the resulting mixture was stirred at room temperature for 1 hour. The reaction mixture was concentrated under reduced pressure and the residue was neutralized with a 1 N aqueous solution of sodium hydroxide. The product was extracted twice with ethyl acetate. The combined organic layers were washed with water, dried on magnesium sulfate, filtered, and concentrated under reduced pressure to obtain 0.37 g of an oily free base. The free base (0.37 g, 0.96 mmol) was dissolved into a hydrochloric acid-methanol mixed solution and the solvent was concentrated under reduced pressure. The resulting residue was crystallized from methanol to obtain 0.21 g (27% yield) of the title compound. Melting point: 200-203°C.
¹H-NMR (CDCl₃) δ: 1.97 (3H, s), 2.30 (3H, s), 2.34 (3H, s), 2.37 (3H, s), 3.73 (3H, s), 4.53 (3H, s), 6.69 (2H, d, J = 8.4 Hz), 7.11-7.25 (4H, m), 7.32-7.37 (3H, m), 1H unidentified.

### Example 39b

### 4-Fluoro-N-(2,4,6,7-tetramethyl-3-phenyl-1-benzofuran-5-yl)benzamide

By using 2,4,6,7-tetramethyl-3-phenyl-1-benzofuran-5-amine and 4-fluorobenzoyl chloride, the title compound was synthesized according to Example 1b. Yield: 80%. Melting point: 242-245°C. (Ethyl acetate-hexane)
¹H-NMR (CDCl₃) δ: 1.96 (3H, s), 2.25 (3H, s), 2.32 (3H, s), 2.45 (3H, s), 7.04-7.14 (2H, m), 7.24-7.50 (6H, m), 7.84-7.93 (2H, m).

### Example 40b

### N-(4-Fluorobenzyl)-2,4,6,7-tetramethyl-3-phenyl-1-benzofuran-5-amine

By using 4-fluoro-N-(2,4,6,7-tetramethyl-3-phenyl-1-benzofuran-5-yl)benzamide, the title compound was synthesized according to Example 2b. Yield: 56%. Melting point: 135-136°C. (Methanol)
¹H-NMR (CDCl₃) δ: 2.00 (3H, s), 2.30 (3H, s), 2.35 (3H, s), 2.45 (3H, s), 3.08 (1H, br s), 3.92 (2H, s), 6.95-7.06 (2H, m), 7.28-7.47 (7H, m).

### Example 41b

### N-[3-(4-Isopropylphenyl)-2,4,6,7-tetramethyl-1-benzofuran-5-yl]benzamide

By using 3-(4-isopropylphenyl)-2,4,6,7-tetramethyl-1-benzofuran-5-amine and benzoyl chloride, the title compound was synthesized according to Example 1b. Yield: 91%. Melting point: 225-227°C. (Ethyl acetate)
¹H-NMR (CDCl₃) δ: 1.29 (6H, d, J = 7.0 Hz), 2.01 (3H, s), 2.30 (3H, s), 2.33 (3H, s), 2.47 (3H, s), 2.95 (1H, septet, J = 7.0 Hz), 7.25 (4H, s), 7.39 (1H, br s),7.41-7.62 (3H, m), 7.88-7.97 (2H, m).

### Example 42b

### N-Benzyl-3-(4-isopropylphenyl)-2,4,6,7-tetramethyl-1-benzofuran-5-amine

By using N-[3-(4-isopropylphenyl)-2,4,6,7-tetramethyl-1-benzofuran-5-yllbenzamide, the title compound was synthesized according to Example 2b. Yield: 55%. Melting point: 94-95°C. (Ethanol)
¹H-NMR (CDCl₃) δ: 1.31 (6H, d, J = 7.0 Hz), 1.95 (1H, br s), 2.04 (3H, s), 2.31 (3H, S), 2.37 (3H, s), 2.45 (3H, s), 2.97 (1H, septet, J = 7.0 Hz), 3.96 (2H, s), 7.23-7.44 (9H, m).

### Example 43b

### N-[3-(4-Isopropylphenyl)-2,4,6,7-tetramethyl-1-benzofuran-5-yl]-4-methoxybenzamide

By using 3-(4-isopropylphenyl)-2,4,6,7-tetramethyl-1-benzofuran-5-amine and 4-methoxybenzoyl chloride, the title compound was synthesized according to Example 1b. Yield: 49%. Amorphous.
¹H-NMR (CDCl₃) δ: 1.29 (6H, d, J = 7.0 Hz), 1.99 (3H, S), 2.28 (3H, s), 2.32 (3H, s), 2.46 (3H, s), 2.95 (1H, septet, J = 7.0 Hz), 3.86 (3H, s), 6.95 (2H, d, J = 8.8 Hz), 7.24 (4H, s), 7.33 (1H, br s), 7.88 (2H, d, J = 8.8 Hz).

### Example 44b

### N-[3-(4-Isopropylphenyl)-2,4,6,7-tetramethyl-1-benzofuran-5-yl]-4-methoxyphenylacetamide

By using 3-(4-isopropylphenyl)-2,4,6,7-tetramethyl-1-benzofuran-5-amine and 4-methoxyphenylacetyl chloride, the title compound was synthesized according to Example 1b. Yield: 42%. Melting point: 202-204°C. (Methanol)
¹H-NMR (CDCl₃) δ: 1.30 (6H, d, J = 7.0 Hz), 1.84 (3H, s), 2.13 (3H, s), 2.28 (3H, s), 2.40 (3H, s), 2.95 (1H, septet, J = 7.0 Hz), 3.72 (2H, s), 3.81 (3H, s), 6.58 (1H, br s), 6.92 (2H, d, J = 8.8 Hz), 7.20-7.33 (6H, m).

### Example 45b

### 3-(4-Isopropylphenyl)-N-(4-methoxybenzyl)-2,4,6,7-tetramethyl-1-benzofuran-5-amine hydrochloride.

By using [N-[3-(4-isopropylphenyl)-2,4,6,7-tetramethyl-1-benzofuran-5-yl]]-4-methoxyphenylacetamide, the title compound was synthesized according to Example 6b. Yield: 87%. Amorphous.
¹H-NMR (CDCl₃) δ: 1.28 (6H, d, J = 7.0 Hz), 2.00 (3H, S), 2.30 (3H, s), 2.32 (3H, s), 2.35 (3H, s), 2.94 (1H, septet, J = 7.0 Hz), 3.72 (3H, s), 4.53 (2H, s), 6.68-6.72 (4H, m), 7.07-7.25 (4H, m), 10.9 (1H, br s), 1H unidentified.

### Example 46b

### 4-Fluoro-N-[3-(4-isopropylphenyl)-2,4,6,7-tetramethyl-1-benzofuran-5-yl]benzamide

By using 3-(4-isopropylphenyl)-2,4,6,7-tetramethyl-1-benzofuran-5-amine and 4-fluorobenzoyl chloride, the title compound was synthesized according to Example 1b. Yield: 72%. Melting point: 242-245°C. (Ethyl acetate-hexane)
¹H-NMR (CDCl₃) δ: 1.28 (6H, d, J = 7.0 Hz), 1.98 (3H, s), 2.26 (3H, s), 2.33 (3H, s), 2.45 (3H, s), 2.95 (1H, septet, J = 7.0 Hz), 7.06-7.17 (2H, m), 7.24 (4H, s), 7.39 (1H, br s), 7.86-7.95 (2H, m) .

### Example 47b

### N-(4-Fluorobenzyl)-3-(4-isopropylphenyl)-2,4,6,7-tetramethyl-1-benzofuran-5-amine

To a suspension of aluminum chloride (807 mg, 6.05 mmol) in tetrahydrofuran (10 ml) was gradually added lithium aluminum hydride (230 mg, 6.05 mmol) under ice cooling and the resulting mixture was stirred at the same temperature for 10 minutes. To this mixture was added 4-fluoro-N-[3-(4-isopropylphenyl)-2,4,6,7-tetramethyl-1-benzofuran-5-yl]benzamide (0.52 g, 1.21 mmol) and the mixture was refluxed with heating for 3 hours. The reaction mixture was poured into ice water and neutralized with an 8 N aqueous solution of sodium hydroxide. The product was extracted twice with ethyl acetate. The combined organic layers were washed with water, dried on magnesium sulfate, filtered, and then concentrated under reduced pressure. The residue was crystallized from ethanol to obtain 0.27 g (54% yield) of the title compound. Melting point: 95-97°C.
¹H-NMR (CDCl₃) δ: 1.30 (6H, d, J = 7.0 Hz), 1.98 (1H, br s), 2.02 (3H, s), 2.31 (3H, s), 2.34 (3H, s), 2.45 (3H, s), 2.96 (1H, septet, J = 7.0 Hz), 3.92 (2H, s), 6.95-7.06 (2H, m), 7.24-7.40 (6H, m).

### Example 48b

### N-[3-(4-Fluorophenyl)-2,4,6,7-tetramethyl-1-benzofuran-5-yl]-4-methoxybenzamide

By using 3-(4-fluorophenyl)-2,4,6,7-tetramethyl-1-benzbfuran-5-amine and 4-methoxybenzoyl chloride, the title compound was synthesized according to Example 1b. Yield: 75%. Melting point: 225-227°C. (Ethyl acetate)
¹H-NMR (CDCl₃) δ: 1. 96 (3H, s), 2.27 (3H, s), 2.30 (3H, s), 2.45 (3H, s), 3.86 (3H, s), 6.95 (2H, d, J = 8.8 Hz), 7.03-7.13 (2H, m), 7.24-7.36 (3H, m), 7.88 (2H, d, J = 8.8 Hz).

### Example 49b

### N-(4-Methoxybenzyl)-3-(4-fluorophenyl)-2,4,6,7-tetramethyl-1-benzofuran-5-amine

By using N-[3-(4-fluorophenyl)-2,4,6,7-tetramethyl-1-benzofuran-5-yl]-4-methoxybenzamide, the title compound was synthesized according to Example 2b. Yield: 75%. Melting point: 100-102°C. (Ethanol)
¹H-NMR (CDCl₃) δ: 2.01 (3H, s), 2.20-2.60 (10H, m), 3.81 (3H, s), 3.89 (2H, s), 6.87 (2H, d, J = 8.8 Hz), 7.05-7.16 (2H, s), 7.23-7.34 (4H, m).

### Example 50b

### 4-Fluoro-N-[3-(4-fluorophenyl)-2,4,6,7-tetramethyl-1-benzofuran-5-yl]benzamide

By using 3-(4-fluorophenyl)-2,4,6,7-tetramethyl-1-benzofuran-5-amine and 4-fluorobenzoyl chloride, the title compound was synthesized according to Example 1b. Yield: 75%. Melting point: 232-234°C. (Ethyl acetate-hexane)
¹H-NMR (CDCl₃) δ: 1.97 (3H, s), 2.28 (3H, s), 2.31 (3H, s), 2.46 (3H, s), 7.03-7.37 (7H, m), 7.86-7.98 (2H, m).

### Example 51b

### N-(4-Fluorobenzyl)-3-(4-fluorophenyl)-2,4,6,7-tetramethyl-1-benzofuran-5-amine

By using 4-fluoro-N-[3-(4-fluorophenyl)-2,4,6,7-tetramethyl-1-benzofuran-5-yl]benzamide, the title compound was synthesized according to Example 2b. Yield: 66%. Melting point: 107-109°C. (Ethanol)
¹H-NMR (CDCl₃) δ: 1.87 (1H, br s), 1.99 (3H, s), 2.28 (3H, s), 2.34 (3H, s), 2.45 (3H,s), 3.92 (2H, s), 6.94-7.13 (4H, m), 7.20-7.43 (4H, m).

### Example 52b

### N-[3-(4-Isopropylphenyl)-1',4,6,7-tetramethylspiro[benzofuran-2(3H),4'-piperidine]-5-yl]-4-methoxybenzamide

By using 3-(4-isopropylphenyl)-1',4,6,7-tetramethylspiro[benzofuran-2(3H),4'-piperidine]-5-amine, the title compound was synthesized according to Example 1b. Yield: 40%. Melting point: 277-278°C. (Ethanol-isopropyl ether)
¹H-NMR (CDCl₃) δ: 1.20 (6H, d, J = 7.0 Hz), 1.35-1.45 (2H, m), 1.81 (3H, s), 2.18-2.91 (16H, m), 3.86 (3H, s), 4.09 (1H, s), 6.6-7.1 (6H, m), 7.19 (1H, br), 7.83-7.88 (2H, m).

### Example 53b

### 3-(4=Isopropylphenyl)-N-(methoxybenzyl)-1',4,6,7-tetramethylspiro[benzofuran-2(3H),4'-piperidine]-5-amine

By using N-[3-(4-isopropylphenyl)-1',4,5,7-tetramethylspiro[benzofuran-2(3H),4'-piperidine]-5-yl]-4-methoxybenzamide, the title compound was synthesized according to Example 2b. Yield: 59%. Amorphous.
¹H-NMR (CDCl₃) δ: 1.21 (6H, d, J = 7.0 Hz), 1.3-1.4 (2H, m), 1.79 (3H, s), 1.8-2.0 (3H, m), 2.21 (3H, s), 2.27 (3H, s), 2.31 (3H, s), 2.4-2.7 (4H, m), 2.85 (1H, septet, J = 7.0 Hz), 3.79 (3H, s), 3.85 (2H, s), 4.05 (1H, s), 6.6-7.1 (6H, m), 7.23-7.27 (2H, m).

### Example 54b

### 4-Fluoro-N-[3-(4-isopropylphenyl)-1',4,6,7-tetramethylspiro[benzofuran-2(3H),4'-piperidine]-5-yl]benzamide

By using 3-(4-isopropylphenyl)-1',4,6,7-tetramethylspiro[benzofuran-2(3H),4'-piperidine]-5-amine and 4-fluorobenzoyl chloride, the title compound was synthesized according to Example 1b. Yield: 38%. Melting point: 271-272°C. (Methanol-isopropyl ether)
¹H-NMR (CDCl₃) δ: 1.20 (6H, d, J = 7.0 Hz), 1.30-1.40 (2H, m), 1.81 (3H, s), 2.02-2.12 (2H, m), 2.18 (3H, s), 2.22 (3H, s), 2.30 (3H, s), 2.37-2.71 (4H, m), 2.85 (1H, septet, J = 7.0 Hz), 4.10 (1H, s), 6.6-7.2 (6H, m), 7.24 (1H, br), 7.86-7.93 (2H, m).

### Example 55b

### N-(4-Fluorobenzyl)-3-(4-isopropylphenyl)-1',4,6,7-tetramethylspiro[benzofuran-2(3H),4'-piperidine]-5-amine

By using 4-fluoro-N-[3-(4-isopropylphenyl)-1',4,6,7-tetramethylspiro[benzofuran-2(3H),4'-piperidine]-5-yl]benzamide, the title compound was synthesized according to Example 2b. Yield: 83%. Amorphous.
¹H-NMR (CDCl₃) δ: 1.21 (6H, d, J = 7.0 Hz), 1.34-1.42 (2H, m), 1.75 (3H, s), 1.80-2.05 (3H, m), 2.21 (3H, s), 2.26 (3H, s), 2.31 (3H, s), 2.35-2.72 (4H, m), 2.85 (1H, septet, J = 7.0 Hz), 3.87 (2H, s), 4.04 (1H, s), 6.5-7.1 (6H, m), 7.23-7.30 (2H, m).

### Example 56b

### 4-Chloro-N-[3-(4-isopropylphenyl)-1',4,6,7-tetramethylspiro[benzofuran-2(3H),4'-piperidine]-5-yl]benzamide

By using 3-(4-isopropylphenyl)-1',4,6,7-tetramethylspiro[benzofuran-2(3H),4'-piperidine]-5-amine and 4-chlorobenzoyl chloride, the title compound was synthesized according to Example 1b. Yield: 58%. Melting point: 293-295°C. (Methanol)
¹H-NMR (CDCl₃) δ: 1.09 (6H, d, J = 7.0 Hz), 1.3-1.4 (2H, m), 1.7-2.7 (18H, m), 2.84 (1H, septet, J = 7.0 Hz), 4.09 (1H, s), 6.6-7.1 (4H, m), 7.33 (1H, br), 7.40-7.44 (2H, m), 7.79-7.83 (2H, m).

### Example 57b

### N-(4-Chlorobenzyl)-3-(4-isopropylphenyl)-1',4,6,7-tetramethylspiro[benzofuran-2(3H),4'-piperidine]-5-amine

By using 4-chloro-N-[3-(4-isopropylphenyl)-1',4,6,7-tetramethylspiro[benzofuran-2(3H),4'-piperidine]-5-yl]benzamide, the title compound was synthesized according to Example 2b. Yield: 96%. Amorphous.
¹H-NMR (CDCl₃) δ: 1.22 (6H, d, J = 7.0 Hz), 1.3-1.4 (2H, m), 1.74 (3H, s), 1.8-2.1 (3H, m), 2.21 (3H, s), 2.25 (3H, s), 2.30 (3H, s), 2.34-2.69 (4H, m), 2.86 (1H, septet, J = 7.0 Hz), 3.83 (2H, s), 4.04 (1H, s), 6.6-7.1 (4H, m), 7.24 (4H, s).

### Example 58b

### N-[3-(4-Isopropylphenyl)-2,2,4;6,7-pentamethyl-2,3-dihydro-1-benzofuran-5-yl]-3,4-dimethoxybenzamide

By using 3-(4-isopropylphenyl)-2,2,4,6,7-pentamethyl-2,3-dihydro-1-benzofuran-5-amine and 3,4-dimethoxybenzoyl chloride, the title compound was synthesized according to Example 1b. Yield: 71%. Melting point: 171-173°C. (Ethyl acetate-hexane)
¹H-NMR (CDCl₃) δ: 1.03 (3H, s), 1.21 (6H, d, J = 7.0 Hz), 1.52 (3H, s), 1.82 (3H, s), 2.19 (6H, s), 2.85 (1H, septet, J = 7.0 Hz), 3.938 (3H, s), 3.943 (3H, s), 4.13 (1H, s), 6.80-7.00 (3H, m), 7.09 (2H, d, J = 7.6 Hz), 7.22 (1H, br s), 7.42 (1H, dd, J = 8.4, 2.2 Hz), 7.52 (1H, d, J = 2.2 Hz).

### Example 59b

### N-(3,4-Dimethoxybenzyl)-3-(4-isopropylphenyl)-2,2,4,6,7-pentamethyl-2,3-dihydro-1-benzofuran-5-amine hydrochloride

By using N-[3-(4-isopropylphenyl)-2,2,4,6,7-pentamethyl-2,3-dihydro-1-benzofuran-]-5-yl]-3,4-dimethoxybenzamide, the title compound was synthesized according to Example 6b. Yield: 76%. Melting point: 181-184°C. (Ethanol-hexane)
¹H-NMR (DMSO-d₆) δ: 0.92 (3H, s), 1.19 (6H, d, J = 7.0 Hz), 1.42 (3H, s), 1.69 (3H, s), 2.10 (3H, s), 2.22 (3H, s), 2.85 (1H, septet, J = 7.0 Hz), 3.66 (3H, s), 3.75 (3H, s), 4.17 (1H, s), 4.20-4.42 (2H, m), 6.40-6.90 (5H, m), 7.13 (2H, d, J = 7.4 Hz), 10.0 (1H, br s), 1H unidentified.

### Example 60b

### (+)-4-Fluoro-N-[3-(4-isopropylphenyl)-2,2,4,6,7-pentamethyl-2,3-dihydro-1-benzofuran-5-yl]benzamide

By using (+)-3-(4-isopropylphenyl)-2,2,4,6,7-pentamethyl-2,3-dihydro-1-benzofuran-5-amine and 4-fluorobenzoyl chloride, the title compound was synthesized according to Example 1b. Yield: 91%. Melting point: 251-253°C. (Ethyl acetate-hexane) [α]D = +74.4° (c = 0.501, methanol)
¹H-NMR (CDCl₃) δ: 1.01 (3H, s), 1.19 (6H, d, J = 6.8 Hz), 1.50 (3H, s), 1.78 (3H, s), 2.15 (3H, s), 2.17 (3H, s), 2.83 (1H, septet, J = 6.8 Hz), 4.12 (1H, s), 6.60-7.40 (7H, m), 7.80-7.91 (2H, m).

### Example 61b

### (+)-N-(4-Fluorobenzyl)-3-(4-isopropylphenyl)-2,2,4,6,7-pentamethyl-2,3-dihydro-1-benzofuran-5-amine hydrochloride

To a suspension of aluminum chloride (0.67 g, 5.05 mmol) in tetrahydrofuran (15 ml) was gradually added lithium aluminum hydride (190 mg, 5.05 mmol) under ice cooling and the resulting mixture was stirred at the same temperature for 10 minutes. To this mixture was added (+)-4-fluoro-N-[3-(4-isopropylphenyl)-2,2,4,6,7-pentamethyl-2,3-dihydro-1-benzofuran-5-yl]benzamide (0.45 g, 1.01 mmol) and the mixture was refluxed with heating for 3 hours. The reaction mixture was poured into ice water and neutralized with an 8 N aqueous solution of sodium hydroxide. The product was extracted twice with ethyl acetate. The combined organic layers were washed with water, dried on magnesium sulfate, filtered, and then concentrated under reduced pressure to obtain 0.29 g of an oily free base. The free base (0.29 g, 0.67 mmol) was dissolved into a mixed solution of hydrochloric acid and methanol and solvent was concentrated under reduced pressure. The resulting residue was crystallized from methanol to obtain 0.27 g (56% yield) of the title compound. Melting point: 158-160°C. [α]D = +70.7° (c = 0.461, methanol)
¹H-NMR (DMSO-d₆) δ: 0.93 (3H, s), 1.20 (6H, d, J = 6.6 Hz), 1.41 (3H, s), 1.55 (3H, s), 2.11 (3H, S), 2.31 (3H, s), 2.85 (1H, septet, J = 6.6 Hz), 4.13 (1H, s), 4.31 (1H, d, J = 12.8 Hz), 4.45 (1H, d, J = 12.8 Hz), 7.02-7.29 (8H, m), 10.3 (1H, br s), 10.8 (1H, br s).

### Example 62b

### (-)-4-Fluoro-N-[3-(4-isopropylphenyl)-2,2,4,6,7-pentamethyl-2,3-dihydro-1-benzofuran-5-yl]benzamide

By using (-)-3-(4-isopropylphenyl)-2,2,4,6,7-pentamethyl-2,3-dihydro-1-benzofuran-5-amine and 4-fluorobenzoyl chloride, the title compound was synthesized according to Example 1b. Yield: 91%. Melting point: 253-254°C. [α]D = -77.4° (c = 0.500, methanol)
¹H-NMR (CDCl₃) δ: 1.03 (3H, s), 1.21 (6H, d, J = 7.0 Hz), 1.51 (3H, s), 1.81 (3H, s), 2.18 (6H, s), 2.85 (1H, septet, J = 7.0 Hz), 4.13 (1H, s), 6.8-7.4 (7H, m), 7.86-7.93 (2H, m).

### Example 63b

### (-)-N-(4-Fluorobenzyl)-3-(4-isopropylphenyl)-2,2,4,6,7-pentamethyl-2,3-dihydro-1-benzofuran-5-amine hydrochloride

To a suspension of aluminum chloride (351 mg, 2.63 mmol) in tetrahydrofuran (35 ml) was gradually added lithium aluminum hydride (101 mg, 2.67 mmol) under ice cooling and the resulting mixture was stirred at the same temperature for 10 minutes. To this mixture was added (-)-4-fluoro-N-[3-(4-isopropylphenyl)-2,2,4,6,7-pentamethyl-2,3-dihydro-1-benzofuran-5-yl]benzamide (528 mg, 1.19 mmol) and the mixture was refluxed with heating for 2 hours. The reaction mixture was poured into ice water and neutralized with an 8 N aqueous solution of sodium hydroxide. The product was extracted twice with ethyl acetate. The combined organic layers were washed with water, dried on magnesium sulfate, filtered, and then concentrated under reduced pressure to obtain 502 mg of an oily free base. The free base (502 mg, 1.17 mmol) was dissolved into a mixed solution of hydrochloric acid and methanol and the solvent was concentrated under reduced pressure. The resulting residue was crystallized from methanol to obtain 115 mg (21% yield) of the title compound. Melting point: 148-151°C. [α] D = -70.5° (c = 0.503, methanol)
¹H-NMR (DMSO-d₆) δ: 0.92 (3H, s), 1.19 (6H, d, J = 6.8 Hz), 1.41 (3H, s), 1.54 (3H, s), 2.11 (3H, s), 2.32 (3H, s), 2.85 (1H, septet, J = 6.8 Hz), 4.16 (1H, s), 4.29-4.45 (2H, m), 6.6-7.4 (8H, m), 10.2-10.6 (2H, m).

### Example 64b

### 3,4-Dimethoxy-N-[2,2,4,6,7-pentamethyl-3-(4-methylphenyl)-2,3-dihydro-1-benzofuran-5-yl]benzamide

By using 2,2,4,6,7-pentamethyl-3-(4-methylphenyl)-2,3-dihydro-1-benzofuran-5-amine and 3,4-dimethoxybenzoyl chloride, the title compound was synthesized according to Example 1b. Yield: 90%. Melting point: 169-171°C. (Ethyl acetate-hexane)
¹H-NMR (CDCl₃) δ: 1.03 (3H, s), 1.51 (3H, s), 1.80 (3H, s), 2.19 (6H, s), 2.29 (3H, s), 3.92 (6H, s), 4.13 (1H, s), 6.60-7.20 (5H, m), 7.29 (1H, br s), 7.42 (1H, dd, J = 8.2, J=2.0 Hz), 7.51 (1H, d, J=2.0Hz).

### Example 65b

### N-(3,4-Dimethoxybenzyl)-2,2,4,6,7-pentamethyl-3-(4-methylphenyl)-2,3-dihydro-1-benzofuran-5-amine hydrochloride

By using 3,4-dimethoxy-N-[2,2,4,6,7-pentamethyl-3-(4-methylphenyl)-2,3-dihydro-1-benzofuran-5-yl]benzamide, the title compound was synthesized according to Example 6b. Yield: 68%. Melting point: 195-198°C. (Ethanol-hexane)
¹H-NMR (DMSO-d₆) δ: 0.93 (3H, s), 1.41 (3H, s), 1.65 (3H, s), 2.10 (3H, s), 2.23 (3H, s), 2.27 (3H, s), 3.66 (3H, s), 3.73 (3H, s), 4.16 (1H, s), 4.23 (1H, d, J = 12.4 Hz), 4.35 (1H, d, J = 12.4 Hz), 6.40-6.82 (5H, m), 7.08 (2H, d, J = 7.0 Hz), 10.2 (1H, br s), 1H unidentified.

### Example 66b

### N-[2,2,4,6,7-Pentamethyl-3-(4-methylphenyl)-2,3-dihydro-1-benzofuran-5-yl]-1,3-benzodioxol-5-carboxamide

By using 2,2,4,6,7-pentamethyl-3-(4-methylphenyl)-2,3-dihydro-1-benzofuran-5-amine and 1,3-benzodioxol-5-carbonyl chloride, the title compound was synthesized according to Example 1b. Yield: 65%. Melting point: 164-165°C. (Ethyl acetate-hexane)
¹H-NMR (CDCl₃) δ: 1.03 (3H, s), 1.51 (3H, s), 1.79 (3H, s), 2.17 (3H, s), 2.18 (3H, s), 2.30 (3H, s), 4.12 (1H, s), 6.03 (2H, s), 6.62-7.12 (5H, m), 7.16 (1H, br s), 7.34-7.45 (2H, m).

### Example 67b

### N-(1,3-Benzodioxol-5-ylmethyl)-2,2,4,6,7-pentamethyl-3-(4-methylphenyl)-2,3-dihydro-1-benzofuran-5-amine hydrochloride

By using N-[2,2,4,6,7-pentamethyl-3-(4-methylphenyl)-2,3-dihydro-1-benzofuran-5-yl]-1,3-benzodioxol-5-carboxamide, the title compound was synthesized according to Example 6b. Yield: 62%. Melting point: 147-149°C. (Ethanol-hexane)
¹H-NMR (CDCl₃) δ: 0.95 (3H, s), 1.42 (3H, s), 1.72 (3H, s), 2.10 (3H, s), 2.25 (3H, s), 2.27 (3H, s), 4.17 (1H, s), 4.28 (1H, s), 5.97 (1H, s), 6.01 (1H, s), 6.40-7.18 (8H, m), 10.2 (1H, br s).

The chemical structures of the compounds obtained in the above-described Examples are shown below.

**Table 3**

| | | | | | | | | |
|---|---|---|---|---|---|---|---|---|
| 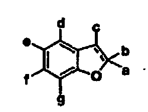 | | | | | | | | |

| example number | a | b | c | d | e | f | g | -̅ -̅ -̅ |
|---|---|---|---|---|---|---|---|---|
| 1b | Me | Me | 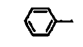 | Me | 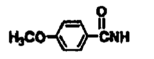 | Me | Me | --- |
| 2b | Me | Me | 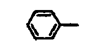 | Me | 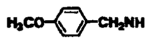 | Me | Me | --- |
| 3b | Me | Me | 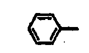 | Me | 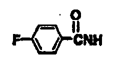 | Me | Me | --- |
| 4b | Me | Me | 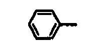 | Me | 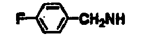 | Me | Me | --- |
| 5b | Me | Me | 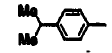 | Me | 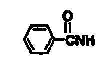 | Me | Me | --- |
| 6b | Me | Me | 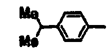 | Me | 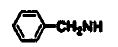 | Me | Me | --- |
| 7b | Me | Me | 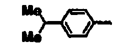 | Me | 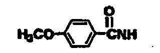 | Me | Me | --- |
| 8b | Me | Me | 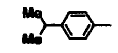 | Me | 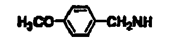 | Me | Me | --- |
| 9b | Me | Me | 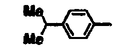 | Me | 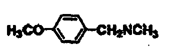 | Me | Me | --- |
| 10b | Me | Me | 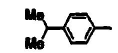 | Me | 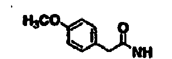 | Me | Me | --- |
| 11b | Me | Me | 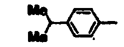 | Me | 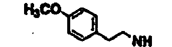 | Me | Me | --- |
| 12b | Me | Me | 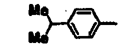 | Me | 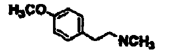 | Me | Me | --- |
| 13b | Me | Me | 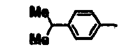 | Me | 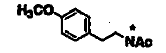 | Me | Me | --- |
| 14b | Me | Me | 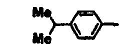 | Me | 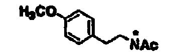 | Me | Me | --- |
| 15b | Me | Me | 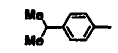 | Me | 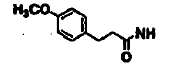 | Me | Me | --- |
| 16b | Me | Me | 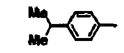 | Me | 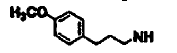 | Me | Me | --- |
| 17b | Me | Me | 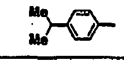 | Me | 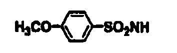 | Me | Me | --- |

**Table 4**

| | | | | | | | | |
|---|---|---|---|---|---|---|---|---|
| 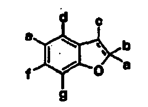 | | | | | | | | |

| example number | a | b | c | d | e | f | g | -̅ -̅ -̅ |
|---|---|---|---|---|---|---|---|---|
| 18b | Me | Me | 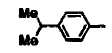 | Me | 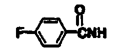 | Me | Me | --- |
| 19b | Me | Me | 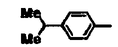 | Me | 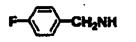 | Me | Me | --- |
| 20b | Me | Me | 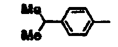 | Me | 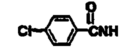 | Me | Me | --- |
| 21b | Me | Me | 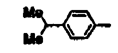 | Me | 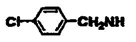 | Me | Me | --- |
| 22b | Me | Me | 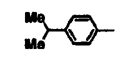 | Me | 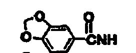 | Me | Me | --- |
| 23b | Me | Me | 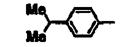 | Me | 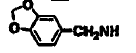 | Me | Me | --- |
| 24b | Me | Me | 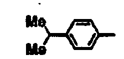 | Me | 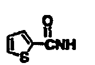 | Me | Me | --- |
| 25b | Me | Me | 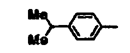 | Me | 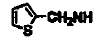 | Me | Me | --- |
| 26b | Me | Me | 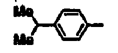 | Me | 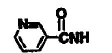 | Me | Me | --- |
| 27b | Me | Me | 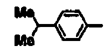 | Me | 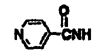 | Me | Me | --- |
| 28b | Me | Me | 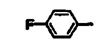 | Me | 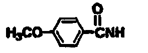 | Me | Me | --- |
| 29b | Me | Me | 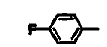 | Me | 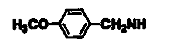 | Me | Me | --- |
| 30b | Me | Me | 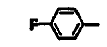 | Me | 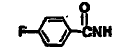 | Me | Me | --- |
| 31b | Me | Me | 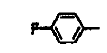 | Me | 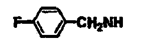 | Me | Me | --- |
| 32b | Me | Me | 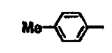 | Me | 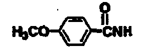 | Me | Me | --- |
| 33b | Me | Me | 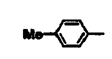 | Me | 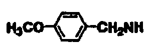 | Me | Me | --- |
| 34b | Me | Me | 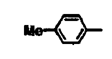 | Me | 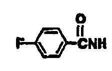 | Me | Me | --- |

**Table 5**

| | | | | | | | | |
|---|---|---|---|---|---|---|---|---|
| 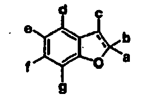 | | | | | | | | |

| example number | a | b | c | d | e | f | g | -̅ -̅ -̅ |
|---|---|---|---|---|---|---|---|---|
| 35b | Me | Me | 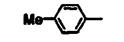 | Me | 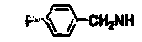 | Me | Me | --- |
| 36b | Me | Me | 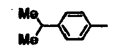 | Me | 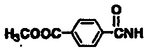 | Me | Me | --- |
| 37b | Me | Me | 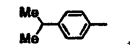 | Me | 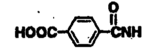 | Me | Me | --- |
| 38b | Me | - | 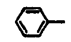 | Me | 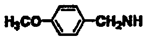 | Me | Me | -̅-̅-̅ |
| 39b | Me | - | 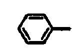 | Me | 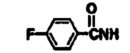 | Me | Me | -̅-̅-̅ |
| 40b | Me | - | 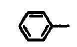 | Me | 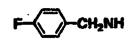 | Me | Me | -̅-̅-̅ |
| 41b | Me | - | 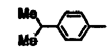 | Me | 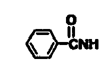 | Me | Me | -̅-̅-̅ |
| 42b | Me | - | 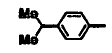 | Me | 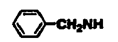 | Me | Me | -̅-̅-̅ |
| 43b | Me | - | 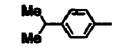 | Me | 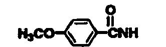 | Me | Me | -̅-̅-̅ |
| 44b | Me | - | 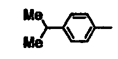 | Me | 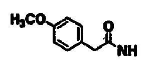 | Me | Me | -̅-̅-̅ |
| 45b | Me | - | 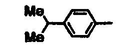 | Me | 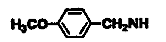 | Me | Me | -̅-̅-̅ |
| 46b | Me | - | 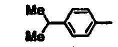 | Me | 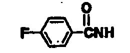 | Me | Me | -̅-̅-̅ |
| 47b | Me | - | 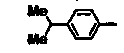 | Me | 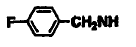 | Me | Me | -̅-̅-̅ |
| 48b | Me | - | 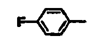 | Me | 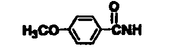 | Me | Me | -̅-̅-̅ |
| 49b | Me | - | 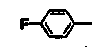 | Me | 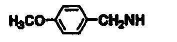 | Me | Me | -̅-̅-̅ |
| 50b | Me | - | 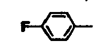 | Me | 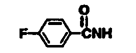 | Me | Me | -̅-̅-̅ |
| 51b | Me | - | 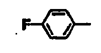 | Me | 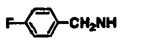 | Me | Me | -̅-̅-̅ |

**Table 6**

| | | | | | | |
|---|---|---|---|---|---|---|
| 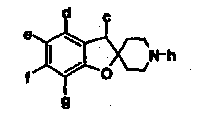 | | | | | | |

| example number | c | d | e | f | g | h |
|---|---|---|---|---|---|---|
| 52b | 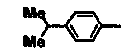 | Me | 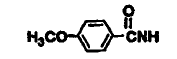 | Me | Me | Me |
| 53b | 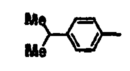 | Me | 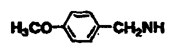 | Me | Me | Me |
| 54b | 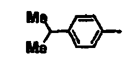 | Me | 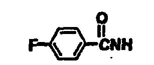 | Me | Me | Me |
| 55b | 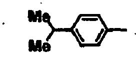 | Me | 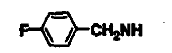 | Me | Me | Me |
| 56b | 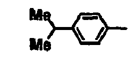 | Me | 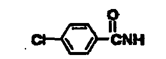 | Me | Me | Me |
| 57b | 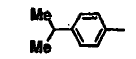 | Me | 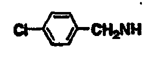 | Me | Me | Me |

**Table 7**

| | | | | | | | | | |
|---|---|---|---|---|---|---|---|---|---|
| 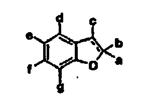 | | | | | | | | | |

| example number | a | b | c | d | e | f | g | -̅ -̅ -̅ | optical rotatory power |
|---|---|---|---|---|---|---|---|---|---|
| 58b | Me | Me | 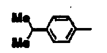 | Me | 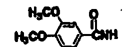 | Me | Me | --- | |
| 59b | Me | Me | 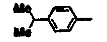 | Me | 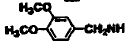 | Me | Me | --- | |
| 60b | Me | Me | 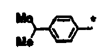 | Me | 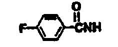 | Me | Me | --- | + |
| 61b | Me | Me | 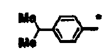 | Me | 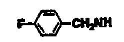 | Me | Me | --- | + |
| 62b | Me | Me | 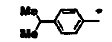 | Me | 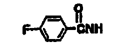 | Me | Me | --- | - |
| 63b | Me | Me | 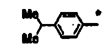 | Me | 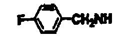 | Me | Me | --- | - |
| 64b | Me | Me | 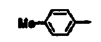 | Me | 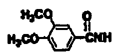 | Me | Me | --- | |
| 65b | Me | Me | 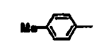 | Me | 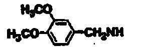 | Me | Me | --- | |
| 66b | Me | Me | 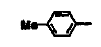 | Me | 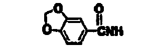 | Me | Me | --- | |
| 67b | Me | Me | 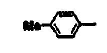 | Me | 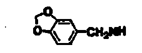 | Me | Me | --- | |

### Formulation Example 1b

| | | |
|---|---|---|
| (1) | The compound obtained in Example 19b | 50 mg |
| (2) | Lactose | 34 mg |
| (3) | Corn starch | 10.6 mg |
| (4) | Corn starch (paste) | 5 mg |
| (5) | Magnesium Stearate | 0.4 mg |
| (6) | Calcium carboxymethyl cellulose | 20 mg |
| | Total | 120 mg |

According to a conventional method, tablets were prepared by mixing the above-described substances (1) to (6), and then subjecting the resulting mixture to a tablet compression process By using a tablet compression machine.

### [Compounds (Ic)]

### Reference Example 1c

### Methyl α-bromophenylacetate

Concentrated sulfuric acid (0.5 mL) was added to a solution of α-bromophenylacetic acid (3.00 g, 13.9 mmol) in ethanol (30 mL) at room temperature, and the mixture was heated under reflux for 1 hour. The reaction mixture was cooled, and extracted twice with ethyl acetate. The organic layers were combined, washed with an aqueous saturated sodium hydrogencarbonate, then dried over magnesium sulfate, filtered, and concentrated under reduced pressure to obtain the title compound (2.50 g, yield 79 %). This was oily.
¹H-NMR (CDCl₃) δ: 3.78 (3H, s), 5.36 (1H, s), 7.29-7.42 (3H, m), 7.48-7.61 (2H, m).

### Reference Example 2c

### 1-Bromo-4-(4-morpholinyl)benzene

Bromine (10.8 g, 67.4 mmol) was added to a solution of (4-morpholinyl)benzene (10.0 g, 61.3 mmol) in ethanol (100 mL) at 0°C, and the mixture was stirred for 1 hour at room temperature. Water (100 mL) was poured into the reaction mixture, which was then extracted twice with ethyl acetate. The organic layers were combined, washed with an aqueous saturated sodium hydrogencarbonate and water, then dried over magnesium sulfate, filtered, and concentrated under reduced pressure. The residue was crystallized from ethyl acetate-hexane to obtain the title compound (10.7 g, yield 72 %).
m.p.: 118-120°C.
¹H-NMR (CDCl₃) δ: 2.98-3.22 (4H, m), 3.71-3.92 (4H, m), 6.72-6.83 (2H, m), 7.31-7.42 (2H, m).

### Reference Example 3c

### 1-Bromo-4-(4-methyl-1-piperazinyl)benzene

Sodium hydride (60 % liquid paraffin dispersion, 2.70 g, 67.8 mmol) was added to a solution of 1-phenylpiperazine (10.0 g, 61.6 mmol) in N,N-dimethylformamide (80 mL) at 0°C, and the mixture was stirred for 10 minutes at the same temperature. To the reaction mixture was added iodomethane (8.74 g, 67.8 mmol), and the mixture was stirred for 30 minutes at room temperature. The reaction mixture was poured into water (80 mL), and extracted twice with ethyl acetate. The organic layers were combined, washed with water, dried over magnesium sulfate, filtered, and concentrated under reduced pressure. The residue was crystallized from hexane-isopropyl ether to obtain 1-methyl-4-phenylpiperazine (7.40 g). Bromine (7.00 g, 43.8 mmol) was added to a solution of this compound in ethanol (80 mL) at 0°C, and the mixture was stirred for 1 hour at room temperature. Water (80 mL) was poured into the reaction mixture, which was then extracted twice with ethyl acetate. The organic layer was combined, washed with an aqueous saturated sodium hydrogencarbonate and water, then dried over magnesium sulfate, filtered, and concentrated under reduced pressure. The residue was crystallized from ethyl acetate-hexane to obtain the title compound (8.1 g, yield 52 %).
m.p.: 78-80°C.
¹H-NMR (CDCl₃) δ: 2.35 (3H, s), 2.52-2.63 (4H, m), 3.13-3.26 (4H, m), 6.78 (2H, d, J=8.8Hz), 7.33 (2H, d, J = 8.8 Hz).

### Reference Example 4c

### 2-Methyl-1-[4-(4-morpholinyl)phenyl]propan-1-one

n-Butyllithium (1.6 M, 25.8 mL, 41.3 mmol) was added to a solution of 1-bromo-4-(4-morpholinyl)benzene (10.0 g, 41.3 mmol) in tetrahydrofuran (100 mL) at -78°C, and the mixture was stirred for 20 minutes at the same temperature. To the reaction mixture was added N-isobutyrylpropyleneimine (5.77 g, 45.4 mmol), and the mixture was stirred for 30 minutes at room temperature. Water (40 mL) was poured into the reaction mixture, which was then extracted twice with ethyl acetate. The organic layers were combined, washed with water, dried over magnesium sulfate, filtered, and concentrated under reduced pressure. The residue was crystallized from hexane to obtain the title compound (6.50 g, yield 67 %).
m.p.: 75-77°C.
¹H-NMR (CDCl₃) δ: 1.19 (6H, d, J = 7.0 Hz), 3.22-3.33 (4H, m), 3.50 (1H, septet, J = 7.0 Hz), 3.81-3.92 (4H, m), 6.81-6.92 (2H, m), 7.85-8.95 (2H, m).

### Reference Example 5c

### 2-Methyl-1-[4-(4-methyl-1-piperazinyl)phenyl]propan-1-one

Using 1-bromo-4-(4-methyl-1-piperazinyl)benzene the title compound was obtained in the same manner as in Reference Example 4.
Yield: 81 %.
m.p.: 74-76°C (from methanol).
¹H-NMR (CDCl₃) δ: 1.19 (6H, d, J = 6.6 Hz), 2.35 (3H, s), 2.46-2.63 (4H, m), 3.32-3.41 (4H, m), 3.50 (1H, septet, J = 7.0 Hz), 6.84-6.92 (2H, m), 7.85-7.95 (2H, m).

### Reference Example 6c

### 1-(2,5-Dimethoxy-3,4,6-trimethylphenyl)-2-methyl-1-[4-(4-morpholinyl)phenyl]propan-1-ol

n-Butyllithium (1.6 M, 18.1 mL, 29.0 mmol) was added to a solution of 1-bromo-2,5-dimethoxy-3,4,6-trimethylbenzene (7.52 g, 29.0 mmol) in tetrahydrofuran (50 mL) at -78°C, and the mixture was stirred for 20 minutes at the same temperature. To the reaction mixture was added 2-methyl-1-[4-(4-morpholinyl)phenyl]propan-1-one (6.15 g, 26.4 mmol), and the mixture was stirred for 30 minutes at room temperature. Water (40 mL) was poured into the reaction mixture, which was then extracted three times with ethyl acetate. The organic layers were combined, washed with water, dried over magnesium sulfate, filtered, and concentrated under reduced pressure. The residue was crystallized from ethanol to obtain the title compound (8.40 g, yield 90 %).
m.p.: 191-193°C.
¹H-NMR (CDCl₃) δ: 0.87-1.10 (6H, m), 2.11 (3H, s), 2.18 (3H, s), 2.45 (3H, s), 2.80-3.18 (8H, m), 3.62 (3H, s), 3.75-3.90 (4H, m), 6.41 (1H, br s), 6.82 (2H, d, J = 8.8 Hz), 7.34 (2H, d, J = 8.8 Hz).

### Reference Example 7c

### 1-(2,5-Dimethoxy-3,4,6-trimethylphenyl)-2-methyl-1-[4-(4-methyl-1-piperazinyl)phenyl]propan-1-ol

Using 2-methyl-1-[4-(4-methyl-1-piperazinyl)phenyl]propan-1-one, the title compound was obtained in the same manner as in Reference Example 6.
Yield: 43 %.
m.p.: 114-116°C (from methanol).
¹H-NMR (CDCl₃) δ: 0.97 (6H, t, J = 6.6 Hz), 2.11 (3H, s), 2.18 (3H, s), 2.34 (3H, s), 2.45 (3H, s), 2.50-2.62 (4H, m), 2.76-3.00 (1H, m), 3.02 (3H, s), 3.10-3.28 (4H, m), 3.62 (3H, s), 6.40 (1H, br s), 6.84 (2H, d, J = 8.8 Hz), 7.33 (2H, d, J = 8.8 Hz).

### Reference Example 8c

### 3-(4-Isopropylphenyl)-2,2-dimethyl-2,3-dihydrobenzofuran-5-ol

n-Butyllithium (1.6 M, 20.8 mL, 33.2 mmol) was added to a solution of 1-bromo-2,5-dimethoxybenzene (7.2 g, 33.2 mmol) in tetrahydrofuran (20 mL) at -78°C, and the mixture was stirred for 20 minutes at the same temperature. To the reaction mixture was added 1-(4-isopropylphenyl)-2-methylpropan-1-one (5.70 g, 30.0 mol), and the mixture was stirred for 30 minutes at room temperature. Water (30 mL) was poured into the reaction mixture, which was then extracted three times with ethyl acetate. The organic layers were combined, washed with water, dried over magnesium sulfate, filtered, and concentrated under reduced pressure. A mixture of the residue and 48 % hydrobromic acid (30 mL) was heated under reflux for 24 hours in an argon atmosphere. After cooled, water (30 mL) was added to the reaction mixture, which was then extracted twice with ethyl acetate. The organic layers were combined, washed with water, dried over magnesium sulfate, filtered, and concentrated under reduced pressure. The residue was crystallized from isopropyl ether-hexane to obtain the title compound (2.1 g, yield 70 %).
m.p.: 102-104°C.
¹H-NMR (CDCl₃) δ: 0.96 (3H, s), 1.25 (6H, d, J = 7.0 Hz), 1.57 (3H, s), 2.90 (1H, septet, J = 7.0 Hz), 4.2.8 (1H, s), 4.67 (1H, s), 6.53-6.85 (3H, m), 7.02 (2H, d, J = 8.0 Hz), 7.16 (2H, d, J = 8.0 Hz).

### Reference Example 9c

### 2,2,4,6,7-Pentamethyl-3-[4-(4-morpholinyl)phenyl]-2,3-dihydrobenzofuran-5-ol

A mixture of 1-(2,5-dimethoxy-3,4,6-trimethylphenyl)-2-methyl-1-[4-(4-morpholinyl)phenyl]propan-1-ol (8.00 g, 19.3 mmol) and 48 % hydrobromic acid (100 mL) was heated under reflux for 3 hours in an argon atmosphere. After cooled, an aqueous saturated sodium hydrogencarbonate (30 mL) was added to the reaction mixture, which was then extracted twice with ethyl acetate. The organic layers were combined, washed with water, dried over magnesium sulfate, filtered, and concentrated under reduced pressure. The residue was crystallized from isopropyl ether-hexane to obtain the title compound (6.40 g, yield 90 %).
m.p.: 91-93°C.
¹H-NMR (CDCl₃) δ: 1.00 (3H, s), 1.46 (3H, s), 1.82 (3H, s), 2.15 (3H, s), 2.17 (3H, s), 2.98-3.24 (4H, m), 3.71-3.99 (4H, m), 4.04 (1H, s), 4.18 (1H, s), 6.44-7.10 (4H, m).

### Reference Example 10c

### 2,2,4,6,7-Pentamethyl-3-[4-(4-methyl-1-piperazinyl)phenyl]-2,3-dihydrobenzofuran-5-ol

Using 1-(2,5-dimethoxy-3,4,6-trimethylphenyl)-2-methyl-1-[4-(4-methyl-1-piperazinyl)phenyl]propan-1-ol the title compound was obtained in the same manner as in Reference Example 9.
Yield: 55 %.
m.p.: 159-161°C (from ethyl acetate-hexane).
¹H-NMR (CDCl₃) δ: 1.00 (3H, s), 1.46 (3H, s), 1.81 (3H, s), 2.17 (6H, s), 2.34 (3H, s), 2.48-2.65 (4H, m), 3.08-3.22 (4H, m), 4.03 (1H, s), 6.58-7.20 (4H, m), 1H not confirmed.

### Reference Example 11c

### 1-(4-Isopropylphenyl)propan-1-ol

Propionyl chloride (11.6 g, 125 mmol) was dropwise added to a suspension of aluminum chloride (16.7 g, 125 mmol) and cumene (18.0 g, 150 mmol) in carbon disulfide (30 mL) at -5°C, and the mixture was stirred for 30 minutes at room temperature. The reaction mixture was poured into ice water, and the organic layer was separated, washed with an aqueous saturated sodium hydrogencarbonate and water, dried over magnesium sulfate, filtered, and concentrated under reduced pressure to obtain 1-(4-isopropylphenyl)propan-1-one (24.7 g). Sodium borohydride (1.29 g, 34.2 mmol) was added to a solution of the thus-obtained compound (13.0 g, 68.4 mmol) in ethanol (80 mL) under ice cooling, and the mixture was stirred for 30 minutes at room temperature. Water was added to the reaction mixture, which was then extracted with ethyl acetate. The organic layer was washed with water, dried over magnesium sulfate, filtered, and concentrated under reduced pressure to obtain the title compound (11.5 g, yield 79 %). This was oily.
¹H-NMR (CDCl₃) δ: 0.91 (3H, t, J = 7.4 Hz), 1.25 (6H, d, J = 7.0 Hz), 1.63-1.92 (2H, m), 1.94 (1H, br s), 2.90 (1H, septet, J = 7.0 Hz), 4.47-4.61 (1H, m), 7.16-7.29 (4H, m).

### Reference Example 12c

### 2-[1-(4-Isopropylphenyl)propyl]-3,5,6-trimethyl-1,4-benzoquinone

Boron trifluoride/ethyl ether complex (1.30 g, 9.33 mmol) was dropwise added to a suspension of 1-(4-isopropylphenyl)propan-1-ol (5.00 g, 28.0 mmol) and trimethylhydroquinone (4.30 g, 28.0 mmol) in 1,2-dichloroethane (100 mL) at 60°C under a nitrogen atmosphere, and the mixture was stirred for 3 hours at the same temperature. After cooling, the reaction mixture was washed with an aqueous solution of iron(III) chloride and water, dried over magnesium sulfate, filtered, and concentrated under reduced pressure. The residue was subjected to silica gel column chromatography (hexane/ethyl acetate = 30/1) to obtain the title compound (5.40 g, yield 62 %).
m.p.: 61-63°C (from methanol).
¹H-NMR (CDCl₃) δ: 0.91 (3H, t, J = 7.4 Hz), 1.22 (6H, d, J = 6.8 Hz), 1.83-2.11 (11H, m), 2.85 (1H, septet, J = 6.8 Hz), 4.02-4.23 (1H, m), 7.02-4.24 (4H, m).

### Reference Example 13c

### 3-(4-Isopropylphenyl)-2,4,6,7-tetramethylbenzofuran-5-ol

A solution of 2-[1-(4-isopropylphenyl)propyl]-3,5,6-trimethyl-1,4-benzoquinone (1.00 g, 0.324 mmol) in ethanol (1.00 liter) was stirred for 5 hours while cooling it with ice-water to keep the solution at room temperature and while exposing it to light from 400 W Bromcinelight Deluxe (manufactured by LPL Co.). The solvent was removed under reduced pressure, and the residue was subjected to silica gel column chromatography (hexane/ethyl acetate = 20/1) to obtain the title compound (0.90 g, yield 90 %). This was oily.
¹H-NMR(CDCl₃) δ: 1.31 (6H, d, J = 7.0 Hz), 1.98 (3H, s), 2.28 (3H, s), 2.30 (3H, s), 2.43 (3H, s), 2.97 (1H, septet, J = 7.0 Hz), 4.43 (1H, s), 7.26 (4H, s).

### Reference Example 14c

### 2,3,6-Trimethyl-4-[(3-phenyl-2-propenyl)oxy]phenyl acetate

To a solution of 4-hydroxy-2,3,6-trimethylphenyl acetate (10.0 g, 51.5 mmol) in N,N-dimethylformamide (100 mL) was added 1-chloro-3-phenyl-2-propene (7.86 g, 51.5 mmol) and potassium carbonate (7.10 g, 51.5 mmol) and the mixture was stirred under an argon atmosphere at 60°C for 2 hours. To this reaction mixture was added water and the product was extracted twice with ethyl acetate. The combined extracts was washed with water, dried over magnesium sulfate, and concentrated under reduced pressure. The residue was crystallized from methanol to obtain the title compound (13.0 g, yield 81%).
m.p.: 104-107°C.
¹H-NMR (CDCl₃) δ: 2.06 (3H, s), 2.13 (3H, s), 2.18 (3H, s), 2.34 (3H, s), 4.66 (2H, dd, J = 5.6, 1.2 Hz), 6.43 (1H, dt, J = 16.2, 5.6 Hz), 5.63 (1H, s), 6.74 (1H, d, J = 16.2 Hz), 7.24-7.46 (5H, m).

### Reference Example 15c

### 4-Hydroxy-2,3,6-trimethyl-5-(1-phenyl-2-propenyl)phenyl acetate

A solution of 2,3,6-trimethyl-4-[(3-phenyl-2-propenyl)oxy]phenyl acetate (10.0 g, 32.2 mmol) in N,N-dimethylaniline (70 mL) was stirred under an argon atmosphere at 200°C for 3 h. After the reaction mixture was cooled, it was diluted with ethyl acetate, washed with 2N hydrochloric acid and water, and dried over magnesium sulfate, and concentrated under reduced pressure. The residue was recrystallized from ethyl acetate-hexane to obtain the title compound (7.80 g, yield 78 %).
m.p.: 136-138°C.
¹H-NMR(CDCl₃) δ: 2.06 (6H, s), 2.11 (3H, s), 2.33 (3H, s), 4.83-5.18 (2H, m), 5.36 (1H, d, J = 10.0 Hz), 6.32-6.58 (1H, m), 7.18-7.37 (5H, m), 1H not confirmed.

### Reference Example 16c

### 2,4,6,7-Tetramethyl-3-phenylbenzofuran-5-yl acetate

To a suspension of 4-hydroxy-2,3,6-trimethyl-5-(1-phenyl-2-propenyl)phenyl acetate (5.10 g, 16.4 mmol) and calcium carbonate (2.13 g, 21.3 mmol) in tetrahydrofuran (20 mL) and methanol (20 mL) was added benzyltrimethylammonium dichloroiodate (6.28 g, 18.0 mmol) slowly. The mixture was stirred at room temperature for 30 minutes. The insoluble material was removed by filtration and the filtrate was concentrated under reduced pressure. To the residue was added ethyl acetate and water. The organic layer was separated and the aqueous layer was extracted twice with ethyl acetate. The combined organic layers were washed with 10% aqueous sodium hydrogen sulfite, water, an aqueous saturated solution of sodium bicarbonate and an aqueous saturated solution of sodium chloride, dried over magnesium sulfate, and then concentrated under reduced pressure to provide 5.30 g of 2-iodomethyl-4,6,7-trimethyl-3-phenyl-2,3-dihydrobenzofuran-5-yl acetate. A mixture of this compound (5.30 g, 12.1 mmol) and 1,8-diazabicyclo[5,4,0]-7-undecene (9.0 m, 60.0 mmol) in toluene (20 mL) was stirred under an argon atmosphere at 100°C for 3 hours. To the reaction mixture was added water, and the mixture was extracted twice with ethyl acetate. The extract was washed with 2N hydrochloric acid and water, dried over magnesium sulfate, and concentrated under reduced pressure. The residue was purified by silica gel column chromatography (hexane/ethyl acetate = 20/1) to obtain the title compound (4.0 g, yield 79 %). This was oily.
¹H-NMR (CDCl₃) δ: 1.85 (3H, s), 2.15 (3H, s), 2.30 (3H, s), 2.33 (3H, s), 2.44 (3H, s), 7.32-7.48 (5H, m).

### Reference Example 17c

### 2,4,6,7-Tetramethyl-3-phenylbenzofuran-5-ol

To a solution of 2,4,6,7-tetramethyl-3-phenylbenzofuran-5-yl acetate (4.00 g, 13.0 mmol) in a mixture of tetrahydrofuran (32 mL) and methanol (8 mL) was added 8N sodium hydroxide solution (2.0 mL) dropwise and the mixture was stirred at 40°C for 1 hour. The solvent was then distilled off under reduced pressure. To the residue was added 2N hydrochloric acid, and the mixture was extracted with ethyl acetate. The extract was washed with water and an aqueous saturated solution of sodium chloride, dried over magnesium sulfate, and concentrated under reduced pressure. The residue was recrystallized from isopropyl ether-hexane to obtain the title compound (3.0 g, yield 87 %).
m.p.: 102-104°C.
¹H-NMR (CDCl₃) δ: 1.96 (3H, s), 2.28 (3H, s), 2.29 (3H, s), 2.44 (3H, s), 4.42 (1H, s), 7.28-7.43 (5H, m).

### Reference Example 18c

### 1-(2,4-Dimethoxyphenyl)-1-(4-isopropylphenyl)-2-methylpropan-1-ol

Using 1-bromo-2,4-dimethoxybenzene and 1-(4-isopropylphenyl)-2-methylpropan-1-one the title compound was obtained in the same manner as in Reference Example 6. Yield 56 %.
m.p.: 80-81°C (from methanol).
¹H-NMR(CDCl₃) δ: 0.75 (3H, d, J = 6.6 Hz), 1.08 (3H, d, J = 6.6 Hz), 1.20 (6H, d, J = 7.0 Hz), 2.66 (1H, septet, J = 7.0 Hz), 2.80 (1H, septet, J = 6.6 Hz) , 3.48 (3H, s), 3.79 (3H, s), 4.71 (1H, s), 6.39-6.40 (1H, m), 6.50-6.56 (1H, m), 7.04-7.08 (2H, m), 7.19-7.23 (2H, m), 7.40-7.44 (1H, m).

### Reference Example 19c

### 3-(4-Isopropylphenyl)-2,2-dimethyl-2,3-dihydrobenzofuran-6-ol

A mixture of 1-(2,4-dimethoxyphenyl)-1-(4-isopropylphenyl)-2-methylpropan-1-ol (5.58 g, 17.0 mmol) and 48 % hydrobromic acid (30 mL) was heated under reflux for 24 hours under an argon atmosphere. After the reaction mixture was cooled to room temperature, an aqueous saturated sodium hydrogencarbonate was added to the mixture, which was then extracted twice with ethyl acetate. The extracts were combined, washed with an aqueous saturated sodium hydrogencarbonate, dried over magnesium sulfate, and concentrated under reduced pressure. The residue was subjected to silica gel column chromatography (hexane/ ethyl acetate = 20/1 to 10/1) to obtain the title compound (2.43 g, yield 51 %).
m.p.: 114-115°C (from hexane).
¹H-NMR (CDCl₃) δ: 0.95 (3H, s), 1.24 (6H, d, J = 7.0 Hz), 1.57 (3H, s), 2.89 (1H, septet, J = 7.0 Hz), 4.25 (1H, s), 6.15 (1H, br), 6.34-6.38 (2H, m), 6.84-6.88 (1H, m), 6.99-7.03 (2H, m), 7.13-7.17 (2H, m).

### Reference Example 20c

### 4-(4-Isopropylbenzoyl)piperidine

To 1-acetylisonipecotic acid (41.74 g, 243.8 mmol) was added thionyl chloride (200 mL), and the resulting mixture was stirred for 30 minutes. The mixture was diluted with petroleum ether. The precipitated solid was collected and washed with petroleum ether to afford 1-acetylisonipecotoyl chloride. This was added to a suspension of cumene (120 mL) and aluminum chloride (69.6 g, 522 mmol) and the resulting mixture was stirred at 110°C for 1 hour. After cooling to room temperature, the reaction mixture was poured into ice water, and extracted twice with ethyl acetate. The organic layers were combined, washed with an aqueous saturated solution of sodium chloride, dried over magnesium sulfate, filtered, and concentrated under reduced pressure. To the residue was added concentrated hydrochloric acid (100 mL) , and the mixture was refluxed for 12 hours. The mixture was cooled to room temperature and was washed twice with diethyl ether. The aqueous solution was made basic with 8N sodium hydroxide solution and then extracted twice with ethyl acetate. The organic layers were combined, washed with an aqueous saturated sodium hydrogencarbonate, dried over magnesium sulfate, filtered, and concentrated under reduced pressure. The residue was crystallized from ethyl acetate-hexane to obtain the title compound (23.5 g, yield 41 %).
m.p.: 55-57°C.
¹H-NMR(CDCl₃) δ: 1.27 (6H, d, J = 6. 8 Hz), 1.57-2.70 (5H, m), 2.70-2.83 (2H, m), 2.97 (1H, septet, J = 6.8 Hz), 3.16-3.22 (2H, m), 3.34-3.46 (1H, m), 7.30-7.34 (2H, m), 7.87-7.91 (2H, m).

### Reference Example 21c

### 1-Benzyl-4-(4-isopropylbenzoyl)piperidine

To a solution of 4-(4-isopropylbenzoyl)piperidine in N,N-dimethylformamide (100 mL), potassium carbonate (9.60 g, 69.5 mmol) and benzyl bromide (8.50 g, 71.5 mmol) were added, and the resulting mixture was stirred for 20 hours at room temperature. The mixture was poured into water, and extracted twice with ethyl acetate. The organic layers were combined, washed with an aqueous saturated sodium hydrogencarbonate, dried over magnesium sulfate, filtered, and concentrated under reduced pressure. The residue was crystallized from hexane to obtain the title compound (13.53 g, yield 66 %).
m.p.: 76-77°C.
¹H-NMR(CDCl₃) δ: 1.26 (6H, d, J = 7.0 Hz), 1.79-1.90 (4H, m), 2.07-2.20 (2H, m), 2.92-2.99 (3H, m), 3.15-3.30 (1H, m), 3.55 (2H, s), 7.24-7.32 (7H, m), 7.85-7.89 (2H, m).

### Reference Example 22c

### (1-Benzyl-4-piperidyl)(2,5-dimethoxy-3,4,6-trimethylphenyl)(4-isopropylphenyl)methanol

n-Butyllithium (1.6 M, 12.0 mL, 19.2 mmol) was added to a solution of 1-bromo-2,5-dimethoxy-3,4,6-trimethylbenzene (4.89 g, 18.87 mmol) in tetrahydrofuran (100 mL) at -78°C under an argon atmosphere, and the mixture was stirred for 30 minutes at the same temperature. To the reaction mixture was added a solution of 1-benzyl-4-(4-isopropylbenzoyl)piperidine (5.02 g, 15.6 mmol) in tetrahydrofuran (10 ml) and the mixture was stirred for 30 minutes at room temperature. To the mixture was then added water, and the product was extracted twice with ethyl acetate. The extracts were combined, washed with an aqueous saturated solution of sodium chloride, dried over magnesium sulfate, and concentrated under reduced pressure. The residue was crystallized from ethyl acetate-hexane to obtain the title compound (6.54 g, yield 83 %).
m.p.: 105-108°C.
¹H-NMR(CDCl₃) δ: 1.19 (6H, d, J = 6.6 Hz), 1.2-1.5 (2H, m), 1.8-2.0 (4H, m), 2.09 (3H, s), 2.17 (3H, s), 2.39 (3H, s), 2.4-2.5 (1H, m), 2.78-2.88 (3H, m), 2.97 (3H, s), 3.51 (2H, s), 3.60 (3H, s), 6.37 (1H, br), 7.08-7.12 (2H, m), 7.26-7.34 (7H, m).

### Reference Example 23c

### 1'-Benzyl-3-(4-isopropylphenyl)-4,6,7-trimethylspiro[benzofuran-2(3H),4'-piperidine]-5-ol

To a solution of (1-benzyl-4-piperidyl)(2,5-dimethoxy-3,4,6-trimethylphenyl)(4-isopropylphenyl)methanol (6.41 g, 12.8 mmol) in acetic acid (50 mL) was added 48% hydrobromic acid (60 mL), and the mixture was heated under reflux for 15 hours under an argon atmosphere. The reaction mixture was cooled to room temperature, made basic with 8N sodium hydroxide solution, and extracted twice with ethyl acetate. The extracts were combined, washed with an aqueous saturated sodium hydrogencarbonate, dried over magnesium sulfate, and concentrated under reduced pressure. The residue was crystallized from ethyl acetate-hexane to obtain the title compound (4.44 g, yield 76 %).
m.p.: 190-192°C.
¹H-NMR(CDCl₃) δ: 1.19 (6H, d, J = 7.0 Hz), 1.21-1.41 (2H, m), 1.71-2.00 (5H, m), 2.17 (3H, s), 2.20 (3H, s), 2.27-2.90 (5H, m), 2.97 (3H, s), 3.54 (2H, s), 4.02 (1H, s), 6.6-7.1 (4H, m), 7.20-7.32 (5H, m), 1H not confirmed.

### Reference Example 24c

### 3-(4-Isopropylphenyl)-4,6,7-trimethylspiro[benzofuran-2(3H),4'-piperidine]-5-ol hydrochloride

To a solution of 1'-benzyl-3-(4-isopropylphenyl)-4,6,7-trimethylspiro[benzofuran-2(3H),4'-piperidine]-5-ol (3.51 g, 7.70 mmol) and triethylamine (1.1 mL, 7.9 mmol) in chloroform (40 mL), which was precooled at 0°C, 1-chloroethyl chloroformate (2.30 g, 16.1 mmol) was added. The mixture was heated under reflux for 1 hour and concentrated under reduced pressure. The residue was heated under reflux in methanol (20 mL) for 1 hour and concentrated under reduced pressure. The residue was crystallized from ethanol-ethyl acetate to obtain the title compound (2.80 g, yield 90 %).
m.p.: >245°C (dec.)
¹H-NMR(d₆-DMSO) δ: 1.18 (6H, d, J = 6.6 Hz), 1.34 (2H, br), 1.71 (3H, s), 1.97 (2H, br), 2.08 (3H, s), 2.11 (3H, s), 2.8-3.3 (5H, m), 4.26 (1H, s), 6.6-7.2 (4H, m), 7.53 (1H, s), 8.78 (1H, s), 1H not confirmed.

### Reference Example 25c

### 3-(4-Isopropylphenyl)-1',4,6,7-tetramethylspiro[benzofuran-2(3H),4'-piperidine]-5-ol

A mixture of 3-(4-isopropylphenyl)-4,6,7-trimethylspiro[benzofuran-2(3H),4'-piperidine]-5-ol hydrochloride (2.80 g, 6.97 mmol), formic acid (30 mL) and 37% formalin (30 mL) was stirred for 15 hours at 100°C. The reaction mixture was cooled to room temperature, made basic with 8N sodium hydroxide solution, and extracted twice with ethyl acetate. The extracts were combined, washed with an aqueous saturated sodium hydrogencarbonate, dried over magnesium sulfate, and concentrated under reduced pressure. The residue was subjected to column chromatography (Chromatorex NH DM1020, Fuji Silysia Chemical LTD) (hexane/ethyl acetate = 1/1) to obtain the title compound (2.05 g, yield 77 %).
m.p.: 114-117°C (from ethyl acetate-hexane).
¹H-NMR(CDCl₃) δ: 1.18-1.39 (8H, m), 1.72-2.91 (19H, m), 4.02 (1H, m), 6.6-7.1 (4H, m), 1H not confirmed.

### Reference Example 26c

### (1-Benzyl-4-piperidyl)(2,5-dimethoxy-3,4,6-trimethylphenyl)methanol

n-Butyllithium (1.6 M, 19.5 mL, 31.2 mmol) was added to a solution of 1-bromo-2,5-dimethoxy-3,4,6-trimethylbenzene (8.00 g, 30.87 mmol) in tetrahydrofuran (80 mL) at -78°C, and the mixture was stirred for 30 minutes at the same temperature. To the reaction mixture was added a solution of 1-benzyl-4-formylpiperidine (6.23 g, 30.65 mmol) in tetrahydrofuran (20 ml). The mixture was stirred for 30 minutes at room temperature, then poured into water, and extracted twice with ethyl acetate. The extracts were combined, washed with an aqueous saturated sodium hydrogencarbonate, dried over magnesium sulfate, and concentrated under reduced pressure. The residue was subjected to silica gel column chromatography (ethyl acetate) to obtain the title compound (6.17 g, yield 52 %). This was oily.
¹H-NMR(CDCl₃) δ: 1.17-2.05 (7H, m), 2.16 (3H, s), 2.17(3H, s), 2.24 (3H, s), 2.79-2.85 (1H, m), 2.98-3.05 (1H, m), 3.48 (2H, s), 3.61 (3H, s), 3.75 (3H, s), 4.59 (1H, m), 7.23-7.32 (5H, m), 1H not confirmed.

### Reference Example 27c

### 1'-Benzyl-4,6,7-trimethylspiro[benzofuran-2(3H),4'-piperidine]-5-ol

To a solution of (1-benzyl-4-piperidyl)(2,5-dimethoxy-3,4,6-trimethylphenyl)methanol (6.10 g, 15.9 mmol) in acetic acid (30 mL) was added 48% hydrobromic acid (40 mL), and the mixture was heated under reflux for 2.5 hours under an argon atmosphere. The reaction mixture was cooled to room temperature, made basic with 8N sodium hydroxide solution, and extracted twice with ethyl acetate. The extracts were combined, washed with an aqueous saturated sodium hydrogencarbonate, dried over magnesium sulfate, and concentrated under reduced pressure. The residue was subjected to silica gel column chromatography (hexane/ethyl acetate = 1/1) to obtain the title compound (4.60 g, yield 86 %). This was amorphous.
¹H-NMR(CDCl₃) δ: 1.71-2.00 (6H, m), 2.10 (3H, s), 2.11 (3H, s), 2.12 (3H, s), 2.58 (2H, m), 2.87 (2H, s), 3.56 (2H, s), 7.25-7.38 (5H, m), 1H not confirmed.

### Example 1c

### 5-Benzyloxy-3-(4-isopropylphenyl)-2,2,4,6,7-pentamethyl-2,3-dihydrobenzofuran

Sodium hydride (60 % liquid paraffin dispersion, 68 mg, 1.70 mmol) was added to a solution of 3-(4-isopropylphenyl)-2,2,4,6,7-pentamethyl-2,3-dihydrobenzofuran-5-ol (0.5 g, 1.54 mmol) in N,N-dimethylformamide (20 mL) at 0°C, and the mixture was stirred for 10 minutes at the same temperature. To the reaction mixture was added benzyl bromide (290 mg, 1.70 mmol) and the mixture was stirred for further 30 minutes at room temperature. The reaction mixture was poured into water (30 mL), and extracted twice with ethyl acetate. The organic layers were combined, washed with water, dried over magnesium sulfate, filtered, and concentrated under reduced pressure. The residue was crystallized from methanol to obtain the title compound (380 mg, yield 60 %).
m.p.: 79-81°C.
¹H-NMR (CDCl₃) δ: 1.01 (3H, s), 1.22 (6H, d, J = 6.8 Hz), 1.50 (3H, s), 1.83 (3H, s), 2.16 (3H, s), 2.24 (3H, s), 2.86 (1H, septet, J = 6.8 Hz), 4.09 (1H, s), 4.70 (2H, s), 6.70-7.00 (2H, br), 7.09 (2H, d, J = 8.4 Hz), 7.30-7.50 (5H, m).

### Example 2c

### 5-Benzyloxy-3-[4-(dimethylamino)phenyl]-2,2,4,6,7-pentamethyl-2,3-dihydrobenzofuran

Using 3-[4-(dimethylamino)phenyl]-2,2,4,6,7-pentamethyl-2,3-dihydrobenzofuran-5-ol and benzyl bromide, the title compound was obtained in the same manner as in Example 1c.
Yield: 40 %.
m.p.: 110-112°C (from methanol).
¹H-NMR (CDCl₃) δ: 1.03 (3H, s), 1.48 (3H, s), 1.87 (3H, s), 2.16 (3H, s), 2.23 (3H, s), 2.91 (6H, s), 4.04 (1H, s), 4.70 (2H, s), 6.48-7.16 (4H, m), 7.20-7.48 (5H, m).

### Example 3c

### 5-Benzyloxy-2,4,6,7-tetramethyl-2-(4-phenyl-1-piperazinyl)methyl-2,3-dihydrobenzofuran

Using 2,4,6,7-tetramethyl-2-(4-phenyl-1-piperazinyl)methyl-2,3-dihydrobenzofuran-5-ol and benzyl bromide, the title compound was obtained in the same manner as in Example 1c.
Yield: 48 %.
m.p.: 120-121°C (from methanol).
¹H-NMR (CDCl₃) δ: 1.47 (3H, s), 2.09 (3H, s), 2.16 (3H, s), 2.20 (3H, s), 2.58-2.92 (7H, m), 3.08-3.22 (5H, m), 4.71 (2H, s), 6.78-6.94 (3H, m), 7.20-7.52 (7H, m).

### Example 4c

### 3-(4-Isopropylphenyl)-5-(4-methoxybenzyloxy)-2,2,4,6,7-pentamethyl-2,3-dihydrobenzofuran

Using 3-(4-isopropylphenyl)-2,2,4,6,7-pentamethyl-2,3-dihydrobenzofuran-5-ol and 4-methoxybenzyl chloride, the title compound was obtained in the same manner as in Example 1c.
Yield: 49 %.
m.p.: 95-96°C (from methanol).
¹H-NMR (CDCl₃) δ: 1.00 (3H, s), 1.22 (6H, d, J = 7.0 Hz), 1.49 (3H, s), 1.82 (3H, s), 2.16 (3H, s), 2.23 (3H, s), 2.86 (1H, septet, J = 7.0 Hz), 3.81 (3H, s), 4.08 (1H, s), 4.63 (2H, s), 6.70-7.18 (6H, m), 7.35 (2H, d, J = 8.8 Hz).

### Example 5c

### 3-(4-Isopropylphenyl)-5-(4-methoxybenzyloxy)-2,2-dimethyl-2,3-dihydrobenzofuran

Using 3-(4-isopropylphenyl)-2,2-dimethyl-2,3-dihydrobenzofuran-5-ol and 4-methoxybenzyl chloride, the title compound was obtained in the same manner as in Example 1c.
Yield: 75 %.
m.p.: 124-126°C (from ethyl acetate-hexane).
¹H-NMR (CDCl₃) δ: 0.95 (3H, s), 1.25 (6H, d, J = 7.0 Hz), 1.57 (3H, s), 2.90 (septet, 1H, J = 7.0 Hz), 3.71 (3H, s), 4.30 (1H, s), 4.87 (2H, s), 6.65-7.35 (11H, m).

### Example 6c

### 3-[4-(Dimethylamino)phenyl]-5-(4-methoxybenzyloxy)-2,2,4,6,7-pentamethyl-2,3-dihydrobenzofuran

Using 3-[4-(dimethylamino)phenyl]-2,2,4,6,7-pentamethyl-2,3-dihydrobenzofuran-5-ol and 4-methoxybenzyl chloride, the title compound was obtained in the same manner as in Example 1c.
Yield: 42 %.
m.p.: 105-107°C (from ethanol).
¹H-NMR (CDCl₃) δ: 1.02 (3H, s), 1.48 (3H, s), 1.84 (3H, s), 2.15 (3H, s), 2.23 (3H, s), 2.92 (6H, s), 3.81 (3H, s), 4.04 (1H, s), 4.58-4.69 (2H, m), 6.54-6.93 (6H, m), 7.30-7.42 (2H, m).

### Example 7c

### 5-(4-Methoxybenzyloxy)-3-[4-(4-morpholinyl)phenyl]-2,2,4,6,7-pentamethyl-2,3-dihydrobenzofuran

Using 2,2,4,6,7-pentamethyl-3-[4-(4-morpholinyl)phenyl]-2,3-dihydrobenzofuran-5-ol and 4-methoxybenzyl chloride, the title compound was obtained in the same manner as in Example 1c.
Yield: 38 %.
m.p.: 110-112°C (ethanol).
¹H-NMR (CDCl₃) δ: 1.01 (3H, s), 1.48 (3H, s), 1.83 (3H, s), 2.15 (3H, s), 2.23 (3H, s), 3.02-3.26 (4H, m), 3.71-3.99 (7H, m), 4.05 (1H, s), 4.57-4.90 (2H, m), 6.60-7.00 (6H, m), 7.35 (2H, d, J = 6.8 Hz) .

### Example 8c

### 5-(4-Methoxybenzyloxy)-2,2,4,6,7-pentamethyl-3-[4-(4-methyl-1-piperazinyl)phenyl]-2,3-dihydrobenzofuran

Using 2,2,4,6,7-pentamethyl-3-[4-(4-methyl-1-piperazinyl)phenyl]-2,3-dihydrobenzofuran-5-ol and 4-methoxybenzyl chloride, the title compound was obtained in the same manner as in Example 1c.
Yield: 42 %.
m.p.: 121-122°C (from ethyl ether-hexane).
¹H-NMR (CDCl₃) δ: 1.01 (3H, s), 1.48 (3H, s), 1.83 (3H, s), 2.15 (3H, s), 2.23 (3H, s), 2.34 (3H, s), 2.52-2.63 (4H, m), 3.13-3.24 (4H, m), 3.81 (3H, s), 4.05 (1H, s), 4.58-4.67 (2H, m), 6.60-7.07 (6H, m), 7.35 (2H, d, J = 8.8 Hz).

### Example 9c

### 3-(4-Isopropylphenyl)-2,2,4,6,7-pentamethyl-5-(4-methylthiobenzyloxy)-2,3-dihydrobenzofuran

Using 3-(4-isopropylphenyl)-2,2,4,6,7-pentamethyl-2,3-dihydrobenzofuran-5-ol and 4-(bromomethyl)phenyl methyl sulfide, the title compound was obtained in the same manner as in Example 1c.
Yield: 70 %.
m.p.: 118-120°C (from ethanol).
¹H-NMR (CDCl₃) δ: 1.01 (3H, s), 1.22 (6H, d, J = 7.0 Hz), 1.49 (3H, s), 1.82 (3H, s), 2.16 (3H, s), 2.22 (3H, s), 2.48 (3H, s), 2.86 (1H, septet, J = 7.0 Hz), 4.08 (1H, s), 4.65 (2H, s), 6.80-7.02 (2H, br), 7.08 (2H, d, J = 8.0 Hz), 7.25 (2H, d, J = 8.4 Hz), 7.36 (2H, d, J = 8.4 Hz).

### Example 10c

### 3-(4-Isopropylphenyl)-2,2,4,6,7-pentamethyl-5-[4-(methylsulfinyl)benzyloxy]-2,3-dihydrobenzofuran

Sodium periodate (0.766 g, 3.58 mmol) was added to a solution of 3-(4-isopropylphenyl)-2,2,4,6,7-pentamethyl-5-(4-methylthiobenzyloxy)-2,3-dihydrobenzofuran (1.50 g, 3.26 mmol) in a mixture of ethanol (80 mL) and water (8 mol), and the mixture was heated under reflux for 2 hours. To the reaction mixture were added ethyl acetate and water to separate it into two layers, and the aqueous layer was extracted with ethyl acetate. The organic layers were combined, washed with water, dried over magnesium sulfate, filtered, and concentrated under reduced pressure. The resulting residue was recrystallized from ethyl acetate-hexane to obtain the title compound (1.23 g, yield 79 %).
m.p.: 132-134°C.
¹H-NMR (CDCl₃) δ: 1.02 (3H, s), 1.22 (6H, d, J = 6.8 Hz), 1.50 (3H, s), 1.82 (3H, s), 2.17 (3H, s), 2.23 (3H, s), 2.71, 2.72 (1.5H x2, s x2), 2.86 (1H, septet, J = 6.8 Hz), 4.09 (1H, s), 4.76 (2H, s), 6.71-7.15 (4H, m), 7.57-7.69 (4H, m).

### Example 11c

### 3-(4-Isopropylphenyl)-2,2,4,6,7-pentamethyl-5-[4-(methylsulfonyl)benzyloxy]-2,3-dihydrobenzofuran

Sodium periodate (2.02 g, 9.45 mmol) was added to a solution of 3-(4-isopropylphenyl)-2,2,4,6,7-pentamethyl-5-[(4-methylsulfinyl)benzyloxy]-2,3-dihydrobenzofuran (1.50 g, 3.15 mmol) in a mixture of ethanol (80 mL) and water (8 mol), and the mixture was heated under reflux for 18 hours. To the reaction mixture were added ethyl acetate and water to separate it into two layers, and the aqueous layer was extracted with ethyl acetate. The organic layers were combined, washed with water, dried over magnesium sulfate, filtered, and concentrated under reduced pressure. The resulting residue was recrystallized from ethyl acetate-hexane to obtain the title compound (1.05 g, yield 68 %).
m.p.: 161-162C.
¹H-NMR (CDCl₃) δ: 1.02 (3H, s), 1.22 (6H, d, J = 7.0 Hz), 1.50 (3H, s), 1.82 (3H, s), 2.17 (3H, s), 2.22 (3H, s), 2.87 (1H, septet, J = 7.0 Hz), 3.05 (3H, s), 4.09 (1H, s), 4.80 (2H, s), 6.70-7.13 (4H, m), 7.67 (2H, d, J = 8.4 Hz), 7.95 (2H, d, J = 8.4 Hz).

### Example 12c

### 3-(4-Isopropylphenyl)-2,2,4,6,7-pentamethyl-5-(3-phenyl-2-propen-1-yloxy)-2,3-dihydrobenzofuran

Using 3-(4-isopropylphenyl)-2,2,4,6,7-pentamethyl-2,3-dihydrobenzofuran-5-ol and 3-bromo-1-phenyl-1-propene, the title compound was obtained in the same manner as in Example 1c.
Yield: 71 %.
m.p.: 106-107°C (from methanol).
¹H-NMR (CDCl₃) δ: 1.00 (3H, s), 1.21 (6H, d, J = 7.0 Hz), 1.49 (3H, s), 1.86 (3H, s), 2.16 (3H, s), 2.24 (3H, s), 2.85 (1H; septet, J = 7.0 Hz), 4.08 (1H, s), 4.36 (2H, d, J = 6.0 Hz), 6.42 (1H, dt, J = 15.4, 6.0 Hz), 6.66-7.15 (5H, m), 7.20-7.48 (5H, m).

### Example 13c

### 3-(4-Isopropylphenyl)-2,2,4,6,7-pentamethyl-5-(2-quinolylmethyloxy)-2,3-dihydrobenzofuran hydrochloride

Sodium hydride (60 % liquid paraffin dispersion, 136 mg, 3.39 mmol) was added to a solution of 3-(4-isopropylphenyl)-2,2,4,6,7-pentamethyl-2,3-dihydrobenzofuran-5-ol (1.0 g, 3.08 mmol) in N,N-dimethylformamide (30 mL) at 0°C, and the mixture was stirred for 10 minutes at the same temperature. To the reaction mixture was added 2-(chloromethyl)quinoline hydrochloride (730 mg, 3.39 mmol) and the mixture was stirred for 30 minutes at 80°C. The reaction mixture was poured into water (40 mL), and extracted twice with ethyl acetate. The organic layers were combined, washed with water, dried over magnesium sulfate, filtered, and concentrated under reduced pressure. To the residue was added 4 N HCl-ethanol, and the solvent was removed through distillation. The residue was crystallized from ethanol-hexane to obtain the title compound (1.1 g, yield 71 %).
m.p.: 136-139°C.
¹H-NMR (DMSO-d₆) δ: 0.94 (3H, s), 1.18 (6H, d, J = 7.0 Hz), 1.45 (3H, s), 1.78 (3H, s), 2.11 (3H, s), 2.22 (3H, s), 2.85 (1H, septet, J = 7.0 Hz), 4.19 (1H, s), 4.20-4.90 (1H, br), 5.10 (1H, d, J = 15.8 Hz), 5.19 (1H, d, J = 15.8 Hz), 6.65-7.05 (2H, br), 7.13 (2H, d, J = 8.8 Hz), 7.72-7.85 (1H, m), 7.91-8.02 (2H, m), 8.15-8.30 (2H, m), 8.80 (1H, d, J = 8.8 Hz).

### Example 14c

### 5-(3,3-Diphenylpropyloxy)-3-(4-isopropylphenyl)-2,2,4,6,7-pentamethyl-2,3-dihydrobenzofuran

Using 3-(4-isopropylphenyl)-2,2,4,6,7-pentamethyl-2,3-dihydrobenzofuran-5-ol and 3,3-diphenylpropyl methanesulfonate, the title compound was obtained in the same manner as in Example 1c. This was oily.
Yield: 55 %.
¹H-NMR (CDCl₃) δ : 0.99 (3H, s), 1.21 (6H, d, J = 7.0 Hz), 1.45 (3H, s), 1.71 (3H, s), 2.08 (3H, s), 2.10 (3H, s), 2.48 (1H, d, J = 6.6 Hz), 2.55 (1H, d, J = 6.6 Hz), 2.76-2.93 (1H, m), 3.60 (2H, t, J = 6.6 Hz), 4.07 (1H, s), 4.25 (1H, t, J = 8.0 Hz), 6.60-7.00 (2H, br), 7.06 (2H, d, J = 7.6 Hz), 7.10-7.34 (10H, m).

### Example 15c

### Methyl 4-[[3-(4-isopropylphenyl)-2,2,4,6,7-pentamethyl-2,3-dihydrobenzofuran-5-yl]oxymethyl]benzoate

Using methyl 3-(4-isopropylphenyl)-2,2,4,6,7-pentamethyl-2,3-dihydrobenzofuran-5-ol and methyl 4-(bromomethyl)methylbenzoate, the title compound was obtained in the same manner as in Example 1c.
Yield: 82 %.
m.p.: 108-110°C (from methanol).
¹H-NMR (CDCl₃) δ: 1.01 (3H, s), 1.22 (6H, d, J = 7.0 Hz), 1.50 (3H, s), 1.82 (3H, s), 2.16 (3H, s), 2.22 (3H, s), 2.86 (1H, septet, J = 7.0 Hz), 3.92 (3H, s), 4.09 (1H, s), 4.76 (2H, s), 6.65-7.00 (2H, br), 7.08 (2H, d, J = 8.0 Hz), 7.51 (2H, d, J = 8.0 Hz), 8.04 (2H, d, J = 8.2 Hz). 07 (1H, s), 4.21-4.37 (4H, m), 6.63-6.98 (2H, br), 7.07 (2H, d, J = 8.0 Hz).

### Example 16c

### Methyl α-[[3-(4-isopropylphenyl)-2,2,4,6,7-pentamethyl-2,3-dihydrobenzofuran-5-yl]oxy]phenylacetate

Using 3-(4-isopropylphenyl)-2,2,4,6,7-pentamethyl-2,3-dihydrobenzofuran-5-ol and methyl α-bromophenylacetate, the title compound was obtained in the same manner as in Example 1c. This was oily.
Yield: 82 %.
¹H-NMR (CDCl₃) δ: 0.99 (3H, s), 1.21, 1.23 (6H, each d, J = 7.0 Hz), 1.47 (3H, s), 1.57, 1.60 (3H, each s), 2.00, 2.04 (3H, each s), 2.09, 2.11 (3H, each s), 2.75-2.98 (1H, m), 3.70, 3.74 (3H, each s), 4.01 (1H, s), 5.07 (1H, s), 6.60-6.95 (2H, br), 7.06 (2H, d, J = 8.0 Hz), 7.24-7.50 (5H, m).

### Example 17c

### 3-(4-Isopropylphenyl)-2,2,4,6,7-pentamethyl-5-(2-pyridylmethyloxy)-2,3-dihydrobenzofuran

Using 3-(4-isopropylphenyl)-2,2,4,6,7-pentamethyl-2,3-dihydrobenzofuran-5-ol and 2-chloromethylpyridine hydrochloride, the title compound was obtained in the same manner as in Example 1c.
Yield: 17 %.
m.p.: 88-89°C (from methanol).
¹H-NMR (CDCl₃) δ: 1.02 (3H, s), 1. 22 (6H, d, J = 7.0 Hz), 1.51 (3H, s), 1.83 (3H, s), 2.17 (3H, s), 2.24 (3H, s), 2.86 (1H, septet, J = 7.0 Hz), 4.10 (1H, s), 4.80 (1H, d, J = 15.8 Hz), 4.89 (1H, d, J = 15.8 Hz), 6.72-7.02 (2H, br), 7.09 (2H, d, J = 8.2 Hz), 7.15-7.25 (1H, m), 7.67-7.81 (2H, m), 8.50-8.58 (1H, m).

### Example 18c

### 3-(4-Isopropylphenyl)-2,2,4,6,7-pentamethyl-5-(3-pyridylmethyloxy)-2,3-dihydrobenzofuran

Using 3-(4-isopropylphenyl)-2,2,4,6,7-pentamethyl-2,3-dihydrobenzofuran-5-ol and 3-chloromethylpyridine hydrochloride, the title compound was obtained in the same manner as in Example 1c. This was oily.
Yield: 76 %.
¹H-NMR (CDCl₃) δ: 1.02 (3H, s), 1.22 (6H, d, J = 7.0 Hz), 1.50 (3H, s), 1.82 (3H, s), 2.16 (3H, s), 2.22 (3H, s), 2.86 (1H, septet, J = 7.0 Hz), 4.09 (1H, s), 4.73 (2H, s), 6.63-7.02 (2H, br), 7.09 (2H, d, J = 8.2 Hz), 7.24 (1H, dd, J = 7.8, 5.0 Hz), 7.78 (1H, d, J = 7.6 Hz), 8.56 (1H, d, J = 4.0 Hz), 8.60-8.71 (1H, br).

### Example 19c

### 3-(4-Isopropylphenyl)-2,2,4,6,7-pentamethyl-5-(4-pyridylmethyloxy)-2,3-dihydrobenzofuran

Using 3-(4-isopropylphenyl)-2,2,4,6,7-pentamethyl-2,3-dihydrobenzofuran-5-ol and 4-chloromethylpyridine hydrochloride, the title compound was obtained in the same manner as in Example 1c. This was oily.
Yield: 52 %.
¹H-NMR (CDCl₃) δ: 1.02 (3H, s), 1.22 (6H, d, J = 7.0 Hz), 1.50 (3H, s), 1.82 (3H, s), 2.16 (3H, s), 2.21 (3H, s), 2.78-2.93 (1H, m), 4.08 (1H, s), 4.73 (2H, s), 6.62-7.01 (2H, br), 7.09 (2H, d, J = 8.4 Hz), 7.38 (2H, d, J = 5.8 Hz), 8.60 (2H, d, J = 5.8 Hz).

### Example 20c

### 3-(4-Isopropylphenyl)-5-(2,4-dinitrophenyloxy)-2,2,4,6,7-pentamethyl-2,3-dihydrobenzofuran

Sodium hydride (60 % liquid paraffin dispersion, 270 mg, 6.75 mmol) was added to a solution of 3-(4-isopropylphenyl)-2,2,4,6,7-pentamethyl-2,3-dihydrobenzofuran-5-ol (2.0 g, 6.16 mmol) in N,N-dimethylformamide (30 mL) at 0°C, and the mixture was stirred for 20 minutes at the same temperature. To the reaction mixture was added 1-chloro-2,4-dinitrobenzene (1.37 g, 6.78 mmol) and the mixture was stirred for 20 minutes at room temperature. The reaction mixture was poured into water (50 mL), and extracted twice with ethyl acetate. The organic layers were combined, washed with water, dried over magnesium sulfate, filtered, and concentrated under reduced pressure. The residue was crystallized from ethyl acetate-hexane to obtain the title compound (1.5 g, yield 50 %).
m.p.: 137-139°C.
¹H-NMR (CDCl₃) δ: 1.04 (3H, s), 1.22 (6H, d, J = 7.0 Hz), 1.57 (3H, s), 1.66 (3H, s), 2.03 (3H, s), 2.19 (3H, s), 2.86 (1H, septet, J = 7.0 Hz), 4.13 (1H, s), 6.62-6.95 (3H, m), 7.11 (2H, d, J = 8. 0 Hz), 8.26 (1H, dd, J = 9.2, 2.6 Hz), 8.75-8.86 (1H, m).

### Example 21c

### 5-(2,4-Bisacetylaminophenyloxy)-3-(4-isopropylphenyl)-2,2,4,6,7-pentamethyl-2,3-dihydrobenzofuran

3-(4-Isopropylphenyl)-5-(2,4-dinitrophenyloxy)-2,2,4,6,7-pentamethyl-2,3-dihydrobenzofuran (800 mg, 1.63 mmol) and 10 % palladium-carbon (hydrate) (80 mg) were dispersed in ethanol (40 mL), and the mixture was stirred under a hydrogen atmosphere at 60°C for 4 hours. The reaction mixture, from which was removed the catalyst through filtration, was concentrated under reduced pressure to obtain 5-(2,4-diaminophenoxy)-3-(4-isopropylphenyl)-2,2,4,6,7-pentamethyl-2,3-dihydrobenzofuran (710 mg). Acetyl chloride (0.26 mL, 3.63 mmol) was added to a solution of the thus-obtained compound (710 mg, 1.65 mmol) and triethylamine (290 mg, 1.70 mmol) in chloroform (30 mL) at 0°C, and the mixture was stirred for 1 hour at the same temperature. The reaction mixture was poured into water (30 mL), and extracted twice with ethyl acetate. The organic layers were combined, washed with an aqueous saturated sodium hydrogencarbonate, dried over magnesium sulfate, filtered, and concentrated under reduced pressure. The residue was subjected to silica gel column chromatography (hexane/ethyl acetate = 1/5) to obtain the title compound (640 mg, yield 76 %). This was amorphous.
¹H-NMR (CDCl₃) δ: 1.04 (3H, s), 1.22 (6H, d, J = 6.8 Hz), 1.52 (3H, s), 1.64 (3H, s), 2.00 (3H, s), 2.12 (3H, s), 2.18 (3H, s), 2.23 (3H, s), 2.86 (1H, septet, J = 6.8 Hz), 4.11 (1H, s), 6.30 (1H, d, J = 9.2 Hz), 6.60-7.03 (2H, br), 7.05 (2H, d, J = 8.4 Hz), 7.54 (1H, dd, J = 9.2, 2.6 Hz), 7.69 (1H, br s), 8.02 (1H, s), 8.21 (1H, d, J = 2.6 Hz).

### Example 22c

### α-[3-(4-isopropylphenyl)-2,2,4,6,7-pentamethyl-2,3-dihydrobenzofuran-5-yloxy]phenylacetic acid

An aqueous solution of 2 N sodium hydroxide (2.5 mL) was added dropwise to a solution of methyl α-[3-(4-isopropylphenyl)-2,2,4,6,7-pentamethyl-2,3-dihydrobenzofuran-5-yloxy]phenylacetate (1.20 g, 2.54 mmol) in a mixture of tetrahydrofuran (24 mL) and methanol (6 mL), and the mixture was stirred for 30 minutes at room temperature. The reaction mixture was concentrated under reduced pressure, to which was added 2 N hydrochloric acid. Then, this was extracted twice with ethyl acetate. The organic layers were washed with water, dried over magnesium sulfate, filtered, and concentrated under reduced pressure. The resulting residue was recrystallized from hexane to obtain the title compound (0.31 g, yield 27 %), which was a mixture of diastereomers (ratio: 8/1).
m.p.: 163-166°C.
¹H-NMR (CDCl₃) δ: 0.98 (3H, s), 1.12-1.25 (6H, m), 1.41-1.56 (6H, m), 1.92-2.10 (6H, m), 2.87 (1H, septet, J = 6. 6 Hz), 3.99 (1H, s), 5.08-5.10 (1H, m), 5.20-6.00 (1H, br), 6.60-7.17 (4H, m), 7.20-7.39 (5H, m).

### Example 23c

### α-[3-(4-isopropylphenyl)-2,2,4,6,7-pentamethyl-2,3-dihydrobenzofuran-5-yloxy]phenylacetic acid

The filtrate obtained in Example 22c was concentrated under reduced pressure to obtain the title compound (0.50 g, yield 43 %), which was amorphous and was a mixture of diastereomers (ratio: 1/3).
¹H-NMR (CDCl₃) δ: 0.98 (3H, s), 1.16-1.26 (6H, m), 1.39-1.56 (6H, m), 1.91- 2.10 (6H, m), 2.84 (1H, septet, J = 6.8 Hz), 4.00 (1H, m), 5.07-5.10 (1H, s), 5.40-6.30 (1H, br), 6.50-7.14 (4H, m), 7.20-7.40 (5H, m).

### Example 24c

### 3-(4-Isopropylphenyl)-2,2,4,6,7-pentamethyl-5-(3-phenyl-1-propyl)oxy-2,3-dihydrobenzofuran

3-(4-Isopropylphenyl)-2,2,4,6,7-pentamethyl-5-(3-phenyl-2-propen-1-yl)oxy-2,3-dihydrobenzofuran (800 mg, 1.82 mmol) and 10 % palladium-carbon (hydrate) (80 mg) were suspended in ethanol (20 mL), and the mixture was stirred for 3 hours under a hydrogen atmosphere at room temperature. The catalyst was removed through filtration, and the filtrate was concentrated under reduced pressure. The residue was crystallized from methanol to obtain the title compound (610 mg, yield 76 %).
m.p.: 78-80°C.
¹H-NMR (CDCl₃) δ: 0.99 (3H, s), 1.22 (6H, d, J = 6.8 Hz), 1.48 (3H, s), 1.81 (3H, s), 2.02-2.22 (8H, m), 2.76-2.91 (3H, m), 3.68 (2H, t, J = 6.4 Hz), 4.07 (1H, s), 6.70-6.92 (2H, br), 7.07 (2H, d, J = 8.8 Hz), 7.15-7. 32 (5H, m).

### Example 25c

### 3-(4-Isopropylphenyl)-2,2,4,6,7-pentamethyl-5-(2-phenylethyl)oxy-2,3-dihydrobenzofuran

A solution of 3-(4-isopropylphenyl)-2,2,4,6,7-pentamethyl-2,3-dihydrobenzofuran-5-ol (1.0 g, 3.08 mmol), 2-phenylethanol (414 mg, 3.39 mmol), triphenylphosphine (890 mg, 3.39 mmol) and diethyl azodicarboxylate (590 mg, 3.39 mmol) in tetrahydrofuran (20 mL) was stirred for 30 minutes at room temperature. The reaction mixture was concentrated under reduced pressure, and the residue was subjected to silica gel column chromatography (hexane/ethyl acetate = 100/1) to obtain the title compound (150 mg, yield 11 %).
m.p.: 72-74°C (from methanol).
¹H-NMR (CDCl₃) δ: 0.98 (3H, s), 1.21 (6H, d, J = 7.0 Hz), 1.46 (3H, s), 1.72 (3H, s), 2.10 (3H, s), 2.12 (3H, s), 2.83 (1H, septet, J = 7.0 Hz), 3.05 (2H, t, J = 7.0 Hz), 3.85 (2H, t, J = 7.0 Hz), 4.03 (1H, s), 6.65-7.00 (2H, br), 7.06 (2H, d, J = 8.0 Hz), 7.15-7.50 (5H, m).

### Example 26c

### 3-(4-Isopropylphenyl)-2,4,6,7-tetramethylbenzofuran-5-yl 4-methoxybenzoate

Triethylamine (0.45 mL, 3.21 mmol) was added to a solution of 3-(4-isopropylphenyl)-2,4,6,7-tetramethylbenzofuran-5-ol (0.90 g, 2.92 mmol) and 4-methoxybenzoyl chloride (0.55 g, 3,21 mmol) in chloroform (15 mL) at room temperature, and the mixture was stirred for 3 hours at 60°C. Water (30 mL) was poured into the reaction mixture, which was then extracted twice with ethyl acetate. The organic layers were combined, washed with 1 N hydrochloric acid and saturated sodium hydrogencarbonate, dried over magnesium sulfate, filtered, and concentrated under reduced pressure. The residue was crystallized from ethanol to obtain the title compound (0.52 g, yield 79 %).
m.p.: 113-115°C.
¹H-NMR (CDCl₃) δ: 1.28 (6H, d, J = 6.8 Hz), 1.90 (3H, s), 2.18 (3H, s), 2.33 (3H, s), 2.46 (3H, s), 2.95 (1H, septet, J = 6.8 Hz), 3.89 (3H, s), 6.99 (2H, d, J = 9.0 Hz), 7.25 (4H, s), 8.20 (2H, d, J = 8.8 Hz).

### Example 27c

### 3-(4-Isopropylphenyl)-5-(4-methoxybenzyloxy)-2,4,6,7-tetramethylbenzofuran

Using 3-(4-isopropylphenyl)-2,4,6,7-tetramethylbenzofuran-5-ol and 4-methoxybenzyl chloride, the title compound was obtained in the same manner as in Example 1c. This was oily.
Yield: 64 %.
¹H-NMR (CDCl₃) δ: 1.31 (6H, d, J = 6.8 Hz), 2.06 (3H, s), 2.31 (3H, s), 2.34 (3H, s), 2.43-(3H, s), 2.97 (1H, septet, J = 6.8 Hz), 3.82 (3H, s), 4.66 (2H, s), 6.91 (2H, d, J = 8.8 Hz), 7.26 (4H, s), 7.40 (2H, d, J = 8.8 Hz).

### Example 28c

### 2,4,6,7-Tetramethyl-3-phenylbenzofuran-5-yl 4-methoxybenzoate

Using 2,4,6,7-tetramethyl-3-phenylbenzofuran-5-ol and 4-methoxybenzoyl chloride, the title compound was obtained in the same manner as in Example 26c.
Yield 64%.
m.p.: 152-154°C (from methanol).
¹H₋NMR (CDCl₃) δ: 1.88 (3H, s), 2.18 (3H, s), 2.32 (3H, s), 2.46 (3H, s), 3.89 (3H, s), 6.99 (2H, d, J = 9.2 Hz), 7.29-7.43 (5H, m), 8.20 (2H, d, J = 9.2 Hz).

### Examples 29c

### 3-(4-Isopropylphenyl)-6-(4-methoxybenzyloxy)-2,2-dimethyl-2,3-dihydrobenzofuran

Sodium hydride (60 % liquid paraffin dispersion, 179.0 mg, 4.48 mmol) was added to a solution of 3-(4-isopropylphenyl)-2,2-dimethyl-2,3-dihydrobenzofuran-6-ol (1.12 g, 4.00 mmol) in N,N-dimethylformamide (15 mL) at 0°C, and the mixture was stirred for 30 minutes at the same temperature. To the reaction mixture was added 4-methoxybenzyl chloride (636.8 mg, 4.07 mmol) and the mixture was stirred for further 30 minutes at room temperature. The reaction mixture was poured into water, and extracted twice with ethyl acetate. The extracts were combined, washed with an aqueous saturated sodium chloride, dried over magnesium sulfate, and concentrated under reduced pressure. The residue was subjected to silica gel column chromatography (hexane/ethyl acetate = 5/1) to obtain the title compound (1.19 g, yield 74 %).
m.p.: 86-88°C (from hexane).
¹H-NMR(CDCl₃) δ: 0.95 (3H, s), 1.24 (6H, d, J = 7.0 Hz); 1.58 (3H, s), 2.89 (1H, septet, J = 7.0 Hz), 3.82 (3H, s), 4.27 (1H, s), 4.96 (2H, s), 6.47-6.52 (2H, m), 6.90-6.95 (3H, m), 7.02 (2H, d, J = 8.1 Hz) , 7.16 (2H, d, J = 8.1 Hz), 7.37 (2H, d, J = 8.8 Hz) .

### Example 30c

### 1'-Benzyl-3-(4-isopropylphenyl)-5-(4-methoxybenzyloxy)-4,6,7-trimethylspiro[benzofuran-2(3H),4'-piperidine]

Sodium hydride (60 % liquid paraffin dispersion, 81.4 mg, 1.81 mmol) was added to a solution of 1'-benzyl-3-(4-isopropylphenyl)-4,6,7-trimethylspiro[benzofuran-2(3H),4'-piperidine]-5-ol (824.0 mg, 1.81 mmol) in N,N-dimethylformamide (15 mL) at 0°C, and the mixture was stirred for 30 minutes at the same temperature. To the reaction mixture was added 4-methoxybenzyl chloride (319.9 mg, 2.04 mmol) and the mixture was stirred for further 30 minutes at room temperature. The reaction mixture was poured into water, and extracted twice with ethyl acetate. The extracts were combined, washed with an aqueous saturated sodium chloride, dried over magnesium sulfate, and concentrated under reduced pressure. The residue was subjected to silica gel column chromatography (hexane/ethyl acetate = 3/1) to obtain the title compound (539 mg, yield 52 %). This was amorphous.
¹H-NMR(CDCl₃) δ: 1.20 (6H, d, J = 6.8 Hz), 1.27-1.39 (2H, m), 1.81 (3H, s), 1.86-1.96 (2H, m), 2.19 (3H, s), 2.23 (3H, s), 2.35-2.87 (5H, m), 3.52 (2H, s), 3.80 (3H, s), 4.04 (1H, s), 4.62 (2H, s), 6.6-6.9 (4H, m), 7.04-7.08 (2H, m), 7.22-7.36 (7H, m).

### Example 31c

### 1'-Benzyl-5-(4-methoxybenzyloxy)-4,6,7-trimethylspiro[benzofuran-2(3H),4'-piperidine]

Sodium hydride (60 % liquid paraffin dispersion, 134.6 mg, 3.37 mmol) was added to a solution of 1'-benzyl-4,6,7-trimethylspiro[benzofuran-2(3H),4'-piperidine]-5-ol (1.01 g, 2.98 mmol) in N,N-dimethylformamide (15 mL) at 0°C, and the mixture was stirred for 30 minutes at the same temperature. To the reaction mixture was added 4-methoxybenzyl chloride (584.9 mg, 3.43 mmol) and the mixture was stirred for further 30 minutes at room temperature. The reaction mixture was poured into water, and extracted twice with ethyl acetate. The extracts were combined, washed with an aqueous saturated sodium chloride, dried over magnesium sulfate, and concentrated under reduced pressure. The residue was subjected to silica gel column chromatography (hexane/ethyl acetate = 2/1) to obtain the title compound (1.15 g, yield 85 %).
m.p.: 85-86°C (from hexane).
¹H-NMR(CDCl₃) δ: 1.80-2.00 (4H, m), 2.10 (3H, s), 2.15 (3H, s), 2.18 (3H, s), 2.60 (4H, br), 2.87 (2H, s), 3.58 (2H, s), 3.83 (3H, s), 4.62 (2H, s), 6.90-6.95 (2H, m), 7.30-7.43 (7H, m).

### Example 32c

### 3-(4-Isopropylphenyl)-5-(4-methoxybenzyloxy)-1',4,6,7-tetramethylspiro[benzofuran-2(3H),4'-piperidine]

Sodium hydride (60 % liquid paraffin dispersion, 64.3 mmol, 1.61 mmol) was added to a solution of 3-(4-isopropylphenyl)-1',4,6,7-tetramethylspiro[benzofuran-2(3H),4'-piperidine]-5-ol (509.0 mg, 1.34 mmol) in N,N-dimethylformamide (25 mL) at 0°C, and the mixture was stirred for 30 minutes at the same temperature. To the reaction mixture was added 4-methoxybenzyl chloride (244.0 mg, 1.56 mmol) and the mixture was stirred for further 30 minutes at room temperature. The reaction mixture was poured into water, and extracted twice with ethyl acetate. The extracts were combined, washed with an aqueous saturated sodium chloride, dried over magnesium sulfate, and concentrated under reduced pressure. The residue was subjected to column chromatography (Chromatorex NH DM1020, Fuji Silysia Chemical LTD) (hexane/ethyl acetate = 1/1) to obtain the title compound (262 mg, yield 39 %). This was amorphous.
¹H-NMR(CDCl₃) δ: 1.21 (6H, d, J = 7.0 Hz), 1.3-1.4 (2H, m), 1.82 (3H, s), 1.99-2.04 (2H, m), 2.19 (3H, s), 2.23 (3H, s), 2.30 (3H, s), 2.37-2.70 (4H, m), 2.82 (1H, septet, J = 7.0 Hz), 3.81 (3H, s), 4.05 (1H, s), 4.62 (2H, s), 6.6-6.9 (4H, m), 7.05-7.09 (2H, m), 7.33-7.37 (2H, m).

### Example 33c

### 3-(4-Isopropylphenyl)-1',4,6,7-tetramethyl-5-(4-pyridylmethyloxy)spiro[benzofuran-2(3H),4'-piperidine]

Sodium hydride (60 % liquid paraffin dispersion, 187.3 mg, 4.98 mmol) was added to a solution of 3-(4-isopropylphenyl)-1',4,6,7-tetramethylspiro[benzofuran-2(3H),4'-piperidine]-5-ol (817.7 mg, 2.15 mmol) in N,N-dimethylformamide (30 mL) at 0°C, and the mixture was stirred for 30 minutes at the same temperature. To the reaction mixture was added 4-chloromethylpyridine hydrochloride (364.5 mg, 2.22 mmol) and the mixture was stirred for further 30 minutes at room temperature. The reaction mixture was poured into water, and extracted twice with ethyl acetate. The extracts were combined, washed with an aqueous saturated sodium chloride, dried over magnesium sulfate, and concentrated under reduced pressure. The residue was subjected to column chromatography (Chromatorex NH DM1020, Fuji Silysia Chemical LTD) (hexane/ethyl acetate = 4/1) to obtain the title compound (575 mg, yield 57 %).
m.p.: 96-98°C (from hexane).
¹H-NMR (CDCl₃) δ: 1.21 (6H, d, J = 7.0 Hz),1.34-1.41 (2H, m), 1.82 (3H, s), 1.92-2.11 (2H, m), 2.19 (3H, s), 2.21 (3H, s), 2.30 (3H, s), 2.37-2.65 (4H, m), 2.85 (1H, septet, J = 7.0 Hz), 4.05 (1H, s), 4.72 (2H, s), 6.6-7.1 (4H, m), 7.36-7.39 (2H, m), 8.58-8.61 (2H, m).

The chemical structures of the compounds obtained in the above described Examples are shown below.

**Table 8**

| | | | | | | | | |
|---|---|---|---|---|---|---|---|---|
| 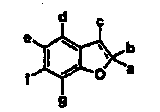 | | | | | | | | |

| example number | a | b | c | d | e | f | g | -̅ -̅ -̅ |
|---|---|---|---|---|---|---|---|---|
| 1c | Me | Me | 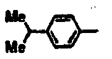 | Me | 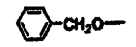 | Me | Me | --- |
| 2c | Me | Me | 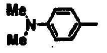 | Me | 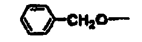 | Me | Me | --- |
| 3e | Me | 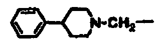 | H | Me | 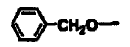 | Me | Me | --- |
| 4c | Me | Me | 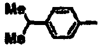 | Me | 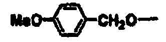 | Me | Me | --- |
| 5c | Me | Me | 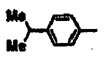 | H | 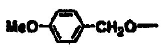 | H | H | --- |
| 6c | Me | Me | 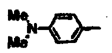 | Me | 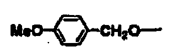 | Me | Me | --- |
| 7c | Me | Me | 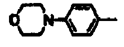 | Me | 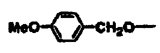 | Me | Me | --- |
| 8c | Me | Me | 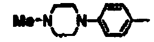 | Me | 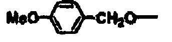 | Me | Me | --- |
| 9c | Me | Me | 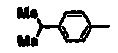 | Me | 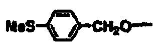 | Me | Me | --- |
| 10c | Me | Me | 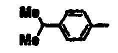 | Me | 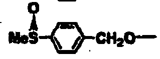 | Me | Me | --- |
| 11c | Me | Me | 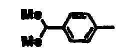 | Me | 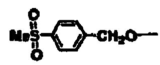 | Me | Me | --- |
| 12c | Me | Me | 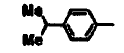 | Me | 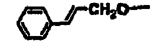 | Me | Me | --- |
| 13c | Me | Me | 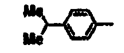 | Me | 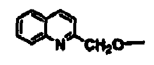 | Me | Me | --- |
| 14c | Me | Me | 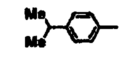 | Me | 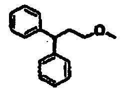 | Me | Me | --- |

**Table 9**

| | | | | | | | | |
|---|---|---|---|---|---|---|---|---|
| 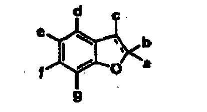 | | | | | | | | |

| example number | a | b | c | d | e | f | g | -̅ -̅ -̅ |
|---|---|---|---|---|---|---|---|---|
| 15c | Me | Me | 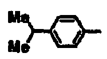 | Me | 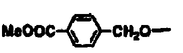 | Me | Me | --- |
| 16c | Me | Me | 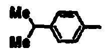 | Me | 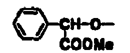 | Me | Me | --- |
| 17c | Me | Me | 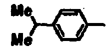 | Me | 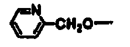 | Me | Me | --- |
| 18c | Me | Me | 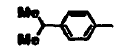 | Me | 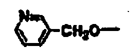 | Me | Me | --- |
| 19c | Me | Me | 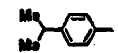 | Me | 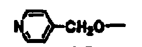 | Me | Me | --- |
| 20c | Me | Me | 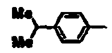 | Me | 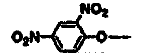 | Me | Me | --- |
| 21c | Me | Me | 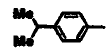 | Me | 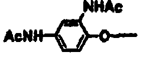 | Me | Me | --- |
| 22c | Me | Me | 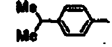 | Me | 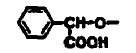 | Me | Me | --- |
| 23c | Me | Me | 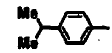 | Me | 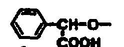 | Me | Me | --- |
| 24c | Me | Me | 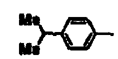 | Me | 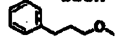 | Me | Me | --- |
| 25c | Me | Me | 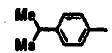 | Me | 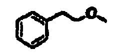 | Me | Me | --- |
| 26c | Me | Me | 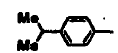 | Me | 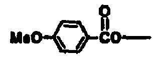 | Me | Me | -̅-̅-̅ |
| 27c | Me | Me | 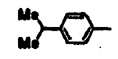 | Me | 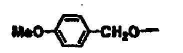 | Me | Me | -̅-̅-̅ |
| 28c | Me | Me | 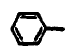 | Me | 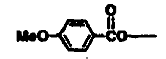 | Me | Me | -̅-̅-̅ |
| 29c | Me | Me | 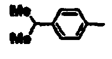 | H | H | 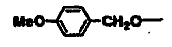 | H | --- |

**Table 10**

| | | | | | | |
|---|---|---|---|---|---|---|
| 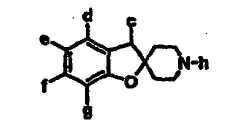 | | | | | | |

| example number | c | d | e | f | g | h |
|---|---|---|---|---|---|---|
| 30c | 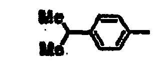 | Me | 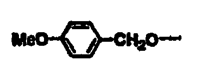 | Me | Me | 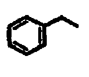 |
| 31c | H | Me | 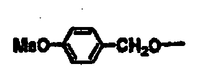 | Me | Me | 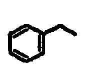 |
| 32c | 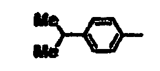 | Me | 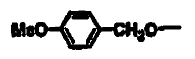 | Me | Me | Me |
| 33c | 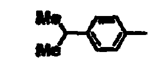 | Me | 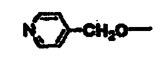 | Me | Me | Me |

### Formulation Example 1c

| | | |
|---|---|---|
| (1) | Compound obtained in Example 4c | 50 mg |
| (2) | Lactose | 34 mg |
| (3) | Corn starch | 10.6 mg |
| (4) | Corn starch (paste) | 5 mg |
| (5) | Magnesium stearate | 0.4 mg |
| (6) | Calcium carboxymethyl cellulose | 20 mg |
| | Total | 120 mg |

According to a conventional method, tablets were prepared by mixing the above-described substances (1) to (6), and then subjecting the resulting mixture to a tablet compression process by using a tablet compression machine.

### Experimental Example 1

Dopamine neuron regeneration promoting effect after MPP⁺ human induced neurodegeneration in rat fetal mesencephalic dopamine neuron culture immobilized on rat neonatal gliacyte

### Experimental Methods

A rat neonatal gliacyte was prepared from a cerebrum of a 1 to 2 days old SD rat. Fourteen days after DIV, said cell was subcultured and inoculated onto a 96-well culture plate coated with poly-L-lysine. A rat fetal dopamine nerve was prepared from a mesencephalon of a 14 days old SD rat fetus, and inoculated onto the gliacyte described above. 2 Days after initiation of the incubation, 3mM MMP⁺ was added and incubated for 24 hours whereby destroying the dopamine nerve. After 24 hours, the culture medium was replaced with a medium containing a compound of the present invention, and then the incubation was further continued for 4 days. After completion of the incubation followed by fixation with p-formaldehyde, the dopamine nerve was stained with an anti-tyrosine hydroxylase antibody and the tyrosine hydroxylase positive dopamine neurons were counted. The results are shown in Figure 1.

As appeared from Figure 1, an agent for promoting the proliferation or differentiation of a stem cell or neural progenitor cell comprising a Compound (I) of the present invention can promote the differentiation of a neural stem cell.

### Experimental Example 2

Neural neogenesis promoting effect in rat mixture glia culture

### a) Experimental Materials

A neonatal SD rat was purchased from Charles River Japan, Inc. A nylon cell strainer with 40 micron in diameter was purchased from Becton Dickinson. DMEM/F12 Medium, antibiotics, N2 additives were purchased from LIFE TECHNOLOGY. Anti-β III-tubulin antibody was purchased from Sigma. DAKO EnVision+/HRP kit was purchased from DAKO Japan. Other reagents were commercial products of analytical grade.

### b) Experimental Methods

### 1. A rat glia mixed culture

A rat mixed glia culture was made from a hippocampus of a neonatal SD rat of 2 days old. The neonate was anesthetized by ice-cooling, sacrificed by decapitation and the brain was taken out immediately. The meninx was removed carefully, and the cerebral cortex was separated. The hippocampus was pulverized mechanically by passing through a nylon cell strainer with 40 micron in diameter. The cell dispersion was overlaid on serum, and the cells were fractionated by a non-continuous gradient centrifugation. The pellet was washed twice with a growth medium (DMEM/F12 supplemented with 10% FBS and antibiotics) and then dispersed. The mixed glia culture was inoculated onto a collagen-coated 96-well multiplate at the density of 1 x 10⁵ cells per well, and incubated for 5 days.

### 2. Differentiation assay

After incubating for 5 days, the mixed glia culture was subjected to a differentiation assay. The growth medium was replaced with a serum-free medium (DMEM/F12 supplemented with N2 additives and antibiotics) and the test compound was added simultaneously. After allowing to undergo the differentiation for 5 days followed by fixation with 4% p-formaldehyde, a mouse anti-β III tubulin monoclonal antibody and DAKO EnVision+/HRP kit was used to effect an immunostaining.

The β III tubulin positive cells were counted, and the data in the presence (1 µM) of the Compound (I) and the absence (control) were compared. The % activity of each Compound (I) based on the non-treatment control activity is indicated in the table shown below.

**Table 11**

| Example compound | Differentiation or neogenesis promoting activity (%) |
|---|---|
| 11a | 478 |
| 17a | 344 |
| 19b | 468 |

Based on the results described above, an agent for promoting the proliferation or differentiation of a stem cell or neural progenitor cell comprising a Compound (I) of the present invention or a salt or prodrug thereof has an ability to promote the differentiation to or the neogenesis of a β III tubulin-positive neural progenitor cell.

### Industrial Applicability

An agent for promoting the proliferation or differentiation of a stem cell or neural progenitor cell comprising a Compound (I) of the present invention or a salt or prodrug thereof has excellent promoting effects on the proliferation or differentiation of an intrinsic neural stem cell and the engraftment or differentiation in neural stem cell or neurocyte transplantation, and thus is useful in preventing or treating a central nervous system disease such as a neurodegenerative disease.

## Claims

1. A use of a compound represented by formula : 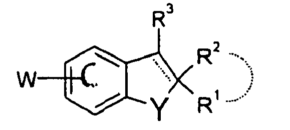 wherein R¹ and R² are same or different and each is a hydrogen atom, an optionally substituted hydrocarbon group or an optionally substituted heterocyclic group or R¹ and R² may be taken together with the adjacent carbon atom to form an optionally substituted 3- to 8-membered homocyclic or heterocyclic ring
R³ is a hydrogen atom, an optionally substituted hydrocarbon group or an optionally substituted heterocyclic group,
--- is a single bond or a double bond,
W is (i) a group represented by Formula: 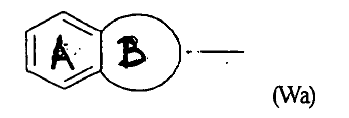 wherein Ring A is an optionally substituted benzene ring Ring B is an optionally substituted 5-to7-membere nitrogen-containing heterocyclic ring
(ii) a group represented by Formula: 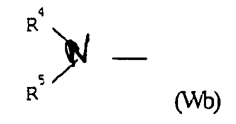 wherein R⁴ is (1) an aliphatic hydrocarbon group which is substituted by an optionally substituted aromatic group and which may have a further substituent or (2) an optionally substituted aromatic ring-containing acyl group, R⁵ is a hydrogen atom, a C₁₋₆ alkyl or an acyl group, or,
(iii) a group represented by Formula: 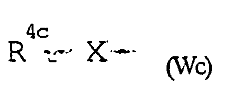 wherein R^{4c} is an optionally substituted aromatic group, an optionally substituted aliphatic hydrocarbon group or an acyl group, X is an oxygen atom or an optionally oxidized sulfur atom,
Y is an oxygen atom, an optionally oxidized sulfur atom or an optionally substituted imino,
Ring C is a benzene ring which may have a further substituent in addition to the group represented by W or a salt thereof in the production of an agent for promoting the engraftment or differentiation in neural stem cell and/or neurocyte transplantation or in the production of an agent for promoting the proliferation or differentiation of a neural stem cell and/or neurocyte for transplantation, wherein the cells are not of human embryonic origin.

2. The use according to Claim 1 wherein — is a single bond.

3. The use according to Claim 1 wherein Y is an oxygen atom.

4. The use according to Claim 1 wherein W is a group represented by Formula (Wa).

5. The use according to Claim 1 wherein each of R¹ and R² is a hydrogen atom or a C₁₋₆ alkyl group, R³ is a hydrogen atom or a phenyl group which may have 1 to 3 substituents selected from C₁₋₆ alkyl and halogen, the Ring C is a benzene ring which may further have 1 to 3 substituents selected from C₁₋₆ alkyl and C₁₋₆ alkoxy, --- is a single bond, Y is an oxygen atom, the group represented by Formula (Wa) is a group represented by Formula: 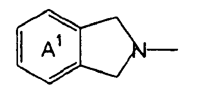 wherein Ring A¹ is a benzene ring which may have 1 to 3 substituents selected from halogen, C₁₋₆ alkoxy and C₁₋₆ alkylenedioxy.

6. The use according to Claim 5 wherein the group represented by Formula (Wa) is a substituent on the 5-position of the benzofuran ring.

7. The use according to Claim 4 comprising [1] 2-[2,2,4,6,7-pentamethyl-3-(4-methylphenyl)-2,3-dihydro-1-benzofuran-5-yl]isoindoline, [2] 5,6-dimethoxy-2-[2,2,4,6,7-pentamethyl-3-(4-methylphenyl)-2,3-dihydro-1-benzofuran-5-yl]isoindoline, [3] 5,6-dimethoxy-2-[3-(4-isopropylphenyl)-2,2,4,6,7-pentamethyl-2,3-dihydro-1-benzofuran-5-yl]isoindoline, [4] 5,6-dimethoxy-2-[2,2,4,6,7-pentamethyl-3-(4-methylphenyl)-2,3-dihydro-1-benzofuran-5-yl]-2H-isoindole, [5] 6-[3-(4-isopropylphenyl)-2,2,4,6,7-pentamethyl-2,3-dihydro-1-benzofuran-5-yl]-6,7-dihydro-5H-[1,3]dioxolo[4,5-f]isoindole, [6] 6-[2,2,4,6,7-pentamethyl-3-(4-methylphenyl)-2,3-dihydro-1-benzofuran-5-yl]-6H-[1,3]dioxolo[4,5-f]isoindole, [7] 6-(2,2,4,6,7-pentamethyl-3-phenyl-2,3-dihydro-1-benzofuran-5-yl)-6,7-dihydro-5H-[1,3]dioxolo[4,5-f]isoindole, [8] (R)-5,6-dimethoxy-2-[2,2,4,6,7-pentamethyl-3-(4-methylphenyl)-2,3-dihydro-1-benzofuran-5-yl]isoindoline or[9] (R)-5,6-dimethoxy-2-[2,2,4,6,7-pentamethyl-3-(4-methylphenyl)-2,3-dihydro-1-benzofuran-5-yl]isoindolinehydrochloride.

8. The use according to Claim 4 comprising (R)-5,6-dimethoxy-2-[2,2,4,6,7-pentamethyl-3-(4-metylphenyl)-2,3-dihydro-1-benzofuran-5-yl]isoindoline.

9. The use according to Claim 1 wherein W is a group represented by Formula (Wb).

10. The use according to Claim 9 wherein each of R¹ and R² is a methyl group, R³ is a phenyl group which may have 1 to 3 substituents selected from fluorine, methyl and isopropyl, the Ring C is a benzene ring which may further have 1 to 3 substituents selected from C₁₋₆ alkyl and C₁₋₆ alkoxy, Y is an oxygen atom, R⁴ is a benzyl or phenethyl group which may have 1 to 3 substituents selected from fluorine, methoxy and methylenedioxy and R⁵ is a hydrogen atom or a methyl group.

11. The use according to Claim 9 comprising (1) N-(4-fluorobenzyl)-2,2,4,6,7-pentamethyl-3-phenyl-2,3-dihydro-1-benzofuran-5-amine,(2)N-benzyl-3-(4-isopropylphenyl)-2,2,4,6,7-pentamethyl-2,3-dihydro-1-benzofuran-5-amine, (3) 3-(4-isopropylphenyl)-N-(4-methoxybenzyl)-N,2,2,4,6,7-hexamethyl-2,3-dihydro-1-benzofuran-5-amine, (4) 3-(4-isopropylphenyl)-N-[2-(4-metoxyphenyl)ethyl]-2,2,4,6,7-pentamethyl-2,3-dihydro-1-benzofuran-5-amine, (5) N-(4-fluorobenzyl)-3-(4-isopropylphenyl)-2,2,4,6,7-pentamethyl-2,3-dihydro-1-benzofuran-5-amine, (6) N-(1,3-benzodioxol-5-ylmethyl)-3-(4-isopropylphenyl)-2,2,4,6,7-pentamethyl-2,3-dihydro-1-benzofuran-5-amine, (7) N-(4-fluorobenzyl)-3-(4-fluorophenyl)-2,2,4,6,7-pentamethyl-2,3-dihydro-1-benzofuran-5-amine,(8) N-(4-methoxybenzyl)-2,2,4,6,7-pentamethyl-3-(4-methylphenyl)-2,3-dihydro-1-benzofuran-5-amine, (9) N-(4-fluorobenzyl)-2,2,4,6,7-pentamethyl-3-(4methylphenyl)-2,3-dihydro-1-benzofuran-5-amine, (10) 3-(4-isopropylphenyl)-N-(4-methoxybenzyl)-2,4,6,7-tetramethyl-1-benzofuran-5-amine, (11)N-(4-fluorobenzyl)-3-(4-isopropylphenyl)-2,4,6,7-tetramethyl-1-benzofuran-5-amine, (12) N-(4-fluorobenzyl)-3-(4-fluorophenyl)-2,4,6,7-tetramethyl-1-benzofuran-5-amine, (13) N-(4-fluorobenzyl)-3-(4-isopropylphenyl)-1',4,6,7-tetramethylspiro[benzofuran-2(3H),4'-pyperidine]-5-amine or (14) (R)-N-(4-fluorobenzyl)-3-(4-isopropylphenyl)-2,2,4,6,7-pentamethyl-2,3-dihydro-1-benzofuran-5-amine hydrochloride.

12. The use according to Claim 1 wherein W is a group represented by Formula(Wc).

13. The use according to Claim 12 comprising a compound represented by Formula: 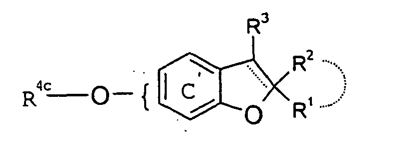 wherein each of R¹ and R² is C₁₋₆ alkyl which may have a phenyl-substituted 6-membered saturated cyclic amino,
or R¹ and R² are taken together with the adjacent carbon atom to form a C₁₋₆ alkyl- or C₇₋₁₆ aralkyl-substituted piperidine;
R³ is (i) a hydrogen atom, or;
(ii) phenyl which may have 1 to 3 substituents selected from (1) C₁₋₆ alkyl, (2) di-C₁₋₆ alkylamino and (3)6-membered saturated cyclic amino which may have C₁₋₆ alkyl;
R^{4c} is (i) phenyl which may have 1 to 3 substituents selected from nitro and C₁₋₆ alkyl-carboxamide,
(ii) C₁₋₆ alkyl or C₂₋₆alkenyl having 1 to 3 phenyl, quinolyl or pyridyl which may have 1 to 3 substituents selected from C₁₋₆ alkoxy, C₁₋₆alkylthio, C₁₋₆alkoxy-carbonyl, C₁₋₆alkylsulfonyl and C₁₋₆ alkylsulfinyl and optionally further having phenyl, carboxy or C₁₋₆alkoxy-carbonyl as additional substituents, or,
(iii)acyl represented by Formula:-(C=O)-R^{5"} wherein R^{5"} is C₁₋₆alkoxy-substituted phenyl; and,
the Ring C is a benzene ring which may further have 1 to 3 C₁₋₆ alkyl, or a salt thereof.

14. The use according to Claim 12 comprising:
3-(4-isopropylphenyl)-5-(4-methoxybenzyloxy)-2,2,4,6,7-pentamethyl-2,3-dihydrobenzofuran, 3-(4-methylphenyl)-2,4,6,7-tetramethylbenzofuran-5-yl4-methoxybenzoate,3-(4-isopropylpenyl)-2,4,6,7-tetramethylbenzofuran-5-yl4-methoxybenzoate, 3-(4-isopropylphenyl)-5-(4-methoxybenzyloxy)-2,4,6,7-tetramethylbenzofuran, 3-(4-isopropylphenyl)-5-(4-isopropylphenyl)1'4,6,7-tetramethylspiro[benzofuran-2(3H),4'-piperidine] or a salt thereof.

15. A method for culturing a stem cell, neural progenitor cell and/or neurocyte wherein the cells are not of human embryonic origin, comprising culturing the stem cell, neural progenitor cell and/or neurocyte in the presence of a compound represented by Formula: 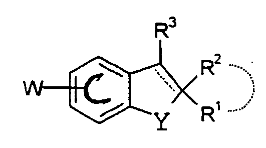 wherein R¹ and R² are same or different and each is a hydrogen atom, an optionally substituted hydrocarbon group or an optionally substituted heterocyclic group or R¹ and R² may be taken together with the adjacent carbon atom to form an optionally substituted 3- to 8-membered homocyclic or heterocyclic ring
R³ is a hydrogen atom, an optionally substituted hydrocarbon group or an optionally substituted heterocyclic group,
--- is a single bond or a double bond,
W is (i) a group represented by Formula: 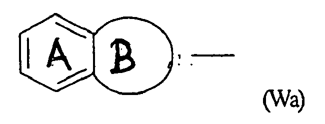 wherein Ring A is an optionally substituted benzene ring Ring B is an optionally substituted 5-to 7-membered nitrogen-containing heterocyclic ring
(ii) a group represented by Formula: 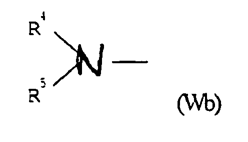 wherein R⁴ is (1) an aliphatic hydrocarbon group which is substituted by an optionally substituted aromatic group and which may have a further substituent or (2) an optionally substituted aromatic ring-containing acyl group, R⁵ is a hydrogen atom, a C₁₋₆ alkyl or an acyl group, or,
(iii) a group represented by Formula: 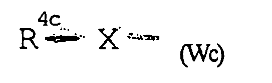 wherein R^{4c} is an optionally substituted aromatic group, an optionally substituted aliphatic hydrocarbon group or an acyl group, Xis an oxygen atom or an optionally oxidized sulfur atom,
Y is an oxygen atom, an optionally oxidized sulfur atom or an optionally substituted imino,
Ring C is a benzene ring which may have a further substituent in addition to the group represented by W or a salt thereof

16. The method according to Claim 15 wherein ^{...} is a single bond.

17. The method according to Claim 15 wherein Y is an oxygen atom.

18. The method according to Claim 15 wherein W is a group represented by Formula (Wa).

19. The method according to Claim 18 wherein each of R¹ and R² is a hydrogen atom or a C₁₋₆ alkyl group, R³ is a hydrogen atom or a phenyl group which may have 1 to 3 substituents selected from C₁₋₆alkyl and halogen, the Ring C is a benzene ring which may further have 1 to 3 substituents selected from C₁₋₆ alkyl and C₁₋₆ alkoxy, ^{...} is a single bond, Y is an oxygen atom, the group represented by Formula(Wa) is a group represented by Fomula: 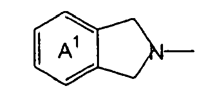 wherein Ring A¹ is a benzene ring which may have 1 to 3 substituents selected from halogen, C₁₋₆alkoxy and C₁₋₆ alkylenedioxy.

20. The method according to Claim 19 wherein the group represented by Formula (Wa) is a substituent on the 5-position of the benzofuran ring

21. The method according to Claim 18 comprising [1] 2-[2,2,4,6,7-pentamethyl-3-(4-methylphenyl)-2,3-dihydro-1-benzofuran-5-yl]isoindoline, [2] 5,6-dimethoxy-2-[2,2,4,6,7-pentamethyl-3-(4 methylphenyl)-2,3-dihydro-1-benzofuran-5-yl]isoindoline, [3] 5,6-dimethoxy-2-[3-(4-isopropylphenyl)-2,2,4,6,7-pentamethyl-2,3-dihydro-1-benzofuran-5-yl]isoindoline, [4] 5,6-dimethoxy-2-[2,2,4,6,7-pentamethyl-3-(4-methylphenyl)-2,3-dihydro-1-benzofuran-5-yl]-2H-isoindole, [5] 6-[3-(4-isopropylphenyl)-2,3-dihydro-1-benzofuran-5-yl]-6,7-dihydro-5H-[1,3]dioxolo[4,5-f]isoindole, [6] 6-[2,2,4,6,7-pentamethyl-3-(4-methylphenyl)-2,3-dihydro-1-benzofuran-5-y]-6H-[1,3]dioxolo[4,5-f]isoindole, [7] 6-(2,2,4,6,7-pentamethyl-3-phenyl-2,3-dihydro-1-benzofuran-5-yl)-6,7-dihydro-5H-[1,3]dioxolo[4,5-f]isoindole, [8] (R)-5,6-dimethoxy-2-[2,2,4,6,7-pentamethyl-3-(4-methylphenyl)-2,3-dihydro-1-benzofuran-5-yl]isoindoline or [9] (R)-5,6-dimethoxy-2-[2,2,4,6,7-pentamethyl-3-(4-methylphenyl)-2,3-dihydro-1-benzofuran-5-yl]isoindoline hydrochloride.

22. The method according to Claim 18 comprising (R)-5,6-dimethoxy-2-[2,2,4,6,7-pentamethyl-3-(4-methylphenyl)-2,3-dihydro-1-benzofuran-5-yl]isoindoline.

23. The method according to Claim 15 wherein W is a group represented by Formula (Wb).

24. The method according to Claim 23 wherein each of R¹ and R² is a methyl group, R³ is a phenyl group which may have 1 to 3 substituents selected from fluorine, methyl and isopropyl, the Ring C is a benzene ring which may further have 1 to 3 substituents selected from C₁₋₆ alkyl and C₁₋₆ alkoxy, Y is an oxygen atom, R⁴ is a benzyl or phenethyl group which may have 1 to 3 substituents selected from fluorine, methoxy and methylenedioxy and R⁵ is a hydrogen atom or a methyl goup.

25. The method according to Claim 23 comprising (1) N-(4-fluorobenzyl)-2,2,6,7-pentamethyl-3-phenyl-2,3-dihydro-1-benzofuran-5-amine, (2)N-benzyl-3-(4-isopropylphenyl)-2,2,6,7-pentamethyl-2,3-dihydro-1-benzofuran-5-amine,(3)3-(4-isopropylphenyl)-N-(4-methoxybenzyl)-N,2,2,4,6,7-hexamethyl-2,3-dihydro-1-benzofuran-5-amine, (4) 3-(4-isopropylphenyl)-N-[2-(4-methoxyphenyl)ethyl]-2,2,4,6,7-pentamethyl-2,3-dihydro-1-benzofuran-5-amine, (5) N-(4-fluorobenzyl)-3-(4-isopropylphenyl)-2,2,4,6,7-pentamethyl-2,3-dihydro-1-benzofuran-5-amine, (6)N-(1,3-benzodioxol-5-ylmethyl)-3-(4-isopropylphenyl)-2,2,4,6,7-pentamethyl-2,3-dihydro-1-benzofuran-5-amine, (7) N-(4-fluorobenzyl)-3-(4-fluorophenyl)-2,2,4,6,7-pentamethyl-2,3-dihydro-1-benzofuran-5-amine, (8) N-(4-methoxybenzyl)-2,2,4,6,7-pentamethyl-3-(4-methylphenyl)-2,3-dihydro-1-benzofuran-5-amine, (9) N-(4-fluorobenzyl)-2,2,4,6,7-pentamethyl-3-(4-methylphenyl)-2,3-dihydro-1-benzofuran-5-amine, (10) 3-(4-isopropylphenyl)-N-(4-methoxybenzyl)-2,4,6,7-tetramethyl-1-benzofuran-5-amine, (11) N-(4-fluorobenzyl)-3-(4-isopropylphenyl)-2,4,6,7-tetramethyl-1-benzofuran-5-amine, (12) N-(4-fluorobenzyl)-3-(4-fluorophenyl)-2,4,6,7-tetramethyl-1-benzofuran-5-amine, (13) N-(4-fluorobenzyl)-3-(4-isopropylphenyl)-1',4,6,7-tetramethylspiro[benzofuran-2(3H),4'-pyperidine]-5-amine or (14) (R)-N-(4-fluorobenzyl-3-(4-isopropylphenyl)-2,2,4,6,7-pentamethyl-2,3-dihydro-1-benzufuran-5-amine hydrochloride.

26. The method according to Claim 15 wherein W is a group represented by Formula (Wc).

27. The method according to Claim 26 comprising a compound represented by Formula: 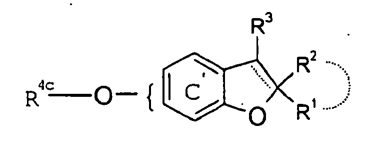 wherein each of R¹ and R² is C₁₋₆ alkyl which may have a phenyl-substituted 6-membered saturated cyclic amino,
or R¹ and R² are taken together with the adjacent carbon atom to form a C₁₋₆ alkyl- or C₇₋₁₆ aralkyl-substituted piperidine;
R³ is (i) a hydrogen atom, or,
(ii) phenyl which may have 1 to 3 substituents selected from (1) C₁₋₆ alkyl, (2) di-C₁₋₆ alkylamino and (3) 6-membered saturated cyclic amino which may have C₁₋₆ alkyl;
R^{4c} is (1) phenyl which may have 1 to 3 substituents selected from nitro and C₁₋₆ alkyl-carboxamide,
(ii)C₁₋₆alkyl or C₂₋₆ alkenyl having 1 to 3 phenyl, quinolyl or pyridyl which may have 1 to 3 substituents selected fromC₁₋₆alkoxy, C₁₋₆alkylthio, C₁₋₆alkoxy-carbonyl, C₁₋₆ alkylsulfonyl and C₁₋₆ alkylsulfinyl and optionally further having phenyl, carboxy or C₁₋₆ alkoxy-carbonyl as additional substituents, or,
(iii)acyl represented by Formula: -(C=O)-R^{5"} wherein R^{5"} is C₁₋₆alkoxy-substituted phenyl; and,
the Ring C is a benzene ring which may further have 1 to 3 C₁₋₆ alkyl, or a salt thereof

28. The method according to Claim 26 comprising
3-(4-isopropylphenyl)-5-(4-methoxybenzyloxy)-2,2,4,6,7-pentamethyl-2,3-dihydrobenzofuran, 3-(4-methylphenyl)-2,4,6,7-tetramethylbenzofuran-5-yl 4-methoxybenzoate, 3-(4-isopropylphenyl)-2,4,6,7-tetramethylbenzofuran-5-yl4-methoxybenzoate, 3-(4-isopropylphenyl)-5-(4-methoxybenzyloxy)-2,4,6,7-tetramethylbenzofuran, 3-(4-isoproprylphenyl)-5-(4-methoxybenzyloxy)-1',4,6,7-tetramethylspiro[benzofuran-2(3H),4'-piperidine] or a salt thereof.

29. The method according to Claim 15 wherein the stem cell is an embryonic stem cell or a neural stem cell.

30. The method according to Claim 15 whereby a cell for transplantation therapy is prepared

## Patentansprüche

1. Verwendung einer Verbindung, die durch die Formel 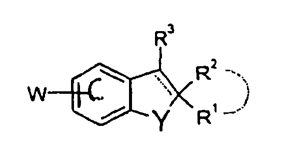 repräsentiert wird, wobei R¹ und R² gleich oder verschieden sind und jeweils ein Wasserstoffatom, eine gegebenenfalls substituierte Kohlenwasserstoffgruppe oder eine gegebenenfalls substituierte heterocyclische Gruppe sind oder R¹ und R² zusammen mit dem benachbarten Kohlenstoffatom einen gegebenenfalls substituierten drei- bis achtgliedrigen homocyclischen oder heterocyclischen Ring bilden können;
R³ ein Wasserstoffatom, eine gegebenenfalls substituierte Kohlenwasserstoffgruppe oder eine gegebenenfalls substituierte heterocyclische Gruppe ist;
^{....} eine Einfachbindung oder eine Doppelbindung ist;
W Folgendes ist: (i) eine Gruppe, die durch die Formel 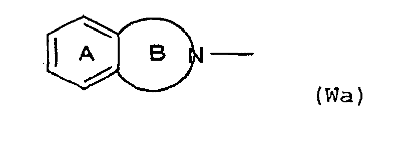 repräsentiert wird, wobei Ring A ein gegebenenfalls substituierter Benzolring ist, Ring B ein gegebenenfalls substituierter fünf- bis siebengliedriger stickstoffhaltiger heterocyclischer Ring ist;
(ii) eine Gruppe, die durch die Formel 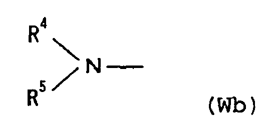 repräsentiert wird, wobei R⁴ (1) eine aliphatische Kohlenwasserstoffgruppe, die mit einer gegebenenfalls substituierten aromatischen Gruppe substituiert ist und die einen weiteren Substituenten aufweisen kann, oder (2) eine Acylgruppe, die einen gegebenenfalls substituierten aromatischen Ring enthält, ist, R⁵ ein Wasserstoffatom, ein C₁₋₆-Alkyl oder eine Acylgruppe ist; oder
(iii) eine Gruppe, die durch die Formel
R^{4c} - X - (Wc)
repräsentiert wird, wobei R^{4c} eine gegebenenfalls substituierte aromatische Gruppe, eine gegebenenfalls substituierte aliphatische Kohlenwasserstoffgruppe oder eine Acylgruppe ist, X ein Sauerstoffatom oder ein gegebenenfalls oxidiertes Schwefelatom ist;
Y ein Sauerstoffatom, ein gegebenenfalls oxidiertes Schwefelatom oder ein gegebenenfalls substituiertes Imino ist;
Ring C ein Benzolring ist, der neben der durch W dargestellten Gruppe noch einen weiteren Substituenten haben kann;
oder eines Salzes davon bei der Herstellung eines Mittels zur Förderung der Integration ins Gewebe oder der Differenzierung bei der Transplantation von neuralen Stammzellen und/oder Neurocyten oder bei der Herstellung eines Mittels zur Förderung der Proliferation oder Differenzierung einer neuralen Stammzelle und/oder eines Neurocyten für die Transplantation, wobei die Zellen nicht aus humanen Embryonen stammen.

2. Verwendung gemäß Anspruch 1, wobei ---- eine Einfachbindung ist.

3. Verwendung gemäß Anspruch 1, wobei Y ein Sauerstoffatom ist.

4. Verwendung gemäß Anspruch 1, wobei W eine durch Formel (Wa) repräsentierte Gruppe ist.

5. Verwendung gemäß Anspruch 1, wobei R¹ und R² jeweils ein Wasserstoffatom oder eine C₁₋₆-Alkylgruppe sind, R³ ein Wasserstoffatom oder eine Phenylgruppe, die 1 bis 3 Substituenten, die aus C₁₋₆-Alkyl und Halogen ausgewählt sind, haben kann, ist, Ring C ein Benzolring ist, der weiterhin 1 bis 3 Substituenten haben kann, die aus C₁₋₆-Alkyl und C₁₋₆-Alkoxy ausgewählt sind, ---- eine Einfachbindung ist, Y ein Sauerstoffatom ist, die durch Formel (Wa) repräsentierte Gruppe eine Gruppe ist, die durch die Formel 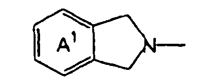 repräsentiert wird, wobei Ring A¹ ein Benzolring ist, der 1 bis 3 Substituenten haben kann, die aus Halogen, C₁₋₆-Alkoxy und C₁₋₆-Alkylendioxy ausgewählt sind.

6. Verwendung gemäß Anspruch 5, wobei die durch Formel (Wa) repräsentierte Gruppe ein Substituent an der 5-Position des Benzofuranrings ist.

7. Verwendung gemäß Anspruch 4, umfassend [1] 2-[2,2,4,6,7-Pentamethyl-3-(4-methylphenyl)-2,3-dihydro-1-benzofuran-5-yl]isoindolin, [2] 5,6-Dimethoxy-2-[2,2,4,6,7-pentamethyl-3-(4-methylphenyl)-2,3-dihydro-1-benzofuran-5-yl]isoindolin, [3] 5,6-Dimethoxy-2-[3-(4-isopropylphenyl)-2,2,4,6,7-pentamethyl-2,3-dihydro-1-benzofuran-5-yl]isoindolin, [4] 5,6-Dimethoxy-2-[2,2,4,6,7-pentamethyl-3-(4-methylphenyl)-2,3-dihydro-1-benzofuran-5-yl]-2H-isoindol, [5] 6-[3-(4-Isopropylphenyl)-2,2,4,6,7-pentamethyl-2,3-dihydro-1-benzofuran-5-yl]-6,7-dihydro-5H-[1,3]dioxo-lo[4,5-f]isoindol, [6] 6-[2,2,4,6,7-Pentamethyl-3-(4-methylphenyl)-2,3-dihydro-1-benzofuran-5-yl]-6H-[1,3]dioxolo[4,5-f]isoindol, [7] 6-(2,2,4,6,7-Pentamethyl-3-phenyl-2,3-dihydro-1-benzofuran-5-yl)-6,7-dihydro-5H-[1,3]dioxolo[4,5-f]isoindol, [8] (R)-5,6-Dimethoxy-2-[2,2,4,6,7-pentamethyl-3-(4-methylphenyl)-2,3-dihydro-1-benzofuran-5-yl]isoindolin oder [9] (R)-5,6-Dimethoxy-2-[2,2,4,6,7-pentamethyl-3-(4-methylphenyl)-2,3-dihydro-1-benzofuran-5-yl]isoindolin-Hydrochlorid.

8. Verwendung gemäß Anspruch 4, umfassend (R)-5,6-Dimethoxy-2-[2,2,4,6,7-pentamethyl-3-(4-methylphenyl)-2,3-dihydro-1-benzofuran-5-yl]isoindolin.

9. Verwendung gemäß Anspruch 1, wobei W eine durch Formel (Wb) repräsentierte Gruppe ist.

10. Verwendung gemäß Anspruch 9, R¹ und R² jeweils eine Methylgruppe sind, R³ eine Phenylgruppe ist, die 1 bis 3 Substituenten haben kann, die aus Fluor, Methyl und Isopropyl ausgewählt sind, Ring C ein Benzolring ist, der weiterhin 1 bis 3 Substituenten haben kann, die aus C₁₋₆-Alkyl und C₁₋₆-Alkoxy ausgewählt sind, Y ein Sauerstoffatom ist, R⁴ eine Benzyl- oder Phenethylgruppe ist, die 1 bis 3 Substituenten haben kann, die aus Fluor, Methoxy und Methylendioxy ausgewählt sind, und R⁵ ein Wasserstoffatom oder eine Methylgruppe ist.

11. Verwendung gemäß Anspruch 9, umfassend (1) N-(4-Fluorbenzyl)-2,2,4,6,7-pentamethyl-3-phenyl-2,3-dihydro-1-benzofuran-5-amin, (2) N-Benzyl-3-(4-isopropylphenyl)-2,2,4,6,7-pentamethyl-2,3-dihydro-1-benzofuran-5-amin, (3) 3-(4-Isopropylphenyl)-N-(4-methoxybenzyl)-N,2,2,4,6,7-hexamethyl-2,3-dihydro-1-benzofuran-5-amin, (4) 3-(4-Isopropylphenyl)-N-[2-(4-methoxyphenyl)ethyl]-2,2,4,6,7-pentamethyl-2,3-dihydro-1-benzofuran-5-amin, (5) N-(4-Fluorbenzyl)-3-(4-isopropylphenyl)-2,2,4,6,7-pentamethyl-2,3-dihydro-1-benzofuran-5-amin, (6) N-(1,3-Benzodioxol-5-ylmethyl)-3-(4-isopropylphenyl)-2,2,4,6,7-pentamethyl-2,3-dihydro-1-benzofuran-5-amin, (7) N-(4-Fluorbenzyl)-3-(4-fluorphenyl)-2,2,4,6,7-pentamethyl-2,3-dihydro-1-benzofuran-5-amin, (8) N-(4-Methoxybenzyl)-2,2,4,6,7-pentamethyl-3-(4-methylphenyl)-2,3-dihydro-1-benzofuran-5-amin, (9) N-(4-Fluorbenzyl)-2,2,4,6,7-pentamethyl-3-(4-methylphenyl)-2,3-dihydro-1-benzofuran-5-amin, (10) 3-(4-Isopropylphenyl)-N-(4-methoxybenzyl)-2,4,6,7-tetramethyl-1-benzofuran-5-amin, (11) N-(4-Fluorbenzyl)-3-(4-isopropylphenyl)-2,4,6,7-tetramethyl-1-benzofuran-5-amin, (12) N-(4-Fluorbenzyl)-3-(4-fluorphenyl)-2,4,6,7-tetramethyl-1-benzofuran-5-amin, (13) N-(4-Fluorbenzyl)-3-(4-isopropylphenyl)-1',4,6,7-tetramethylspiro[benzofuran-2(3H),4'-pyperidin]-5-amin oder (14) (R)-N-(4-Fluorbenzyl)-3-(4-isopropylphenyl)-2,2,4,6,7-pentamethyl-2,3-dihydro-1-benzofuran-5-amin-Hydrochlorid.

12. Verwendung gemäß Anspruch 1, wobei W eine durch Formel (Wc) repräsentierte Gruppe ist.

13. Verwendung gemäß Anspruch 12, umfassend eine Verbindung, die durch die Formel 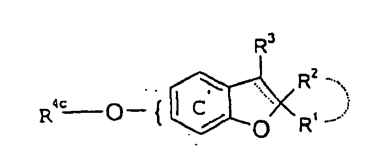 repräsentiert wird, wobei R¹ und R² jeweils C₁₋₆-Alkyl sind, das ein phenylsubstituiertes sechsgliedriges gesättigtes cyclisches Amino aufweisen kann, oder R¹ und R² zusammen mit dem benachbarten Kohlenstoffatom ein C₁₋₆-alkyl- oder C₇₋₁₆-aralkylsubstituiertes Piperidin bilden;
R³ Folgendes ist: (i) ein Wasserstoffatom; oder
(ii) Phenyl, das 1 bis 3 Substituenten haben kann, die aus (1) C₁₋₆-Alkyl, (2) Di-C₁₋₆-alkylamino und (3) sechsgliedriges gesättigtes cyclisches Amino, das C₁₋₆-Alkyl aufweisen kann, ausgewählt sind;
R^{4c} Folgendes ist: (i) Phenyl, das 1 bis 3 Substituenten haben kann, die aus Nitro und C₁₋₆-Alkylcarboxamid ausgewählt sind; (ii) C₁₋₆-Alkyl oder C₂₋₆-Alkenyl, das 1 bis 3 Phenyl-, Chinolyl- oder Pyridylgruppen aufweisen kann, die 1 bis 3 Substituenten, die aus C₁₋₆-Alkoxy, C₁₋₆-Alkylthio, C₁₋₆-Alkoxycarbonyl, C₁₋₆-Alkylsulfonyl und C₁₋₆-Alkylsulfinyl ausgewählt sind, haben können und gegebenenfalls weiterhin Phenyl, Carboxy oder C₁₋₆-Alkoxycarbonyl als zusätzliche Substituenten aufweisen können; oder
(iii) Acyl, das durch die Formel -(C=O)-R^{5"} repräsentiert ist, wobei R^{5"} = C₁₋₆-alkoxysubstituiertes Phenyl ist; und
Ring C ein Benzolring ist, der weiterhin 1 bis 3 C₁₋₆-Alkylgruppen aufweisen kann, oder ein Salz davon.

14. Verwendung gemäß Anspruch 12, umfassend: 3-(4-Isopropylphenyl)-5-(4-methoxybenzyloxy)-2,2,4,6,7-pentamethyl-2,3-dihydrobenzofuran, 3-(4-Methylphenyl)-2,4,6,7-tetramethylbenzofuran-5-yl-4-methoxybenzoat, 3-(4-Isopropylphenyl)-2,4,6,7-tetramethylbenzofuran-5-yl-4-methoxybenzoat, 3-(4-Isopropylphenyl)-5-(4-methoxybenzyloxy)-2,4,6,7-tetramethylbenzofuran, 3-(4-Isopropylphenyl)-5-(4-methoxybenzyloxy)-1',4,6,7-tetramethylspiro[benzofuran-2(3H),4'-piperidin] oder ein Salz davon.

15. Verfahren zum Kultivieren einer Stammzelle, einer neuralen Vorläuferzelle und/oder eines Neurocyten, wobei die Zellen nicht aus humanen Embryonen stammen, umfassend das Kultivieren der Stammzelle, der neuralen Vorläuferzelle und/oder des Neurocyten in Gegenwart einer Verbindung, die durch die Formel 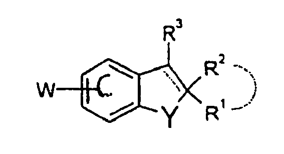 repräsentiert wird, wobei R¹ und R² gleich oder verschieden sind und jeweils ein Wasserstoffatom, eine gegebenenfalls substituierte Kohlenwasserstoffgruppe oder eine gegebenenfalls substituierte heterocyclische Gruppe sind oder R¹ und R² zusammen mit dem benachbarten Kohlenstoffatom einen gegebenenfalls substituierten drei- bis achtgliedrigen homocyclischen oder heterocyclischen Ring bilden können;
R³ ein Wasserstoffatom, eine gegebenenfalls substituierte Kohlenwasserstoffgruppe oder eine gegebenenfalls substituierte heterocyclische Gruppe ist;
^{....} eine Einfachbindung oder eine Doppelbindung ist;
W Folgendes ist: (i) eine Gruppe, die durch die Formel 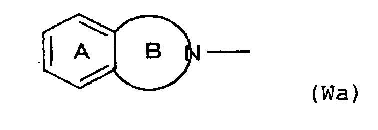 repräsentiert wird, wobei Ring A ein gegebenenfalls substituierter Benzolring ist, Ring B ein gegebenenfalls substituierter fünf- bis siebengliedriger stickstoffhaltiger heterocyclischer Ring ist;
(ii) eine Gruppe, die durch die Formel 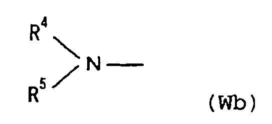 repräsentiert wird, wobei R⁴ (1) eine aliphatische Kohlenwasserstoffgruppe, die mit einer gegebenenfalls substituierten aromatischen Gruppe substituiert ist und die einen weiteren Substituenten aufweisen kann, oder (2) eine Acylgruppe, die einen gegebenenfalls substituierten aromatischen Ring enthält, ist, R⁵ ein Wasserstoffatom, ein C₁₋₆-Alkyl oder eine Acylgruppe ist; oder
(iii) eine Gruppe, die durch die Formel
R^{4c} - X - (Wc)
repräsentiert wird, wobei R^{4c} eine gegebenenfalls substituierte aromatische Gruppe, eine gegebenenfalls substituierte aliphatische Kohlenwasserstoffgruppe oder eine Acylgruppe ist, X ein Sauerstoffatom oder ein gegebenenfalls oxidiertes Schwefelatom ist;
Y ein Sauerstoffatom, ein gegebenenfalls oxidiertes Schwefelatom oder ein gegebenenfalls substituiertes Imino ist;
Ring C ein Benzolring ist, der neben der durch W dargestellten Gruppe noch einen weiteren Substituenten haben kann;
oder eines Salzes davon.

16. Verfahren gemäß Anspruch 15, wobei ---- eine Einfachbindung ist.

17. Verfahren gemäß Anspruch 15, wobei Y ein Sauerstoffatom ist.

18. Verfahren gemäß Anspruch 15, wobei W eine durch Formel (Wa) repräsentierte Gruppe ist.

19. Verfahren gemäß Anspruch 18, wobei R¹ und R² jeweils ein Wasserstoffatom oder eine C₁₋₆-Alkylgruppe sind, R³ ein Wasserstoffatom oder eine Phenylgruppe, die 1 bis 3 Substituenten, die aus C₁₋₆-Alkyl und Halogen ausgewählt sind, haben kann, ist, Ring C ein Benzolring ist, der weiterhin 1 bis 3 Substituenten haben kann, die aus C₁₋₆-Alkyl und C₁₋₆-Alkoxy ausgewählt sind, ^{....} eine Einfachbindung ist, Y ein Sauerstoffatom ist, die durch Formel (Wa) repräsentierte Gruppe eine Gruppe ist, die durch die Formel 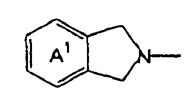 repräsentiert wird, wobei Ring A¹ ein Benzolring ist, der 1 bis 3 Substituenten haben kann, die aus Halogen, C₁₋₆-Alkoxy und C₁₋₆-Alkylendioxy ausgewählt sind.

20. Verfahren gemäß Anspruch 19, wobei die durch Formel (Wa) repräsentierte Gruppe ein Substituent an der 5-Position des Benzofuranrings ist.

21. Verfahren gemäß Anspruch 18, umfassend [1] 2-[2,2,4,6,7-Pentamethyl-3-(4-methylphenyl)-2,3-dihydro-1-benzofuran-5-yl]isoindolin, [2] 5,6-Dimethoxy-2-[2,2,4,6,7-pentamethyl-3-(4-methylphenyl)-2,3-dihydro-1-benzofuran-5-yl]isoindolin, [3] 5,6-Dimethoxy-2-[3-(4-isopropylphenyl)-2,2,4,6,7-pentamethyl-2,3-dihydro-1-benzofuran-5-yl]isoindolin, [4] 5,6-Dimethoxy-2-[2,2,4,6,7-pentamethyl-3-(4-methylphenyl)-2,3-dihydro-1-benzofuran-5-yl]-2H-isoindol, [5] 6-[3-(4-Isopropylphenyl)-2,2,4,6,7-pentamethyl-2,3-dihydro-1-benzofuran-5-yl]-6,7-dihydro-5H-[1,3]dioxolo-[4,5-f]isoindol, [6] 6-[2,2,4,6,7-Pentamethyl-3-(4-methylphenyl)-2,3-dihydro-1-benzofuran-5-yl]-6H-[1,3]dioxolo[4,5-f]isoindol, [7] 6-(2,2,4,6,7-Pentamethyl-3-phenyl-2,3-dihydro-1-benzofuran-5-yl)-6,7-dihydro-5H-[1,3]dioxolo[4,5-f]isoindol, [8] (R)-5,6-Dimethoxy-2-[2,2,4,6,7-pentamethyl-3-(4-methylphenyl)-2,3-dihydro-1-benzofuran-5-yl]isoindolin oder [9] (R)-5,6-Dimethoxy-2-[2,2,4,6,7-pentamethyl-3-(4-methylphenyl)-2,3-dihydro-1-benzofuran-5-yl]isoindolin-Hydrochlorid.

22. Verfahren gemäß Anspruch 18, umfassend (R)-5,6-Dimethoxy-2-[2,2,4,6,7-pentamethyl-3-(4-methylphenyl)-2,3-dihydro-1-benzofuran-5-yl]isoindolin.

23. Verfahren gemäß Anspruch 15, wobei W eine durch Formel (Wb) repräsentierte Gruppe ist.

24. Verfahren gemäß Anspruch 23, R¹ und R² jeweils eine Methylgruppe sind, R³ eine Phenylgruppe ist, die 1 bis 3 Substituenten haben kann, die aus Fluor, Methyl und Isopropyl ausgewählt sind, Ring C ein Benzolring ist, der weiterhin 1 bis 3 Substituenten haben kann, die aus C₁₋₆-Alkyl und C₁₋₆-Alkoxy ausgewählt sind, Y ein Sauerstoffatom ist, R⁴ eine Benzyl- oder Phenethylgruppe ist, die 1 bis 3 Substituenten haben kann, die aus Fluor, Methoxy und Methylendioxy ausgewählt sind, und R⁵ ein Wasserstoffatom oder eine Methylgruppe ist.

25. Verfahren gemäß Anspruch 23, umfassend (1) N-(4-Fluorbenzyl)-2,2,4,6,7-pentamethyl-3-phenyl-2,3-dihydro-1-benzofuran-5-amin, (2) N-Benzyl-3-(4-isopropylphenyl)-2,2,4,6,7-pentamethyl-2,3-dihydro-1-benzofuran-5-amin, (3) 3-(4-Isopropylphenyl)-N-(4-methoxybenzyl)-N,2,2,4,6,7-hexamethyl-2,3-dihydro-1-benzofuran-5-amin, (4) 3-(4-Isopropylphenyl)-N-[2-(4-methoxyphenyl)ethyl]-2,2,4,6,7-pentamethyl-2,3-dihydro-1-benzofuran-5-amin, (5) N-(4-Fluorbenzyl)-3-(4-isopropylphenyl)-2,2,4,6,7-pentamethyl-2,3-dihydro-1-benzofuran-5-amin, (6) N-(1,3-Benzodioxol-5-ylmethyl)-3-(4-isopropylphenyl)-2,2,4,6,7-pentamethyl-2,3-dihydro-1-benzofuran-5-amin, (7) N-(4-Fluorbenzyl)-3-(4-fluorphenyl)-2,2,4,6,7-pentamethyl-2,3-dihydro-1-benzofuran-5-amin, (8) N-(4-Methoxybenzyl)-2,2,4,6,7-pentamethyl-3-(4-methylphenyl)-2,3-dihydro-1-benzofuran-5-amin, (9) N-(4-Fluorbenzyl)-2,2,4,6,7-pentamethyl-3-(4-methylphenyl)-2,3-dihydro-1-benzofuran-5-amin, (10) 3-(4-Isopropylphenyl)-N-(4-methoxybenzyl)-2,4,6,7-tetramethyl-1-benzofuran-5-amin, (11) N-(4-Fluorbenzyl)-3-(4-isopropylphenyl)-2,4,6,7-tetramethyl-1-benzofuran-5-amin, (12) N-(4-Fluorbenzyl)-3-(4-fluorphenyl)-2,4,6,7-tetramethyl-1-benzofuran-5-amin, (13) N-(4-Fluorbenzyl)-3-(4-isopropylphenyl)-1',4,6,7-tetramethylspiro[benzofuran-2(3H),4'-pyperidin]-5-amin oder (14) (R)-N-(4-Fluorbenzyl)-3-(4-isopropylphenyl)-2,2,4,6,7-pentamethyl-2,3-dihydro-1-benzofuran-5-amin-Hydrochlorid.

26. Verfahren gemäß Anspruch 15, wobei W eine durch Formel (Wc) repräsentierte Gruppe ist.

27. Verfahren gemäß Anspruch 26, umfassend eine Verbindung, die durch die Formel 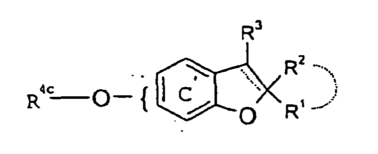 repräsentiert wird, wobei R¹ und R² jeweils C₁₋₆-Alkyl sind, das ein phenylsubstituiertes sechsgliedriges gesättigtes cyclisches Amino aufweisen kann, oder R¹ und R² zusammen mit dem benachbarten Kohlenstoffatom ein C₁₋₆-alkyl- oder C₇₋₁₆-aralkylsubstituiertes Piperidin bilden;
R³ Folgendes ist: (i) ein Wasserstoffatom; oder
(ii) Phenyl, das 1 bis 3 Substituenten haben kann, die aus (1) C₁₋₆-Alkyl, (2) Di-C₁₋₆-alkylamino und (3) sechsgliedriges gesättigtes cyclisches Amino, das C₁₋₆-Alkyl aufweisen kann, ausgewählt sind;
R^{4c} Folgendes ist: (i) Phenyl, das 1 bis 3 Substituenten haben kann, die aus Nitro und C₁₋₆-Alkylcarboxamid ausgewählt sind; (ii) C₁₋₆-Alkyl oder C₂₋₆-Alkenyl, das 1 bis 3 Phenyl-, Chinolyl- oder Pyridylgruppen aufweisen kann, die 1 bis 3 Substituenten, die aus C₁₋₆-Alkoxy, C₁₋₆-Alkylthio, C₁₋₆-Alkoxycarbonyl, C₁₋₆-Alkylsulfonyl und C₁₋₆-Alkylsulfinyl ausgewählt sind, haben können und gegebenenfalls weiterhin Phenyl, Carboxy oder C₁₋₆-Alkoxycarbonyl als zusätzliche Substituenten aufweisen können; oder
(iii) Acyl, das durch die Formel -(C=O)-R^{5"} repräsentiert ist, wobei R^{5"} = C₁₋₆-alkoxysubstituiertes Phenyl ist; und
Ring C ein Benzolring ist, der weiterhin 1 bis 3 C₁₋₆-Alkylgruppen aufweisen kann, oder ein Salz davon.

28. Verfahren gemäß Anspruch 26, umfassend: 3-(4-Isopropylphenyl)-5-(4-methoxybenzyloxy)-2,2,4,6,7-pentamethyl-2,3-dihydrobenzofuran, 3-(4-Methylphenyl)-2,4,6,7-tetramethylbenzofuran-5-yl-4-methoxybenzoat, 3-(4-Isopropylphenyl)-2,4,6,7-tetramethylbenzofuran-5-yl-4-methoxybenzoat, 3-(4-Isopropylphenyl)-5-(4-methoxybenzyloxy)-2,4,6,7-tetramethylbenzofuran, 3-(4-Isopropylphenyl)-5-(4-methoxybenzyloxy)-1',4,6,7-tetramethylspiro[benzofuran-2(3H),4'-piperidin] oder ein Salz davon.

29. Verfahren gemäß Anspruch 15, wobei die Stammzelle eine embryonale Stammzelle oder eine neurale Stammzelle ist.

30. Verfahren gemäß Anspruch 15, wobei eine Zelle für die Transplantationstherapie vorbereitet wird.

## Revendications

1. Utilisation d'un composé représenté par la formule : 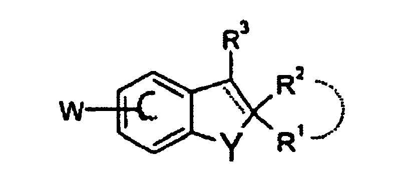 dans laquelle R¹ et R² sont identiques ou différents et chacun est un atome d'hydrogène, un groupe hydrocarbure éventuellement substitué ou un groupe hétérocyclique éventuellement substitué ou R¹ et R² peuvent être pris ensemble avec l'atome de carbone adjacent pour former un noyau homocyclique ou hétérocyclique à 3 à 8 chaînons éventuellement substitué,
R³ est un atome d'hydrogène, un groupe hydrocarbure éventuellement substitué ou un groupe hétérocyclique éventuellement substitué,
^{...} est une liaison simple ou une liaison double,
W est (i) un groupe représenté par la formule : 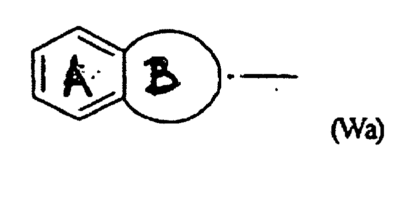 dans laquelle le noyau A est un noyau benzène éventuellement substitué, le noyau B est un noyau hétérocyclique contenant de l'azote à 5 à 7 chaînons éventuellement substitué,
(ii) un groupe représenté par la formule : 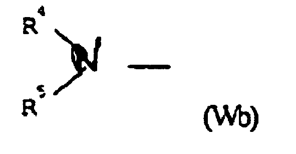 dans laquelle R⁴ est (1) un groupe hydrocarbure aliphatique qui est substitué par un groupe aromatique éventuellement substitué et qui peut présenter un substituant supplémentaire ou (2) un groupe acyle contenant un noyau aromatique éventuellement substitué, R⁵ est un atome d'hydrogène, un groupe alkyle en C₁-C₆ ou un groupe acyle, ou,
(iii) un groupe représenté par la formule : 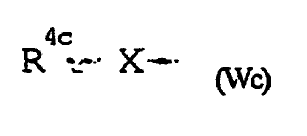 dans laquelle R^{4c} est un groupe aromatique éventuellement substitué, un groupe hydrocarbure aliphatique éventuellement substitué ou un groupe acyle, X est un atome d'oxygène ou un atome de soufre éventuellement oxydé,
Y est un atome d'oxygène, un atome de soufre éventuellement oxydé ou un imino éventuellement substitué,
le noyau C est un noyau benzène qui peut présenter un substituant supplémentaire en plus du groupe représenté par W ou un sel de celui-ci dans la production d'un agent pour la promotion d'une prise de greffe ou une différentiation dans la transplantation de cellules souches neurales et/ou de neurocytes ou dans la production d'un agent pour la promotion de la prolifération ou de la différentiation d'une cellule souche neurale et/ou d'un neurocyte pour une transplantation, dans laquelle les cellules ne sont pas des cellules d'origine embryonnaire humaine.

2. Utilisation selon la revendication 1, dans laquelle ^{...} est une liaison unique.

3. Utilisation selon la revendication 1, dans laquelle Y est un atome d'oxygène.

4. Utilisation selon la revendication 1, dans laquelle W est un groupe représenté par la formule (Wa).

5. Utilisation selon la revendication 1, dans laquelle chacun de R¹ et R² est un atome d'hydrogène ou un groupe alkyle en C₁-C₆, R³ est un atome d'hydrogène ou un groupe phényle qui peut présenter 1 ou 3 substituants sélectionnés parmi un alkyle en C₁-C₆ et un halogène, le noyau C est un noyau benzène qui peut présenter en outre 1 à 3 substituants sélectionnés parmi un alkyle en C₁-C₆ et un alkoxy en C₁-C₆, ^{...} est une liaison unique, Y est un atome d'oxygène, le groupe représenté par la formule (Wa) est un groupe représenté par la formule : 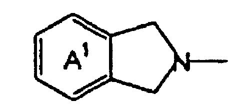 dans laquelle le noyau A¹ est un noyau benzène qui peut présenter 1 à 3 substituants sélectionnés parmi halogène, alkoxy en C₁-C₆ et alkylènedioxy en C₁-C₆.

6. Utilisation selon la revendication 5, dans laquelle le groupe représenté par la formule (Wa) est un substituant sur la position 5 du noyau benzofurane.

7. Utilisation selon la revendication 4, comprenant [1] 2-[2,2,4,6,7-pentaméthyl-3-(4-méthylphényl)-2,3-dihydro-1-benzofuran-5-yl]isoindoline, [2] 5,6-diméthoxy-2-[2,2,4,6,7-pentaméthyl-3-(4-méthylphényl)-2,3-dihydro-1-benzofuran-5-yl]isoindoline, [3] 5,6-diméthoxy-2-[3-(4-isopropylphényl)-2,2,4,6,7-pentaméthyl-2,3-dihydro-1-benzofuran-5-yl]isoindoline, [4] 5,6-diméthoxy-2-[2,2,4,6,7-pentaméthyl-3-(4-méthylphényl)-2,3-dihydro-1-benzofuran-5-yl]-2H-isoindole, [5] 6-[3-(4-isopropylphényl)-2,2,4,6,7-pentaméthyl-2,3-dihydro,1-benzofuran-5-yl]-6,7-dihydro-5H-[1,3]dioxolo[4,5-f]isoindole, [6] 6-[2,2,4,6,7-pentaméthyl-3-(4-méthylphényl)-2,3-dihydro-1-benzofuran-5-yl]-6H-[1,3]dioxolo[4,5-f]isoindole, [7] 6-(2,2,4,6,7-pentaméthyl-3-phényl-2,3-dihydro-1-benzofuran-5-yl)-6,7-dihydro-5H-[1,3]dioxolo[4,5-f]isoindole, [8] (R)-5,6-diméthoxy-2-[2,2,4,6,7-pentaméthyl-3-(4-méthylphényl)-2,3-dihydro-1-benzofuran-5-yl]isoindoline ou [9] chlorhydrate de (R)-5,6-diméthoxy-2-[2,2,4,6,7-pentaméthyl-3-(4-méthylphényl)-2,3-dihydro-1-benzofuran-5-yl]isoindoline.

8. Utilisation selon la revendication 4 comprenant (R)-5,6-diméthoxy-2-[2,2,4,6,7-pentaméthyl-3-(4-méthylphényl)-2,3-dihydro-1-benzofuran-5-yl]isoindoline.

9. Utilisation selon la revendication 1, dans laquelle W est un groupe représenté par la formule (Wb).

10. Utilisation selon la revendication 9, dans laquelle chacun de R¹ et R² est un groupe méthyle, R³ est un groupe phényle qui peut présenter 1 à 3 substituants sélectionnés parmi fluor, méthyle et isopropyle, le noyau C est un noyau benzène qui peut en outre présenter 1 à 3 substituants sélectionnés parmi alkyle en C₁-C₆ et alkoxy en C₁-C₆, Y est un atome d'oxygène, R⁴ est un groupe benzyle ou phénétyle qui peuvent présenter 1 à 3 substituants sélectionnés parmi fluor, méthoxy et méthylènedioxy et R⁵ est un atome d'hydrogène ou un groupe méthyle.

11. Utilisation selon la revendication 9, comprenant (1) N-(4-fluorobenzyl)-2,2,4,6,7-pentaméthyl-3-phényl-2,3-dihydro-1-benzofuran-5-amine, (2) N-benzyl-3-(4-isopropylphényl)-2,2,4,6,7-pentaméthyl-2,3-dihydro-1-benzofuran-5-amine, (3) 3-(4-isopropylphényl)-N-(4-méthoxybenzyl)-N,2,2,4,6,7-hexaméthyl-2,3-dihydro-1-benzofuran-5-amine, (4) 3-(4-isopropylphényl)-N-[2-(4-méthoxyphényl)éthyl]-2,2,4,6,7-pentaméthyl-2,3-dihydro-1-benzofuran-5-amine, (5) N-(4-fluorobenzyl)-3-(4-isopropylphényl)-2,2,4,6,7-pentaméthyl-2,3-dihydro-1-benzofuran-5-amine, (6) N-(1,3-benzodioxol-5-yl-méthyl)-3-(4-isopropylphényl)-2,2,4,6,7-pentaméthyl-2,3-dihydro-1-benzofuran-5-amine, (7) N-(4-fluorobenzyl)-3-(4-fluorophényl)-2,2,4,6,7-pentaméthyl-2,3-dihydro-1-benzofuran-5-amine, (8) N-(4-méthoxybenzyl)-2,2,4,6,7-pentaméthyl-3-(4-méthylphényl)-2,3-dihydro-1-benzofuran-5-amine, (9) N-(4-fluorobenzyl)-2,2,4,6,7-pentaméthyl-3-(4-méthylphényl)-2,3-dihydro-1-benzofuran-5-amine, (10) 3-(4-isopropylphényl)-N-(4-méthoxybenzyl)-2,4,6,7-tétraméthyl-1-benzofuran-5-amine, (11) N-(4-fluorobenzyl)-3-(4-isopropylphényl)-2,4,6,7-tétraméthyl-1-benzofuran-5-amine, (12) N-(4-fluorobenzyl)-3-(4-fluorophényl)-2,4,6,7-tétraméthyl-1-benzofuran-5-amine, (13) N-(4-fluorobenzyl)-3-(4-isopropylphényl)-1',4,6,7-tétraméthylspiro[benzofuran-2(3H),4'-pipéridine]-5-amine ou (14) chlorhydrate de (R)-N-(4-fluorobenzyl)-3-(4-isopropylphényl)-2,2,4,6,7-pentaméthyl-2,3-dihydro-1-benzofuran-5-amine.

12. Utilisation selon la revendication 1, dans laquelle W est un groupe représenté par la formule (Wc).

13. Utilisation selon la revendication 12 comprenant un composé représenté par la formule : 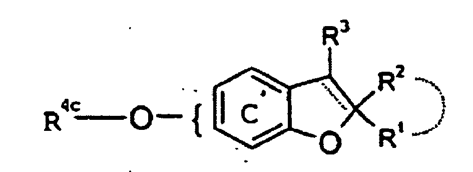 dans laquelle chacun de R¹ et R² est un alkyle en C₁-C₆ qui peut présenter un amino cyclique saturé à 6 chaînons substitué par phényle, ou R¹ et R² sont pris ensemble avec l'atome de carbone adjacent pour former une pipéridine substituée par alkyle en C₁-C₆ ou aralkyle en C₇-C₁₆ ;
R³ est (i) un atome d'hydrogène, ou,
(ii) phényle qui peut présenter 1 à 3 substituants sélectionnés parmi (1) alkyle en C₁-C₆, (2) dialkylamino en C₁-C₆ et (3) amino cyclique saturé à 6 chaînons qui peut présenter un alkyle en C₁-C₆ ;
R^{4c} est (i) un phényle qui peut présenter 1 à 3 substituants sélectionnés parmi nitro et carboxamide-alkyle en C₁-C₆,
(ii) alkyle en C₁-C₆ ou alcényle en C₂-C₆ présentant 1 à 3 phényle, quinolyle ou pyridyle qui peuvent présenter 1 à 3 substituants sélectionnés parmi alkoxy en C₁-C₆, alkylthio en C₁-C₆, alkoxycarbonyle en C₁-C₆, alkylsulfonyle en C₁-C₆ et alkylsulfinyle en C₁-C₆ et éventuellement présentant en outre en tant que substituants additionnels phényle, carboxy ou alkoxycarbonyle en C₁-C₆, ou,
(iii) acyle représenté par la formule : -(C=O)-R^{5'} ou R^{5'} est un phényle substitué par alkoxy en C₁-C₆ ; et,
le noyau C est un noyau benzène qui peut présenter en outre 1 à 3 alkyle en C₁-C₆, ou un sel de celui-ci.

14. Utilisation selon la revendication 12 comprenant :
3-(4-isopropylphényl)-5-(4-méthoxybenzyloxy)-2,2,4,6,7-pentaméthyl-2,3-dihydrobenzofurane, 3-(4-méthylphényl)-2,4,6,7-tétraméthylbenzofuran-5-yl-4-méthoxybenzoate, 3-(4-isopropylphényl)-2,4,6,7-tétraméthylbenzofuran-5-yl-4-méthoxybenzoate, 3-(4-isopropylphényl)-5-(4-méthoxybenzyloxy)-2,4,6,7-tétraméthylbenzofurane, 3-(4-isopropylphényl)-5-(4-méthoxybenzyloxy)-1',4,6,7-tétraméthylspiro[benzofuran-2(3H),4'-pipéridine] ou un sel de ceux-ci.

15. Procédé de mise en culture d'une cellule souche, d'une cellule précurseur neurale et/ou d'un neurocyte, dans lequel les cellules ne sont pas d'origine embryonnaire humaine, comprenant la mise en culture d'une cellule souche, cellule précurseur neurale et/ou neurocyte en présence d'un composé représenté par la formule : 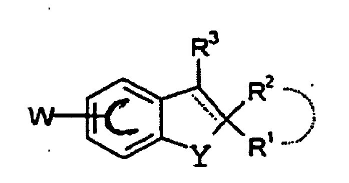 dans laquelle R¹ et R² sont identiques ou différents et chacun est un atome d'hydrogène, un groupe hydrocarbure éventuellement substitué ou un groupe hétérocyclique éventuellement substitué ou R¹ et R² peuvent être pris ensemble avec l'atome de carbone adjacent pour former un noyau homocyclique ou hétérocyclique à 3 à 8 chaînons éventuellement substitué,
R³ est un atome d'hydrogène, un groupe hydrocarbure éventuellement substitué ou un groupe hétérocyclique éventuellement substitué,
^{...} est une liaison simple ou une liaison double,
W est (i) un groupe représenté par la formule : 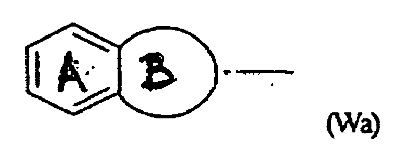 dans laquelle le noyau A est un noyau benzène éventuellement substitué, le noyau B est un noyau hétérocyclique contenant de l'azote à 5 à 7 chaînons éventuellement substitué,
(ii) un groupe représenté par la formule : 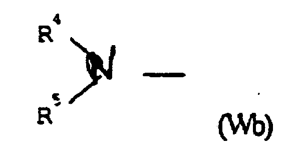 dans laquelle R⁴ est (1) un groupe hydrocarbure aliphatique qui est substitué par un groupe aromatique éventuellement substitué et qui peut présenter un substituant supplémentaire ou (2) un groupe acyle contenant un noyau aromatique éventuellement substitué, R⁵ est un atome d'hydrogène, un groupe alkyle en C₁-C₆ ou un groupe acyle, ou,
(iii) un groupe représenté par la formule : 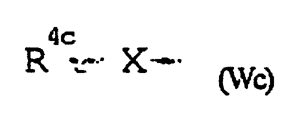 dans laquelle R^{4c} est un groupe aromatique éventuellement substitué, un groupe hydrocarbure aliphatique éventuellement substitué ou un groupe acyle, X est un atome d'oxygène ou un atome de soufre éventuellement oxydé,
Y est un atome d'oxygène, un atome de soufre éventuellement oxydé ou un imino éventuellement substitué,
le noyau C est un noyau benzène qui peut présenter un substituant supplémentaire en plus du groupe représenté par W ou un sel de celui-ci.

16. Procédé selon la revendication 15, dans lequel ^{...} est une liaison unique.

17. Procédé selon la revendication 15, dans lequel Y est un atome d'oxygène.

18. Procédé selon la revendication 15, dans lequel W est un groupe représenté par la formule (Wa).

19. Procédé selon la revendication 18, dans lequel chacun de R¹ et R² est un atome d'hydrogène ou un groupe alkyle en C₁-C₆, R³ est un atome d'hydrogène ou un groupe phényle qui peut présenter 1 ou 3 substituants sélectionnés parmi un alkyle en C₁-C₆ et un halogène, le noyau C est un noyau benzène qui peut présenter en outre 1 à 3 substituants sélectionnés parmi un alkyle en C₁-C₆ et un alkoxy en C₁-C₆, ^{...} est une liaison unique, Y est un atome d'oxygène, le groupe représenté par la formule (Wa) est un groupe représenté par la formule : 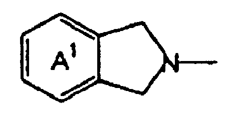 dans laquelle le noyau A¹ est un noyau benzène qui peut présenter 1 à 3 substituants sélectionnés parmi halogène, alkoxy en C₁-C₆ et alkylènedioxy en C₁-C₆.

20. Procédé selon la revendication 19, dans lequel le groupe représenté par la formule (Wa) est un substituant sur la position 5 du noyau benzofurane.

21. Procédé selon la revendication 18 comprenant [1] 2-[2,2,4,6,7-pentaméthyl-3-(4-méthylphényl)-2,3-dihydro-1-benzofuran-5-yl]isoindoline, [2] 5,6-diméthoxy-2-[2,2,4,6,7-pentaméthyl-3-(4-méthylphényl)-2,3-dihydro-1-benzofuran-5-yl]isoindoline, [3] 5,6-diméthoxy-2-[3-(4-isopropylphényl)-2,2,4,6,7-pentaméthyl-2,3-dihydro-1-benzofuran-5-yl]isoindoline, [4] 5,6-diméthoxy-2-[2,2,4,6,7-pentaméthyl-3-(4-méthylphényl)-2,3-dihydro-1-benzofuran-5-yl]-2H-isoindole, [5] 6-[3-(4-isopropylphényl)-2,2,4,6,7-pentaméthyl-2,3-dihydro-1-benzofuran-5-yl]-6,7-dihydro-5H-[1,3]dioxolo[4,5-f]isoindole, [6] 6-[2,2,4,6,7-pentaméthyl-3-(4-méthylphényl)-2,3-dihydro-1-benzofuran-5-yl]-6H-[1,3]dioxolo[4,5-f]isoindole, [7] 6-(2,2,4,6,7-pentaméthyl-3-phényl-2,3-dihydro-1-benzofuran-5-yl)-6,7-dihydro-5H-[1,3]dioxolo[4,5-f]isoindole, [8] (R)-5,6-diméthoxy-2-[2,2,4,6,7-pentaméthyl-3-(4-méthylphényl)-2,3-dihydro-1-benzofuran-5-yl]isoindoline ou [9] chlorhydrate de (R)-5,6-diméthoxy-2-[2,2,4,6,7-pentaméthyl-3-(4-méthylphényl)-2,3-dihydro-1-benzofuran-5-yl]isoindoline.

22. Procédé selon la revendication 18, comprenant (R)-5,6-diméthoxy-2-[2,2,4,6,7-pentaméthyl-3-(4-méthylphényl)-2,3-dihydro-1-benzofuran-5-yl]isoindoline.

23. Procédé selon la revendication 15, dans lequel W est un groupe représenté par la formule (Wb).

24. Procédé selon la revendication 23, dans lequel chacun de R¹ et R² est un groupe méthyle, R³ est un groupe phényle qui peut présenter 1 à 3 substituants sélectionnés parmi fluor, méthyle et isopropyle, le noyau C est un noyau benzène qui peut en outre présenter 1 à 3 substituants sélectionnés parmi alkyle en C₁-C₆ et alkoxy en C₁-C₆, Y est un atome d'oxygène, R⁴ est un groupe benzyle ou phénétyle qui peuvent présenter 1 à 3 substituants sélectionnés parmi fluor, méthoxy et méthylènedioxy et R⁵ est un atome d'hydrogène ou un groupe méthyle.

25. Procédé selon la revendication 23, comprenant (1) N-(4-fluorobenzyl)-2,2,4,6,7-pentaméthyl-3-phényl-2,3-dihydro-1-benzofuran-5-amine, (2) N-benzyl-3-(4-isopropylphényl)-2,2,4,6,7-pentaméthyl-2,3-dihydro-1-benzofuran-5-amine, (3) 3-(4-isopropylphényl)-N-(4-méthoxybenzyl)-N,2,2,4,6,7-hexaméthyl-2,3-dihydro-1-benzofuran-5-amine, (4) 3-(4-isopropylphényl)-N-[2-(4-méthoxyphényl)éthyl]-2,2,4,6,7-pentaméthyl-2,3-dihydro-1-benzofuran-5-amine, (5) N-(4-fluorobenzyl)-3-(4-isopropylphényl)-2,2,4,6,7-pentaméthyl-2,3-dihydro-1-benzofuran-5-amine, (6) N-(1,3-benzodioxol-5-yl-méthyl)-3-(4-isopropylphényl)-2,2,4,6,7-pentaméthyl-2,3-dihydro-1-benzofuran-5-amine, (7) N-(4-fluorobenzyl)-3-(4-fluorophényl)-2,2,4,6,7-pentaméthyl-2,3-dihydro-1-benzofuran-5-amine, (8) N-(4-méthoxybenzyl)-2,2,4,6,7-pentaméthyl-3-(4-méthylphényl)-2,3-dihydro-1-benzofuran-5-amine, (9) N-(4-fluorobenzyl)-2,2,4,6,7-pentaméthyl-3-(4-méthylphényl)-2,3-dihydro-1-benzofuran-5-amine, (10) 3-(4-isopropylphényl)-N-(4-méthoxybenzyl)-2,4,6,7-tétraméthyl-1-benzofuran-5-amine, (11) N-(4-fluorobenzyl)-3-(4-isopropylphényl)-2,4,6,7-tétraméthyl-1-benzofuran-5-amine, (12) N-(4-fluorobenzyl)-3-(4-fluorophényl)-2,4,6,7-tétraméthyl-1-benzofuran-5-amine, (13) N-(4-fluorobenzyl)-3-(4-isopropylphényl)-1',4,6,7-tétraméthylspiro[benzofuran-2(3H),4'-pipéridine]-5-amine ou (14) chlorhydrate de (R)-N-(4-fluorobenzyl)-3-(4-isopropylphényl)-2,2,4,6,7-pentaméthyl-2,3-dihydro-1-benzofuran-5-amine.

26. Procédé selon la revendication 15, dans lequel W est un groupe représenté par la formule (Wc).

27. Procédé selon la revendication 26, comprenant un composé représenté par la formule : 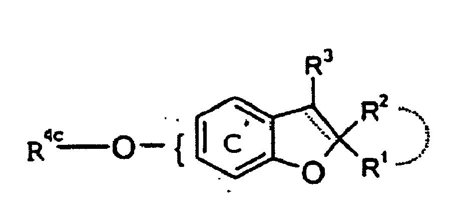 dans laquelle chacun de R¹ et R² est un alkyle en C₁-C₆ qui peut présenter un amino cyclique saturé à 6 chaînons substitué par phényle, ou R¹ et R² sont pris ensemble avec l'atome de carbone adjacent pour former une pipéridine substituée par alkyle en C₁-C₆ ou aralkyle en C₇-C₁₆ ;
R³ est (i) un atome d'hydrogène, ou,
(ii) phényle qui peut présenter 1 à 3 substituants sélectionnés parmi (1) alkyle en C₁-C₆, (2) dialkylamino en C₁-C₆ et (3) amino cyclique saturé à 6 chaînons qui peut présenter un alkyle en C₁-C₆ ;
R^{4c} est (i) un phényle qui peut présenter 1 à 3 substituants sélectionnés parmi nitro et carboxamide-alkyle en C₁-C₆,
(ii) alkyle en C₁-C₆ ou alcényle en C₂-C₆ présentant 1 à 3 phényle, quinolyle ou pyridyle qui peuvent présenter 1 à 3 substituants sélectionnés parmi alkoxy en C₁-C₆, alkylthio en C₁-C₆, alkoxycarbonyle en C₁-C₆, alkylsulfonyle en C₁-C₆ et alkylsulfinyle en C₁-C₆ et éventuellement présentant en outre en tant que substituants additionnels phényle, carboxy ou alkoxycarbonyle en C₁-C₆, ou,
(iii) acyle représenté par la formule : -(C=O)-R^{5'} ou R^{5'} est un phényle substitué par alkoxy en C₁-C₆ ; et,
le noyau C est un noyau benzène qui peut présenter en outre 1 à 3 alkyle en C₁-C₆, ou un sel de celui-ci.

28. Procédé selon la revendication 26, comprenant :
3-(4-isopropylphényl)-5-(4-méthoxybenzyloxy)-2,2,4,6,7-pentaméthyl-2,3-dihydrobenzofurane, 3-(4-méthylphényl)-2,4,6,7-tétraméthylbenzofuran-5-yl-4-méthoxybenzoate, 3-(4-isopropylphényl)-2,4,6,7-tétraméthylbenzofuran-5-yl-4-méthoxybenzoate, 3-(4-isopropylphényl)-5-(4-méthoxybenzyloxy)-2,4,6,7-tétraméthylbenzofurane, 3-(4-isopropylphényl)-5-(4-méthoxybenzyloxy)-1',4,6,7-tétraméthylspiro[benzofuran-2(3H),4'-pipéridine] ou un sel de ceux-ci.

29. Procédé selon la revendication 15, dans lequel la cellule souche est une cellule souche embryonnaire ou une cellule souche neurale.

30. Procédé selon la revendication 15 par lequel une cellule pour une thérapie de greffe est préparée.
